(19) Europäisches Patentamt
European Patent Office
Office européen des brevets

(11) EP 1 704 236 B1

(12) EUROPEAN PATENT SPECIFICATION

(45) Date of publication and mention of the grant of the patent:
**29.11.2017 Bulletin 2017/48**

(21) Application number: **04806537.9**

(22) Date of filing: **23.12.2004**

(51) Int Cl.:
*C12N 15/54* (2006.01) *C12N 15/55* (2006.01)
*C12N 9/10* (2006.01) *C12N 9/14* (2006.01)
*C12N 11/00* (2006.01) *C12P 7/64* (2006.01)

(86) International application number:
**PCT/IB2004/004378**

(87) International publication number:
**WO 2005/066347 (21.07.2005 Gazette 2005/29)**

(54) **PROTEINS**

PROTEINE

PROTEINES

(84) Designated Contracting States:
**AT BE BG CH CY CZ DE DK EE ES FI FR GB GR HU IE IS IT LI LT LU MC NL PL PT RO SE SI SK TR**

(30) Priority:
**24.12.2003 GB 3300167**
**15.01.2004 PCT/IB2004/000655**
**16.07.2004 GB 0415999**
**02.08.2004 US 911160**

(43) Date of publication of application:
**27.09.2006 Bulletin 2006/39**

(60) Divisional application:
**09011127.9 / 2 119 771**

(73) Proprietor: **DuPont Nutrition Biosciences ApS**
**1411 Copenhagen K (DK)**

(72) Inventors:
- **KELLET-SMITH, Anja Hemmingsen**
  **2860 Søborg (DK)**
- **LORENTSEN, Rikke, Høegh**
  **2000 Frederiksberg (DK)**
- **SØE, Jørn, Borch**
  **8381 Tilst (DK)**
- **MIKKELSON, Jørn, Dalgaard**
  **2650 Hividovre (DK)**
- **DE KREIJ, Arno**
  **3353 XK Papendrecht (NL)**

(74) Representative: **D Young & Co LLP**
**120 Holborn**
**London EC1N 2DY (GB)**

(56) References cited:
**EP-A- 1 275 711 WO-A-00/05396**

- **BRUMLIK MICHAEL J ET AL: "Identification of the catalytic triad of the lipase/acyltransferase from Aeromonas hydrophila" JOURNAL OF BACTERIOLOGY, vol. 178, no. 7, 1996, pages 2060-2064, XP002315734 ISSN: 0021-9193 cited in the application**
- **ROBERTSON D L ET AL: "Influence of Active Site and Tyrosine Modification on the Secretion and Activity of the Aeromonas hydrophila Lipase/Acyltransferase" JOURNAL OF BIOLOGICAL CHEMISTRY, AMERICAN SOCIETY OF BIOLOGICAL CHEMISTS, BALTIMORE, MD, US, vol. 269, no. 3, 21 January 1994 (1994-01-21), pages 2146-2150, XP002318365 ISSN: 0021-9258 cited in the application**
- **UPTON C ET AL: "A new family of lipolytic enzymes?" TIBS TRENDS IN BIOCHEMICAL SCIENCES, ELSEVIER PUBLICATION, CAMBRIDGE, EN, vol. 20, no. 5, May 1995 (1995-05), pages 178-179, XP004222260 ISSN: 0968-0004 cited in the application**
- **LO Y-C ET AL: "Crystal Structure of Escherichia coli Thioesterase I/Protease I/Lysophospholipase L1: Consensus Sequence Blocks Constitute the Catalytic Center of SGNH-hydrolases through a Conserved Hydrogen Bond Network" JOURNAL OF MOLECULAR BIOLOGY, LONDON, GB, vol. 330, no. 3, 11 July 2003 (2003-07-11), pages 539-551, XP004434203 ISSN: 0022-2836**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- NERLAND A H: "The nucleotide sequence of the gene GCAT from Aeromonas salmonicida ssp. salmonicida", JOURNAL OF FISH DESEASES, vol. 19, no. 2, 1996, pages 145-150,
- DATABASE UNIPROT [Online] 01 March 2002 SALANOUBAT M. ET AL.: 'SubName: Full= Putative lipase / esterase protein' Database accession no. Q8Y1V9

**Description**

REFERENCE TO RELATED APPLICATIONS

Reference is made to the following related applications: United States Application Serial Number 09/750,990 filed on 20 July 1999; United States Application Serial Number 10/409,391; United States Application Serial Number 60/489,441 filed on 23 July 2003; United Kingdom Application Number GB 0330016.7 filed on 24 December 2003 and International Patent Application Number PCT/IB2004/000655 filed on 15 January 2004.

FIELD OF INVENTION

The present invention relates to methods of producing variant enzymes. The present invention further relates to novel variant enzymes and to the use of these novel variant enzymes.

TECHNICAL BACKGROUND

Lipid:cholesterol acyltransferase enzymes have been known for some time (see for example Buckley - Biochemistry 1983, 22, 5490-5493). In particular, glycerophospholipid:cholesterol acyl transferases (GCATs) have been found, which like the plant and/or mammalian lecithin: cholesterol acyltransferases (LCATs), will catalyse fatty acid transfer between phosphatidylcholine and cholesterol.

Upton and Buckley (TIBS 20, May 1995, p178-179) and Brumlik and Buckley (J. of Bacteriology Apr. 1996, p2060-2064) teach a lipase/acyltransferase from *Aeromonas hydrophila* which has the ability to carry out acyl transfer to alcohol receptors in aqueous media.

A putative substrate binding domain and active site of the *A. hydrophila* acyltransferase have been identified (see for example Thornton et al 1988 Biochem. et Biophys. Acta. 959, 153-159 and Hilton & Buckley 1991 J. Biol. Chem. 266, 997-1000) for this enzyme.

Buckley et al (J. Bacteriol 1996, 178(7) 2060-4) taught that Ser16, Asp116 and His291 are essential amino acids which must be retained for enzyme activity to be maintained.

Robertson et al (J. Biol. Chem. 1994, 269, 2146-50) taught some specific mutations, namely Y226F, Y230F, Y30F, F13S, S18G, S18V, of the *A. hydrophila* acyltransferase, none of which are encompassed by the present invention.

SUMMARY ASPECTS OF THE PRESENT INVENTION

The present invention is predicated upon the finding of specific variants of a GDSx containing lipid acyltransferase enzyme, which variants have an increased transferase activity compared with a parent enzyme. In particular, the variants described herein have an enhanced transferase activity using galactolipid as an acyl donor as compared with a parent enzyme. These lipid acyltransferases are referred to herein as glycolipid acyltransferases. The variants described herein may additionally have an enhanced ratio of transferase activity using galactolipids as an acyl donor as compared with phospholipid transferase activity (GL:PL ratio) and/or an enhanced ratio of transferse activity using galactolipids as an acyl donor as compared with galactolipid hydrolysis activity (GLt:GLh ratio) compared with a parent enzyme.

The present invention provides a method of producing a variant glycolipid acyltransferase enzyme comprising: (a) selecting a parent enzyme which is a lipid acyltransferase enzyme characterised in that the enzyme comprises the amino acid sequence motif GDSX, wherein X is one or more of the following amino acid residues L, A, V, I, F, Y, H, Q, T, N, M or S; (b) modifying one or more amino acids to produce a variant lipid acyltransferase; (c) testing the variant lipid acyltransferase for transferase activity, and optionally hydrolytic activity, on a galactolipid substrate, and optionally a phospholipid substrate and/or optionally a triglyceride substrate; (d) selecting a variant enzyme with an enhanced activity towards galactolipids compared with the parent enzyme; and optionally (e) preparing a quantity of the variant enzyme;
wherein the method further comprises one or more of the following steps: structural homology mapping or sequence homology alignment;
wherein the structural homology mapping comprises one or more of the following steps:

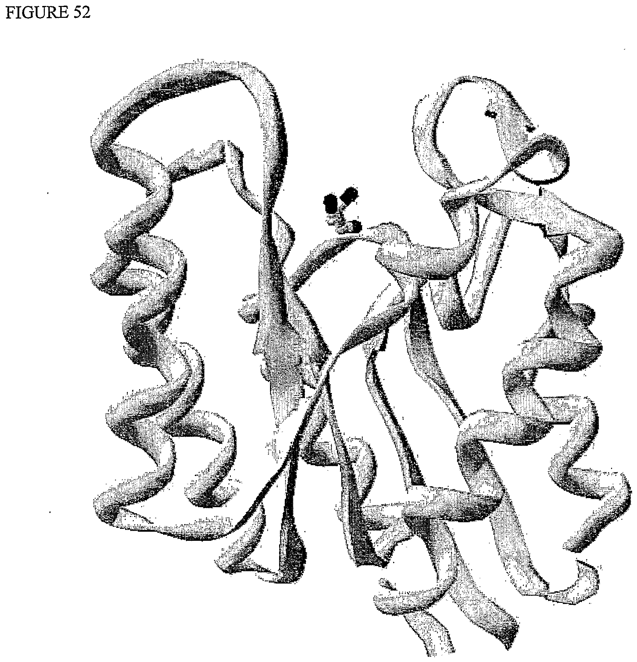

(a) aligning a parent sequence with a structural model of the amino acid sequence SEQ ID No. 2 (P10480) obtained by structural alignment of the crystal structure co-ordinates of SEQ ID No. 2 with 1IVN.PDB shown in figure 52;
(b) selecting one or more amino acids within a 10Å sphere centred on the central carbon atom of the glycerol molecule in the active site;
(c) determining if one or more amino acid residues selected in accordance with step (b) are highly conserved; and
(d) modifying one or more amino acids selected in accordance with step (b), excluding conserved regions identified in accordance with step (c) in said parent sequence;

and wherein the sequence homology alignment comprises one or more of the following steps:

(i) selecting a first parent lipid acyltransferase;
(ii) identifying a second related lipid acyltransferase having a desirable activity;
(iii) aligning said first parent lipid acyltransferase and the second related lipid acyltransferase;
(iv) identifying amino acid residues that differ between the two sequences;
(v) determining if one or more amino acid residues selected in accordance with step (iv) are highly conserved; and
(vi) modifying one or more of the amino acid residues identified in accordance with step (iv) excluding conserved regions identified in accordance with step (v) in said parent sequence,

and wherein the following amino acid residues identified by alignment with SEQ ID No. 2 is modified compared with a parent sequence: S18 M or T; Y30 G, I, L, S, E, M, A or R; G40 L, R, M, Q or V; N80 R, D, P G or E; N87 R, D, E, M, C or G; N88W; Y179 V, E, R, N or Q; H180, T, K, Q or I; M209 Y, L, K or M; L210 N, D, H, R, E, A, Q, P, K, G, T, W, I, V, S or M; R211 G, Q, K, D or T; N215 H, L, G, V, R, or Y.

In one embodiment, the method comprises testing the variant lipid acyltransferase for:

(i) transferase activity from a galactolipid substrate, and
(ii) transferase activity from a phospholipids substrate; and

selecting a variant enzyme, which when compared with the parent enzyme, has an enhanced ratio of transferase activity from galactolipids compared with phospholipids.

The ratio of transferase activity from galactolipids compared with phospholipids may be at least 3.

In some embodiments, the method comprises testing the variant lipid acyltransferase for:

(a) transferase activity from a galactolipid substrate, and
(b) hydrolytic activity on a galactolipid substrate; and

selecting a variant enzyme with an enhanced ratio of transferase activity from galactolipids compared with its hydrolytic activity on glycolipids, compared with the parent enzyme.

The enhanced ratio of transferase activity on galactolipids compared to hydrolytic activity on galactolipids may be at least 1.5.

In some embodiments, the parent enzyme comprises an amino acid sequence as shown as SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45, or an amino acid sequence which has at least 70% identity therewith.

In some embodiments, the parent enzyme is an enzyme which comprises the amino acid sequence shown as SEQ ID No. 2 and/or SEQ ID No. 28.

In some embodiments, the parent lipid acyltransferase enzyme is encoded by any one of the following nucleotide sequences: SEQ ID No. 7, SEQ ID No. 8, SEQ ID No. 9, SEQ ID No. 10, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No. 27, SEQ ID No. 29, SEQ ID No. 31, SEQ ID No. 32, SEQ ID No. 35, SEQ ID No. 38, SEQ ID No. 40, SEQ ID No. 42, SEQ ID No. 44 or SEQ ID No. 46 or a nucleotide sequence which has at least 75% or more identity with any one of the sequences shown as SEQ ID No. 7, SEQ ID No. 8, SEQ ID No. 9, SEQ ID No. 10, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No. 27, SEQ ID No. 29, SEQ ID No. 31, SEQ ID No. 32, SEQ ID No. 35, SEQ ID No. 38, SEQ ID No. 40, SEQ ID No. 42, SEQ ID No. 44 or SEQ ID No. 46.

The X of the GDSX motif may be L.

Further provided by the present invention is a variant glycolipid acyltransferase enzyme characterised in that the enzyme comprises the amino acid sequence motif GDSX, wherein X is one or more of the following amino acid residues L, A, V, I, F, Y, H, Q, T, N, M or S, wherein the variant enzyme comprises an amino acid sequence, which amino acid sequence is shown as SEQ ID No. 2, SEQ ID No. 3, SEQ ID No. 26, SEQ ID No. 28 or SEQ ID No. 30 or is encoded by any one of the following nucleotide sequences: SEQ ID No. 7, SEQ ID No. 8, SEQ ID No. 27, SEQ ID No. 29 or SEQ ID No. 31, except for one or more amino acid modifications at any one or more of the amino acid residues defined S18, M or T; Y30 G, I, L, S, E, M, A or R; G40 L, R, M, Q or V; N80 R, D, P, G or E; N87 R, D, E M, C or G; N88W; Y179 V, E, R, N or Q; H180 T, K, Q or I; M209 Y, L, K or M; L210 N, D, H, R, E, A, Q, P K, G, T, W, I, V, S or M; R211 G, Q, K, D or T; N215 H, L, G, V, R, or Y, wherein the numbering is that obtained from alignment of the variant sequence with the reference sequence shown as SEQ ID No. 2.

The variant enzyme may have an enhanced ratio of activity on galactolipids to either phospholipids and/or triglycerides when compared with the parent enzyme.

In some embodiments, the variant enzyme has a higher galactolipid transferase activity compared with its galactolipid hydrolytic activity compared with the parent enzyme.

The present invention also provides use of a variant glycolipid acyltransferase enzyme of the present invention in a substrate for preparing a lyso-glycolipid, for example digalactosyl monoglyceride (DGMG) or monogalactosyl monoglyceride (MGMG) by treatment of a glycolipid (e.g. digalactosyl diglyceride (DGDG) or monogalactosyl diglyceride (MGDG)) with the variant lipolytic enzyme of the present invention to produce the partial hydrolysis product, i.e. the lyso-glycolipid.

The substrate may be a foodstuff.

The present invention provides a method of preparing a foodstuff the method comprising adding a variant lipid acyltransferase enzyme of the invention to one or more ingredients of the foodstuff.

Further provided by the present invention is a method of preparing a baked product from a dough, the method comprising adding a variant glycolipid acyltransferase enzyme of the invention to the dough.

The present invention also provides use of a variant glycolipid acyltransferase enzyme of the invention in a process of treating egg or egg-based products to produce lysophospholipids.

The present invention further provides a process of enzymatic degumming of vegetable or edible oils, comprising treating the edible or vegetable oil with a variant glycolipid acyltransferase enzyme of the invention so as to hydrolyse a major part of the polar lipids (e.g. phospholipid and/or glycolipid).

The present invention further provides use of a variant glycolipid acyltransferase enzyme of the invention in a process for reducing the content of a phospholipid in an edible oil, comprising treating the oil with said variant lipolytic enzyme so as to hydrolyse a major part of the phospholipid, and separating an aqueous phase containing the hydrolysed phospholipid from the oil.

The present invention further provides use of a variant glycolipid acyltransferase enzyme of the invention in the bioconversion of polar lipids (preferably glycolipids) to make high value products, such as carbohydrate esters and/or protein esters and/or protein subunit esters and/or a hydroxy acid ester.

Further provided by the present invention is an immobilised variant glycolipid acyltransferase enzyme of the invention.

Further described herein is a method of producing a variant glycolipid acyltransferase enzyme comprising: (a) selecting a parent enzyme which is a lipid acyltransferase enzyme characterised in that the enzyme comprises the amino acid sequence motif GDSX, wherein X is one or more of the following amino acid residues L, A, V, I, F, Y, H, Q, T, N, M or S; (b) modifying one or more amino acids to produce a variant lipid acyltransferase; (c) testing the variant lipid acyltransferase for activity on a galactolipid substrate, and optionally a phospholipid substrate and/or optionally a triglyceride substrate; (d) selecting a variant enzyme with an enhanced activity towards galactolipids compared with the parent enzyme; and optionally (e) preparing a quantity of the variant enzyme.

Described herein is a variant glycolipid acyltransferase enzyme characterised in that the enzyme comprises the amino acid sequence motif GDSX, wherein X is one or more of the following amino acid residues L, A, V, I, F, Y, H, Q, T, N, M or S, and wherein the variant enzyme comprises one or more amino acid modifications compared with a parent sequence at any one or more of the amino acid residues defined in set 2 or set 4 or set 6 or set 7 (defined hereinbelow).

Described herein is a variant glycolipid acyltransferase enzyme characterised in that the enzyme comprises the amino acid sequence motif GDSX, wherein X is one or more of the following amino acid residues L, A, V, I, F, Y, H, Q, T, N, M or S, and wherein the variant enzyme comprises one or more amino acid modifications compared with a parent sequence at any one or more of the amino acid residues detailed in set 2 or set 4 or set 6 or set 7 (defined hereinbelow) identified by said parent sequence being structurally aligned with the structural model of P10480 defined herein, which is preferably obtained by structural alignment of P10480 crystal structure coordinates with 1IVN.PDB and/or 1DEO.PDB as taught herein.

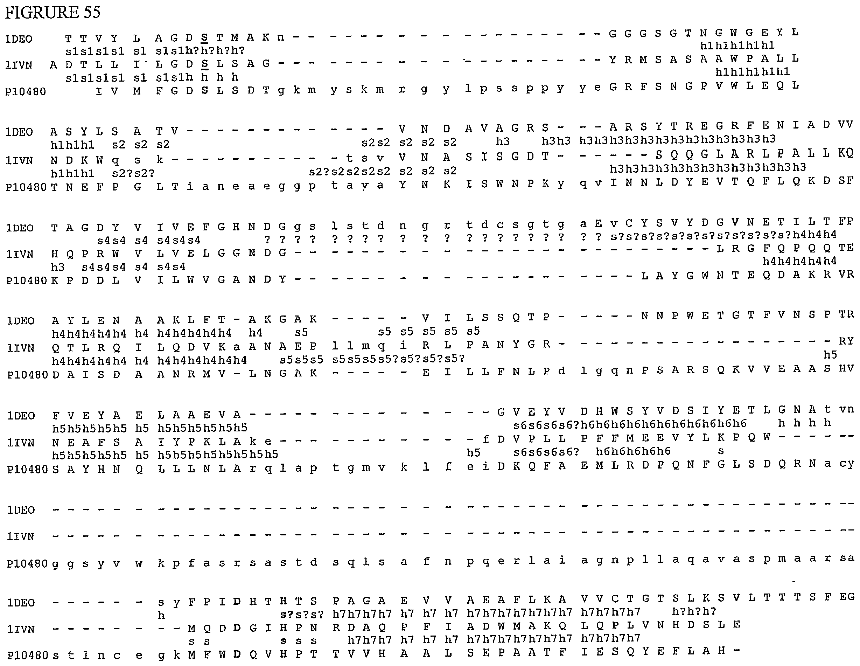

Described herein is a variant glycolipid acyltransferase enzyme characterised in that the enzyme comprises the amino acid sequence motif GDSX, wherein X is one or more of the following amino acid residues L, A, V, I, F, Y, H, Q, T, N, M or S, and wherein the variant enzyme comprises one or more amino acid modifications compared with a parent sequence at any one or more of the amino acid residues taught in set 2 identified when said parent sequence is aligned to the pfam consensus sequence (SEQ ID No. 1) and modified according to a structural model of P10480 to ensure best fit overlap (see Figure 55) as taught

Described herein is a variant glycolipid acyltransferase enzyme wherein the variant enzyme comprises an amino acid sequence, which amino acid sequence is shown as SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ED No. 37, SEQ ID No. 39, SEQ ID No. 41, SEQ ID No. 43, or SEQ ID No. 45 except for one or more amino acid modifications at any one or more of the amino acid residues defined in set 2 or set 4 or set 6 or set 7 (hereinafter defined) identified by sequence alignment with SEQ ID No. 2.

Described herein is a variant glycolipid acyltransferase enzyme wherein the variant enzyme comprises an amino acid sequence, which amino acid sequence is shown as SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45 except for one or more amino acid modifications at any one or more of the amino acid residues defined in set 2 or set 4 or set 6 or set 7 identified by said parent sequence being structurally aligned with the structural model of P10480 defined herein, which is preferably obtained by structural alignment of P10480 crystal structure coordinates with 1IVN.PDB and/or 1DEO.PDB as taught herein.

Described herein is a variant glycolipid acyltransferase enzyme wherein the variant enzyme comprises an amino acid sequence, which amino acid sequence is shown as SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45 except for one or more amino acid modifications at any one or more of the amino acid residues taught in set 2 identified when said parent sequence is aligned to the pfam consensus sequence (SEQ ID No. 1) and modified according to a structural model of F10480 to ensure best fit overlap (see Figure 55) as taught

Described herein is a variant glycolipolytic enzyme according to the present invention or obtained by a method according to the present invention in the manufacture of a substrate (preferably a foodstuff) to prepare a lyso-glycolipid, for example digalactosyl monoglyceride (DGMG) or monogalactosyl monoglyceride (MGMG) by treatment of a glycolipid (e.g. digalactosyl diglyceride (DGDG) or monogalactosyl diglyceride (MGDG)) with the variant lipolytic enzyme described herein to produce the partial hydrolysis product, i.e. the lyso-glycolipid.

Further described herein is the use of a variant lipolytic enzyme described herein in the manufacture of a substrate (preferably a foodstuff) to prepare a lyso-phospholipid, for example lysolecithin, by treatment of a phospholipid (e.g. lecithin) with the variant lipolytic enzyme described herein to produce a partial hydrolysis product, i.e a lyso-phospholipid.

Described herein is a method of preparing a foodstuff the method comprising adding a variant lipolytic enzyme described herein to one or more ingredients of the foodstuff.

Described herein is a method of preparing a baked product from a dough, the method comprising adding a variant lipolytic enzyme according to the present invention or obtained by a method according to the present invention to the dough.

Also described herein is the use of a variant lipolytic enzyme described herein in the manufacture of an egg-based product for producing lysophospholipids.

Described herein is a method of treating eggs or egg-based products comprising adding a variant lipolytic enzyme described herein to an egg or an egg-based product to produce a lysophospholipid.

The variants described herein may be used in a process of production of a snack food such as instant noodles in analogy with WO02/065854.

The present description relates to the use of the variant lipid acyltransferase described herein to result in a preferred technical effect or combination of technical effects in for example the foodstuff (such as those listed herein under Technical Effects').

Further described herein is a process of enzymatic degumming of vegetable or edible oils, comprising treating the edible or vegetable oil with a variant lipolytic enzyme described herein so as to hydrolyse a major part of the polar lipids (e.g. phospholipid and/or glycolipid).

Described herein is a process comprising treating a phospholipid so as to hydrolyse fatty acyl groups, which process comprising admixing said phospholipids with a variant lipolytic enzyme described herein.

Described herein is a process of reducing the content of a phospholipid in an edible oil, comprising treating the oil with a variant lipolytic enzyme described herein so as to hydrolyse a major part of the phospholipid, and separating an aqueous phase containing the hydrolysed phospholipid from the oil.

Described herein is a method of preparing a variant lipolytic enzyme described herein the method comprising transforming a host cell with a recombinant nucleic acid comprising a nucleotide sequence coding for said variant lipolytic enzyme, the host cell being capable of expressing the nucleotide sequence coding for the polypeptide of the lipolytic enzyme, cultivating the transformed host cell under conditions where the nucleic acid is expressed and harvesting the variant lipolytic enzyme.

The present description relates to the use of a variant lipolytic enzyme described herein in the bioconversion of polar lipids (preferably glycolipids) to make high value products, such as carbohydrate esters and/or protein esters and/or protein subunit esters and/or a hydroxy acid ester.

A method of bioconverting polar lipids (preferably glycolipids) to high value products, which method comprises admixing said polar lipid with a variant lipolytic enzyme is described herein which method comprises admixing said polar lipid with a variant lipolytic enzyme described herein.

The present description yet further relates to an immobilised variant lipolytic enzyme described herein.

Other aspects concerning the nucleotide sequences which can be used in the present description include: a construct comprising the sequences as described herein a vector comprising the sequences for use as described herein a plasmid comprising the sequences for use as described herein a transformed cell comprising the sequences for use as described herein a transformed tissue comprising the sequences for use as described herein a transformed organ comprising the sequences for use as described herein a transformed host comprising the sequences for use as described herein a transformed organism comprising the sequences for use as described herein. Also described herein are methods of expressing the nucleotide sequence for use as described herein using the same, such as expression in a host cell; including methods for transferring same. The present description further encompasses methods of isolating the nucleotide sequence, such as isolating from a host cell.

Other aspects concerning the amino acid sequence for use as described herein include: a construct encoding the amino acid sequences for use as described herein ; a vector encoding the amino acid sequences for use as described herein a plasmid encoding the amino acid sequences for use as described herein a transformed cell expressing the amino acid sequences for use as described herein a transformed tissue expressing the amino acid sequences for use as described herein a transformed organ expressing the amino acid sequences for use as described herein a transformed host expressing the amino acid sequences for use as described herein a transformed organism expressing the amino acid sequences for use as described herein. The present description also encompasses methods of purifying the amino acid sequence for use as described herein using the same, such as expression in a host cell; including methods of transferring same, and then purifying said sequence.

For the ease of reference, these and further aspects described herein are now discussed under appropriate section headings. However, the teachings under each section are not necessarily limited to each particular section.

DEFINITION OF SETS

Amino acid set 1:

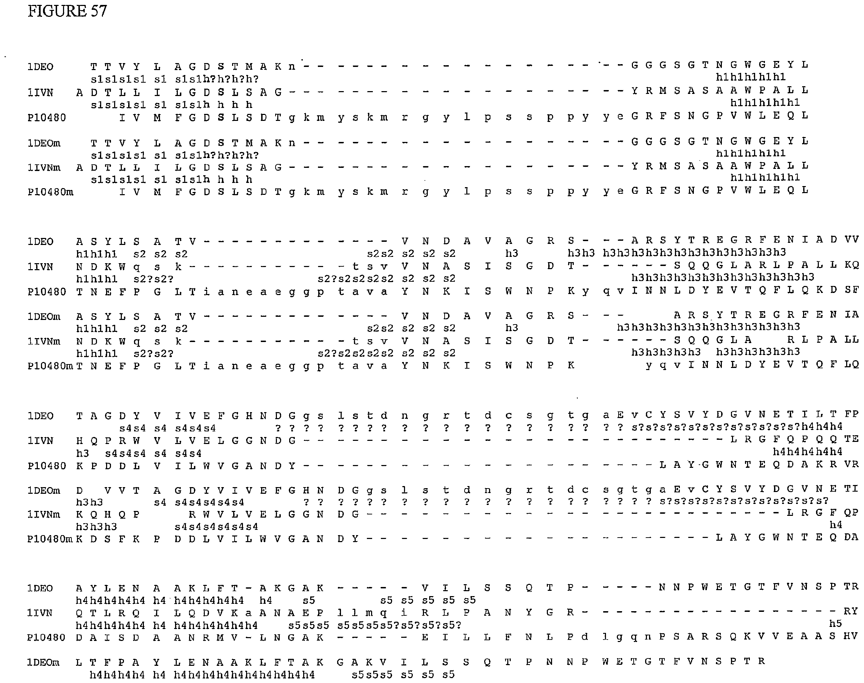

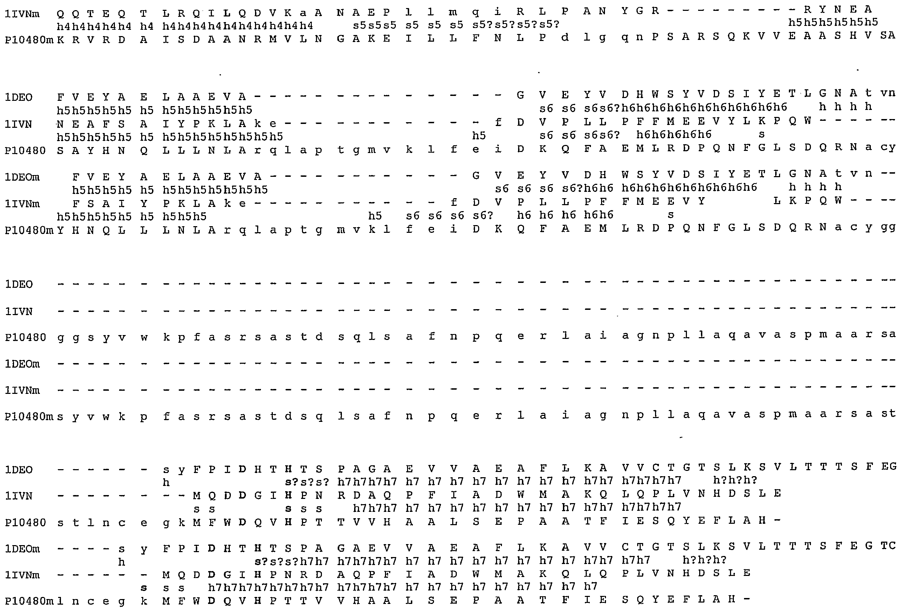

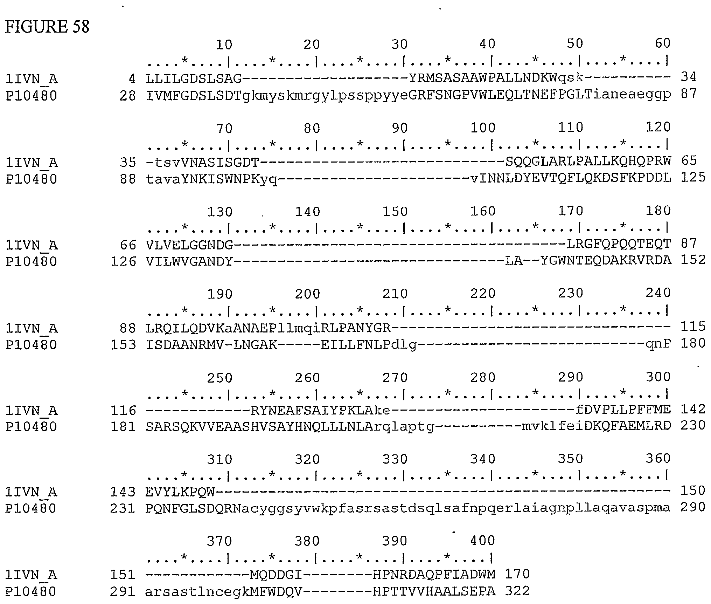

Amino acid set 1 (note that these are amino acids in 1IVN - Figure 57 and Figure 58.) Gly8, Asp9, Ser10, Leu11, Ser12, Tyr15, Gly44, Asp45, Thr46, Glu69, Leu70, Gly71, Gly72, Asn73, Asp74, Gly75, Leu76, Gln106, Ile107, Arg108, Leu109, Pro110, Tyr113, Phe121, Phe139, Phe140, Met141, Tyr145, Met151, Asp154, His157, Gly155, Ile156, Pro158

The highly conserved motifs, such as GDSx and catalytic residues, were deselected from set 1 (residues underlined). For the avoidance of doubt, set 1 defines the amino acid residues within 10Å of the central carbon atom of a glycerol in the active site of the 1IVN model.

Amino acid set 2:

Amino acid set 2 (note that the numbering of the amino acids refers to the amino acids in the P10480 mature sequence)
Leu17, Lys22, Met23, Gly40, Asn80, Pro81, Lys82, Asn87, Asn88, Trp111, Val112, Alga114, Tyr117, Leu118, Pro156, Gly159, Gln160, Asn161, Pro162, Ser163, Ala164, Arg165, Ser166, Gln167, Lys168, Val169, Val170, Glu171, Ala172, Tyr179, His180, Asn181, Met209, Leu210, Arg211, Asn215, Lys284, Met285, Gln289 and Val290.

Table of selected residues in Set 1 compared with Set 2:

| IVN model<br>IVN | A.hyd homologue | | P10480 |
|---|---|---|---|
| | PFAM | Structure | Mature sequence Residue Number |
| Gly8 | Gly32 | | |
| Asp9 | Asp33 | | |
| Ser10 | Ser34 | | |
| Leu11 | Leu35 | | Leu17 |
| Ser12 | Ser36 | | Ser18 |
| | | | Lys22 |
| | | | Met23 |

(continued)

| IVN model IVN | A.hyd homologue | | P10480 |
|---|---|---|---|
| | PFAM | Structure | Mature sequence Residue Number |
| Tyr15 | Gly58 | | Gly40 |
| Gly44 | Asn98 | | Asn80 |
| Asp45 | Pro99 | | Pro81 |
| Thr46 | Lys100 | | Lys82 |
| | | | Asn87 |
| | | | Asn88 |
| Glu69 | Trp129 | | Trp111 |
| Leu70 | Val130 | | Val112 |
| Gly71 | Gly131 | | |
| Gly72 | Ala132 | | Ala114 |
| Asn73 | Asn133 | | |
| Asp74 | Asp 134 | | |
| Gly75 | Tyr135 | | Tyr117 |
| Leu76 | Leu136 | | Leu118 |
| Gln106 | | Pro 174 | Pro156 |
| Ile107 | | Gly177 | Gly159 |
| Arg108 | | Gln178 | Gln160 |
| Leu109 | | Asn179 | Asn161 |
| Pro110 | | 180 to 190 | Pro162 |
| Tyr113 | | | Ser163 |
| | | | Ala164 |
| | | | Arg165 |
| | | | Ser166 |
| | | | Gln167 |
| | | | Lys168 |
| | | | Val169 |
| | | | Val170 |
| | | | Glu171 |
| | | | Ala172 |
| Phe121 | His198 | Tyr197 | Tyr179 |
| | | His198 | His180 |
| | | Asn199 | Asn181 |
| Phe139 | Met227 | | Met209 |
| Phe140 | Leu228 | | Leu210 |
| Met141 | Arg229 | | Arg211 |
| Tyr145 | Asn233 | | Asn215 Lys284 |
| Met151 | Met303 | | Met285 |
| Asp154 | Asp306 | | |
| Gly155 | Gln307 | | Gln289 |
| Ile156 | Val308 | | Val290 |
| His157 | His309 | | |
| Pro158 | Pro310 | | |

Amino acid set 3:

Amino acid set 3 is identical to set 2 but refers to the *Aeromonas salmonicida* (SEQ ID No. 28) coding sequence, i.e. the amino acid residue numbers are 18 higher in set 3 as this reflects the difference between the amino acid numbering in the mature protein (SEQ ID No. 2) compared with the protein including a signal sequence (SEQ ID No. 28).

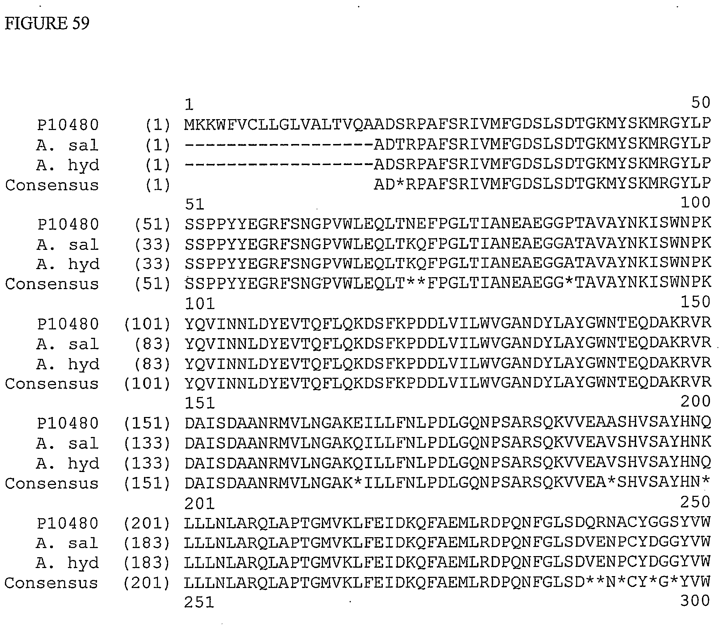

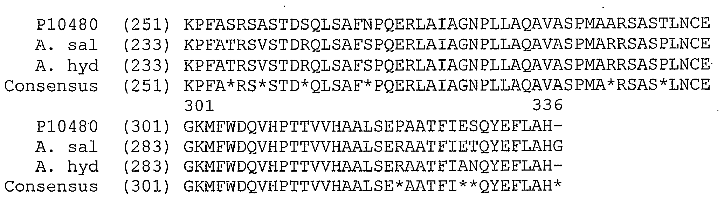

The mature proteins of *Aeromonas salmonicida* GDSX (SEQ ID No. 28) and *Aeromonas hydrophila* GDSX (SEQ ID No. 26) differ in five amino acids. These are Thr3Ser, Gln182Lys, Glu309Ala, Ser310Asn, Gly318-, where the *salmonicida* residue is listed first and the *hydrophila* residue is listed last (FIGURE 59). The *hydrophila* protein is only 317 amino acids long and lacks a residue in position 318. The *Aeromonas salmonicidae* GDSX has considerably high activity on polar lipids such as galactolipid substrates than the *Aeromonas hydrophila* protein. Site scanning was performed on all five amino acid positions.

Amino acid set 4:

Amino acid set 4 is S3, Q182, E309, S310, and -318.

Amino acid set 5:

F13S, D15N, S18G, S18V, Y30F, D116N, D116E, D157N, Y226F, D228N Y230F.

Amino acid set 6:

Amino acid set 6 is Ser3, Leu17, Lys22, Met23, Gly40, Asn80, Pro81, Lys82, Asn 87, Asn88, Trp111, Val112, Ala114, Tyr117, Leu118, Pro156, Gly159, Gln160, Asn161, Pro162, Ser163, Ala164, Arg165, Ser166, Gln167, Lys168, Val169, Val170, Glu171, Ala172, Tyr179, His180, Asn181, Gln182, Met209, Leu210, Arg211, Asn215, Lys284, Met285, Gln289, Val290, Glu309, Ser310, -318.

The numbering of the amino acids in set 6 refers to the amino acids residues in P10480 (SEQ ID No. 2) - corresponding amino acids in other sequence backbones can be determined by homology alignment and/or structural alignment to P10480 and/or 1IVN.

Amino acid set 7:

Amino acid set 7 is Ser3, Leu17, Lys22, Met23, Gly40, Asn80, Pro81, Lys82, Asn 87, Asn88, Trp111, Val112, Ala114, Tyr117, Leu118, Pro156, Gly159, Gln160, Asn161, Pro162, Ser163, Ala164, Arg165, Ser166, Gln167, Lys168, Val169, Val170, Glu171, Ala172, Tyr179, His180, Asn181, Gln182, Met209, Leu210, Arg211, Asn215, Lys284, Met285, Gln289, Val290, Glu309, Ser310, -318, Y30X (where X is selected from A, C, D, E, G, H, I, K, L, M, N, P, Q, R, S, T, V, or W), Y226X (where X is selected from A, C, D, E, G, H, I, K, L, M, N, P, Q, R, S, T, V, or W), Y230X (where X is selected from A, C, D, E, G, H, I, K, L, M, N, P, Q, R, S, T, V, or W), S18X (where X is selected from A, C, D, E, F, H, I, K, L, M, N, P, Q, R, T, W or Y), D157X (where X is selected from A, C, E, F, G, H, I, K, L, M, P, Q, R, S, T, V, W or Y).

The numbering of the amino acids in set 7 refers to the amino acids residues in P10480 (SEQ ID No. 2) - corresponding amino acids in other sequence backbones can be determined by homology alignment and/or structural alignment to P10480 and/or 1IVN).

DETAILED ASPECTS OF THE PRESENT INVENTION

Preferably, the parent lipid acyltransferase enzyme comprises any one of the following amino acid sequences: SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45 or an amino acid sequence which has 75% or more identity with any one of the sequences shown as SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45.

Suitably, the parent lipid acyltransferase enzyme comprises an amino acid sequence which has at least 80%, preferably at least 85%, more preferably at least 90%, more preferably at least 95%, more at least 98% homology with any one of the sequences shown as SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45. Suitably, the parent lipid acyltransferase enzyme may be encoded by any one of the following nucleotide sequences: SEQ ID No. 7, SEQ ID No. 8, SEQ ID No. 9, SEQ ID No. 10, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No.27, SEQ ID No. 29, SEQ ID No. 31, SEQ ID No. 32, SEQ ID No. 35, SEQ ID No. 38, SEQ ID No.

40, SEQ ID No. 42, SEQ ID No. 44 or SEQ ID No. 46 or a nucleotide sequence which has at least 75% or more identity with any one of the sequences shown as SEQ ID No. 7, SEQ ID No. 8, SEQ ID No. 9, SEQ ID No. 10, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No.27, SEQ ID No. 29, SEQ ID No. 31, SEQ ID No. 32, SEQ ID No. 35, SEQ ID No. 38, SEQ ID No. 40, SEQ ID No. 42, SEQ ID No. 44 or SEQ ID No. 46.

Suitably, the nucleotide sequence may have 80% or more, preferably 90% or more, more preferably 95% or more, even more preferably 98% or more identity with any one of the sequences shown as SEQ ID No. 7, SEQ ID No. 8, SEQ ID No. 9, SEQ ID No. 10, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No.27, SEQ ID No. 29, SEQ ID No. 31, SEQ ID No. 32, SEQ ID No. 35, SEQ ID No. 38, SEQ ID No. 40, SEQ ID No. 42, SEQ ID No. 44 or SEQ ID No. 46.

Preferably, the parent enzyme is modified at one or more of the amino acid residues defined in set 2 or set 4 or set 6 or set 7 when aligned to the reference sequence (SEQ ID No. 2) or structurally aligned to the structural model of P10480, or aligned to the pfam consensus sequence and modified according to the structural model of P10480.

Suitably the variant enzyme may have an enhanced ratio of activity on galactolipids compared with the activity on either phospholipids and/or triglycerides when compared with the parent enzyme.

Suitably, the method described herein may comprise testing the variant lipid acyltransferase for:

(i) transferase activity from a galactolipid substrate, and
(ii) transferase activity from a phospholipids substrate; and

selecting a variant enzyme, which when compared with the parent enzyme, has an enhanced ratio of transferase activity from galactolipids compared with phospholipids.

Suitably, the ratio of transferase activity from galactolipids compared with phospholipids of the variant enzyme described herein may be at least 1, at least 2, at least 3, at least 4 or at least 5.

Suitably, the method described herein may comprise testing the variant lipid acyltransferase for:

(a) transferase activity from a galactolipid substrate, and
(b) hydrolytic activity on a galactolipid substrate; and

selecting a variant enzyme with an enhanced ratio of transferase activity from galactolipids compared with its hydrolytic activity on glycolipids, compared with the parent enzyme.

Suitably, the ratio of transferase activity on galactolipids compared to hydrolytic activity on galactolipids may be great than 1, at least 1.5, at least 2, at least 4 or at least 5.

An assay for determining the transferase and hydrolytic activities from galactolipids and/or phospholipids is/are taught in Example 8 for example.

The term "enhanced activity towards galactolipids" means the enzyme has an enhanced (i.e. higher) transferase activity when the lipid acyl donor is a galactolipid compared with the parent enzyme (galactolipid transferase activity) and/or has an increased ratio of galactolipid transferase activity when compared with phospholipids transferase activity compared with the parent enzyme (GLt:PLt ratio) and/or has an increased ratio of galactolipid transferase activity when compared with galactolipid hydrolysis activity compared with the parent enzyme (GLt:GLh ratio).

Suitably, the variant enzyme compared with the parent enzyme may have an increased galactolipid transferase activity and either the same or less galactolipid hydrolytic activity. In other words, suitably the variant enzyme may have a higher galactolipid transferase activity compared with its galactolipid hydrolytic activity compared with the parent enzyme. Suitably, the variant enzyme may preferentially transfer an acyl group from a galactolipid to an acyl acceptor rather than simply hydrolysing the galactolipid.

The enzyme described herein may have an increased transferase activity towards phospholipids (i.e an. increased phospholipid transferase activity) as compared with the parent enzyme. This increased phospholipid transferase activity may be independent of the enhanced activity towards galactolipids. Suitably, however, the variant enzyme may have an increased galactolipid transferase activity and an increased phospholipid transferase activity.

Described herein is a variant lipid acyltransferase enzyme characterised in that the enzyme comprises the amino acid sequence motif GDSX, wherein X is one or more of the following amino acid residues L, A, V, I, F, Y, H, Q, T, N, M or S, wherein the variant has an enhanced activity towards phospholipids, preferably enhanced phospholipid transferase activity, compared with the parent enzyme and wherein the variant enzyme comprises one or more amino acid modifications compared with a parent sequence at any one or more of the amino acid residues defined in set 2 or set 4 or set 6 or set 7.

The term "modifying" as used herein means adding, substituting and/or deleting. Preferably the term "modifying" means "substituting".

For the avoidance of doubt, when an amino acid is substituted in the parent enzyme it is preferably substituted with an amino acid which is different from that originally found at that position in the parent enzyme thus to produce a variant enzyme. In other words, the term "substitution" is not intended to cover the replacement of an amino acid with the same amino acid.

Preferably, the parent enzyme is an enzyme which comprises the amino acid sequence shown as SEQ ID No. 2 and/or SEQ ID No. 28.

Preferably, the variant enzyme is an enzyme which comprises an amino acid sequence, which amino acid sequence is shown as SEQ ID No. 2 except for one or more amino acid modifications at any one or more of the amino acid residues defined in set 2 or set 4 or set 6 or set 7.

In one embodiment, preferably the variant enzyme comprises one or more amino acid modifications compared with the parent sequence at at least one of the amino acid residues defined in set 4.

Suitably, the variant enzyme comprises one or more of the following amino acid modifications compared with the parent enzyme:

S3E, A, G, K, M, Y, R, P, N, T or G
E309Q, R or A, preferably Q or R
-318Y, H, S or Y, preferably Y.

Preferably, X of the GDSX motif is L. Thus, preferably the parent enzyme comprises the amino acid motif GDSL.

Preferably the method of producing a variant lipid acyltransferase enzyme further comprises one or more of the following steps:

1) structural homology mapping or
2) sequence homology alignment.

Suitably, the structural homology mapping may comprise one or more of the following steps:

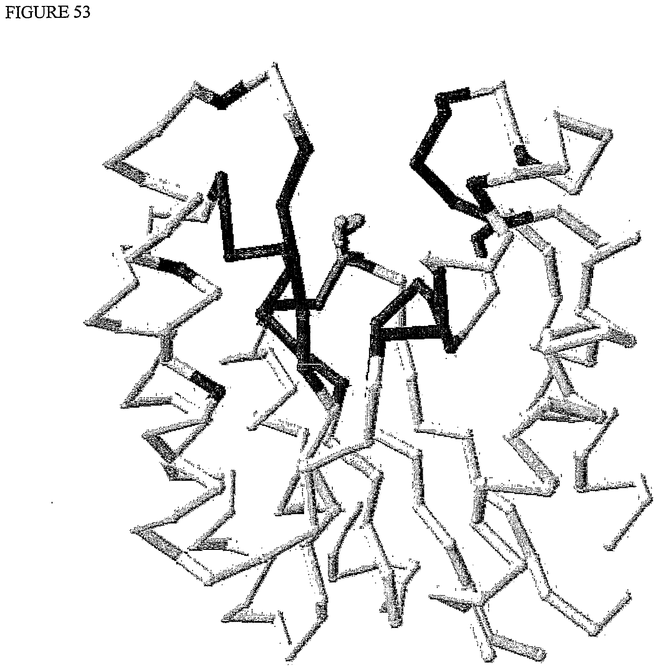

i) aligning a parent sequence with a structural model (1IVN.PDB) shown in Figure 52;
ii) selecting one or more amino acid residue within a 10Å sphere centred on the central carbon atom of the glycerol molecule in the active site (see Figure 53) (such as one or more of the amino acid residues defined in set 1 or set 2); and
iii) modifying one or more amino acids selected in accordance with step (ii) in said parent sequence.

The amino acid residue selected may reside within a 9, preferably within a 8, 7, 6, 5, 4, or 3 A sphere centred on the central carbon atom of the glycerol molecule in the active site (see Figure 53).

Suitably, the structural homology mapping may comprise one or more of the following steps:

i) aligning a parent sequence with a structural model (1IVN.PDB) shown in Figure 52;
ii) selecting one or more amino acids within a 10Å sphere centred on the central carbon atom of the glycerol molecule in the active site (see Figure 53) (such as one or more of the amino acid residues defined in set 1 or set 2);
iii) determining if one or more amino acid residues selected in accordance with step (ii) are highly conserved (particularly are active site residues and/or part of the GDSx motif and/or part of the GANDY motif); and
iv) modifying one or more amino acids selected in accordance with step (ii), excluding conserved regions identified in accordance with step (iii) in said parent sequence.

The amino acid residue selected may reside within a 9, preferably within a 8, 7, 6, 5, 4, or 3 A sphere centred on the central carbon atom of the glycerol molecule in the active site (see Figure 53).

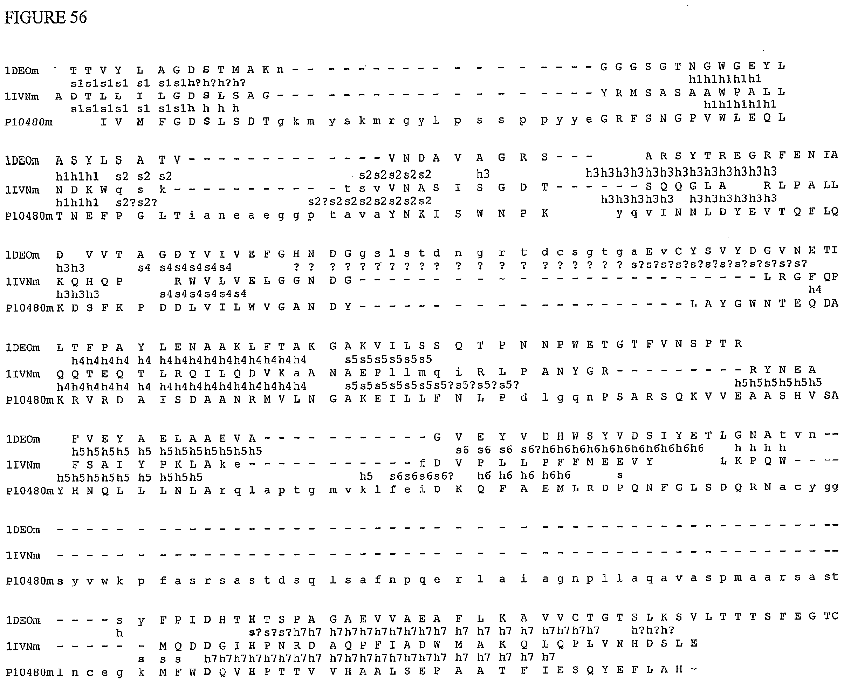

Alternatively to, or in combination with, the structural homology mapping described above, the structureal homology mapping can be performed by selecting specific loop regions (LRs) or intervening regions (IVRs) derived from the pfam aligmment (Alignment 2, Figure 56) overlayed with the P10480 model and 1IVN. The loop regions (LRs) or intervening regions (IVRs) are defined in the Table below:

| | P10480 amino acid positions (SEQ ID No 2) |
|---|---|
| IVR1 | 1-19 |
| Loop1 (LR1) | 20-41 |
| IVR2 | 42-76 |
| Loop2 (LR2) | 77-89 |

(continued)

| | P10480 amino acid positions (SEQ ID No 2) |
|---|---|
| IVR3 | 90-117 |
| Loop3 (LR3) | 118-127 |
| IVR4 | 128-145 |
| Loop4 (LR4) | 146-176 |
| IVR5 | 177-207 |
| Loop5 (LR5) | 208-287 |
| IVR6 | 288-317 |

The variant acyltransferase enzyme may not only comprises an amino acid modifications at one or more of the amino acids defined in any one of sets 1-4 and 6-7, but may also comprises at least one amino acid modification in one or more of the above defined intervening regions (IVR1-6) (preferably in one or more of the IVRs 3, 5 and 6, more preferably in IVR 5 or IVR 6) and/or in one or more of the above-defined loop regions (LR1-5) (preferably in one or more of LR1, LR2 or LR5, more preferably in LR5).

The variant acyltransferase described herein or obtained by a method according to the present invention may comprise one or more amino acid modification which is not only defined by one or more of set 2, 4, 6 and 7, but also is within one or more of the IVRs 1-6 (preferably within IVR 3, 5 or 6, more preferably within in IVR 5 or IVR 6) or within one or more of the LRs 1-5 (preferably within LR1, LR2 or LR5, more preferably within LR5).

Suitably, the variant acyltransferase described herein may comprise one or more amino acid modification which is not only in set 1 or 2, but also is within IVR 3.

Suitably, the variant acyltransferase described herein may comprise one or more amino acid modification which is not only in set 1 or 2, but also is within IVR 5.

Suitably, the variant acyltransferase described herein may comprise one or more amino acid modification which is not only in set 1 or 2, but also is within IVR 6.

Suitably, the variant acyltransferase described herein may comprise one or more amino acid modification which is not only in set 1 or 2, but also is within LR 1.

Suitably, the variant acyltransferase described herein may comprise one or more amino acid modification which is not only in set 1 or 2, but also is within LR 2.

Likewise, the variant acyltransferse enzyme may not only comprises an amino acid modification at one or more amino acid residues which reside within a 10, preferably within a 9, 8, 7, 6, 5, 4, or 3, A sphere centred on the central carbon atom of the glycerol molecule in the active site (see Figure 53), but may also comprises at least one amino acid modification in one or more of the above defined intervening regions (IVR1-6) (preferably in one or more of IVRs 3, 5 and 6, more preferably in IVR 5 or IVR 6) and/or in one or more of the above-defined loop regions (LR1-5) (preferably in one or more of LR1, LR2 or LR5, more preferably in LR5).

Preferably the amino acid modification is at one or more amino acid residues which reside within a 10Å sphere and also within LR5.

Thus, the structural homology mapping may comprise one or more of the following steps:

i) aligning a parent sequence with a structural model (1IVN.PDB) shown in Figure 52;
ii) selecting one or more amino acid residue within a 10Å sphere centred on the central carbon atom of the glycerol molecule in the active site (see Figure 53) (such as one or more of the amino acid residues defined in set 1 or set 2); and/or selecting one or more amino acid residues within IVR1-6) (preferably within IVR 3, 5 or 6, more preferably within in IVR 5 or IVR 6); and/or selecting one or more amino acid residues within LR1-5 (preferably within LR1, LR2 or LR5, more preferably within LR5); and
iii) modifying one or more amino acids selected in accordance with step (ii) in said parent sequence.

The amino acid residue selected may reside within a 9, preferably within a 8, 7, 6, 5, 4, or 3 Å sphere centred on the central carbon atom of the glycerol molecule in the active site (see Figure 53).

Suitably, the structural homology mapping may comprise one or more of the following steps:

i) aligning a parent sequence with a structural model (1IVN.PDB) shown in Figure 52;
ii) selecting one or more amino acids within a 10Å sphere centred on the central carbon atom of the glycerol molecule in the active site (see Figure 53) (such as one or more of the amino acid residues defined in set 1 or set 2); and/or selecting one or more amino acid residues within IVR1-6) (preferably within IVR 3, 5 or 6, more preferably within in IVR 5 or IVR 6); and/or selecting one or more amino acid residues within LR1-5 (preferably within LR1, LR2 or LR5, more preferably within LR5);

iii) determining if one or more amino acid residues selected in accordance with step (ii) are highly conserved (particularly are active site residues and/or part of the GDSx motif and/or part of the GANDY motif); and

modifying one or more amino acids selected in accordance with step (ii), excluding conserved regions identified in accordance with step (iii) in said parent sequence.

Suitably, the one or more amino acids selected in the methods detailed above are not only within a 10A sphere centred on the central carbon atom of the glycerol molecule in the active site (see Figure 53) (such as one or more of the amino acid residues defined in set 1 or set 2), but are also within one or more of the IVRs 1-6 (preferably within IVR 3, 5 or 6, more preferably within in IVR 5 or IVR 6) or within one or more of the LRs 1-5 (preferably within LR1, LR2 or LR5, more preferably within LR5).

Preferably the one or more amino acid modifications is/are within LR5. When it is the case that the modification(s) is within LR5, the modification is not one which is defined in set 5. Suitably, the one or more amino acid modifications not only fall with the region defined by LR5, but also constitute an amino acid within one or more of set 2, set 4, set 6 or set 7.

Suitably, the sequence homology alignment may comprise one or more of the following steps:

i) selecting a first parent lipid acyltransferase;
ii) identifying a second related lipid acyltransferase having a desirable activity;
iii) aligning said first parent lipid acyltransferase and the second related lipid acyltransferase;
iv) identifying amino acid residues that differ between the two sequences; and
v) modifying one or more of the amino acid residues identified in accordance with step (iv) in said parent lipid acyltransferase.

Suitably, the sequence homology alignment may comprise one or more of the following steps:

i) selecting a first parent lipid acyltransferase;
ii) identifying a second related lipid acyltransferase having a desirable activity;
iii) aligning said first parent lipid acyltransferase and the second related lipid acyltransferase;
iv) identifying amino acid residues that differ between the two sequences;
v) determining if one or more amino acid residues selected in accordance with step (iv) are highly conserved (particularly are active site residues and/or part of the GDSx motif and/or part of the GANDY motif); and
vi) modifying one or more of the amino acid residues identified in accordance with step (iv) excluding conserved regions identified in accordance with step (v) in said parent sequence.

Suitably, said first parent lipid acyltransferase may comprise any one of the following amino acid sequences: SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45.

Suitably, said second related lipid acyltransferase may comprise any one of the following amino acid sequences: SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45.

The variant enzyme must comprise at least one amino acid modification compared with the parent enzyme. In some embodiments, the variant enzyme may comprise at least 2, preferably at least 3, preferably at least 4, preferably at least 5, preferably at least 6, preferably at least 7, preferably at least 8, preferably at least 9, preferably at least 10 amino acid modifications compared with the parent enzyme.

Suitably the methods according to the present invention may comprise a further step of formulating the variant enzyme into an enzyme composition and/or a foodstuff composition, such as a bread improving composition.

In order to align a GDSx polypeptide sequence (parent sequence) with SEQ ID No. 2 (P01480), sequence alignment such as pairwise alignment can be used (http://www.ebi.ac.uk/emboss/align/index.html). Thereby, the equivalent amino acids in alternative parental GDSx polypeptides, which correspond to one or more of the amino acids defined in set 2 or set 4 or set 6 or set 7 in respect of SEQ ID No. 2 can be determined and modified. As the skilled person will readily appreciate, when using the emboss pairwise alignment, standard settings usually suffice. Corresponding residues can be identified using "needle" in order to make an alignment that covers the whole length of both sequences. However, it is also possible to find the best region of similarity between two sequences, using "water".

Alternatively, particularly in instances where parent GDSx polypeptides share low homology with SEQ ID No. 2, the corresponding amino acids in alternative parental GDSx polypeptides which correspond to one or more of the amino acids defined in set 2, set 4, set 6 or set 7 in respect of SEQ ID No. 2 can be determined by structural alignment to the structural model of P10480, obtained by comparison of P10480 derived structural model with the structural coordinates of 1IVN.PDB and 1DEO.PDB using the 'Deep View Swiss-PDB viewer' (obtained from www.expasy.org/spdbv/) (Figure 53 and Example 1). Equivalent residues are identified as those overlapping or in closest proximity to the residues in the obtained structural model of P010480, as illustrated in the Table comparing Set 1 and Set 2 (see section entitled "Definition of Sets" hereinabove). In this way other GDSX polypeptides can be compared against the 1IVN.PBD crystal co-ordinates, and equivalent residues to Set 1 determined.

Alternatively, particularly in instances where a parent GDSx polypeptide shares a low homology with SEQ ID No. 2, the equivalent amino acids in alternative parental GDSx polypeptides, which correspond to one or more of the amino acids defined in set 2 or set 4 or set 6 or set 7 in respect of SEQ ID No. 2 can be determined from an alignment obtained from the PFAM database (PFAM consensus) modified based on the structural alignment as shown in Alignment 1 (Figure 55). The modification based on the structural models may be necessary to slightly shift the alignment in order to ensure a best fit overlap. Alignment 1 (Figure 55) provides guidance in this regard.

The variant enzyme described herein preferably does not comprise one or more of the amino acid modifications defined in set 5.

Suitably the variant enzyme may be prepared using site directed mutagenesis.

Alternatively, one can introduce mutations randomly for instance using a commercial kit such as the GeneMorph PCR mutagenesis kit from Stratagene, or the Diversify PCR random mutagenesis kit from Clontech. EP 0 583 265 refers to methods of optimising PCR based mutagenesis, which can also be combined with the use of mutagenic DNA analogues such as those described in EP 0 866 796. Error prone PCR technologies are suitable for the production of variants of lipid acyl transferases with preferred characteristics. WO0206457 refers to molecular evolution of lipases.

A third method to obtain novel sequences is to fragment non-identical nucleotide sequences, either by using any number of restriction enzymes or an enzyme such as Dnase I, and reassembling full nucleotide sequences coding for functional proteins (hereinafter referred to as "shuffling"). Alternatively one can use one or multiple non-identical nucleotide sequences and introduce mutations during the reassembly of the full nucleotide sequence. DNA shuffling and family shuffling technologies are suitable for the production of variants of lipid acyl transferases with preferred characteristics. Suitable methods for performing 'shuffling' can be found in EP0 752 008, EP1 138 763, EP1 103 606. Shuffling can also be combined with other forms of DNA mutagenesis as described in US 6,180,406 and WO 01/34835.

Thus, it is possible to produce numerous site directed or random mutations into a nucleotide sequence, either *in vivo* or *in vitro,* and to subsequently screen for improved functionality of the encoded variant polypeptide by various means.

As a non-limiting example, In addition, mutations or natural variants of a polynucleotide sequence can be recombined with either the wild type or other mutations or natural variants to produce new variants. Such new variants can also be screened for improved functionality of the encoded polypeptide.

The following regions may preferably be selected for localised random mutagenesis and/or shuffling: IVR3, IVR 5, IVR 6, LR1, LR2, and/or LR5, most preferably LR5.

For the production of libraries of variants microbial eukaryotic or prokaryotic expression hosts may be used. In order to ensure uniform expression within a library of variants, low copy number, preferably single event chromosomal expression systems may be preferred. Expression systems with high transformation frequencies are also preferred, particularly for the expression of large variant libraries (>1000 colonies), such as those prepared using random mutagenesis and/or shuffling technologies.

Suitable methods for the use of a eukaryotic expression host, namely yeast, in the production of enzymes are described in EP1131416. Microbial eukaryotic expression hosts, such as yeast, may be preferred for the expression of variant libraries produced using a eukaryotic acyltransferase parent gene.

Suitable methods using *Bacillus,* i.e. *Bacillus subtilis,* as an expression host in the production of enzymes are described in WO02/14490. Microbial prokaryotic expression hosts, such as *Bacillus,* may be preferred for the expression of variant libraries produced using a prokaryotic acyltransferase parent gene, for example the P10480 reference sequence (SEQ ID No 2).

Suitably, the variant lipid acyltransferase described herein retains at least 70%, preferably at least 80%, preferably at least 90%, preferably at least 95%, preferably at least 97%, preferably at least 99% homology with the parent enzyme.

Suitable parent enzymes may include any enzyme with esterase or lipase activity.

Preferably, the parent enzyme aligns to the pfam00657 consensus sequence.

A variant lipid acyltransferase enzyme may retain or incorporates at least one or more of the pfam00657 consensus sequence amino acid residues found in the GDSx, GANDY and HPT blocks.

Enzymes, such as lipases with no or low lipid acyltransferase activity in an aqueous environment may be mutated using molecular evolution tools to introduce or enhance the transferase activity, thereby producing a variant lipid acyltransferase enzyme with significant transferase activity suitable for use in the compositions and methods of the present invention.

Suitably, the lipid acyltransferase for use as described herein may be a variant with enhanced enzyme activity on polar lipids, preferably glycolipids, when compared to the parent enzyme. Preferably, such variants also have low or no activity on lyso polar lipids. The enhanced activity on polar lipids, preferably glycolipids may be the result of hydrolysis and/or transferase activity or a combination of both.

Variant lipid acyltransferases for use as described herein may have decreased activity on triglycerides, and/or monoglycerides and/or diglycerides compared with the parent enzyme.

Suitably the variant enzyme may have no activity on triglycerides and/or monoglycerides and/or diglycerides. Low activity on triglycerides is preferred in variant enzymes which are to be used for bakery applications, for treatment of egg or egg-based products and/or for degumming oils.

Suitably the variant enzyme may have a high activity on diglycerdies and no or low activity on triglycerides.

When referring to specific amino acid residues herein the numbering is that obtained from alignment of the variant sequence with the reference sequence shown as SEQ ID No. 2.

In one aspect preferably the variant enzyme comprises one or more of the following amino acid substitutions:

S3A, C, D, E, F, G, H, I, K, L, M, N, P, Q, R, T, V, W, or Y; and/or
L17A, C, D, E, F, G, H, I, K, M, N, P, Q, R, S, T, V, W, or Y; and/or
S18A, C, D, E, F, H, I, K, L, M, N, P, Q, R, T, W, or Y; and/or
K22A, C, D, E, F, G, H, I, L, M, N, P, Q, R, S, T, V, W, or Y; and/or
M23A, C, D, E, F, G, H, I, K, L, N, P, Q, R, S, T, V, W, or Y; and/or
Y30A, C, D, E, G, H, I, K, L, M, N, P, Q, R, S, T, V, or W; and/or
G40A, C, D, E, F, H, I, K, L, M, N, P, Q, R, S, T, V, W, or Y; and/or
N80A, C, D, E, F, G, H, I, K, L, M, P, Q, R, S, T, V, W, or Y; and/or
P81A, C, D, E, F, G, H, I, K, L, M, N, Q, R, S, T, V, W, or Y; and/or
K82A, C, D, E, F, G, H, I, L, M, N, P, Q, R, S, T, V, W, or Y; and/or
N87A, C, D, E, F, G, H, I, K, L, M, P, Q, R, S, T, V, W, or Y; and/or
N88A, C, D, E, F, G, H, I, K, L, M, P, Q, R, S, T, V, W, or Y; and/or
W111A, C, D, E, F, G, H, I, K, L, M, N, P, Q, R, S, T, V, W or Y; and/or
V112A, C, D, E, F, G, H, I, K, L, M, N, P, Q, R, S, T, W, or Y; and/or
A114C, D, E, F, G, H, I, K, L, M, N, P, Q, R, S, T, V, W, or Y; and/or
Y117A, C, D, E, F, G, H, I, K, L, M, N, P, Q, R, S, T, V, or W; and/or
L118A, C, D, E, F, G, H, I, K, M, N, P, Q, R, S, T, V, W, or Y; and/or
P156A, C, D, E, F, G, H, I, K, L, M, N, Q, R, S, T, V, W, or Y; and/or
D157A, C, E, F, G, H, I, K, L, M, P, Q, R, S, T, V, W, or Y; and/or
G159A, C, D, E, F, H, I, K, L, M, N, P, Q, R, S, T, V, W, or Y; and/or
Q160A, C, D, E, F, G, H, I, K, L, M, N, P, R, S, T, V, W, or Y; and/or
N161A, C, D, E, F, G, H, I, K, L, M P, Q, R, S, T, V, W, or Y; and/or
P162A, C, D, E, F, G, H, I, K, L, M, N, Q, R, S, T, V, W, or Y; and/or
S163A, C, D, E, F, G, H, I, K, L, M, N, P, Q, R, T, V, W, or Y; and/or
A164C, D, E, F, G, H, I, K, L, M, N, P, Q, R, S, T, V, W, or Y; and/or
R165A, C, D, E, F, G, H, I, K, L, M, N, P, Q, S, T, V, W, or Y; and/or
S166A, C, D, E, F, G, H, I, K, L, M, N, P, Q, R, T, V, W, or Y; and/or
Q167A, C, D, E, F, G, H, I, K, L, M, N, P, R, S, T, V, W, or Y; and/or
K168A, C, D, E, F, G, H, I, L, M, N, P, Q, R, S, T, V, W, or Y; and/or
V169A, C, D, E, F, G, H, I, K, L, M, N, P, Q, R, S, T, W, or Y; and/or
V170A, C, D, E, F, G, H, I, K, L, M, N, P, Q, R, S, T, W, or Y; and/or
E171A, C, D, F, G, H, I, K, L, M, N, P, Q, R, S, T, V, W, or Y; and/or
A172C, D, E, F, G, H, I, K, L, M, N, P, Q, R, S, T, V, W, or Y; and/or
Y179A, C, D, E, F, G, H, I, K, L, M, N, P, Q, R, S, T, V, or W; and/or
H180A, C, D, E, F, G, I, K, L, M, P, Q, R, S, T, V, W, or Y; and/or
N181A, C, D, E, F, G, H, I, K, L, M, P, Q, R, S, T, V, W, or Y; and/or
Q182A, C, D, E, F, G, H, I, K, L, M, N, P, R, S, T, V, W, or Y, preferably K; and/or
M209A, C, D, E, F, G, H, I, K, L, N, P, Q, R, S, T, V, W, or Y; and/or
L210 A, C, D, E, F, G, H, I, K, M, N, P, Q, R, S, T, V, W, or Y; and/or

R211 A, C, D, E, F, G, H, I, K, L, M, N, P, Q, R, S, T, V, W, or Y; and/or
N215 A, C, D, E, F, G, H, I, K, L, M, N, P, Q, R, S, T, V, W, or Y; and/or
Y226A, C, D, E, G, H, I, K, L, M, N, P, Q, R, S, T, V, or W; and/or
Y230A, C, D, E, G, H, I, K, L, M, N, P, Q, R, S, T, V or W; and/or
K284A, C, D, E, F, G, H, I, L, M, N, P, Q, R, S, T, V, W, or Y; and/or
M285A, C, D, E, F, G, H, I, K, L, N, P, Q, R, S, T, V, W, or Y; and/or
Q289A, C, D, E, F, G, H, I, K, L, M, N, P, R, S, T, V, W, or Y; and/or
V290A, C, D, E, F, G, H, I, K, L, M, N, P, Q, R, S, T, W, or Y; and/or
E309A, C, D, F, G, H, I, K, L, M, N, P, Q, R, S, T, V, W, or Y; and/or
S310A, C, D, E, F, G, H, I, K, L, M, N, P, Q, R, T, V, W, or Y.

In addition or alternatively thereto there may be one or more C-terminal extensions. Preferably the additional C-terminal extension is comprised of one or more aliphatic amino acids, preferably a non-polar amino acid, more preferably of I, L, V or G. Thus, the present invention further provides for a variant enzyme comprising one or more of the following C-terminal extensions: 318I, 318L, 318V, 318G.

When it is the case that the residues in the parent backbone differ from those in P10480 (SEQ ID No. 2), as determined by homology alignment and/or structural alignment to P10480 and/or 1IVN, it may be desirable to replace the residues which align to any one or more of the following amino acid residues in P10480 (SEQ ID No. 2): Ser3, Leu17, Lys22, Met23, Gly40, Asn80, Pro81, Lys82, Asn 87, Asn88, Trp111, Val112, Ala114, Tyr117, Leu118, Pro156, Gly159, Gln160, Asn161, Pro162, Ser163, Ala164, Arg165, Ser166, Gln167, Lys168, Val169, Val170, Glu171, Ala172, Tyr179, His180, Asn181, Gln182, Met209, Leu210, Arg211, Asn215, Lys284, Met285, Gln289, Val290, Glu309 or Ser310, with the residue found in P10480 respectively.

The following wildtype residues of P10480 have been found to be preferable for retaining good activity, particularly good transferase activity from a galactolipid:, L17, W111, R221, S3, G40, N88, K22, Y117, L118, N181, M209, M285, E309, M23. Thus preferably the variant enzyme comprises the amino acid residue found in P10480 at any one or more of these sites.

Variant enzymes which have an increased hydrolytic activity against a polar lipid may also have an increased transferase activity from a polar lipid.

Variant enzymes which have an increased hydrolytic activity against a phospholipid, such as phosphatidylcholine (PC) may also have an increased transferase activity from a phospholipid.

Variant enzymes which have an increased hydrolytic activity against a galactolipid, such as DGDG, may also have an increased transferase activity from a galactolipid.

Variants enzymes which have an increased transferase activity from a phospholipid, such as phosphatidylcholine (PC), may also have an increased hydrolytic activity against a phospholipid.

Variants enzymes which have an increased transferase activity from a galactolipid, such as DGDG, may also have an increased hydrolytic activity against a galactolipid.

Variants enzymes which have an increased transferase activity from a polar lipid may also have an increased hydrolytic activity against a polar lipid.

Suitably, one or more of the following sites may be involved in substrate binding: Leu17; Ala114; Tyr179; His180; Asn181; Met209; Leu210; Arg211; Asn215; Lys284; Met285; Gln289; Val290.

The variant enzyme in accordance with the present description may have one or more of the following functionalities compared with the parent enzyme:

1) an increased relative transferase activity against galactolipid (DG) compared to PC calculated as % $T_{DG}/T_{PC}$ (as illustrated in Example 8)
2) an increased absolute transferase activity against galactolipid (DG) (as illustrated in Example 8)
3) an increased transferase activity using galactolipid as donor ($T_{DG}$) relative to the hydrolytic activity $H_{DG}$ on galactolipid (DO) (as illustrated in Example 8)
4) an increased absolute transferase activity against PC (as illustrated in Example 8)

Wherein DG is galactolipid (e.g. DGDG) (and may be herein also referred to as GL) and PC is phospholipid (e.g. lecithin). Variants with an increased activity towards galactolipid include variants within categories 1), 2) and 3) above. Variants with an increased activity on galactolipids may also have an increased activity in phospholipids (as per category 4) above).

**1. A modification to one or more of the following residues may result in a variant enzyme having an increased relative transferase activity against DG compared to PC calculated as % $T_{DG}/T_{PC}$:**

-318, N215, L210, S310, E309, H180, N80, V112, Y30X (where X is selected from A, C, D, E, G, H, I, K, L, M, N, P, Q, R, S, T, V, or W), V290, Q289, K22, G40, Y179, M209, R211, K22, P81, N87, Y117, N181, Y230X (where X is selected from A, C, D, E, G, H, I, K, L, M, N, P, Q, R, S, T, V, or W), Q182.

Typically, one or more of the following substitutions may be preferred:

S3A, C, D, E, F, G, H, I, K, L, M, N, P, Q, R, T, V, W or Y, preferably M, R, N, G, T, Q, P, Y, S, L, E, W, most preferably Q
K22A, E, C, F, G, H, I, L, M, N, P, Q, R, S, T, V, W or Y, preferably A, C, E or R
Y30A, C, D, H, K, M, N, P, Q, R, T, V, W, G, I, L, S, M, A, R or E, preferably H, T, W, N, D, C, Q G, I, L, S, M, A, R or E
G40 L, N, T, V or A
N80N, R, D, A, C, E, F, G, H, I, K, L, M, P, Q, S, T, V, W or Y, preferably H, I, Y, C, Q, M, S, W, L, N, R, D or F
P81A, C, D, E, F, G H, I, K, L, M, N, Q, R, S, T, V, W or Y, preferably I, M, F, G, V, Y, D, C or A
K82A, C, D, E, F, G, H, I, L, M, N, P, Q, R, S, T, V, W or Y, preferably H, K, S or R
N87A, C, D, E, F, G, H, I, K, L, M, P, Q, R, S, T, V, W or Y, preferably I, Y, M, T, Q, S, W, F, V or P
N88A, C, D, E, F, G, H, I, K, L, M, P, Q, R, S, T, V, W or Y, preferably C, V, A or F V112C
Y117A, C, D, E, F, H, T, G, I, K, L, M, N, P, Q, R, S, V or W, preferably A, N, E, H, T, I, F, C, P or S
L118A, C, D, E, F, G, H, I, K, M, N, P, Q, R, S, T, V, W, or Y, preferably F
V112A, D, E, F, G, H, I, K, L, M, N, P, Q, R, S, T, W or Y, preferably I, M, F, Y, N, E, T, Q, H or P
Y179A, C, D, E, F, G, H, I, K, L, M, N, P, Q, R, S, T, V or W, preferably F, C, H, I, L, M, P, V or W
H180K, Q, A, C, D, E, F, G, I, L, M, P, R, S, T, V, W, or Y, preferably M, F, C, K or Q
N181A or V
Q182A, C, D, E, F, G, H, I, K, M, N, P, Q, R, S, T, V, W, or Y, preferably K
M209L, K, M, A, C, D, E, F, G, H, I, N, P, Q, R, S, T, V, W, or Y, preferably I, F, T, D, C, H, L, K, M or P
L210 G, I, H, E, M, S, W, V, A, R, N, D, Q ,T, C, F, K, P or Y, preferably G, I, H, E, M, S, W, V, A, R, N, D, Q,T, Y or F
R211G, Q, K, D, A, C, E, F, H, I, L, M, N, P, R, S, T, V, W or Y, preferably G, Q, K, D, H, I, M, F, P, S, Y, N, C, L or W
N215A, C, D, E, F, G, H, I, K, L, M, N, P, Q, R, S, T, V, W or Y, preferably I, F, P, T, W,HorA
Y230A, C, D, E, G, H, I, K, L, M, N, P, Q, R, S, T, V or W, preferably I, T, G, D, R, E, V, M or S, most preferably I, D, R or E
Q289A, C, D, E, F, G, H, I, K, L, M, N, P, R, S, T, V, W or Y, preferably F, W, H, I, Y, L, D, C, K, V, E, G. R, N or P, more preferably R, T, D, K, N or P
V290A, C, D, E, H, F, G, I, K, L, M, N, P, Q, R, S, T, W or Y;
E309S, Q, R, A, C, D, F, G, H, I, K, L, M, N, P, T, V, W or Y, preferably F, W, N, H, I, M, S, Q, R, A or Y
S310A, P, T, H, M, K, G, C, D, E, F, I, L, N, Q, R,V, W or Y, preferably F, Y, C, L, K, A, P, T, H, M, K or G
-318 A, C, D, E, F, G, I, K, L, M, N, P, Q, R, T, V, W, Y, H or S

Preferably, one or more of the following modifications may result in a variant enzyme having an increased relative transferase activity against DG compared to PC calculated as % $T_{DG}/T_{PC}$
S3A, C, D, E, F, G, H, I, K, L, M, N, P, Q, R, T, V, W or Y, preferably M, R, N, G, T, Q, P, Y, S, L, E, W, most preferably Q
G40 L, N, T, V or A
K82A, C, D, E, F, G, H, I, L, M, N, P, Q, R, S, T, V, W or Y, preferably H, K, S or R
N88A, C, D, E, F, G, H, I, K, L, M, P, Q, R, S, T, V, W or Y, preferably C, V, A or F
Y230A, C, D, E, G, H, I, K, L, M, N, P, Q, R, S, T, V or W, preferably I, T, G, D, R, E, V, M or S, most preferably D, R or E
Q289A, C, D, E, F, G, H, I, K, L, M, N, P, R, S, T, V, W or Y, preferably F, W, H, I, Y, L, D, C, K, V, E, G or P, more preferably R, T, D, K or P

Modification of one or more of the following modifications results in a variant enzyme having an increased relative transferase activity against DG compared to PC calculated as % $T_{DG}/T_{PC}$:

-318Y,HorS
N215H
L210G, I, H, E, M, S, W, V, A, R, N, D, Q or T
S310A, P, T, H, M, K or G
E309S, Q, A or R
H180K, T or Q
N80N, R or D
V112C
Y30G, I, L, S, M, A, R or E , more preferably Y30M, A or R
V290R, E, H or A

Q289R, T, D or N
K22E
G40L
Y179V or R
M209L, K or M
R211G, Q, K or D
Y230V
G40Q, L or V
N88W
N87R or D

For some embodiments the following substitutions may also be suitable:

K22A or C

P81G

N87 M

Y117A,N,E,HorT

N181A or V

Y230I

V290H

N87R, D, E or M

Q182T

Preferably, the residues modified in order to increase the ratio of galactolipid transferase compared to phospholipid transferase activity are one or more of the following:: -318, N215, L210, E309, H180, N80.
Typically, one or more of the following substitutions are preferred:

-318 Y, H or S, most preferably Y
N215H
L210D, Q or T
E309Q or R
H180K or Q
N80N, R or D

**2. A modification to one or more of the following residues may result in a variant having an increased absolute transferase activity against DG:**

-318, N215, L210, S310, E309, H180, N80, V112, Y30X (where X is selected from A, C, D, E, G, H, I, K, L, M, N, P, Q, R, S, T, V, or W), V290, Q 289, K22, G40, Y179, M209, R211, K22, P81, N87, Y117, N181, Y230X (where X is selected from A, C, D, E, G, H, I, K, L, M, N, P, Q, R, S, T, V, or W), V290, N87, Q182, S3, S310, K82, A309.
In particular, one or more of the following modifications may result in a variant having an increased absolute transferase activity against DG:

-318Y, H, S, A, C, D, E, F, G, I, K, L, M, N, P, Q, R, T, V or W, preferably Y, H, S or I
N215A, C, D, E, F, G, H, I, K, L, M, N, P, Q, R, S, T, V, W or Y; preferably H, I, F, P, T, W or A, most preferably H, S, L, R, Y
L210G, I, H, E, M, S, W, V, A, R, N, D, Q, T, C, F, K, P or Y, preferably D, Q, T, Y or F
S310A, P, T, H, M, K, G, C, D, E, F, I, L, N, Q, R,V, W or Y. preferably F, Y, C, L, K or P,
E309S, Q, R, A, C, D, F, G, H, I, K, L, M, N, P, S, T, V, W or Y; preferably S, Q, R, F, W, N, H, I, M or Y, most preferably S, Q, R, N, P or A
H180A, C, D, E, F, G, I, K, Q L, M, P, R, S, T, V, W or Y; preferably K, Q, M, F or C, most preferably T, K or Q

N181A or V
N80N, R, D, A, C, E, F, G, H, I, K, L, M, P, Q, S, T, V, W or Y, preferably H, I, Y, C, Q, M, S, W, L, N, R, D or F, most preferably N, R, D, P, V, A or G
V112A, C, D, E, F, G, H, I, K, L, M, N, P, Q, R, S, T, W or Y; preferably I, M, F, Y, N, E, T, Q, H or P
Y30G, I, L, S, A, E, C, D, H, K, M, N, P, Q, R, T, V or W, preferably H, T, W, N, D, C or Q
V290A, C, D, E, F, G, H, I, K, L, M, N, P, Q, R, S, T, W or Y;
Q289A, C, D, E, F, G, H, I, K, L, M, N, P, R, S, T, V, W or Y; preferably R, E, G, P, NorR
K22A, C, E, F, G, H, I, L, M, N, P, Q, R, S, T, V, W or Y, preferably C
Y179A, C, D, E, F, G, H, I, K, L, M, N, P, Q, R, S, T, V or W; preferably F, C, H, I, L, M, P or W, more preferably E, R, N, V, K, S
M209A, C, D, E, F, G, H, I, L, K, M, N, P, Q, R, S, T, V, W or Y; preferably R, N, Y, EorV
R211A, C, E, F, G, H, I, L, M, N, P, Q, K, D, R, S, T, V, W or Y, preferably H, I, M, F, P, S, Y, N, C, L or W, most preferably R
S310 C, D, E, F, I, L, N, Q, R,V, W or Y. preferably F, Y, C, L, K or P
S3A, C, D, E, F, G, H, I, K, L, M, N, P, Q, R, T, V, W or Y, preferably M, R, N, A, G, T, Q, P, Y or S most preferably Q or N
K82A, C, D, E, F, G, H, I, L, M, N, P, Q, R, S, T, V, W or Y, preferably H, K, S, E or R
P81A, C, D, E, F, G,H, I, K, L, M, N, Q, R, S, T, V, W or Y; preferably I, M, F, V, Y, D, C or A
N87A, C, F, G, H, I, K, L, M, P, Q, R, D, E, S, T, V, W or Y; preferably L, G or A
Y117A, N, E, H, T, C, D, F, G, I, K, L, M, P, Q, R, S, V or W; preferably I, F, C, P or S
N87A, C, F, G, H, I, K, L, P, Q, S, T, V, W or Y; preferably I, Y, T, Q, S, W, F, V or P
Q182A, C, D, E, F, G, H, I, K, L, M, N, P, R, S, T, V, W or Y, preferably D or K
Y230A, C, D, E, G, H, I, K, L, M, N, P, Q, R, S, T, V or W, preferably W, H, Q, L, P or C, most preferable T or G
D157A, C, E, F, G, H, I, K, L, M, P, Q, R, S, T, V, W or Y, preferably C
G40L
Y226I

Typically, one or more of the following substitutions are preferred:

-318Y,HorS
N215H
L210G, I, H, E, M, S, W, V, A, R, N, D, Q or T
S310A, P, T, H, M, K or G
E309S, Q or R
H180K or Q
N80N, R or D
V112C
Y30G, I, L, S, M, A, R or E, more preferably Y30M, A or R
V290R, E, H or A
Q289R or N
K22E
G40L
Y179V
M209L, K or M
L211G, Q, K or D

The following substitutions may also be suitable:

K22A or C
P81G
N87M
Y117A, N, E, H or T
N181A or V
Y230 I
V290H
N87R, D, E or M
Q182T

Preferably, the residues modified in order to increase transferase activity from a galactolipid substrate (DGDG) are one or more of the following:: -318, N215, L210, E309, H180, N80.

Typically, one or more of the following substitutions are preferred:

-318 Y, H or S, most preferably Y
N215H
L210D, Q or T
E309Q or R
H180K or Q
N80N, R or D

**3. A modificiation at one or more of the following residues may result in a variant enzyme having an increased transferase activity $T_{DG}$ relative to the hydrolytic activity $H_{DG}$ on DG:**

Y230, S310, H180, Q289, G40, N88, Y179, N215, L210, N80, Y30X (where X is selected from A, C, D, E, G, H, I, K, L, M, N, P, Q, R, S, T, V, or W), N87, M209, R211, S18X (where X is specifically selected from A, C, D, E, F, H, I, K, L, M, N, P, Q, R, T, W or Y).

Preferably, one or more of the following modifications may result in a variant enzyme having an increased transferase activity $T_{DG}$ relative to the hydrolytic activity $H_{DG}$ on DG:

Y230A, C, D, E, G, H, I, K, L, M, N, P, Q, R, S, T or W, preferably W, H, Q, L, P or C
S310A, C, D, E, F, G, H, I, K, L, M, N, Q, R, T, V, W or Y, preferably F, Y, C, L, K or P
Y179A, C, D, E, F, G, H, I, K, L, M, N, P, Q, S, T, V, or W, preferably F, C, H, I, L, M, P or W
H180A, C, D, E, F, G, I, K, L, M, P, Q, R, S, V, W or Y, preferably M, F or C
Q289A, C, E, F, G, H, I, K, L, M, N, P, R, S, V, W or Y; preferably F, W, H, I, Y, L, D, C, K, V, E, G or P
G40A, C, D, E, F, H, I, K, M, N, P, R, S, T, W or Y; preferably I, P, W or Y
N88A, C, D, E, F, G, H, I, K, L, M, P, Q, R, S, T, V or Y; preferably I or H
N87A, C, E, F, G, H, I, K, L, M, P, Q, S, T, V, W or Y; preferably I, Y, T, Q, S, W, F, VorP

Typically, one or more of the following substitutions are preferred (such variant enzymes may have a decreased hydrolytic activity (galactolipid and/or phospholipids) and/or an increased tranferase activity from galactolipid:

Y179E,R,NorQ
N215G
L210D, H, R, E, A, Q, P, N, K, G, R, T, W, I, V or S
N80G
Y30L
N87G

Typically, one or more of the following substitutions are preferred (such variant enzymes may have a decreased hydrolytic activity (galactolipid and/or phospholipids) whilst retaining significant tranferase activity from galactolipid:

Y179 E, R, N, Q
N215 G
L210 D, H, R, E, A, Q, P, N, K, G, R, T, W, I, V and S
N80 G
Y30 L
N87 G
H180 I, T
M209 Y
R211 D, T and G
S18G,MandT
G40 R and M
N88 W
N87 C, D, R, E and G

**4. Modification of one or more of the following residues may result in a variant enzyme having an increased absolute transferase activity against phospholipid:**

S3, D157, S310, E309, Y179, N215, K22, Q289, M23, H180, M209, L210, R211, P81, V112, N80, L82, N88; N87

Specific modifications which may provide a variant enzyme having an improved transferase activity from a phospholipid may be selected from one or more of the following:

S3A, C, D, E, F, G, H, I, K, L, M, N, P, Q, R, T, V, W or Y; preferably N, E, K, R, A, P or M, most preferably S3A
D157A, C, E, F, G, H, I, K, L, M, N, P, Q, R, S, T, V, W or Y ; preferably D157S, R, E, N, G, T, V, Q, K or C
S310A, C, D, E, F, G, H, I, K, L, M, N, P, Q, R, T, V, W or Y; preferably S310T -318 E
E309A, C, D, E, F, G, H, I, K, L, M, N, P, Q, R, T, V, W or Y; preferably E309 R, E, L, R or A
Y179A, C, D, E, F, G, H, I, K, L, M, N, P, Q, R, S, T, V or W; preferably Y179 D, T, E, R, N, V, K, Q or S, more preferably E, R, N, V, K or Q
N215A, C, D, E, F, G, H, I, K, L, M, P, Q, R, S, T, V, W or Y; preferably N215 S, L, RorY
K22A, C, D, E, F, G, H, I, L, M, N, P, Q, R, S, T, V, W or Y; preferably K22 E, R, C or A
Q289A, C, D, E, F, G, H, I, K, L, M, N, P, R, S, T, V, W or Y; preferably Q289 R, E, G, P or N
M23A, C, D, E, F, G, H, I, K, L N, P, Q, R, S, T, V, W or Y; preferably M23 K, Q, L, G, T or S
H180A, C, D, E, F, G, I, K, L, M, P, Q, R, S, T, V, W or Y; preferably H180 Q, R or K
M209 A, C, D, E, F, G, H, I, K, L, N, P, Q, R, S, T, V, W or Y; preferably M209 Q, S, R, A, N, Y, E, V or L
L210A, C, D, E, F, G, H, I, K, M, N, P, Q, R, S, T, V, W or Y; preferably L210 R, A, V,S,T,I,WorM
R211 A, C, D, E, F, G, H, I, K, L, M, N, P, Q, S, T, V, W or Y; preferably R211T
P81A, C, D, E, F, G, H, I, K, L, M, N, Q, R, S, T, V, W or Y; preferably P81G
V112A, C, D, E, F, G, H, I, K, L, M, N, P, Q, R, S, T, W or Y; preferably V112C
N80A, C, D, E, F, G, H, I, K, L, M, P, Q, R, S, T, V, W or Y; preferably N80 R, G, N, D, P, T, E, V, A or G
L82A, C, D, E, F, G, H, I, M, N, P, Q, R, S, T, V, W or Y; preferably L82N, S or E
N88A, C, D, E, F, G, H, I, K, L, M, P, Q, R, S, T, V, W or Y; preferably N88C
N87A, C, D, E, F, G, H, I, K, L, M, P, Q, R, S, T, V, W or Y; preferably N87M or G

Modification of one or more of the following residues results in a variant enzyme having an increased absolute transferase activity against phospholipid:

S3 N, R, A, G
M23 K, Q, L , G, T, S
H180 R
L82 G
Y179 E, R, N, V, K or Q
E309 R, S, L or A

**5. Residues the modification of which results in an increased transferase activity from a galactolipid substrate (DGDG) and an increase in ratio of galactolipid transferase compared to phospholipid transferase activity** include one or more of: - 318, N215, L210, S310, E309, H180, N80, V112, Y30X (where X is selected from A, C, D, E, G, H, I, K, L, M, N, P, Q, R, S, T, V, or W), V290, Q 289, K22, G40, Y179, M209, R211, K22, P81, N87, Y117, N181, Y230X (where X is selected from A, C, D, E, G, H, I, K, L, M, N, P, Q, R, S, T, V, or W), Q182.

Typically, one or more of the following substitutions are preferred:

-318Y,HorS
N215H
L210G, I, H, E, M, S, W, V, A, R, N, D, Q or T
S310A, P, T, H, M, K or G
E309S, A, Q or R
H180K or Q
N80N, R or D
V112C
Y30G, I, L, S, M, A, R or E, more preferably Y30M, A or R
V290R, E, H or A
Q289R or N
K22E
G40L
Y179V
M209L, K or M
L211G, Q, K or D

The following substitutions may also be suitable:

K22A or C

P81G

N87M

Y117A, N, E, H or T

N181A or V

Y230I

V290H

N87R, D, E or M

Q182T

Preferably, the residues modified in order to increase transferase activity from a galactolipid substrate (DGDG) and/or increase the ratio of galactolipid transferase compared to phospholipid transferase activity are one or more of the following:: -318, N215, L210, E309, H180, N80.

Typically, one or more of the following substitutions are preferred:

-318 Y, H or S, most preferably Y
N215H
L210D, Q or T
E309Q or R
H180K or Q
N80N, R or D

**6. The following wildtype residues of P10480 have been found to be preferable for retaining good activity, particularly good transferase activity from a galactolipid:**

W111, R211, N181, S3, L17, G40, N88, Y117, L118, N181, K22, M209, M285, M23

Preferably, these residues are retained in the variant enzyme.

When making variant GDSx acyl-transferases for increased activity transferase from galactolipid substrates, where the parent enzyme has a residue corresponding to residues of the P10480 sequence at positions W111, R211, N181, S3, L17, G40, N88, Y117, L118, N181, K22, M209, M285, M23 other than the residue found in P10480 the variant may preferably contain a substitution at the corresponding position to include the amino acid residue found in the P10480 sequence.

L17 is preferably a hydrophobic amino acid residue

**7. The following combinations may have an increased transferase activity from a galactolipid substrate (DGDG) and/or an increase in ratio of galactolipid transferase activity compared to phospholipid transferase activity**

N215H & -318Y
N215H & L210D, Q, or T
-318Y & L210,D, Q, or T
N215H & -318Y & L210D, Q, or T

The above combinations may optionally also include a C-terminal amino acid addition, such as -318Y, H or S, or preferably -318Y.

The above combinations may optionally also include the following modification:

Suitably one or more of the following combinations may have an increased transferase activity from a galactolipid substrate (DGDG) and/or an increase in ratio of galactolipid transferase activity compared to phospholipid transferase activity:

E309A, Q or R.
N215H & -318Y, H, or S, preferably Y.
L210D, Q or T & -318Y, H, or S, preferably Y.
N215H & E309A, Q or R
L210D, Q or T & E309A, Q or R
-318Y & E309A, Q or R

The above combinations may optionally also include a substitution at position Q182, preferably Q182K.

The following combinations may have an increased transferase activity from a galactolipid substrate (DGDG) and/or an increase in ratio of galactolipid transferase activity compared to phospholipid transferase activity, and/or an increase in ratio of galactolipid transferase compared to hydrolytic activity:

N215H & N80G
-318Y & N80G
L210D or Q & N80G
N215H & N88N
-318Y & N88N
L210D or Q & N88N
N215H & Y30L
-318Y & Y30L
L210D or Q & Y30L
N215H & N87G
-318Y & N87G
L210D or Q & N87G
N215H & Y179E, R, N or Q
-318Y & Y179 E, R, N or Q
L210D or Q & Y179 E, R, N or Q

As noted above, when referring to specific amino acid residues herein the numbering is that obtained from alignment of the variant sequence with the reference sequence shown as SEQ ID No. 2.

For the avoidance of doubt, when a particular amino acid is taught at a specific site, for instance L118 for instance, this refers to the specifc amino acid at residue number 118 in SEQ ID No. 2. However, the amino acid residue at site 118 in a different parent enzyme may be different from leucine.

Thus, when taught to substitute an amino acid at residue 118, although reference may be made to L118 it would be readily understood by the skilled person that when the parent enzyme is other than that shown in SEQ ID No. 2, the amino acid being substituted may not be leucine. It is, therefore, possible that when substituting an amino acid sequence in a parent enzyme which is not the enzyme having the amino acid sequence shown as SEQ ID No. 2, the new (substituting) amino acid may be the same as that taught in SEQ ID No. 2. This may be the case, for instance, where the amino acid at say residue 118 is not leucine and is, therefore different from the amino acid at residue 118 in SEQ ID No. 2. In other words, at residue 118 for example, if the parent enzyme has at that position an amino acid other than leucine, this amino acid may be substituted with leucine

The term "lipid acyltransferase" as used herein means an enzyme which has acyltransferase activity (generally classified as E.C. 2.3.1.x in accordance with the Enzyme Nomenclature Recommendations (1992) of the Nomenclature Committee of the International Union of Biochemistry and Molecular Biology), whereby the enzyme is capable of transferring an acyl group from a lipid to one or more acceptor substrates, such as one or more of the following: a sterol; a stanol; a carbohydrate; a protein; a protein subunit; glycerol.

Preferably the lipid acyltransferase is capable of transferring an acyl group from a lipid to at least a sterol and/or a stanol, for example to cholesterol.

Preferably, the lipid acyltransferase variant described herein and/or for use in the methods and/or uses described herein is capable of transferring an acyl group from a lipid (as defined herein) to one or more of the following acyl acceptor substrates: a sterol, a stanol, a carbohydrate, a protein or subunits thereof, or a glycerol.

For some aspects the "acyl acceptor" described herein may be any compound comprising a hydroxy group (-OH), such as for example, polyvalent alcohols, including glycerol; sterol; stanols; carbohydrates; hydroxy acids including fruit acids, citric acid, tartaric acid, lactic acid and ascorbic acid; proteins or a sub-unit thereof, such as amino acids, protein hydrolysates and peptides (partly hydrolysed protein) for example; and mixtures and derivatives thereof. Preferably, the "acyl acceptor" described herein is not water. The acyl acceptor is preferably also not a monoglyceride and/or a diglyceride. In one aspect, preferably the variant enzyme is capable of transferring an acyl group from a lipid to a sterol and/or a stanol.

In one aspect, preferably the variant enzyme is capable of transferring an acyl group from a lipid to a carbohydrate.

In one aspect, preferably the variant enzyme is capable of transferring an acyl group from a lipid to a protein or a subunit thereof. Suitably the protein subunit may be one or more of the following: an amino acid, a protein hydrolysate, a peptide, a dipeptide, an oligopeptide, a polypeptide.

Suitably in the protein or protein subunit the acyl acceptor may be one or more of the following constituents of the protein or protein subunit: a serine, a threonine, a tyrosine, or a cysteine.

When the protein subunit is an amino acid, suitably the amino acid may be any suitable amino acid. Suitably the amino acid may be one or more of a serine, a threonine, a tyrosine, or a cysteine for example.

In one aspect, preferably the variant enzyme is capable of transferring an acyl group from a lipid to glycerol.

In one aspect, preferably the variant enzyme is capable of transferring an acyl group from a lipid to a hydroxy acid.

In one aspect, preferably the variant enzyme is capable of transferring an acyl group from a lipid to a polyvalent alcohol.

In one aspect, the variant lipid acyltransferase may, as well as being able to transfer an acyl group from a lipid to a sterol and/or a stanol, additionally be able to transfer the acyl group from a lipid to one or more of the following: a carbohydrate, a protein, a protein subunit, glycerol.

Preferably, the lipid substrate upon which the variant lipid acyltransferase described herein acts is one or more of the following lipids: a phospholipid, such as a lecithin, e.g. phosphatidylcholine, a triacylglyceride, a cardiolipin, a diglyceride, or a glycolipid, such as digalactosyldiglyceride (DGDG) or monogalactosyldiglyceride (MGDG) for example. More preferably, the variant enzyme described herein acts on one or both of DGDG and MGDG. Preferably, the variant enzyme described herein has no (or has only limited) activity on digalactosylmonoglyceride (DGMG) and monogalactosylmonoglyceride (MGMG). Thus preferably the lipid substrate is not one or both of DGMG or MGMG. This lipid substrate may be referred to herein as the "lipid acyl donor". The term lecithin as used herein encompasses phosphatidylcholine, phosphatidylethanolamine, phosphatidylinositol, phosphatidylserine and phosphatidylglycerol.

The term "galactolipid" as used herein means one or more of DGDG or DGMG.

The term "phospholipid" as used herein means lecithin, including phosphatidylcholine.

The term "polar lipid" as used herein means a phospholipids and/or a galactolipid, preferably a phospholipids and a galactolipid.

For some aspects, preferably the lipid substrate upon which the variant lipid acyltransferase acts is a phospholipid, such as lecithin, for example phosphatidylcholine.

For some aspects, preferably the lipid substrate is a glycolipid, such as DGDG or MGDG for example.

Preferably the lipid substrate is a food lipid, that is to say a lipid component of a foodstuff.

For some aspects, preferably the variant lipid acyltransferase described herein invention is incapable, or substantially incapable, of acting on a triglyceride and/or a 1-monoglyceride and/or 2-monoglyceride.

Suitably, the lipid substrate or lipid acyl donor may be one or more lipids present in one or more of the following substrates: fats, including lard, tallow and butter fat; oils including oils extracted from or derived from palm oil, sunflower oil, soya bean oil, safflower oil, cotton seed oil, ground nut oil, corn oil, olive oil, peanut oil, coconut oil, and rape seed oil. Lecithin from soya, rape seed or egg yolk is also a suitable lipid substrate. The lipid substrate may be an oat lipid or other plant based material containing galactolipids.

In one aspect the lipid acyl donor is preferably lecithin (such as phosphatidylcholine) in egg yolk.

The lipid may be selected from lipids having a fatty acid chain length of from 8 to 22 carbons.

**[0209]** - The lipid may be selected from lipids having a fatty acid chain length of from 16 to 22 carbons, more preferably of from 16 to 20 carbons.

The lipid may be selected from lipids having a fatty acid chain length of no greater than 14 carbons, suitably from lipids having a fatty acid chain length of from 4 to 14 carbons, suitably 4 to 10 carbons, suitably 4 to 8 carbons.

Suitably, the variant lipid acyltransferase described herein may exhibit one or more of the following lipase activities: glycolipase activity (E.C. 3.1.1.26), triacylglycerol lipase activity (E.C. 3.1.1.3), phospholipase A2 activity (E.C. 3.1.1.4) or phospholipase A1 activity (E.C. 3.1.1.32). The term "glycolipase activity" as used herein encompasses "galactolipase activity".

Suitably, the variant lipid acyltransferase described herein may have at least one or more of the following activities: glycolipase activity (E.C. 3.1.1.26) and/or phospholipase A1 activity (E.C. 3.1.1.32) and/or phospholipase A2 activity (E.C. 3.1.1.4).

For some aspects, the variant lipid acyltransferase described herein may have at least glycolipase activity (E.C. 3.1.1.26).

Suitably, for some aspects the variant lipid acyltransferase described herein may be capable of transferring an acyl group from a glycolipid and/or a phospholipid to one or more of the following acceptor substrates: a sterol, a stanol, a carbohydrate, a protein, glycerol.

For some aspects, preferably the variant lipid acyltransferase described herein is capable of transferring an acyl group from a glycolipid and/or a phospholipid to a sterol and/or a stanol to form at least a sterol ester and/or a stanol ester.

For some aspects, preferably the variant lipid acyltransferase described herein is capable of transferring an acyl group from a glycolipid and/or a phospholipid to a carbohydrate to form at least a carbohydrate ester.

For some aspects, preferably the variant lipid acyltransferase described herein is capable of transferring an acyl group from a glycolipid and/or a phospholipid to a protein to form at least protein ester (or a protein fatty acid condensate).

For some aspects, preferably the variant lipid acyltransferase described herein is capable of transferring an acyl group from a glycolipid and/or a phospholipid to glycerol to form at least a diglyceride and/or a monoglyceride.

For some aspects, preferably the variant lipid acyltransferase described herein does not exhibit triacylglycerol lipase activity (E.C. 3.1.1.3).

In some aspects, the variant lipid acyltransferase may be capable of transferring an acyl group from a lipid to a sterol and/or a stanol. Thus, in one embodiment the "acyl acceptor" described herein may be either a sterol or a stanol or a combination of both a sterol and a stanol.

Suitably the sterol and/or stanol may comprise one or more of the following structural features:

i) a 3-beta hydroxy group or a 3-alpha hydroxy group; and/or
ii) A:B rings in the *cis* position or A:B rings in the *trans* position or $C_5$-$C_6$ is unsaturated.

Suitable sterol acyl acceptors include cholesterol and phytosterols, for example alpha-sitosterol, beta-sitosterol, stigmasterol, ergosterol, campesterol, 5,6-dihydrosterol, brassicasterol, alpha-spinasterol, beta-spinasterol, gamma-spinasterol, deltaspinasterol, fucosterol, dimosterol, ascosterol, serebisterol, episterol, anasterol, hyposterol, chondrillasterol, desmosterol, chalinosterol, poriferasterol, clionasterol, sterol glycosides, and other natural or synthetic isomeric forms and derivatives.

Suitably more than one sterol and/or stanol may act as the acyl acceptor, suitably more than two sterols and/or stanols may act as the acyl acceptor. In other words, suitably more than one sterol ester and/or stanol ester may be produced. Suitably, when cholesterol is the acyl acceptor one or more further sterols or one or more stanols may also act as the acyl acceptor. Thus, described herein is a method for the *in situ* production of both a cholesterol ester and at least one sterol or stanol ester in combination. In other words, the lipid acyltransferase for some aspects of the present description may transfer an acyl group from a lipid to both cholesterol and at least one further sterol and/or at least one stanol.

In one aspect, preferably the sterol acyl acceptor is one or more of the following: alpha-sitosterol, beta-sitosterol, stigmasterol, ergosterol and campesterol.

In one aspect, preferably the sterol acyl acceptor is cholesterol. When it is the case that cholesterol is the acyl acceptor for the variant lipid acyltransferase, the amount of free cholesterol in the foodstuff is reduced as compared with the foodstuff prior to exposure to the variant lipid acyltransferase and/or as compared with an equivalent foodstuff which has not been treated with the variant lipid acyltransferase.

Suitable stanol acyl acceptors include phytostanols, for example beta-sitostanol or ss-sitostanol.

In one aspect, preferably the sterol and/or stanol acyl acceptor is a sterol and/or a stanol other than cholesterol.

In some aspects, the foodstuff prepared in accordance with the present description may be used to reduce blood serum cholesterol and/or to reduce low density lipoprotein. Blood serum cholesterol and low density lipoproteins have both been associated with certain diseases in humans, such as atherosclerosis and/or heart disease for example. Thus, it is envisaged that the foodstuffs prepared in accordance with the present description may be used to reduce the risk of such diseases.

Thus, described herein is the use of a foodstuff described herein for use in the treatment and/or prevention of atherosclerosis and/or heart disease. Thus is one aspect the foodstuff may be considered as a neutraceutical.

Further described herein is a medicament comprising a foodstuff described herein.

Described herein is a method of treating and/or preventing a disease in a human or animal patient which method comprises administering to the patient an effective amount of a foodstuff described herein.

Suitably, the sterol and/or the stanol "acyl acceptor" may be found naturally within the foodstuff. Alternatively, the sterol and/or the stanol may be added to the foodstuff. When it is the case that a sterol and/or a stanol is added to the foodstuff, the sterol and/or stanol may be added before, simultaneously with, and/or after the addition of the lipid acyltransferase described herein

Described herein is the addition of exogenous sterols/stanols, particularly phytosterols/phytostanols, to the foodstuff prior to or simultaneously with the addition of the variant enzyme described herein.

For some aspects, one or more sterols present in the foodstuff may be converted to one or more stanols prior to or at the same time as the variant lipid acyltransferase is added as described herein Any suitable method for converting sterols to stanols may be employed. For example, the conversion may be carried out by chemical hydrogenation for example. The conversion may be conducted prior to the addition of the variant lipid acyltransferase as described herein or simultaneously with the addition of the variant lipid acyltransferase as described herein Suitably enzymes for the conversion of sterol to stanols are taught in WO00/061771.

Suitably the present description may be employed to produce phytostanol esters *in situ* in a foodstuff. Phytostanol esters have increased solubility through lipid membranes, bioavailability and enhanced health benefits (see for example WO92/99640).

The stanol ester and/or the sterol ester may be a flavouring and/or a texturiser. In which instances, the present description encompasses the *in situ* production of flavourings and/or texturisers.

Described herein is a method of producing a plant sterol ester and/or stanol ester and lysolecithin in an edible oil (such as a plant oil, such as soya bean oil for instance) without the formation of free fatty acids by treatment of the oil with a variant enzyme according to the present invention. In such instances the lysolecithin so produced may be removed using a degumming process. Any degumming process may be used, such as one or more of the known degumming processes. Any free fatty acids can be removed by deodorizing if necessary. Notably, any stanol/sterol ester produced in the oil is not removed by the deodorizing process. Thus, the edible oil produced comprises sterol esters and/or stanol esters which may have beneficial nutritional and/or nutriceutical effects, such as lowering blood cholesterol levels.

Suitable oils in which this method could be carried out are those comprising *inter alia* lecithin and a sterol/stanol. Suitably, the oil is a crude oil when treated. Suitably, the edible oil may be one or more of the following: corn germ oil, cotton seed oil, linseed oil, palm oil, peanut oil, rapeseed oil, sesame oil, soybean oil, sunflower oil and wheat germ oil.

The variant lipid acyltransferase described herein may utilise a carbohydrate as the acyl acceptor. The carbohydrate acyl acceptor may be one or more of the following: a monosaccharide, a disaccharide, an oligosaccharide or a polysaccharide. Preferably, the carbohydrate is one or more of the following: glucose, fructose, anhydrofructose, maltose, lactose, sucrose, galactose, xylose, xylooligosacharides, arabinose, maltooligosaccharides, tagatose, microthecin, ascopyrone P, ascopyrone T, cortalcerone.

Suitably, the carbohydrate "acyl acceptor" may be found naturally within the foodstuff. Alternatively, the carbohydrate may be added to the foodstuff. When it is the case that the carbohydrate is added to the foodstuff, the carbohydrate may be added before, simultaneously with, and/or after the addition of the variant lipid acyltransferase described herein.

Carbohydrate esters can function as valuable emulsifiers in foodstuffs. Thus, when it is the case that the enzyme functions to transfer the acyl group to a sugar, the description encompasses the production of a second *in situ* emulsifier in the foodstuff.

The variant lipid acyltransferase may utilise both a sterol and/or stanol and a carbohydrate as an acyl acceptor.

The utilisation of a variant lipid acyltransferase which can transfer the acyl group to a carbohydrate as well as to a sterol and/or a stanol is particularly advantageous for foodstuffs comprising eggs. In particular, the presence of sugars, in particular glucose, in eggs and egg products is often seen as disadvantageous. Egg yolk may comprise up to 1% glucose. Typically, egg or egg based products may be treated with glucose oxidase to remove some or all of this glucose. However, as described herein this unwanted sugar can be readily removed by "esterifying" the sugar to form a sugar ester.

The variant lipid acyltransferase described herein may utilise a protein as the acyl acceptor. Suitably, the protein may be one or more of the proteins found in a food product, for example in a dairy product and/or a meat product. By way of example only, suitable proteins may be those found in curd or whey, such as lactoglobulin. Other suitable proteins include ovalbumin from egg, gliadin, glutenin, puroindoline, lipid transfer proteins from grains, and myosin from meat.

Preferably, the parent lipid acyltransferase enzyme described herein may be characterised using the following criteria:

(i) the enzyme possesses acyl transferase activity which may be defined as ester transfer activity whereby the acyl part of an original ester bond of a lipid acyl donor is transferred to an acyl acceptor to form a new ester; and
(ii) the enzyme comprises the amino acid sequence motif GDSX, wherein X is one or more of the following amino acid residues L, A, V, I, F, Y, H,Q,T,N,MorS.

Preferably, X of the GDSX motif is L. Thus, preferably the enzyme described herein comprises the amino acid sequence motif GDSL.

The GDSX motif is comprised of four conserved amino acids. Preferably, the serine within the motif is a catalytic serine of the lipid acyltransferase enzyme. Suitably, the serine of the GDSX motif may be in a position corresponding to Ser-16 in *Aeromonas hydrophila* lipolytic enzyme taught in Brumlik & Buckley (Journal of Bacteriology Apr. 1996, Vol. 178, No. 7, p 2060-2064).

To determine if a protein has the GDSX motif according to the present invention, the sequence is preferably compared with the hidden markov model profiles (HMM profiles) of the pfam database.

Pfam is a database of protein domain families. Pfam contains curated multiple sequence alignments for each family as well as profile hidden Markov models (profile HMMs) for identifying these domains in new sequences. An introduction to Pfam can be found in Bateman A et al. (2002) Nucleic Acids Res. 30; 276-280. Hidden Markov models are used in a number of databases that aim at classifying proteins, for review see Bateman A and Haft DH (2002) Brief Bioinform 3; 236-245.
http://www.ncbi.nlm.nih.gov/entrez/query.fcgi?cmd=Retrieve&db=PubMed&list_uids =12230032&dopt=Abstract
http://www.ncbi.nlm.nih.gov/entrez/query.fcgi?cmd=Retrieve&db=PubMed&list_uids =11752314&dopt=Abstract

For a detailed explanation of hidden Markov models and how they are applied in the Pfam database see Durbin R, Eddy S, and Krogh A (1998) Biological sequence analysis; probabilistic models of proteins and nucleic acids. Cambridge University Press, ISBN 0-521-62041-4. The Hammer software package can be obtained from Washington University, St Louis, USA.

Alternatively, the GDSX motif can be identified using the Hammer software package, the instructions are provided in Durbin R, Eddy S, and Krogh A (1998) Biological sequence analysis; probabilistic models of proteins and nucleic acids. Cambridge University Press, ISBN 0-521-62041-4 and the references therein, and the HMMER2 profile provided within this specification.

The PFAM database can be accessed, for example, through several servers which are currently located at the following websites.
http://www.sanger.ac.uk/Software/Pfam/index.shtml
http://pfam.wustl.edu/
http://pfam.jouy.inra.fr/
http://pfam.cgb.ki.se/

The database offers a search facility where one can enter a protein sequence. Using the default parameters of the database the protein sequence will then be analysed for the presence of Pfam domains. The GDSX domain is an established domain in the database and as such its presence in any query sequence will be recognised. The database will return the alignment of the Pfam00657 consensus sequence to the query sequence.

A multiple alignment, including *Aeromonas salmonicida* or *Aeromonas hydrophila* can be obtained by:

a) manual
obtain an alignment of the protein of interest with the Pfam00657 consensus sequence and obtain an alignment of P10480 with the Pfam00657 consensus sequence following the procedure described above;
or

b) through the database
After identification of the Pfam00657 consensus sequence the database offers the option to show an alignment of the query sequence to the seed alignment of the Pfam00657 consensus sequence. P10480 is part of this seed alignment and is indicated by GCAT_AERHY. Both the query sequence and P10480 will be displayed in the same window.

The *Aeromonas hydrophila* reference sequence:

The residues of *Aeromonas hydrophila* GDSX lipase are numbered in the NCBI file P10480, the numbers in this text refer to the numbers given in that file which as described herein is used to determine specific amino acids residues which, as described herein are present in the lipid acyltransferase enzymes described herein.

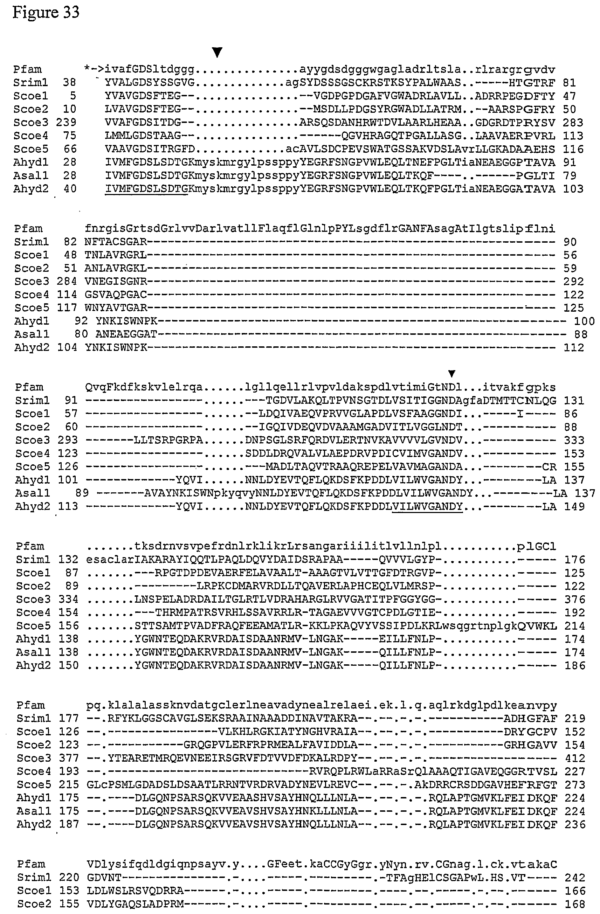

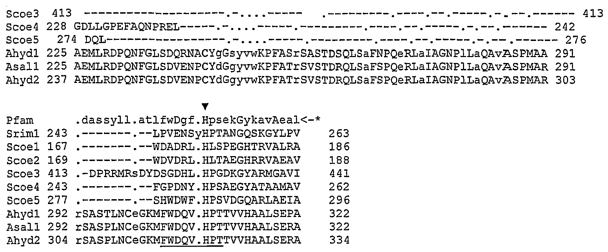

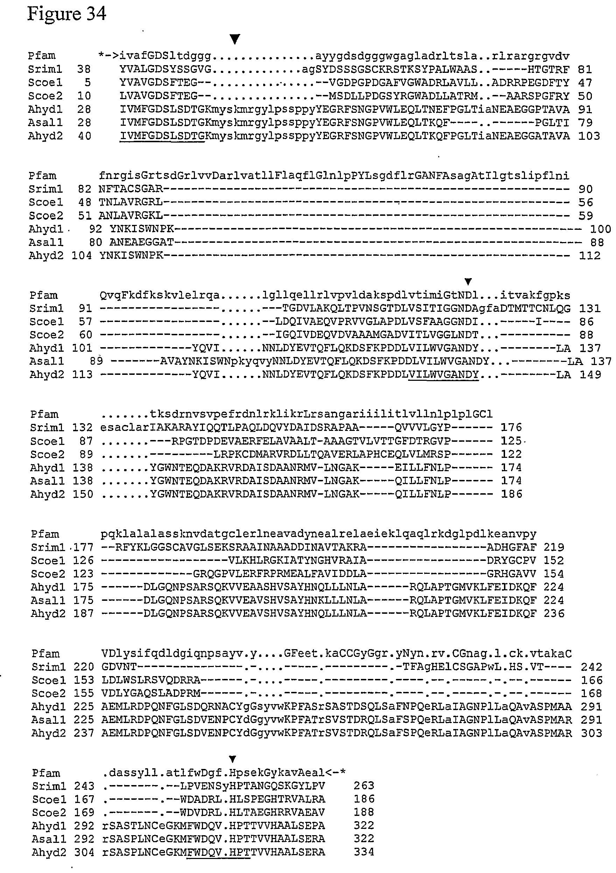

The Pfam alignment was performed (Figure 33 and Figure 34):

The following conserved residues can be recognised and may be present in the variant enzymes for use in the compositions and methods described herein;

Block 1 - GDSX block

| | | | | | | | |
|---|---|---|---|---|---|---|---|
| hid | hid | hid | hid | Gly | Asp | Ser | hid |
| 28 | 29 | 30 | 31 | 32 | 33 | 34 | 35 |

Block 2 - GANDY block

| | | | | | |
|---|---|---|---|---|---|
| hid | Gly | hid | Asn | Asp | hid |
| 130 | 131 | 132 | 133 | 134 | 135 |

Block 3 - HPT block

His

309

Where 'hid' means a hydrophobic residue selected from Met, Ile, Leu, Val, Ala, Gly, Cys, His, Lys, Trp, Tyr, Phe.

Preferably the parent and/or variant lipid acyltransferase enzyme for use in the compositions/methods described herein be aligned using the Pfam00657 consensus sequence.

Preferably, a positive match with the hidden markov model profile (HMM profile) of the pfam00657 domain family indicates the presence of the GDSL or GDSX domain as described herein.

Preferably when aligned with the Pfam00657 consensus sequence the parent and/or variant lipid acyltransferase for use in the compositions/methods described herein have at least one, preferably more than one, preferably more than two, of the following, a GDSx block, a GANDY block, a HPT block. Suitably, the parent and/or variant lipid acyltransferase may have a GDSx block and a GANDY block. Alternatively, the parent and/or variant enzyme may have a GDSx block and a HPT block. Preferably the parent and/or variant enzyme comprises at least a GDSx block.

Preferably, when aligned with the Pfam00657 consensus sequence the parent and/or variant enzyme for use in the compositions/methods described herein have at least one, preferably more than one, preferably more than two, preferably more than three, preferably more than four, preferably more than five, preferably more than six, preferably more than seven, preferably more than eight, preferably more than nine, preferably more than ten, preferably more than eleven, preferably more than twelve, preferably more than thirteen, preferably more than fourteen, of the following amino acid residues when compared to the reference *A. hydrophilia* polypeptide sequence, namely SEQ ID No. 26: 28hid, 29hid, 30hid, 31hid, 32gly, 33Asp, 34Ser, 35hid, 130hid, 131Gly, 132Hid, 133Asn, 134Asp, 135hid, 309His

The pfam00657 GDSX domain is a unique identifier which distinguishes proteins possessing this domain from other enzymes.

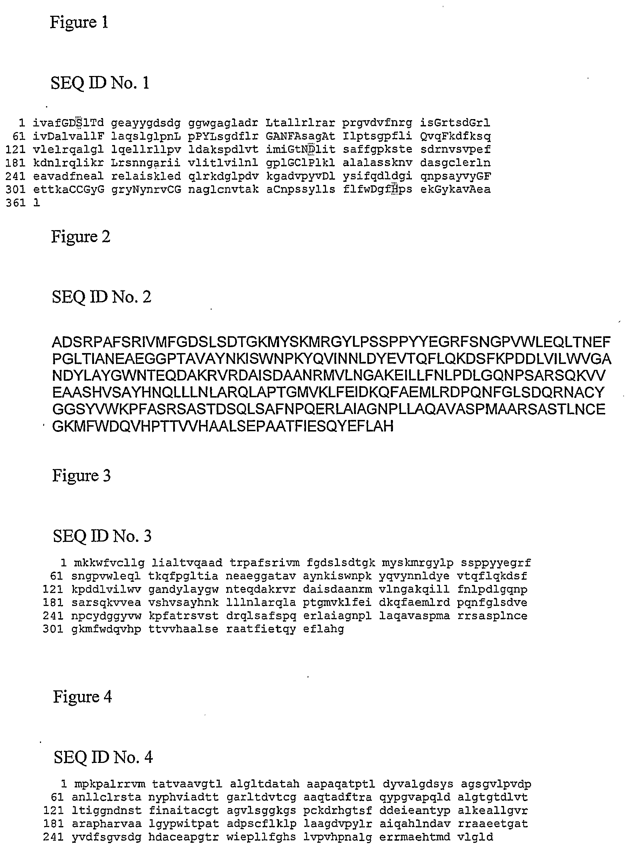

The pfam00657 consensus sequence is presented in Figure 1 as SEQ ID No. 1. This is derived from the identification of the pfam family 00657, database version 6, which may also be referred to as pfam00657.6 herein.

The consensus sequence may be updated by using further releases of the pfam database.

For example, Figures 33 and 34 show the pfam alignment of family 00657, from database version 11, which may also be referred to as pfam00657.11 herein.

The presence of the GDSx, GANDY and HPT blocks are found in the pfam family 00657 from both releases of the database. Future releases of the pfam database can be used to identify the pfam family 00657.

Preferably, the parent lipid acyltransferase enzyme described herein may be characterised using the following criteria:

(i) the enzyme possesses acyl transferase activity which may be defined as ester transfer activity whereby the acyl part of an original ester bond of a lipid acyl donor is transferred to acyl acceptor to form a new ester;

(ii) the enzyme comprises the amino acid sequence motif GDSX, wherein X is one or more of the following amino acid residues L, A, V, I, F, Y, H, Q, T, N, M or S.;

(iii) the enzyme comprises His-309 or comprises a histidine residue at a position corresponding to His-309 in the *Aeromonas hydrophila* lipolytic enzyme shown in Figure 2 (SEQ ID No. 2 or SEQ ID No. 26).

Preferably, the amino acid residue of the GDSX motif is L.

In SEQ ID No. 26 the first 18 amino acid residues form a signal sequence. His-309 of the full length sequence, that is the protein including the signal sequence, equates to His-291 of the mature part of the protein, i.e. the sequence without the signal sequence.

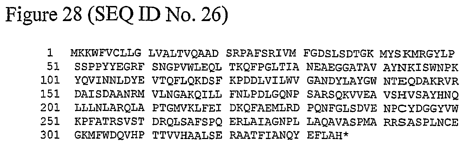

Preferably, the parent lipid acyltransferase enzyme described herein comprises the following catalytic triad: Ser-16, Asp-116 and His-291 or comprises a serine residue, an aspartic acid residue and a histidine residue, respectively, at positions corresponding to Ser-16, Asp-116 and His-291 in the *Aeromonas hydrophila* lipolytic enzyme shown in Figure 2 (SEQ ID No. 2) or at positions corresponding to Ser-34, Asp-134 and His-309 of the full length sequence shown in Figure 28 (SEQ ID No. 26). As stated above, in the sequence shown in SEQ ID No. 26 the first 18 amino acid residues form a signal sequence. Ser-34, Asp-134 and His-309 of the full length sequence, that is the protein including the signal sequence, equate to Ser-16, Asp-116 and His-291 of the mature part of the protein, i.e. the sequence without the signal sequence. In the pfam00657 consensus sequence, as given in Figure 1 (SEQ ID No. 1) the active site residues correspond to Ser-7, Asp-157 and His-348.

Preferably, the parent lipid acyltransferase enzyme described herein may be characterised using the following criteria:

(i) the enzyme possesses acyl transferase activity which may be defined as ester transfer activity whereby the acyl part of an original ester bond of a first lipid acyl donor is transferred to an acyl acceptor to form a new ester; and
(ii) the enzyme comprises at least Gly-14, Asp-15, Ser-16, Asp-116 and His-191 at positions corresponding to *Aeromonas hydrophila* enzyme in Figure 2 (SEQ ID No. 2) which is equivalent to positions Gly-32, Asp-33, Ser-34, Asp-134 and His-309, respectively, in Figure 28 (SEQ ID No. 26).

Suitably, the parent lipid acyltransferase enzyme described herein may be obtainable, preferably obtained, from organisms from one or more of the following genera: *Aeromonas, Corynebacterium, Novosphingobium, Termobifida, Streptomyces, Saccharomyces, Lactococcus, Mycobacterium*, *Streptococcus, Lactobacillus, Desulfitobacterium*, *Bacillus, Campylobacter, Vibrionaceae, Xylella, Sulfolobus, Aspergillus, Schizosaccharomyces, Listeria, Neisseria, Mesorhizobium, Ralstonia, Xanthomonas* and *Candida.*

Suitably, the parent lipid acyltransferase enzyme described herein may be obtainable, preferably obtained, from one or more of the following organisms: *Aeromonas hydrophila, Aeromonas salmonicida, Streptomyces coelicolor, Streptomyces rimosus, Mycobacterium, Streptococcus pyogenes, Lactococcus lactis, Streptococcus pyogenes, Streptococcus thermophilus, Lactobacillus helveticus, Desulfitobacterium dehalogenans, Bacillus sp, Campylobacter jejuni, Vibrionaceae, Xylella fastidiosa, Sulfolobus solfataricus, Saccharomyces cerevisiae, Aspergillus terreus, Schizosaccharomyces pombe*, *Listeria innocua, Listeria monocytogenes*, *Neisseria meningitidis, Mesorhizobium loti, Ralstonia solanacearum, Xanthomonas campestris, Xanthomonas axonopodis, Corynebacterium efficens, Novosphingobium aromaticivorans*, *Termobifida fusca* and *Candida parapsilosis*.

In one aspect, preferably the parent lipid acyltransferase enzyme described herein is obtainable, preferably obtained, from one or more of *Aeromonas hydrophila* or *Aeromonas salmonicida.*

In one aspect, the parent lipid acyltransferase described herein may be a lecithin:cholesterol acyltransferases (LCAT) or variant thereof (for example a variant made by molecular evolution)

Suitable LCATs are known in the art and may be obtainable from one or more of the following organisms for example: mammals, rat, mice, chickens, *Drosophila melanogaster,* plants, including *Arabidopsis* and *Oryza sativa,* nematodes, fungi and yeast.

Preferably, when carrying out a method described herein the product (i.e. foodstuff) is produced without increasing or substantially increasing the free fatty acids in the foodstuff.

The term "transferase" as used herein is interchangeable with the term "lipid acyltransferase".

The term "galactolipid transferase activity" as used herein means the ability of the enzyme to catalyse the transfer of an acyl group from a galactolipid donor to an acceptor molecule (other than water), such as glycerol for instance.

Likewise, the term "phospholipids transferase activity" as used herein means the ability of the enzyme to catalyse the transfer of an acyl group from a phospholipids donor to an acceptor molecule (other than water), such as glycerol for instance.

The term "an increased ratio of galactolipase transferase activity compared with phospholipid transferase activity" as used herein means the variant enzyme when compared with the parent enzyme is able to catalyse galactolipid transferase at a higher rate compared with phospholipid transferase. This may mean that both galactolipid transferase activity and phospholipid transferase activity are increased compared with the parent enzyme or that galactolipid transferase activity is increased whilst phospholipid transferase activity is decreased compared with the parent enzyme. It is the final relation between the two activities which is important.

Suitably, the lipid acyltransferase as defined herein catalyses one or more of the following reactions: interesterification, transesterification, alcoholysis, hydrolysis.

The term "interesterification" refers to the enzymatic catalysed transfer of acyl groups between a lipid donor and lipid acceptor, wherein the lipid donor is not a free acyl group.

The term "transesterification" as used herein means the enzymatic catalysed transfer of an acyl group from a lipid donor (other than a free fatty acid) to an acyl acceptor (other than water).

As used herein, the term "alcoholysis" refers to the enzymatic cleavage of a covalent bond of an acid derivative by reaction with an alcohol ROH so that one of the products combines with the H of the alcohol and the other product combines with the OR group of the alcohol.

As used herein, the term "alcohol" refers to an alkyl compound containing a hydroxyl group.

As used herein, the term "hydrolysis" refers to the enzymatic catalysed transfer of an acyl group from a lipid to the OH group of a water molecule. Acyl transfer which results from hydrolysis requires the separation of the water molecule.

The term "galactolipid hydrolytic activity" as used herein means the the ability of the enzyme to catalyse the hydrolysis of a galactolipid by transferring an acyl group from the galactolipid to the OH group of a water molecule.

Similarly, the term "phospholipid hydrolytic activity" as used herein means the the ability of the enzyme to catalyse the hydrolysis of a phospholipid by transferring an acyl group from the phospholipid to the OH group of a water molecule.

The term "an increased ratio of galactolipase transferase activity compared with galacolipid hydrolysis activity" as used herein means the variant enzyme when compared with the parent enzyme is able to catalyse galactolipid transferase at a higher rate compared with galactolipid hydrolysis. This may mean that both galactolipid transferase activity and galactolipid hydrolysis activity are increased compared with the parent enzyme or that galactolipid transferase activity is increased whilst galactolipid hydrolysis activity is decreased compared with the parent enzyme. It is the final relation between the two activities which is important.

The term "without increasing or without substantially increasing the free fatty acids" as used herein means that preferably the lipid acyl transferase according to the present invention has 100% transferase activity (i.e. transfers 100% of the acyl groups from an acyl donor onto the acyl acceptor, with no hydrolytic activity); however, the enzyme may transfer less than 100% of the acyl groups present in the lipid acyl donor to the acyl acceptor. In which case, preferably the acyltransferase activity accounts for at least 5%, more preferably at least 10%, more preferably at least 20%, more preferably at least 30%, more preferably at least 40%, more preferably 50%, more preferably at least 60%, more preferably at least 70%, more preferably at least 80%, more preferably at least 90% and more preferably at least 98% of the total enzyme activity. The % transferase activity (i.e. the transferase activity as a percentage of the total enzymatic activity) may be determined by the following protocol:

*Protocol for the determination of % acyltransferase activity:*

A foodstuff to which a lipid acyltransferase described herein has been added may be extracted following the enzymatic reaction with $CHCl_3$:$CH_3OH$ 2:1 and the organic phase containing the lipid material is isolated and analysed by GLC according to the procedure detailed hereinbelow. From the GLC analysis (and if necessary HPLC analysis) the amount of free fatty acids and one or more of sterol/stanol esters; carbohydrate esters, protein esters; diglycerides; or monoglycerides are determined. A control foodstuff to which no enzyme described herein has been added, is analysed in the same way.

*Calculation:*

From the results of the GLC (and optionally HPLC analyses) the increase in free fatty acids and sterol/stanol esters and/or carbohydrate esters and/or protein esters and/or diglycerides and/or monoglycerides can be calculated:

Δ % fatty acid = % Fatty acid(enzyme) - % fatty acid(control); Mv fatty acid = average molecular weight of the fatty acids;
A = Δ % sterol ester/Mv sterol ester (where Δ % sterol ester = % sterol/stanol ester(enzyme) - % sterol/stanol ester(control) and Mv sterol ester = average molecular weight of the sterol/stanol esters) - applicable where the acyl acceptor is a sterol and/or stanol;
B = Δ % carbohydrate ester/Mv carbohydrate ester (where Δ % carbohydrate ester = % carbohydrate ester(enzyme) - % carbohydrate ester(control) and Mv carbohydrate ester = average molecular weight of the carbohydrate ester) - applicable where the acyl acceptor is a carbohydrate;
C = Δ % protein ester/Mv protein ester (where Δ % protein ester = % protein ester(enzyme) - % protein ester(control) and Mv protein ester = average molecular weight of the protein ester) - applicable where the acyl acceptor is a protein; and
D = absolute value of diglyceride and/or monoglyceride/Mv di/monoglyceride (where Δ% diglyceride and/or monoglyceride = % diglyceride and/or monoglyceride (enzyme) - % diglyceride and/or monoglyceride (control) and Mv di/monoglyceride = average molecular weight of the diglyceride and/or monoglyceride) - applicable where the acyl acceptor is glycerol.

The transferase activity is calculated as a percentage of the total enzymatic activity:

$$\% \text{ transferase activity} = \frac{A^* + B^* + C^* + D^* \times 100}{A^* + B^* + C^* + D^* + \Delta \% \text{ fatty acid}/(\text{Mv fatty acid})}$$

* - delete as appropriate.

The amino acids which fall within the terms "non-polar", "polar - uncharged", "polar - charged" are given in the table below, as are the amino acids falling within the terms "aliphatic" and "aromatic". The term "polar" refers to both "polar - uncharged" and "polar - charged" amino acids.

| | | |
|---|---|---|
| ALIPHATIC | Non - polar | G A P |
| | | I L V |
| | Polar - uncharged | C S T M |
| | | N Q |
| | Polar - charged | D E |
| | | K R |
| AROMATIC | | H F W Y |

GLC analysis

Perkin Elmer Autosystem 9000 Capillary Gas Chromatograph equipped with WCOT fused silica column 12.5 m x 0.25 mm ID x 0.1 μ film thickness 5% phenyl-methylsilicone (CP Sil 8 CB from Chrompack).
Carrier gas: Helium.
Injector. PSSI cold split injection (initial temp 50°C heated to 385°C), volume 1.0μl
Detector FID: 395°C

| | | | |
|---|---|---|---|
| Oven program: | 1 | 2 | 3 |
| Oven temperature, °C. | 90 | 280 | 350 |
| Isothermal, time, min. | 1 | 0 | 10 |
| Temperature rate, °C/min. | | 15 | 4 |

Sample preparation: 30 mg of sample was dissolved in 9 ml Heptane:Pyridin, 2:1 containing internal standard heptadecane, 0.5 mg/ml. 300μl sample solution was transferred to a crimp vial, 300 μl MSTFA (N-Methyl-N-trimethylsilyl-trifluoraceamid) was added and reacted for 20 minutes at 60°C.
Calculation: Response factors for mono-di-triglycerides and free fatty acid were determined from Standard 2 (mono-di-triglyceride), for Cholesterol, Cholesteryl palmitate and Cholesteryl stearate the response factors were determined from pure reference material (weighing for pure material 10mg).

TECHNICAL EFFECTS

Described herein are one or more of the following unexpected technical effects in egg products, particularly mayonnaise: an improved heat stability during pasteurisation; improved organoleptic properties, an improved consistency.
Variant enzymes with increased phospholipid transferase activity, particularly with increased tranferase activity between a phospholipid and a sterol and/or stanol, such as cholesterol, may be particularly useful in methods for producing lysophospholipids and/or for enzymatic degumming of edible oils and/or for the production of egg products with improved emulsification properties and/or health benefits.
For use in methods of enzymatic degumming, variants with an increased absolute phospholipid transferase to sterol activity are preferred.
Suitably, described herein are one or more of the following unexpected technical effects in egg or in egg products: improved stability of emulsion; thermal stability of emulsion; improved flavour; reduced mal-odour; improved thickening properties, improved consistency.
Further described herein are one or more of the following unexpected technical effects in dough and/or baked products: an improved specific volume of either the dough or the baked products (for example of bread and/or of cake); an improved dough stability; an improved crust score (for example a thinner and/or crispier bread crust), an improved crumb score (for example a more homogenous crumb distribution and/or a finer crumb structure and/or a softer crumb); an improved appearance (for example a smooth surface without blisters or holes or substantially without blisters or holes); a reduced staling; an enhanced softness; an improved odour; an improved taste.
Suitably, described herein are one or more of the following unexpected technical effects in a foodstuff: an improved appearance, an improved mouthfeel, an improved stability, in particular an improved thermal stability, an improved taste, an improved softness, an improved resilience, an improved emulsification.
Suitably, described herein are | one or more of the following unexpected technical effects in dairy products, such as ice cream for example: an improved mouthfeel (preferably a more creamy mouthfeel); an improved taste; an improved meltdown.

Specific technical effects associated with the use of a lipid acyltransferase as defined herein in the preparation of a foodstuff are listed in the table below:

| | Foodstuff | Effect |
|---|---|---|
| 1 | Bread, Muffins and Doughnuts | Strengthens dough and increases mechanical resistance and increases water absorption capacity and/or increases volume of bakery products and maintains softness of crumb and/or reduces blisters on the bread surface. |
| 2 | Frozen dough | Prevents spoiling during refrigeration |
| 3 | Sponge cake | Makes good cake volume and/or a uniform soft texture |
| 4 | Biscuit, cracker and cookie | Makes stable emulsions of fat and/or prevents stickiness to the machine and/or prevents blooming of high fat products |
| 5 | Batter and breading | Improves texture of fried products. |
| 6 | Noodles | Prevents dough from sticking to the machine and/or increases water content, and/or decreases cooking loss |
| 7 | Instant noodles | Prevent noodles form adhering to each other |
| 8 | Pasta | Dough conditioner prevents adhesion on cooking. |
| 9 | Custard cream | Makes starch paste with a smooth and creamy texture, and/or prevents dehydration. |
| 10 | Coffee whitener | Prevent oil and water separation |
| 11 | Whipping cream | Provides stable emulsion |
| 12 | Chocolate | Prevents or reduced blooming |
| 13 | Caramel, candy and nougat | Improves emulsification of molten sugar and oil and/or prevents separation of oil. |
| 14 | Processed meat, sausages | Improves water holding capacity of sausages and pressed ham, and/or prevents separation of oil phase of pastes and pate. |

Suitably, described herein are one or more of the following unexpected technical effects in cheese: a decrease in the oiling-off effect in cheese; an increase in cheese yield; an improvement in flavour; a reduced mal-odour; a reduced "soapy" taste.

In one aspect, the present description is based in part on the realisation that yields of foods - such as cheese - may be improved by the use of a lipid acyl transferase. In addition or alternatively, the flavour, texture, oxidative stability and/or shelf life of the food may be improved. In addition or alternatively, the food may have a reduced cholesterol level or enhanced content of phytosterol/stanol esters.

Described herein is a food additive composition comprising a lipid acyl transferase as defined herein.

Described herein is a cosmetic composition comprising a lipid acyl transferase as defined herein.

In addition, described herein is the use of an acyltransferase as defined herein to produce a cosmetic composition.

ADVANTAGES

Variant transferases described herein have one or more of the following advantageous properties compared with the parent enzyme:

i) an increased activity on polar lipids and/or an increased activity on polar lipids compared to triglycerides.

ii) an increased activity on galactolipids (glycolipids), such as one or more of digalactosyl diglyceride (DGDG) and/or monogalactosyl diglyceride (MGDG).

iii) an increased ratio of activity on galactolipids (glycolipids) compared to either phospholipids and/or triglycerides

Preferably variant transferases described herein have increased activity on digalactosyl diglyceride (DGDG) and/or monogalactosyl diglyceride (MGDG).

Preferably variant transferase described herein has increased activity on DGDG and/or MGDG and decreased activity on DGMG and/or MGMG.

The variant transferases described herein may also have an increased activity on triglycerides.

The variant transferases described herein may also have an increased activity on phospholipids, such as lecithin, including phosphatidyl choline.

Variant transferases described herein may have decreased activity on triglycerides, and/or monoglycerides and/or diglycerides.

The term polar lipid refers to the polar lipids usually found in a dough, preferably galactolipids and phospholipids.

When used in preparation of a dough or baked product the variant transferase described herein may result in one or more of the following unexpected technical effects in dough and/or baked products: an improved specific volume of either the dough or the baked products (for example of bread and/or of cake); an improved dough stability; an improved crust score (for example a thinner and/or crispier bread crust), an improved crumb score (for example a more homogenous crumb distribution and/or a finer crumb structure and/or a softer crumb); an improved appearance (for example a smooth surface without blisters or holes or substantially without blisters or holes); a reduced staling; an enhanced softness; an improved odour; an improved taste.

ISOLATED

In one aspect, preferably the polypeptide or protein for use as described herein is in an isolated form. The term "isolated" means that the sequence is at least substantially free from at least one other component with which the sequence is naturally associated in nature and as found in nature.

PURIFIED

In one aspect, preferably the polypeptide or protein for use as described herein is in a purified form. The term "purified" means that the sequence is in a relatively pure state - e.g. at least about 51% pure, or at least about 75%, or at least about 80%, or at least about 90% pure, or at least about 95% pure or at least about 98% pure.

CLONING A NUCLEOTIDE SEQUENCE ENCODING A POLYPEPTIDE DESCRIBED HEREIN

A nucleotide sequence encoding either a polypeptide which has the specif properties as defined herein or a polypeptide which is suitable for modification may be isolated from any cell or organism producing said polypeptide. Various methods are well known within the art for the isolation of nucleotide sequences.

For example, a genomic DNA and/or cDNA library may be constructed using chromosomal DNA or messenger RNA from the organism producing the polypeptide. If the amino acid sequence of the polypeptide is known, labelled oligonucleotide probes may be synthesised and used to identify polypeptide-encoding clones from the genomic library prepared from the organism. Alternatively, a labelled oligonucleotide probe containing sequences homologous to another known polypeptide gene could be used to identify polypeptide-encoding clones. In the latter case, hybridisation and washing conditions of lower stringency are used.

Alternatively, polypeptide-encoding clones could be identified by inserting fragments of genomic DNA into an expression vector, such as a plasmid, transforming enzyme-negative bacteria with the resulting genomic DNA library, and then plating the transformed bacteria onto agar containing an enzyme inhibited by the polypeptide, thereby allowing clones expressing the polypeptide to be identified.

In a yet further alternative, the nucleotide sequence encoding the polypeptide may be prepared synthetically by established standard methods, e.g. the phosphoroamidite method described by Beucage S.L. et al (1981) Tetrahedron Letters 22, p 1859-1869, or the method described by Matthes et al (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in US 4,683,202 or in Saiki R K et al (Science (1988) 239, pp 487-491).

NUCLEOTIDE SEQUENCES

Described herein are nucleotide sequences encoding polypeptides having the specific properties as defined herein. The term "nucleotide sequence" as used herein refers to an oligonucleotide sequence or polynucleotide sequence, and variant, homologues, fragments and derivatives thereof (such as portions thereof). The nucleotide sequence may be of genomic or synthetic or recombinant origin, which may be doublestranded or single-stranded whether representing the sense or antisense strand.

The term "nucleotide sequence" as used herein includes genomic DNA, cDNA, synthetic DNA, and RNA. Preferably it means DNA, more preferably cDNA for the coding sequence.

Preferably the nucleotide sequence *per se* encoding a polypeptide having the specific properties as defined herein does not cover the native nucleotide sequence in its natural environment when it is linked to its naturally associated sequence(s) that is/are also in its/their natural environment. For ease of reference, we shall call this preferred embodiment the "non-native nucleotide sequence". In this regard, the term "native nucleotide sequence" means an entire nucleotide sequence that is in its native environment and when operatively linked to an entire promoter with which it is naturally associated, which promoter is also in its native environment. Thus, the polypeptide of the present invention can be expressed by a nucleotide sequence in its native organism but wherein the nucleotide sequence is not under the control of the promoter with which it is naturally associated within that organism.

Preferably the polypeptide is not a native polypeptide. In this regard, the term "native polypeptide" means an entire polypeptide that is in its native environment and when it has been expressed by its native nucleotide sequence.

Typically, the nucleotide sequence encoding polypeptides having the specific properties as defined herein is prepared using recombinant DNA techniques (i.e. recombinant DNA). However, the nucleotide sequence could be synthesised, in whole or in part, using chemical methods well known in the art (see Caruthers MH et al (1980) Nuc Acids Res Symp Ser 215-23 and Horn T et al (1980) Nuc Acids Res Symp Ser 225-232).

MOLECULAR EVOLUTION

Once an enzyme-encoding nucleotide sequence has been isolated, or a putative enzyme-encoding nucleotide sequence has been identified, it may be desirable to modify the selected nucleotide sequence, for example it may be desirable to mutate the sequence in order to prepare an enzyme described herein

Mutations may be introduced using synthetic oligonucleotides. These oligonucleotides contain nucleotide sequences flanking the desired mutation sites.

A suitable method is disclosed in Morinaga et al (Biotechnology (1984) 2, p646-649). Another method of introducing mutations into enzyme-encoding nucleotide sequences is described in Nelson and Long (Analytical Biochemistry (1989), 180, p 147-151).

Instead of site directed mutagenesis, such as described above, one can introduce mutations randomly for instance using a commercial kit such as the GeneMorph PCR mutagenesis kit from Stratagene, or the Diversify PCR random mutagenesis kit from Clontech. EP 0 583 265 refers to methods of optimising PCR based mutagenesis, which can also be combined with the use of mutagenic DNA analogues such as those described in EP 0 866 796. Error prone PCR technologies are suitable for the production of variants of lipid acyl transferases with preferred characterisitics. WO0206457 refers to molecular evolution of lipases.

A third method to obtain novel sequences is to fragment non-identical nucleotide sequences, either by using any number of restriction enzymes or an enzyme such as Dnase I, and reassembling full nucleotide sequences coding for functional proteins. Alternatively one can use one or multiple non-identical nucleotide sequences and introduce mutations during the reassembly of the full nucleotide sequence. DNA shuffling and family shuffling technologies are suitable for the production of variants of lipid acyl transferases with preferred characteristics. Suitable methods for performing 'shuffling' can be found in EP0 752 008, EP1 138 763, EP1 103 606. Shuffling can also be combined with other forms of DNA mutagenesis as described in US 6,180,406 and WO 01/34835.

Thus, it is possible to produce numerous site directed or random mutations into a nucleotide sequence, either *in vivo* or *in vitro,* and to subsequently screen for improved functionality of the encoded polypeptide by various means. Using in silico and exo mediated recombination methods (see WO 00/58517, US 6,344,328, US 6,361,974), for example, molecular evolution can be performed where the variant produced retains very low homology to known enzymes or proteins. Such variants thereby obtained may have significant structural analogy to known transferase enzymes, but have very low amino acid sequence homology.

As a non-limiting example, In addition, mutations or natural variants of a polynucleotide sequence can be recombined with either the wild type or other mutations or natural variants to produce new variants. Such new variants can also be screened for improved functionality of the encoded polypeptide.

The application of the above-mentioned and similar molecular evolution methods allows the identification and selection of variants of the enzymes described herein which have preferred characteristics without any prior knowledge of protein structure or function, and allows the production of non-predictable but beneficial mutations or variants. There are numerous examples of the application of molecular evolution in the art for the optimisation or alteration of enzyme activity, such examples include, but are not limited to one or more of the following: optimised expression and/or activity in a host cell or in vitro, increased enzymatic activity, altered substrate and/or product specificity, increased or decreased enzymatic or structural stability, altered enzymatic activity/specificity in preferred environmental conditions, e.g. temperature, pH, substrate

As will be apparent to a person skilled in the art, using molecular evolution tools an enzyme may be altered to improve the functionality of the enzyme.

Suitably, the lipid acyltransferase used as described herein may be a variant, i.e. may contain at least one amino acid substitution, deletion or addition, when compared to a parental enzyme. Variant enzymes retain at least 25%, 30%, 40%, 50 %, 60%, 70%, 80%, 90%, 95%, 97%, 99% homology with the parent enzyme. Suitable parent enzymes may include any enzyme with esterase or lipase activity. Preferably, the parent enzyme aligns to the pfam00657 consensus sequence.

A variant lipid acyltransferase enzyme may retain or incorporates at least one or more of the pfam00657 consensus sequence amino acid residues found in the GDSx, GANDY and HPT blocks.

Enzymes, such as lipases with no or low lipid acyltransferase activity in an aqueous environment may be mutated using molecular evolution tools to introduce or enhance the transferase activity, thereby producing a lipid acyltransferase enzyme with significant transferase activity suitable for use in the compositions and methods described herein.

Suitably, the lipid acyltransferase for use as described herein may be a variant with enhanced enzyme activity on polar lipids, preferably phospholipids and/or glycolipids when compared to the parent enzyme. Preferably, such variants also have low or no activity on lyso polar lipids. The enhanced activity on polar lipids, phospholipids and/or glycolipids may be the result of hydrolysis and/or transferase activity or a combination of both.

Variant lipid acyltransferases for use as described herein may have decreased activity on triglycerides, and/or monoglycerides and/or diglycerides compared with the parent enzyme.

Suitably the variant enzyme may have no activity on triglycerides and/or monoglycerides and/or diglycerides.

Alternatively, the variant enzyme for use as described herein may have increased activity on triglycerides, and/or may also have increased activity on one or more of the following, polar lipids, phospholipids, lecithin, phosphatidylcholine, glycolipids, digalactosyl monoglyceride, monogalactosyl monoglyceride.

Variants of lipid acyltransferases are known, and one or more of such variants may be suitable for use in the methods and uses described herein and/or in the enzyme compositions described herein By way of example only, variants of lipid acyltransferases are described in the following references may be used as described herein : Hilton & Buckley J Biol. Chem. 1991 Jan 15: 266 (2): 997-1000; Robertson et al J. Biol. Chem. 1994 Jan 21; 269(3):2146-50; Brumlik et al J. Bacteriol 1996 Apr; 178 (7): 2060-4; Peelman et al Protein Sci. 1998 Mar; 7(3):587-99.

## AMINO ACID SEQUENCES

Also described herein are amino acid sequences of polypeptides having the specific properties as defined herein.

As used herein, the term "amino acid sequence" is synonymous with the term "polypeptide" and/or the term "protein". In some instances, the term "amino acid sequence" is synonymous with the term "peptide".

The amino acid sequence may be prepared/isolated from a suitable source, or it may be made synthetically or it may be prepared by use of recombinant DNA techniques.

Suitably, the amino acid sequences may be obtained from the isolated polypeptides taught herein by standard techniques.

One suitable method for determining amino acid sequences from isolated polypeptides is as follows:

> Purified polypeptide may be freeze-dried and 100 μg of the freeze-dried material may be dissolved in 50 μl of a mixture of 8 M urea and 0.4 M ammonium hydrogen carbonate, pH 8.4. The dissolved protein may be denatured and reduced for 15 minutes at 50°C following overlay with nitrogen and addition of 5 μl of 45 mM dithiothreitol. After cooling to room temperature, 5 μl of 100 mM iodoacetamide may be added for the cysteine residues to be derivatized for 15 minutes at room temperature in the dark under nitrogen.

135 μl of water and 5 μg of endoproteinase Lys-C in 5 μl of water may be added to the above reaction mixture and the digestion may be carried out at 37°C under nitrogen for 24 hours.

The resulting peptides may be separated by reverse phase HPLC on a VYDAC C18 column (0.46x15cm;10μm; The Separation Group, California, USA) using solvent A: 0.1% TFA in water and solvent B: 0.1% TFA in acetonitrile. Selected peptides may be re-chromatographed on a Develosil C18 column using the same solvent system, prior to N-terminal sequencing. Sequencing may be done using an Applied Biosystems 476A sequencer using pulsed liquid fast cycles according to the manufacturer's instructions (Applied Biosystems, California, USA).

SEQUENCE IDENTITY OR SEQUENCE HOMOLOGY

Also described herein is the use of sequences having a degree of sequence identity or sequence homology with amino acid sequence(s) of a polypeptide having the specific properties defined herein or of any nucleotide sequence encoding such a polypeptide (hereinafter referred to as a "homologous sequence(s)"). Here, the term "homologue" means an entity having a certain homology with the subject amino acid sequences and the subject nucleotide sequences. Here, the term "homology" can be equated with "identity".

The homologous amino acid sequence and/or nucleotide sequence should provide and/or encode a polypeptide which retains the functional activity and/or enhances the activity of the enzyme.

In the present context, a homologous sequence is taken to include an amino acid sequence which may be at least 75, 85 or 90% identical, preferably at least 95 or 98% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 75, 85 or 90% identical, preferably at least 95 or 98% identical to a nucleotide sequence encoding a polypeptide of the present invention (the subject sequence). Typically, the homologues will comprise the same sequences that code for the active sites etc. as the subject sequence. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (Devereux et al 1984 Nuc. Acids Research 12 p387). Examples of other software that can perform sequence comparisons include, but are not limited to, the BLAST package (see Ausubel et al 1999 Short Protocols in Molecular Biology, 4th Ed - Chapter 18), FASTA (Altschul et al 1990 J. Mol. Biol. 403-410) and the GENEWORKS suite of comparison tools. Both BLAST and FASTA are available for offline and online searching (see Ausubel *et al* 1999, pages 7-58 to 7-60). However, for some applications, it is preferred to use the GCG Bestfit program. A new tool, called BLAST 2 Sequences is also available for comparing protein and nucleotide sequence (see FEMS Microbiol Lett 1999 174(2): 247-50; FEMS Microbiol Lett 1999 177(1): 187-8 and tatiana@ncbi.nlm.nih.gov).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). For some applications, it is preferred to use the public default values for the GCG package, or in the case of other software, the default matrix, such as BLOSUM62.

Alternatively, percentage homologies may be calculated using the multiple alignment feature in DNASIS™ (Hitachi Software), based on an algorithm, analogous to CLUSTAL (Higgins DG & Sharp PM (1988), Gene 73(1), 237-244).

Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non - polar | G A P |
| | | I L V |
| | Polar - uncharged | C S T M |
| | | N Q |
| | Polar - charged | D E |
| | | K R |
| AROMATIC | | H F W Y |

Also described herein is homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

Nucleotide sequences for use as described herein or encoding a polypeptide having the specific properties defined herein may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present invention, it is to be understood that the nucleotide sequences described herein may be modified by any method available in the art. Such modifications may be carried out in order to enhance the *in vivo* activity or life span of nucleotide sequences.

Also described herein is the use of nucleotide sequences that are complementary to the sequences discussed herein, or any derivative, fragment or derivative thereof. If the sequence is complementary to a fragment thereof then that sequence can be used as a probe to identify similar coding sequences in other organisms etc.

Polynucleotides which are not 100% homologous to the sequences described herein can be obtained in a number of ways. Other variants of the sequences described herein may be obtained for example by probing DNA libraries made from a range of individuals, for example individuals from different populations. In addition, other viral/bacterial, or cellular homologues particularly cellular homologues found in mammalian cells (e.g. rat, mouse, bovine and primate cells), may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of any one of the sequences in the attached sequence listings under conditions of medium to high stringency. Similar considerations apply to obtaining species homologues and allelic variants of the polypeptide or nucleotide sequences. Variants and strain/species homologues may also be obtained using degenerate PCR which will use primers designed to target sequences within the variants and homologues encoding conserved amino acid sequences within the. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Alternatively, such polynucleotides may be obtained by site directed mutagenesis of characterised sequences. This may be useful where for example silent codon sequence changes are required to optimise codon preferences for a particular host cell in which the polynucleotide sequences are being expressed. Other sequence changes may be desired in order to introduce restriction polypeptide recognition sites, or to alter the property or function of the polypeptides encoded by the polynucleotides.

Polynucleotides (nucleotide sequences) described herein may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides as used herein.

Polynucleotides such as DNA polynucleotides and probes described herein may be produced recombinantly, synthetically, or by any means available to those of skill in the art. They may also be cloned by standard techniques.

In general, primers will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. This will involve making a pair of primers (e.g. of about 15 to 30 nucleotides) flanking a region of the lipid targeting sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

## HYBRIDISATION

Described herein are sequences that are complementary to the sequences described herein or sequences that are capable of hybridising either to the sequences described herein or to sequences that are complementary thereto.

The term "hybridisation" as used herein shall include "the process by which a strand of nucleic acid joins with a complementary strand through base pairing" as well as the process of amplification as carried out in polymerase chain reaction (PCR) technologies.

Also described herein is use of nucleotide sequences that are capable of hybridising to the sequences that are complementary to the subject sequences discussed herein, or any derivative, fragment or derivative thereof.

Also described herein are sequences that are complementary to sequences that are capable of hybridising to the nucleotide sequences discussed herein. Hybridisation conditions are based on the melting temperature (Tm) of the nucleotide binding complex, as taught in Berger and Kimmel (1987, Guide to Molecular Cloning Techniques, Methods in Enzymology, Vol. 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridisation can be used to identify or detect identical nucleotide sequences while an intermediate (or low) stringency hybridisation can be used to identify or detect similar or related polynucleotide sequences.

Described herein are sequences that are complementary to sequences that are capable of hybridising under high stringency conditions or intermediate stringency conditions to nucleotide sequences encoding polypeptides having the specific properties as defined herein.

Described herein are sequences that are complementary to sequences that are capable of hybridising under high stringent conditions (e.g. 65°C and 0.1xSSC {1xSSC = 0.15 M NaCl, 0.015 M Na-citrate pH 7.0}) to nucleotide sequences encoding polypeptides having the specific properties as defined herein.

The present description also relates to nucleotide sequences that can hybridise to the nucleotide sequences discussed herein (including complementary sequences of those discussed herein).

The presentdescriptionalso relates to nucleotide sequences that are complementary to sequences that can hybridise to the nucleotide sequences discussed herein (including complementary sequences of those discussed herein).

Also described herein are polynucleotide sequences that are capable of hybridising to the nucleotide sequences discussed herein under conditions of intermediate to maximal stringency.

Described herein are nucleotide sequences that can hybridise to the nucleotide sequences discussed herein, or the complement thereof, under stringent conditions (e.g. 50°C and 0.2xSSC).

Described herein are nucleotide sequences that can hybridise to the nucleotide sequences discussed herein, or the complement thereof, under high stringent conditions (e.g. 65°C and 0.1xSSC).

EXPRESSION OF POLYPEPTIDES

A nucleotide sequence for use as described herein or for encoding a polypeptide having the specific properties as defined herein can be incorporated into a recombinant replicable vector. The vector may be used to replicate and express the nucleotide sequence, in polypeptide form, in and/or from a compatible host cell. Expression may be controlled using control sequences which include promoters/enhancers and other expression regulation signals. Prokaryotic promoters and promoters functional in eukaryotic cells may be used. Tissue specific or stimuli specific promoters may be used. Chimeric promoters may also be used comprising sequence elements from two or more different promoters described above.

The polypeptide produced by a host recombinant cell by expression of the nucleotide sequence may be secreted or may be contained intracellularly depending on the sequence and/or the vector used. The coding sequences can be designed with signal sequences which direct secretion of the substance coding sequences through a particular prokaryotic or eukaryotic cell membrane.

EXPRESSION VECTOR

The term "expression vector" means a construct capable of *in vivo* or *in vitro* expression.

Preferably, the expression vector is incorporated in the genome of the organism. The term "incorporated" preferably covers stable incorporation into the genome.

The nucleotide sequence described herein or coding for a polypeptide having the specific properties as defined herein may be present in a vector, in which the nucleotide sequence is operably linked to regulatory sequences such that the regulatory sequences are capable of providing the expression of the nucleotide sequence by a suitable host organism, i.e. the vector is an expression vector.

The vectors described herein may be transformed into a suitable host cell as described below to provide for expression of a polypeptide having the specific properties as defined herein.

The choice of vector, e.g. plasmid, cosmid, virus or phage vector, will often depend on the host cell into which it is to be introduced.

The vectors may contain one or more selectable marker genes - such as a gene which confers antibiotic resistance e.g. ampicillin, kanamycin, chloramphenicol or tetracyclin resistance. Alternatively, the selection may be accomplished by co-transformation (as described in WO91/17243).

Vectors may be used *in vitro,* for example for the production of RNA or used to transfect or transform a host cell.

Thus, further embodiment, described herein is a method of making nucleotide sequences described herein or nucleotide sequences encoding polypeptides having the specific properties as defined herein by introducing a nucleotide sequence into a replicable vector, introducing the vector into a compatible host cell, and growing the host cell under conditions which bring about replication of the vector.

The vector may further comprise a nucleotide sequence enabling the vector to replicate in the host cell in question. Examples of such sequences are the origins of replication of plasmids pUC19, pACYC177, pUB110, pE194, pAMB1 and pIJ702.

REGULATORY SEQUENCES

In some applications, a nucleotide sequence for use as described herein or a nucleotide sequence encoding a polypeptide having the specific properties as defined herein may be operably linked to a regulatory sequence which is capable of providing for the expression of the nucleotide sequence, such as by the chosen host cell. By way of example, covers a vector comprising the nucleotide sequence described herein may be operably linked to such a regulatory sequence, i.e. the vector is an expression vector.

The term "operably linked" refers to a juxtaposition wherein the components described are in a relationship permitting them to function in their intended manner. A regulatory sequence "operably linked" to a coding sequence is ligated in such a way that expression of the coding sequence is achieved under conditions compatible with the control sequences.

The term "regulatory sequences" includes promoters and enhancers and other expression regulation signals.

The term "promoter" is used in the normal sense of the art, e.g. an RNA polymerase binding site.

Enhanced expression of the nucleotide sequence encoding the enzyme having the specific properties as defined herein may also be achieved by the selection of heterologous regulatory regions, e.g. promoter, secretion leader and terminator regions.

Preferably, the nucleotide sequence described herein may be operably linked to at least a promoter.

Examples of suitable promoters for directing the transcription of the nucleotide sequence in a bacterial, fungal or yeast host are well known in the art.

CONSTRUCTS

The term "construct" - which is synonymous with terms such as "conjugate", "cassette" and "hybrid" - includes a nucleotide sequence encoding a polypeptide having the specific properties as defined herein for use as described herein directly or indirectly attached to a promoter. An example of an indirect attachment is the provision of a suitable spacer group such as an intron sequence, such as the Sh1-intron or the ADH intron, intermediate the promoter and the nucleotide sequence described herein The same is true for the term "fused" as used herein which includes direct or indirect attachment. In some cases, the terms do not cover the natural combination of the nucleotide sequence coding for the protein ordinarily associated with the wild type gene promoter and when they are both in their natural environment.

The construct may even contain or express a marker which allows for the selection of the genetic construct.

For some applications, preferably the construct comprises at least a nucleotide sequence described herein or a nucleotide sequence encoding a polypeptide having the specific properties as defined herein operably linked to a promoter.

HOST CELLS

The term "host cell" as used herein includes any cell that comprises either a nucleotide sequence encoding a polypeptide having the specific properties as defined herein or an expression vector as described above and which is used in the recombinant production of a polypeptide having the specific properties as defined herein.

Thus, a further described herein are host cells transformed or transfected with a nucleotide sequence described herein or a nucleotide sequence that expresses a polypeptide having the specific properties as defined herein. The cells will be chosen to be compatible with the said vector and may for example be prokaryotic (for example bacterial), fungal, yeast or plant cells. Preferably, the host cells are not human cells.

Examples of suitable bacterial host organisms are gram negative bacterium or gram positive bacteria.

Depending on the nature of the nucleotide sequence encoding a polypeptide having the specific properties as defined herein, and/or the desirability for further processing of the expressed protein, eukaryotic hosts such as yeasts or other fungi may be preferred. In general, yeast cells are preferred over fungal cells because they are easier to manipulate. However, some proteins are either poorly secreted from the yeast cell, or in some cases are not processed properly (e.g. hyperglycosylation in yeast). In these instances, a different fungal host organism should be selected.

The use of suitable host cells, such as yeast, fungal and plant host cells - may provide for post-translational modifications (e.g. myristoylation, glycosylation, truncation, lapidation and tyrosine, serine or threonine phosphorylation) as may be needed to confer optimal biological activity on recombinant expression products of the present invention.

The host cell may be a protease deficient or protease minus strain.

ORGANISM

The term "organism" as used herein includes any organism that could comprise a nucleotide sequence described herein or a nucleotide sequence encoding for a polypeptide having the specific properties as defined herein and/or products obtained therefrom.

Suitable organisms may include a prokaryote, fungus, yeast or a plant.

The term "transgenic organism" as used herein includes any organism that comprises a nucleotide sequence coding for a polypeptide having the specific properties as defined herein and/or the products obtained therefrom, and/or wherein a promoter can allow expression of the nucleotide sequence coding for a polypeptide having the specific properties as defined herein within the organism. Preferably the nucleotide sequence is incorporated in the genome of the organism.

The term "transgenic organism" does not cover native nucleotide coding sequences in their natural environment when they are under the control of their native promoter which is also in its natural environment.

Therefore, the transgenic organism described herein includes an organism comprising any one of, or combinations of, a nucleotide sequence coding for a polypeptide having the specific properties as defined herein, constructs as defined herein, vectors as defined herein, plasmids as defined herein, cells as defined herein, or the products thereof For example the transgenic organism can also comprise a nucleotide sequence coding for a polypeptide having the specific properties as defined herein under the control of a heterologous promoter.

TRANSFORMATION OF HOST CELLS/ORGANISM

As indicated earlier, the host organism can be a prokaryotic or a eukaryotic organism. Examples of suitable prokaryotic hosts include *E. coli* and *Bacillus subtilis.*

Teachings on the transformation of prokaryotic hosts is well documented in the art, for example see Sambrook et al (Molecular Cloning: A Laboratory Manual, 2nd edition, 1989, Cold Spring Harbor Laboratory Press). If a prokaryotic host is used then the nucleotide sequence may need to be suitably modified before transformation - such as by removal of introns.

The transgenic organism can be a yeast.

Filamentous fungi cells may be transformed using various methods know in the art - such as a process involving protoplast formation and transformation of the protoplasts followed by regeneration of the cell wall in a manner known. The use of *Aspergillus* as a host microorganism is described in EP 0 238 023.

Another host organism can be a plant. A review of the general techniques used for transforming plants may be found in articles by Potrykus (Annu Rev Plant Physiol Plant Mol Biol [1991] 42:205-225) and Christou (Agro-Food-Industry Hi-Tech March/April 1994 17-27). Further teachings on plant transformation may be found in EP-A-0449375.

General teachings on the transformation of fungi, yeasts and plants are presented in following sections.

TRANSFORMED BACTERIA

A host organism may be a bacterium, such as *Streptomyces, Bacillus subtillis* or *E.coli.* Suitable methods of heterologous expression in *E.coli* are disclosed in WO04/064537. Suitable methods of heterologous expression in *Bacillus* are disclosed in WO02/214490. Examples of suitable bacterial host organisms are gram positive bacterial species such *as Bacillaceae, including Bacillus subtilis, Bacillus licheniformis, Bacillus lentus, Bacillus brevis, Bacillus stearothermophilus*, *Bacillus alkalophilus, Bacillus amyloliquefaciens, Bacillus coagulans, Bacillus lautus, Bacillus megaterium and Bacillus thuringiensis, Streptomyces* species, such as *Streptomyces murinus,* lactic acid bacterial species, including *Lactococcus* spp., such as *Lactococcus lactis, Lactobacillus* spp., including *Lactobacillus reuteri,, Leuconostoc spp., Pediococcus* spp. and *Streptococcus spp.* Alternatively, strains of a gram-negative bacterial species belonging to *Enterobacteria*ceae, including *E coli,* or to *Pseudomoizadaceae* can be selected as the host organism.

TRANSFORMED FUNGUS

A host organism may be a fungus - such as a filamentous fungus. Examples of suitable such hosts include any member belonging to the genera Thermomyces, Acremonium, Aspergillus, Penicillium, Mucor, Neurospora, Trichoderma and the like.

Teachings on transforming filamentous fungi are reviewed in US-A-5741665 which states that standard techniques for transformation of filamentous fungi and culturing the fungi are well known in the art. An extensive review of techniques as applied to *N. crassa* is found, for example in Davis and de Serres, Methods Enzymol (1971) 17A: 79-143.

Further teachings on transforming filamentous fungi are reviewed in US-A-5674707.

In one aspect, the host organism can be of the genus *Aspergillus,* such as *Aspergillus niger.*

A transgenic *Aspergillus* described herein can also be prepared by following, for example, the teachings of Turner G. 1994 (Vectors for genetic manipulation. In: Martinelli S.D., Kinghorn J.R.(Editors) Aspergillus: 50 years on. Progress in industrial microbiology vol 29. Elsevier Amsterdam 1994. pp. 641-666).

Gene expression in filamentous fungi has been reviewed in Punt et al. (2002) Trends Biotechnol 2002 May;20(5):200-6, Archer & Peberdy Crit Rev Biotechnol (1997) 17(4):273-306.

TRANSFORMED YEAST

The transgenic organism can be a yeast.

A review of the principles of heterologous gene expression in yeast are provided in, for example, Methods Mol Biol (1995), 49:341-54, and Curr Opin Biotechnol (1997) Oct;8(5):554-60

In this regard, yeast - such as the species *Saccharomyces cerevisi or Pichia pastoris* (see FEMS Microbiol Rev (2000 24(1):45-66), may be used as a vehicle for heterologous gene expression.

A review of the principles of heterologous gene expression in *Saccharomyces cerevisiae* and secretion of gene products is given by E Hincheliffe E Kenny (1993, "Yeast as a vehicle for the expression of heterologous genes", Yeasts, Vol 5, Anthony H Rose and J Stuart Harrison, eds, 2nd edition, Academic Press Ltd.).

For the transformation of yeast, several transformation protocols have been developed. For example, a transgenic Saccharomyces can be prepared by following the teachings of Hinnen et al., (1978, Proceedings of the National Academy of Sciences of the USA 75, 1929); Beggs, J D (1978, Nature, London, 275, 104); and Ito, H et al (1983, J Bacteriology 153,163-168).

The transformed yeast cells may be selected using various selective markers - such as auxotrophic markers dominant antibiotic resistance markers.

A suitable yeast host organism can be selected from the biotechnologically relevant yeasts species such as, but not limited to, yeast species selected from *Pichia* spp., *Hansenula* spp., *Kluyveromyces, Yarrowinia* spp., *Saccharomyces* spp., including *S. cerevisiae,* or *Schizosaccharomyce* spp. (including *Schizosaccharomyce pombe.*

A strain of the methylntrophic yeast species *Pichia pastoris* may be used as the host organism.

The host organism may be a *Hansenula* species, such as *H. polymorpha* (as described in WO01/39544).

TRANSFORMED PLANTS/PLANT CELLS

A suitable host organism may be a plant. A review of the general techniques may be found in articles by Potrykus (Annu Rev Plant Physiol Plant Mol Biol [1991] 42:205-225) and Christou (Agro-Food-Industry Hi-Tech March/April 1994 17-27), or in WO01/16308. The transgenic plant may produce enhanced levels of phytosterol esters and phytostanol esters, for example.

Therefore the present description also relates to a method for the production of a transgenic plant with enhanced levels of phytosterol esters and phytostanol esters, comprising the steps of transforming a plant cell with a lipid acyltransferase as defined herein (in particular with an expression vector or construct comprising a lipid acyltransferase as defined herein), and growing a plant from the transformed plant cell.

SECRETION

Often, it is desirable for the polypeptide to be secreted from the expression host into the culture medium from where the enzyme may be more easily recovered.

The secretion leader sequence may be selected on the basis of the desired expression host. Hybrid signal sequences may also be used with the context of the present invention.

Typical examples of heterologous secretion leader sequences are those originating from the fungal amyloglucosidase (AG) gene (*gla*A - both 18 and 24 amino acid versions e.g. from *Aspergillus*), the a-factor gene (yeasts e.g. *Saccharomyces, Kluyveromyces* and *Hansenula*) or the $\alpha$-amylase gene (*Bacillus*).

DETECTION

A variety of protocols for detecting and measuring the expression of the amino acid sequence are known in the art. Examples include enzyme-linked immunosorbent assay (ELISA), radioimmunoassay (RIA) and fluorescent activated cell sorting (FACS).

A wide variety of labels and conjugation techniques are known by those skilled in the art and can be used in various nucleic and amino acid assays.

A number of companies such as Pharmacia Biotech (Piscataway, NJ), Promega (Madison, WI), and US Biochemical Corp (Cleveland, OH) supply commercial kits and protocols for these procedures.

Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US-A-3,817,837; US-A-3,850,752; US-A-3,939,350; US-A-3,996,345; US-A-4,277,437; US-A-4,275,149 and US-A-4,366,241.

Also, recombinant immunoglobulins may be produced as shown in US-A-4,816,567.

FUSION PROTEINS

A polypeptide having the specific properties as defined herein may be produced as a fusion protein, for example to aid in extraction and purification thereof. Examples of fusion protein partners include glutathione-S-transferase (GST), 6xHis, GAL4 (DNA binding and/or transcriptional activation domains) and $\beta$-galactosidase. It may also be convenient to include a proteolytic cleavage site between the fusion protein partner and the protein sequence of interest to allow removal of fusion protein sequences. Preferably the fusion protein will not hinder the activity of the protein sequence.

Gene fusion expression systems in *E. coli* have been reviewed in Curr. Opin. Biotechnol. (1995) 6(5):501-6.

The amino acid sequence of a polypeptide having the specific properties as defined herein may be ligated to a heterologous sequence to encode a fusion protein. For example, for screening of peptide libraries for agents capable of affecting the substance activity, it may be useful to encode a chimeric substance expressing a heterologous epitope that is recognised by a commercially available antibody.

The invention will now be described, by way of example only, with reference to the following Figures and Examples.

Figure 1 shows a pfam00657 consensus sequence from database version 6 (SEQ ID No. 1);

Figure 2 shows an amino acid sequence (SEQ ID No. 2) obtained from the organism *Aeromonas hydrophila* (P10480; GI:121051). This amino acid sequence is a reference enzyme, which may be a parent enzyme in accordance with the present invention;

Figure 3 shows an amino acid sequence (SEQ ID No. 3) obtained from the organism *Aeromonas salmonicida* (AAG098404; GI:9964017);

Figure 4 shows an amino acid sequence (SEQ ID No. 4) obtained from the organism *[Streptomyces coelicolor* A3(2) (Genbank accession number NP_631558);

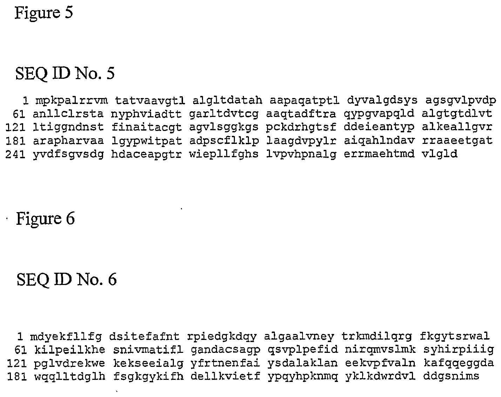

Figure 5 shows an amino acid sequence (SEQ ID No. 5) obtained from the organism *Streptomyces coelicolor* A3(2) (Genbank accession number: CAC42140);

Figure 6 shows an amino acid sequence (SEQ ID No. 6) obtained from the organism *Saccharomyces cerevisiae* (Genbank accession number P41734);

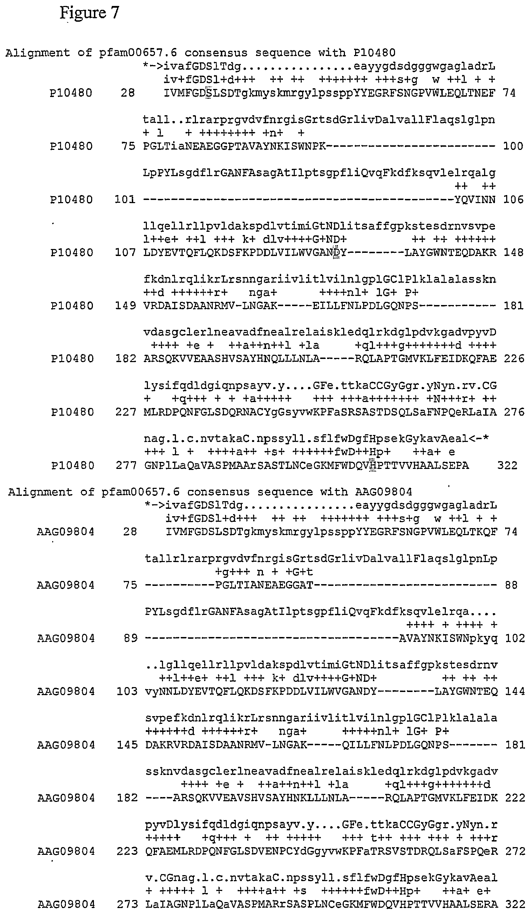

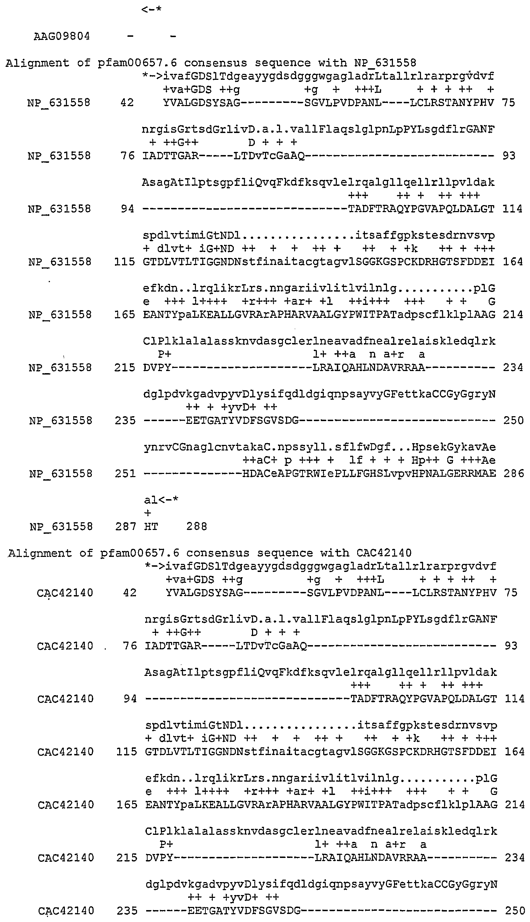

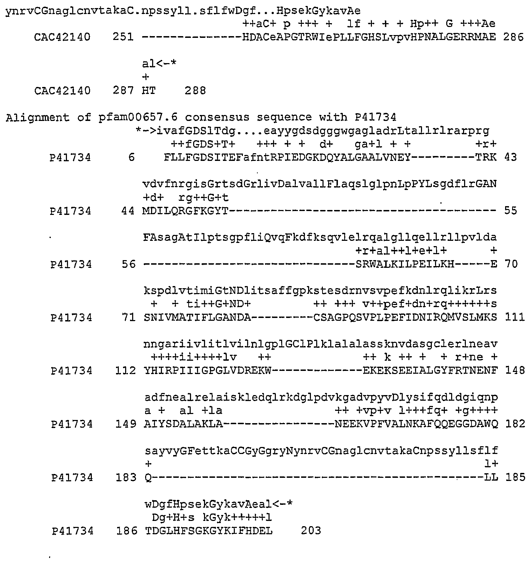

Figure 7 shows an alignment of selected sequences to pfam00657 consensus sequence;

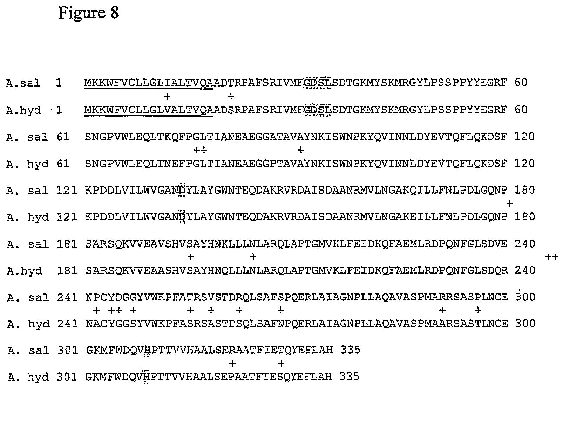

Figure 8 shows a pairwise alignment of SEQ ID No. 3 with SEQ ID No. 2 showing 93% amino acid sequence identity. The signal sequence is underlined. + denotes differences. The GDSX motif containing the active site serine 16, and the active sites aspartic acid 116 and histidine 291 are highlighted (see shaded regions). Numbers after the amino acid is minus the signal sequence;

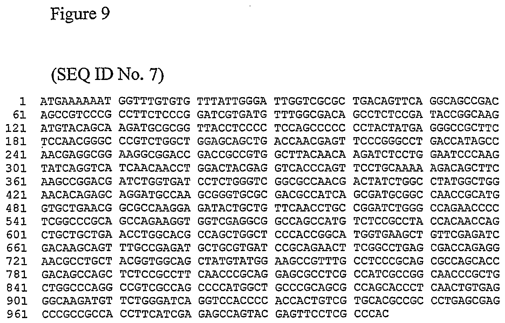

Figure 9 shows a nucleotide sequence (SEQ ID No. 7) encoding a lipid acyl transferase described herein obtained from the organism *Aeromonas hydrophila;*

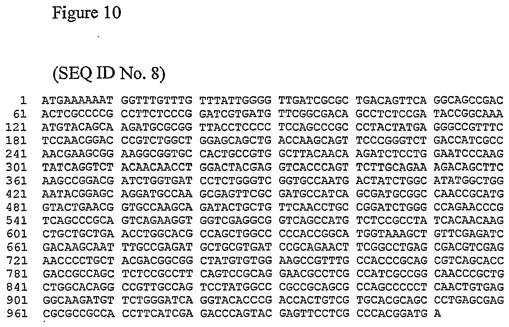

Figure 10 shows a nucleotide sequence (SEQ ID No. 8) encoding a lipid acyl transferase described herein obtained from the organism *Aeromonas salmonicida;*

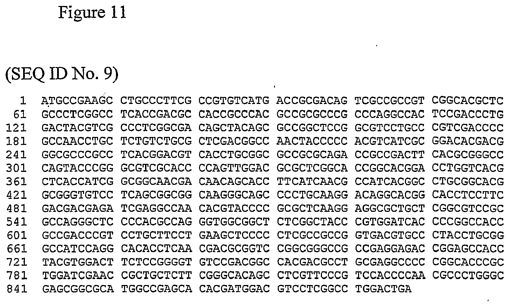

Figure 11 shows a nucleotide sequence (SEQ ID No. 9) encoding a lipid acyl transferase described herein obtained from the organism *Streptomyces coelicolor* A3(2) (Genbank accession number NC_003888.1:8327480..8328367);

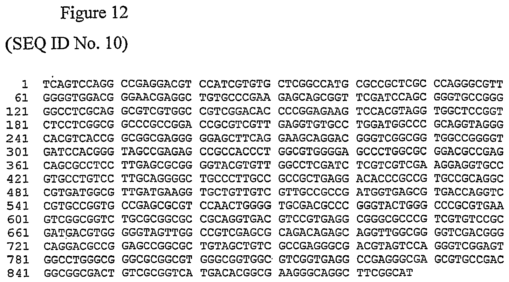

Figure 12 shows a nucleotide sequence (SEQ ID No. 10) encoding a lipid acyl transferase described herein obtained from the organism *Streptomyces coelicolor* A3(2) (Genbank accession number AL939131.1:265480..266367);

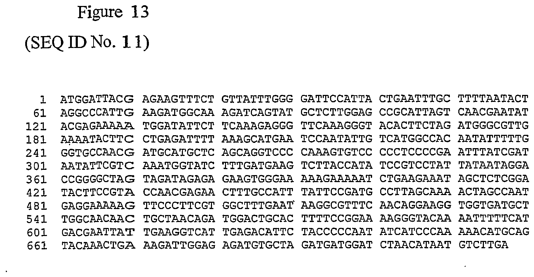

Figure 13 shows a nucleotide sequence (SEQ ID No. 11) encoding a lipid acyl transferase described herein obtained from the organism *Saccharomyces cerevisiae* (Genbank accession number Z75034);

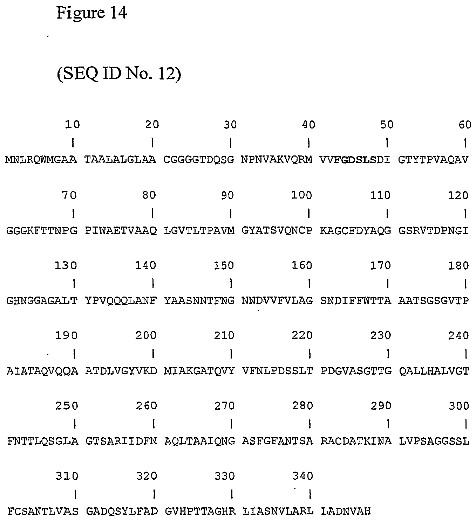

Figure 14 shows an amino acid sequence (SEQ ID No. 12) obtained from the organism *Ralstonia* (Genbank accession number: AL646052);

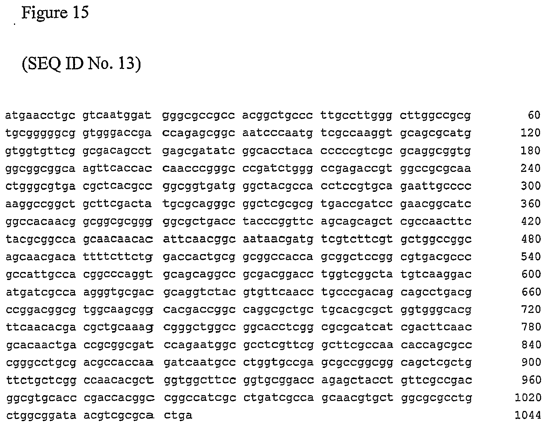

Figure 15 shows a nucleotide sequence (SEQ ID No. 13) encoding a lipid acyl transferase described herein obtained from the organism *Ralstonia*;

Figure 16 shows SEQ ID No. 14. Scoe1 NCBI protein accession code CAB39707.1 GI:4539178 conserved hypothetical protein [Streptomyces coelicolor A3(2)];

Figure 17 shows a nucleotide sequence shown as SEQ ID No. 15 encoding NCBI protein accession code CAB39707.1 GI:4539178 conserved hypothetical protein [Streptomyces coelicolor A3(2)];

Figure 18 shows an amino acid shown as SEQ ID No. 16. Scoe2 NCBI protein accession code CAC01477.1 GI:9716139 conserved hypothetical protein [Streptomyces coelicolor A3(2)];

Figure 19 shows a nucleotide sequence shown as SEQ ID No. 17 encoding Scoe2 NCBI protein accession code CAC01477.1 GI:9716139 conserved hypothetical protein [Streptomyces coelicolor A3(2)];

Figure 20 shows an amino acid sequence (SEQ ID No. 18) Scoe3 NCBI protein accession code CAB88833.1 GI:7635996 putative secreted protein. [Streptomyces coelicolor A3(2)];

Figure 21 shows a nucleotide sequence shown as SEQ ID No. 19 encoding Scoe3 NCBI protein accession code CAB88833.1 GI:7635996 putative secreted protein. [Streptomyces coelicolor A3(2)];

Figure 22 shows an amino acid sequence (SEQ ID No. 20) Scoe4 NCBI protein accession code CAB89450.1 GI:7672261 putative secreted protein. [Streptomyces coelicolor A3(2)];

Figure 23 shows an nucleotide sequence shown as SEQ ID No. 21 encoding Scoe4 NCBI protein accession code CAB89450.1 GI:7672261 putative secreted protein. [Streptomyces coelicolor A3(2)];

Figure 24 shows an amino acid sequence (SEQ ID No. 22) Scoe5 NCBI protein accession code CAB62724.1 GI:6562793 putative lipoprotein [Streptomyces coelicolor A3(2)];

Figure 25 shows a nucleotide sequence shown as SEQ ID No. 23, encoding Scoe5 NCBI protein accession code CAB62724.1 GI:6562793 putative lipoprotein [Streptomyces coelicolor A3(2)];

Figure 26 shows an amino acid sequence (SEQ ID No. 24) Srim1 NCBI protein accession code AAK84028.1 GI:15082088 GDSL-lipase [Streptomyces rimosus];

Figure 27 shows a nucleotide sequence shown as SEQ ID No. 25 encoding Srim1 NCBI protein accession code AAK84028.1 GI:15082088 GDSL-lipase [Streptomyces rimosus];

Figure 28 shows an amino acid sequence (SEQ ID No. 26) - a lipid acyl transferase from *Aeromonas hydrophila* (ATCC #7965);

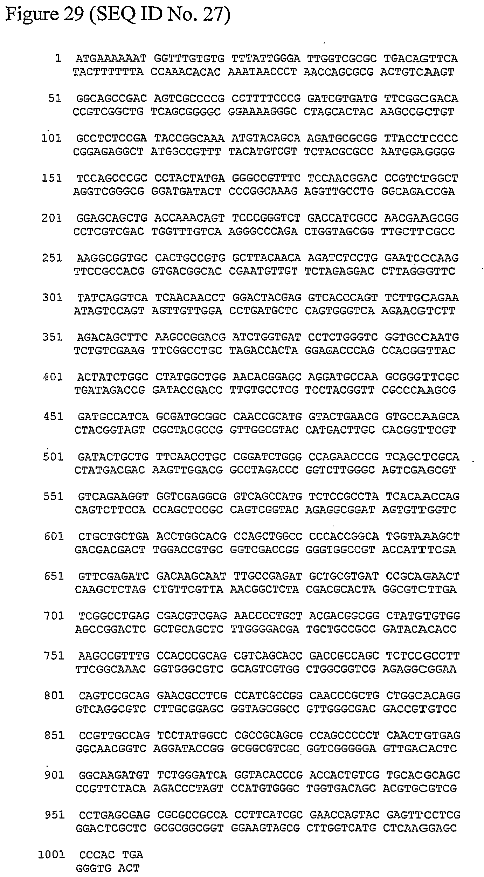

Figure 29 shows a nucleotide sequence (SEQ ID No. 27) encoding a lipid acyltransferase from *Aeromonas hydrophila* (ATCC #7965);

Figure 30 shows an amino acid sequence (SEQ ID No. 28) of a lipid acyltransferase from *Aeromonas salmonicida* subsp. *Salmorzicida* (ATCC#14174);

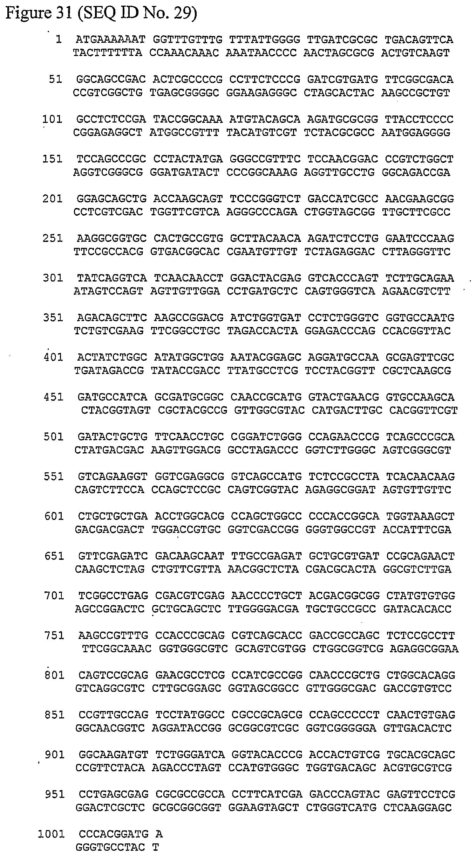

Figure 31 shows a nucleotide sequence (SEQ ID No. 29) encoding a lipid acyltransferase from *Aeromonas salmonicida* subsp. *Salmonicida* (ATCC#14174);

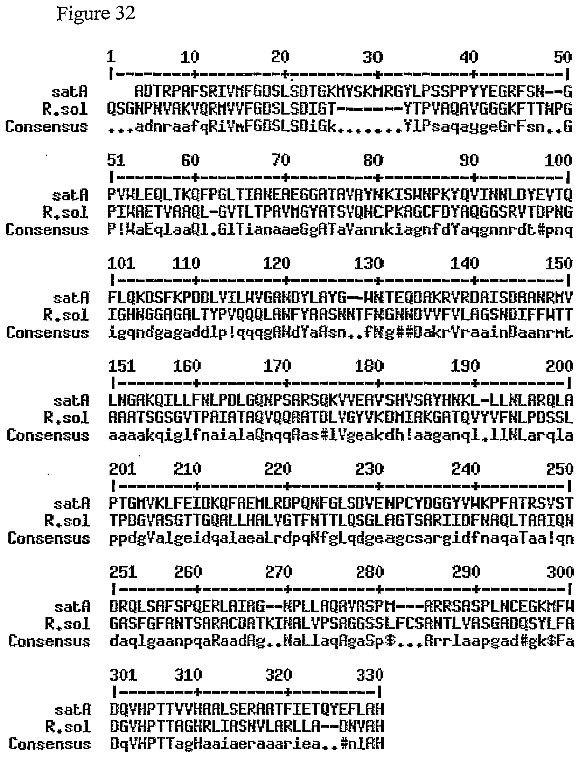

Figure 32 shows that homologues of the *Aeromonas* genes can be identified using the basic local alignment search tool service at the National Center for Biotechnology Information, NIH, MD, USA and the completed genome databases. The GDSX motif was used in the database search and a number of sequences/genes potentially encoding enzymes with lipolytic activity were identified. Genes were identified from the genus Streptomyces, Xanthomonas and Ralstonia. As an example below, the Ralstonia solanacearum was aligned to the Aeromonas salmonicida (satA) gene. Pairwise alignment showed 23% identity. The active site serine is present at the amino terminus and the catalytic residues histidine and aspartic acid can be identified;

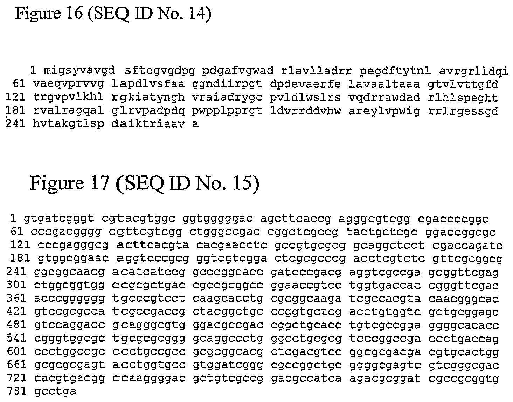

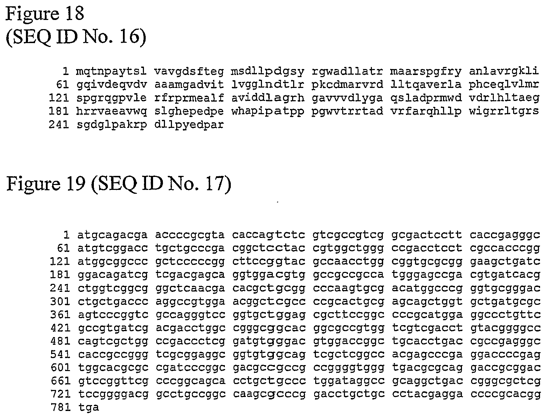

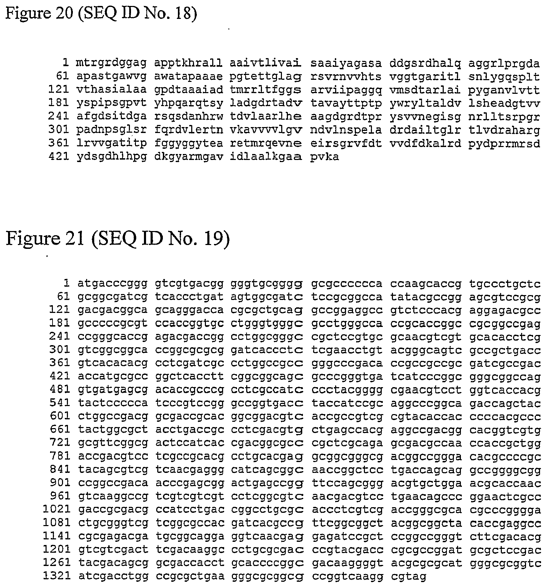

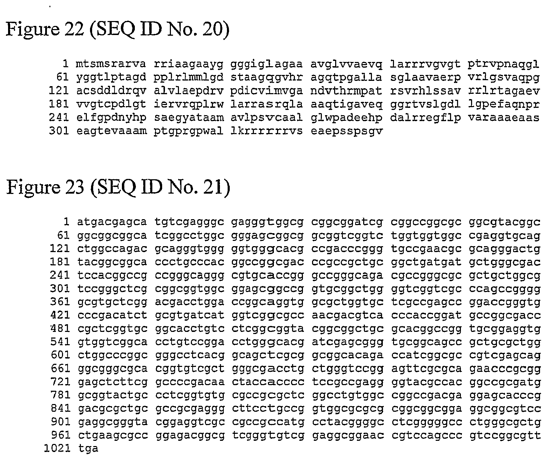

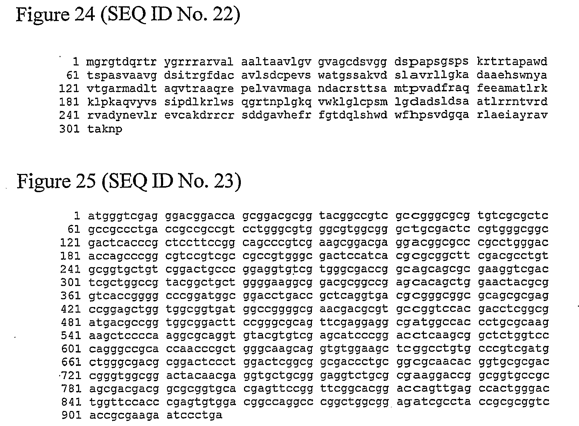

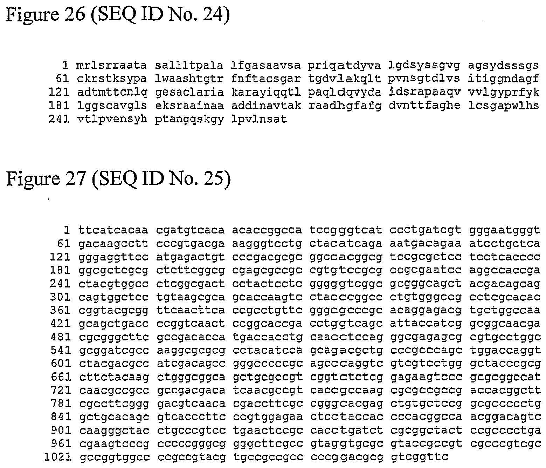

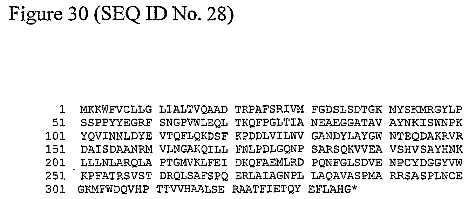

Figure 33 shows the Pfam00657.11 [family 00657, database version 11] consensus sequence (hereafter called Pfam consensus) and the alignment of various sequences to the Pfam consensus sequence. The arrows indicate the active site residues, the underlined boxes indicate three of the homology boxes indicated by [Upton C and Buckley JT (1995) Trends Biochem Sci 20; 179-179]. Capital letters in the Pfam consensus indicate conserved residues in many family members. The - symbol indicates a position where the hidden Markov model of the Pfam consensus expected to find a residue but did not, so a gap is inserted. The . symbol indicates a residue without a corresponding residue in the Pfam consensus. The sequences are the amino acid sequences listed in Figures 16, 18, 20, 22, 24, 26, 28 and 30.

Figure 34 shows the Pfam00657.11 [family 00657, database version 11] consensus sequence (hereafter called Pfam consensus) and the alignment of various sequences to the Pfam consensus sequence. The arrows indicate the active site residues, the underlined boxes indicate three of the homology boxes indicated by [Upton C and Buckley JT (1995) Trends Biochem Sci 20; 179-179]. Capital letters in the Pfam consensus indicate conserved residues in many family members. The - symbol indicates a position where the hidden Markov model of the Pfam consensus expected to find a residue but did not, so a gap is inserted. The . symbol indicates a residue without a corresponding residue in the Pfam consensus. The sequences are the amino acid sequences listed in Figures 2, 16, 18, 20, 26, 28 and 30. All these proteins were found to be active against lipid substrates.

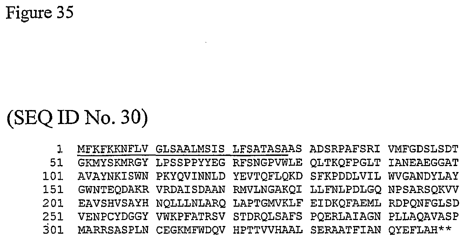

Figure 35 shows an amino acid sequence (SEQ ID No. 30) of the fusion construct used for mutagenesis of the *Aeromonas hydrophila* lipid acyltransferase gene in Example 7. The underlined amino acids is a xylanase signal peptide;

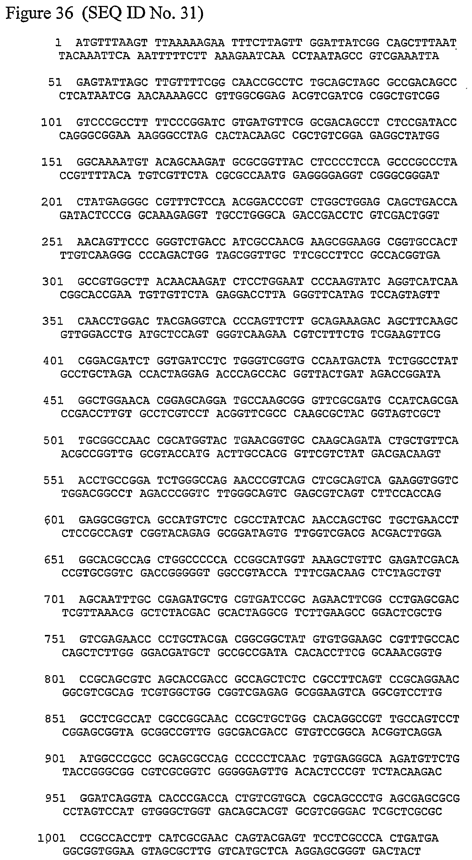

Figure 36 shows a nucleotide sequence (SEQ ID No. 31) encoding a lipid acyltransferase enzyme from *Aeromonas hydrophila* including a xylanase signal peptide;

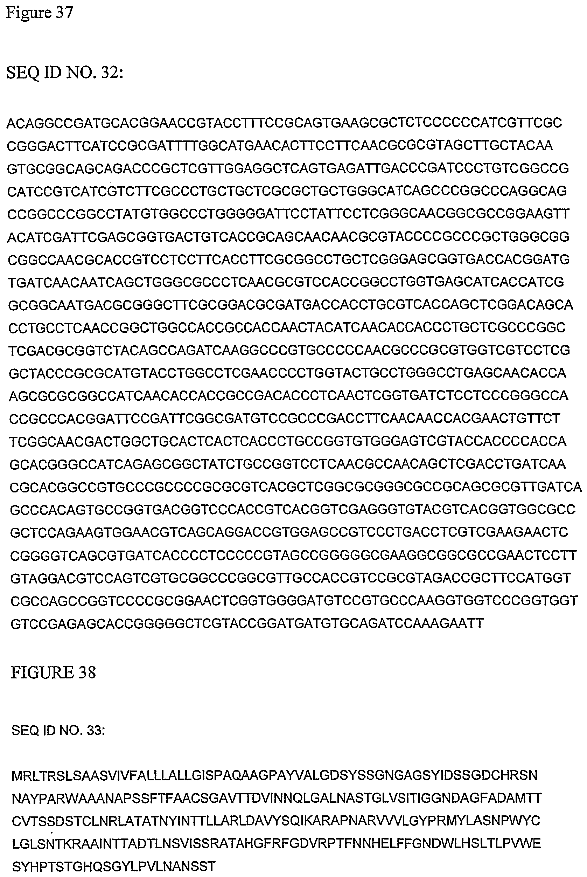

Figure 37 shows a nucleotide sequence encoding a lipid acyltransferase enzyme from *Streptomyces* (SEQ ID No. 32);

Figure 38 shows a polypeptide sequence of a lipid acyltransferase enzyme from *Streptomyces* (SEQ ID No. 33);

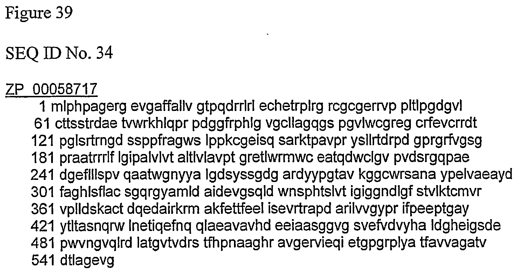

Figure 39 shows a polypeptide sequence of a lipid acyltransferase enzyme from *Termobifida_*(SEQ ID No. 34);

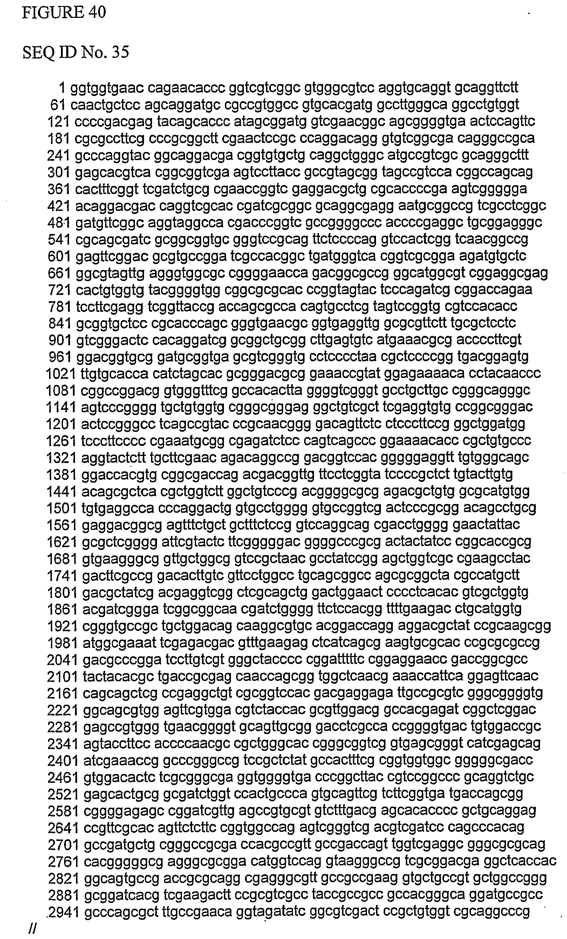

Figure 40 shows a nucleotide sequence encoding a lipid acyltransferase enzyme from *Termobifida_*(SEQ ID No. 35);

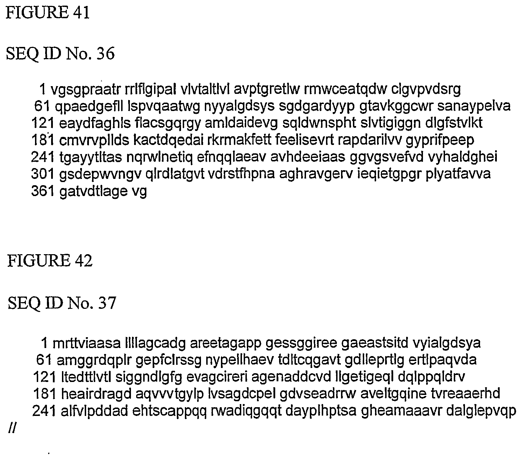

Figure 41 shows a polypeptide sequence of a lipid acyltransferase enzyme from *Termobifida_(SEQ* ID No. 36);

Figure 42 shows a polypeptide of a lipid acyltransferase enzyme from *Corynebacterium\effciens*\ GDSx 300 aa_(SEQ ID No. 37);

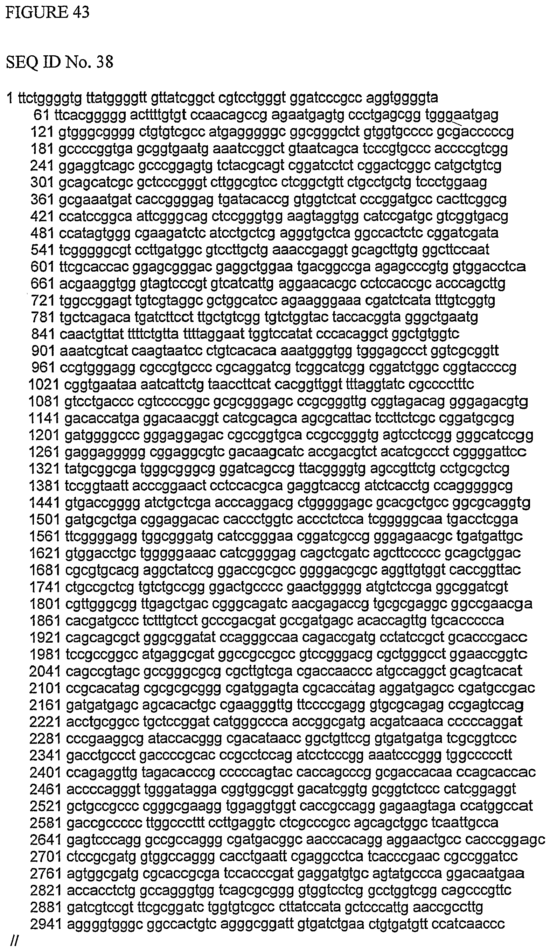

Figure 43 shows a nucleotide sequence encoding a lipid acyltransferase enzyme from *Corynebacterium\effciens*\ GDSx 300 aa_(SEQ ID No. 38);

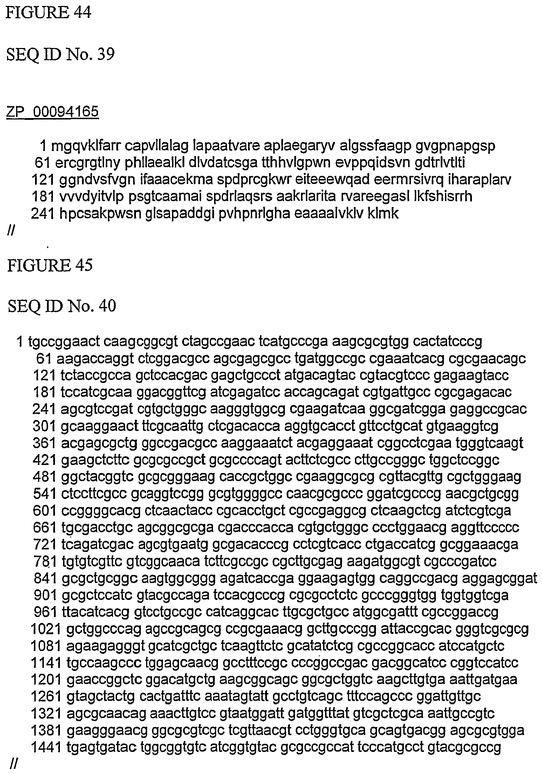

Figure 44 shows a polypeptide of a lipid acyltransferase enzyme from *Novosphingobium\aromaticivorans*\ GDSx 284 aa_(SEQ ID No. 39);

Figure 45 shows a nucleotide sequence encoding a lipid acyltransferase enzyme from *Novosphingobium\aromaticivorans*\ GDSx 284 aa (SEQ ID No. 40);

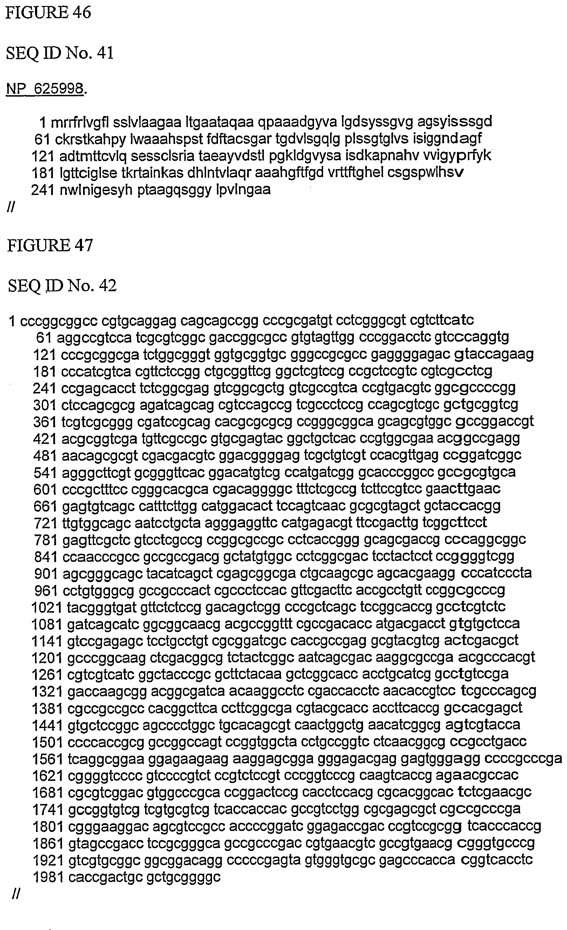

Figure 46 shows a polypeptide of a lipid acyltransferase enzyme from *Streptomyces coelicolor*\ GDSx 268 aa (SEQ ID No. 41);

Figure 47 shows a nucleotide sequence encoding a lipid acyltransferase enzyme from *Streptomyces coelicolor*\ GDSx 268 aa (SEQ ID No. 42);

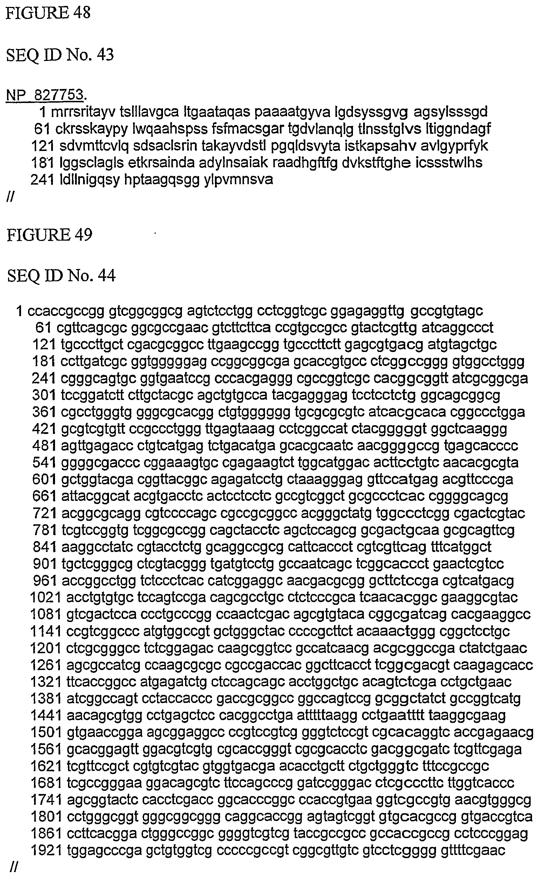

Figure 48 shows a polypeptide of a lipid acyltransferase enzyme from *Streptomyces avermitilis*\GDSx 269 aa (SEQ ID No. 43);

Figure 49 shows a nucleotide sequence encoding a lipid acyltransferase enzyme from *Streptomyces avermitilis*\GDSx 269 aa (SEQ ID No. 44);

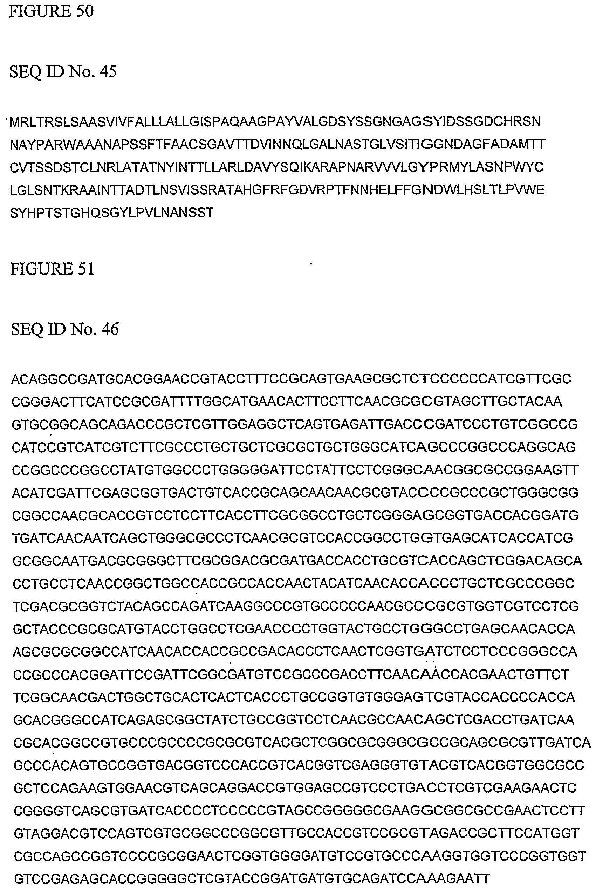

Figure 50 shows a polypeptide of a lipid acyltransferase enzyme from *Streptomyces* (SEQ ID No. 45);

Figure 51 shows a nucleotide sequence encoding a lipid acyltransferase enzyme from *Streptomyces* (SEQ ID No. 46);

Figure 52 shows a ribbon representation of the 1IVN.PDB crystal structure which has glycerol in the active site. The Figure was made using the Deep View Swiss-PDB viewer;

Figure 53 shows 1IVN.PDB Crystal Structure - Side View using Deep View Swiss-PDB viewer, with glycerol in active site - residues within 10Å of active site glycerol are coloured black;

Figure 54 shows 1IVN.PDB Crystal Structure - Top View using Deep View Swiss-PDB viewer, with glycerol in active site - residues within 10Å of active site glycerol are coloured black;

Figure 55 shows alignment 1;

Figure 56 shows alignment 2;

Figures 57 and 58 show an alignment of 1IVN to P10480 (P10480 is the database sequence for *A. hydrophila* enzyme), this alignment was obtained from the PFAM database and used in the model building process;

Figure 59 shows an alignment where P10480 is the database sequence for *Aeromonas hydrophila.* This sequence is used for the model construction and the site selection. Note that the full protein is depicted, the mature protein (equivalent to SEQ ID No. 2) starts at residue 19. A. sal is *Aeromonas salmonicida* (SEQ ID No. 28) GDSX lipase, A. hyd is *Aeromonas hydrophila* (SEQ ID No. 26) GDSX lipase. The consensus sequence contains a * at the position of a difference between the listed sequences;

Figure 60 shows a typical set of 384 clones, the wild type control lies at the intersection of 0.9PC, 0.8DGDG; and

Figure 61 shows three areas of interest. Section 1 contains mutants with an increased ratio R but lower activity towards DGDG. Region 2 contains mutants with an increased ratio R and an increased DGDG activity. Region 3 contains clones with an increased PC or DGDG activity, but no increase in the ratio R.

EXAMPLE 1

Modelling of *Aeromonas hydrophila* GDSx lipase on 1IVN

The alignment of the *Aeromonas hydrophila* GDSX lipase amino acid sequence (P10480) to the *Escherichia coli* Tioesterase amino acid sequence (1IVN) and the *Aspergillus aculeatus* rhamnogalacturonan acetylesterase amino acid sequence (1DEO) was obtained from the PFAM database in FASTA format. The alignment of P10480 and 1IVN was fed into an automated 3D structure modeller (SWISS-MODELLER server at www.expasy.org) together with the 1IVN.PDB crystal structure coordinates file FIGURE 52). The obtained model for P10480 was structurally aligned to the crystal structures coordinates of 1IVN.PDB and 1DEO.PDB using the 'Deep View Swiss-PDB viewer' (obtained from www.expasv.org/spdbv/) (FIGURE 53). The amino acid alignment obtained from the PFAM database (alignment 1 - (FIGURE 55)) was modified based on the structural alignment of 1DEO.PDB and 1IVN.PDB. This alternative amino acid alignment is called alignment 2 (FIGURE 56).

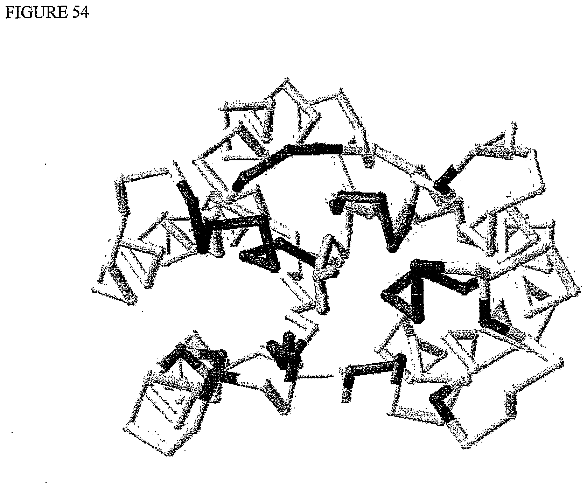

The 1IVN.PDB structure contains a glycerol molecule. This molecule is considered to be in the active site because it is in the vicinity of the catalytic residues. Therefore, a selection can be made of residues that are close to the active site which, due to their vicinity, are likely to have an influence on substrate binding, product release, and/or catalysis. In the 1IVN.PDB structure, all amino acids within a 10 Å sphere centered on the central carbon atom of the glycerol molecule in the active site were selected (amino acid set 1) (See Figure 53 and Figure 54).

The following amino acids were selected from the P10480 sequence; (1) all amino acids in P10480 corresponding to the amino acid set 1 in alignment 1; (2) all amino acids in P10480 corresponding to the amino acid set 1 in alignment 2; (3) from the overlay of the P10480 model and 1IVN all amino acids in the P10480 model within 12Å from the glycerol molecule in 1IVN. All three groups combined give amino acid set 2.

Sequence P10480 was aligned to "AAG09804.1 GI:9964017 glycerophospholipid-cholesterol acyltransferase [Aeromonas salmonicida]" and the residues in AAG09804 corresponding to amino acid set 2 were selected to give amino acid set 3.

Set 1, 2, and 3

Amino acid set 1:

Amino acid set 1 (note that these are amino acids in 1IVN - Figure 57 and Figure 58.) Gly8, Asp9, Leu11, Ser12, Tyr15, Gly44, Asp45, Thr46, Glu69, Leu70, Gly71, Gly72, Asn73, Asp74, Gly75, Leu76, Gln106, Ile107, Arg108, Leu109, Pro110, Tyr113, Phe121, Phe139, Phe140, Met141, Tyr145, Met151, Asp154, His157, Gly155, Ile156, Pro158

The highly conserved motifs, such as GDSx and catalytic residues, were deselected from set 1 (residues underlined). For the avoidance of doubt, set 1 defines the amino acid residues within 10Å of the central carbon atom of a glycerol in the active site of the 1IVN model.

Amino acid set 2:

Amino acid set 2 (note that the numbering of the amino acids refers to the amino acids in the P10480 mature sequence)

Leu17, Lys22, Met23, Gly40, Asn80, Pro81, Lys82, Asn87, Asn88, Trp111, Val112, Ala114, Tyr117, Leu118, Pro156, Gly159, Gln160, Asn161, Pro162, Ser163, Alga164, Arg165, Ser166, Gln167, Lys168, Val169, Val170, Glu171, Ala172, Tyr179, His180, Asn181, Met209, Leu210, Art211, Asn215, Lys284, Met285, Gln289 and Val290.

Table of selected residues in Set 1 compared with Set 2:

| IVN model<br>IVN | A.hyd homologue | | P10480<br>Mature sequence residue Number |
|---|---|---|---|
| | PFAM | Structure | |
| Gly8 | Gly32 | | |
| Asp9 | Asp33 | | |
| Ser10 | Ser34 | | |
| Leu11 | Leu35 | | Leu17 |
| Ser12 | Ser36 | | Ser18 |
| | | | Lys22 |
| | | | Met23 |
| Tyr15 | Gly58 | | Gly40 |
| Gly44 | Asn98 | | Asn80 |
| Asp45 | Pro99 | | Pro81 |
| Thr46 | Lys100 | | Lys82 |
| | | | Asn87 |
| | | | Asn88 |
| Glu69 | Trip129 | | Trp111 |
| Leu70 | Val130 | | Val112 |
| Gly71 | Gly131 | | |
| Gly72 | Ala132 | | Ala114 |
| Asn73 | Asn133 | | |
| Asp74 | Asp134 | | |
| Gly75 | Tyr135 | | Tyr117 |
| Leu76 | Leu136 | | Leu118 |
| Gln106 | | Pro174 | Pro156 |
| Ile107 | | Gly177 | Gly159 |
| Arg108 | | Gln178 | Gln160 |
| Leu109 | | Asn179 | Asn161 |
| Pro110 | | 180 to 190 | Pro162 |
| Tyr113 | | | Ser163 |
| | | | Ala164 |
| | | | Arg165 |
| | | | Ser166 |
| | | | Gln167 |
| | | | Lys168 |
| | | | Val169 |
| | | | Val170 |
| | | | Glu171 |

(continued)

| IVN model | | | P10480 |
|---|---|---|---|
| IVN | A.hyd homologue | | Mature sequence residue Number |
| | PFAM | Structure | |
| | | | Ala172 |
| Phe121 | His198 | Tyr197 | Tyr179 |
| | | His198 | His180 |
| | | Asn199 | Asn181 |
| Phe139 | Met227 | | Met209 |
| Phe140 | Leu228 | | Leu210 |
| Met141 | Arg229 | | Arg211 |
| Tyr145 | Asn233 | | Asn215 |
| | | | Lys284 |
| Met151 Asp154 | Met303 Asp306 | | Met285 |
| Gly155 | Gln307 | | Gln289 |
| Ile156 | Val308 | | Val290 |
| His 157 | His309 | | |
| Pry158 | Pro310 | | |

Amino acid set 3:

Amino acid set 3 is identical to set 2 but refers to the *Aenomonas salmonicida* (SEQ ID No. 28) coding sequence, i.e. the amino acid residue numbers are 18 higher in set 3 as this reflects the difference between the amino acid numbering in the mature protein (SEQ ID No. 2) compared with the protein including a signal sequence (SEQ ID No. 28).

The mature proteins of *Aeromonas salmonicida* GDSX (SEQ ID No. 28) and *Aeromonas hydrophila* GDSX (SEQ ID No. 26) differ in five amino acids. These are Thr3Ser, Gln182Lys, Glu309Ala, Ser310Asn, Gly318-, where the *salmonicida* residue is listed first and the *hydrophila* residue is listed last (FIGURE 59). The *hydrophila* protein is only 317 amino acids long and lacks a residue in position 318. The *Aeromonas salmonicidae* GDSX has considerably high activity on polar lipids such as galactolipid substrates than the *Aeromonas hydrophila* protein. Site scanning was performed on all five amino acid positions.

Amino acid set 4:

Amino acid set 4 is S3, Q182, E309, S310, and -318.

Amino acid set 5:

F13S, D15N, S18G, S18V, Y30F, D116N, D116E, D157 N, Y226F, D228N Y230F.

Amino acid set 6:

Amino acid set 6 is Ser3, Leu17, Lys22, Met23, Gly40, Asn80, Pro81, Lys82, Asn 87, Asn88, Trp111, Val112, Ala114, Tyr117, Leu118, Pro156, Gly159, Gln160, Asn161, Pro162, Ser163, Ala164, Arg165, Ser166, Gln167, Lys168, Val169, Va1170, Glu171, Ala172, Tyr179, His180, Asn181, Gln182, Met209, Leu210, Arg211, Asn215, Lys284, Met285, Gln289, Val290, Glu309, Ser310, -318.

The numbering of the amino acids in set 6 refers to the amino acids residues in P10480 (SEQ ID No. 2) - corresponding amino acids in other sequence backbones can be determined by homology alignment and/or structural alignment to P10480 and/or 1IVN.

Amino acid set 7:

Amino acid set 7 is Ser3, Leu17, Lys22, Met23, Gly40, Asn80, Pro81, Lys82, Asn 87, Asn88, Trp111, Val112, Ala114, Tyr117, Leu118, Pro156, Gly159, Gln160, Asn161, Pro162, Ser163, Ala164, Arg165, Ser166, Gln167, Lys168, Val169, Val170, Glu171, Ala172, Tyr179, His180, Asn181, Gln182, Met209, Leu210, Arg211, Asn215, Lys284, Met285, Gln289, Val290, Glu309, Ser310, -318, Y30X (where X is selected from A, C, D, E, G, H, I, K, L, M, N, P, Q, R, S, T, V, or W), Y226X (where X is selected from A, C, D, E, G, H, I, K, L, M, N, P, Q, R, S, T, V, or W), Y230X (where X is selected from A, C, D, E, G, H, I, K, L, M, N, P, Q, R, S, T, V, or W), S18X (where X is selected from A, C, D, E, F, H, I, K, L, M, N, P, Q, R, T, W or Y), D157X (where X is selected from A, C, E, F, G, H, I, K, L, M, P, Q, R, S, T, V, W or Y).

The numbering of the amino acids in set 7 refers to the amino acids residues in P10480 (SEQ ID No. 2) - corresponding amino acids in other sequence backbones can be determined by homology alignment and/or structural alignment to P10480 and/or 1IVN).

From the crystal structure one can obtain the secondary structure classification. That means, one can classify each amino acid as being part of an alpha-helix or a beta-sheet. Figure 57 shows the PFAM alignment of 1DEO, 1IVN, and P10480 (the database *Aeromonas hydrophila*). Added below each line of sequence is the structural classification.

The PFAM database contains alignments of proteins with low sequence identity. Therefore, these alignments are not very good. Although the alignment algorithms (HAMMER profiles) are well suited for recognizing conserved motifs the algorithm is not very good on a detailed level. Therefore it is not surprising to find a disparity between the PFAM alignment and a structural alignment. As a skilled person would be readily aware, one can modify the PFAM alignment based on the structural data. Meaning that one can align those structural elements that overlap.

FIGURE 55 shows the original PFAM alignment of 1DEO, 1IVN and P10480. Added to the alignment is the secondary structure information from the crystal structures of 1DEO and 1IVN. Alignment 2 in FIGURE 56 shows a manually modified alignment where the match between the secondary structure elements is improved. Based on conserved residues between either 1DEO and P10480 or between 1IVN and P10480 the alignment was modified for P10480 as well. To easily distinguish the sequence blocks the sequence identifiers in alignment 2 have an extra m (IDEOm, 1IVNm, P10480m).

Alignment 3 is a mix of 1 and 2, it gives the alignment per block.

EXAMPLE 2: Construction of site scan libraries

The Quick Change Multi Site-Directed Mutagenesis Kit from Stratagene was used according to the manufacturers instruction. For each library a degenerate primer with one NNK or NNS (nucleotide abbreviations) codon was designed. Primer design was performed using the tools available on the Stratagene web site. Primer quality control was further confirmed using standard analysis tools which analyze the primer for the potential of forming hairpins or of forming primer-dimers.

The main concepts of the method are as follows; using a non-strand displacing high-fidelity DNA polymerase such as Pfu-Turbo and a single primer one will linearly amplify the DNA template. This is in contrast to the normal exponential amplification process of a PCR reaction. This linear amplification process ensures a low error frequency. The product is single stranded non-methylated DNA and double stranded hemi-methylated DNA. If the template is obtained from a suitable host organism, then the template is double stranded methylated DNA. This means that the template DNA can be digested with Dpn I endonuclease without digesting the product DNA. Therefore upon transformation of the DNA into a suitable host only a very low frequency of the transformants with non-mutagenized plasmid.

EXAMPLE 3: Selection of winners from a site scan library

Two alternative approaches are described; library sequencing followed by analysis of unique amino acids, or library analysis followed by sequencing of the winners.

Selection of winners method 1; library sequencing followed by analysis of unique amino acids.

The transformation/expression shuttle vector used for generation of the site scanning libraries/variants in *E. coli* and expression of the variants in *B. subtilis* was derived from pDP66S, Penninaga et al., (Biochemistry (1995), 3368-3376), by replacement of the selection cassette to a kanamycin selection cassette. The vector used to insert the acyl-transferase variant gene in place of the cgt *gene* down-stream of the P32 promoter. The vector uses the P32 promoter to drive expression of the acyl-transferase variant gene in *B. subtilis.*

The expression vector was transformed into nprE-, aprA- *Bacillus subtillis* DB104 (Kawamura and Doi, J. of Bacteriology Oct 1984, p442-444) using transformation methods, as described in Chapter 3, Molecular Biological Methods for Bacillus (Ed. C.R. Harwood and S.M. Cutting), 1990. John Wiley & Sons Ltd. Chichester, UK).

Site scan libraries were constructed using a degenerate oligo containing one NNK codon, where K stands for G or T and N stands for A, C, G, or T. This means that a set of clones constructed from an amplification reaction using an NNK primer (also known as a site scan library') contains in principle 32 unique codons (4x4x2 = 32 combination options). Assuming no bias due, the number of clones that one needs to pick to have a 95% chance of picking every one of the 32 codons at least once is 95. This can be calculated using the following formula

$$\text{Formula 1;}\ n = \{ \log (1\text{-}c) \} / \{ \log (1\text{-}f) \}$$

Where n is the number of clones, c is the fraction value of the confidence interval, for example the 95% confidence interval has a value of 0.95 and the 99% confidence interval has a fraction value of 0.99, and f is the frequency with which each individual codon occurs, which for an NNK primer is 1/32 or 0.03125. Solving the formula for n gives 94.36 or 95 clones. If a 95% confidence interval is deemed to be too low, or if one is unable to avoid bias in one or more steps of the library construction process, one can decide to assay or sequence more clones. For example, in formula 1, if n is set to 384, f to 1/32 or 0.03125 then the confidence interval c is much larger than 99%. Even if 60% of the clones contain the same mutation or the wild type codon, then 363 clones will give a 99% confidence of obtaining all 32 codons. From this one can conclude that, 384 clones will have a 99% confidence of containing each of the 32 codons at least once.

A colony PCR was performed (a PCR reaction on a bacterial colony or on a bacterial liquid culture to amplify a fragment from a plasmid inside a bacterium, and subsequently sequencing that part of the fragment which has been mutagenised is an established procedure. Colony PCR can be routinely performed for sets of 96 due to the availability of prefabricated material (also known as kits) for colony PCR, sequencing, and sequence purification. This entire procedure is offered as a service by several commercial companies such as AGOWA GmbH, Glienicker weg 185, D-12489 Berlin, Germany.

After analysing the 96 sequence reactions, the individual clones were selected representing one for each codon that is available in the set of 96 sequences. Subsequently, for each of the clones representing the mutants, 5 ml of LB broth (Casein enzymatic digest, 10 g/l; low-sodium Yeast extract, 5 g/l; Sodium Chloride, 5 g/l; Inert tableting aids, 2 g/l) supplemented with 50 mg/l kanamycin, was inoculated and incubated at 33 °C for 6 hours at 205 rpm. 0.7 ml of this culture was used to inoculate 50 ml of SAS substrate ($K_2HPO_4$, 10 g/l; MOPS (3-morpholinopropane sulfonic acid), 40 g/l; Sodium Chloride, 5 g/l; Antifoam (Sin 260), 5 drops/l; Soy flour degreased, 20 g/l; Biospringer 106 (100 % dw YE), 20 g/l) supplemented with 50 mg/l kanamycin and a solution of high maltose starch hydrolysates (60 g/l). Incubation was continued for 40 hours at 33 °C and 180 rpm before the culture supernatant was separated by centrifugation at 19000 rpm for 30 min. The supernatant was transferred into a clean tube and directly used for the assay.

Selection of winners method 2; library screening followed by sequencing of the winners

Although one could choose to sequence 384 clones, one may also assay them and select improved variants before sequencing.

A number of issues should be considered when such a number of samples are screened. Without being exhaustive, although it is possible to select variants with altered activity on one substrate, the difference in expression level between 384 cultures can be substantial even if one uses a 384 well microtiter plate, resulting in a high background. Therefore, measuring two activities and selecting winners based on a change in ratio is a preferred method. To illustrate, if two activities have a certain ratio R then regardless of the absolute amount of enzyme present, the ratio between the two activities will always be R. A change in the R value indicates a mutation that changed one activity relative to the second activity.

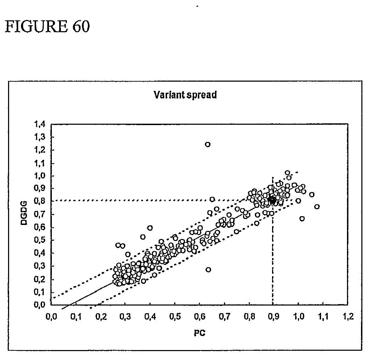

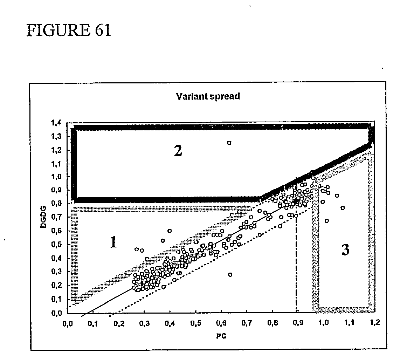

Figure 60 shows a data set obtained from the site scan library. The clones are all tested for activity towards phosphatidyl choline (PC) and digalactosyl diglyceride (DGDG). All clones, which can be mutated or not, that exhibit no change in the R value will lie on a straight line with a certain margin of error. Disregarding these clones three groups of interest appear in Figure 61.

Section 1 in Figure 61 contains all the clones that have a significantly higher R than the wild-type (not mutated) but lower overall DGDG activity. Section 2 contains those clones that have both a higher R value and a higher DGDG activity than the wild type. Section 3 contains clones that do not have a higher R value, but that do have a significantly higher DGDG or PC activity.

If one is interested in variants with an increased activity towards DGDG then section 2 contains the most interesting variants and section 3 contains variants of interest as well. The variants in Section 3 which show a large increase in hydrolytic activity may be accompanied by a decrease in transferase activity.

One thing is worth noticing, if a specificity determining residue is hit, most of the 20 possible amino acids could yield a very different R value. However, if the library contains a large bias towards a single amino acid (for example 60% is Tyrosine) then all those variants will still lie on a straight line.

<u>EXAMPLE 4 : Assays for PC and DGDG activity in a 384 well microtiter plate</u>

Start material

- EM media

- Plate with transformants
- Plate with wild type
- 384 plates
- colony picker
- Waco NEFA-C kit
- PC and DGDG solutions in a 384 plate

Part 1 - picking colonies

- Pick colonies into a 384 plate filled with EM medium
- Skip 4 wells and inoculate those with colonies containing the non-mutated backbone
- Grow o/n at 30°C, 200 rpm shaking speed

Part 2 - Incubation on substrate

- Centrifuge the o/n grown plates; 2500 rpm, 20 min
- Transfer 10 µl supernatant from each well to 2 empty 384 plates
- Add 5 µl 12.5 mM DGDG to one of the plates, add 5 µl 12.5 mM PC to the other plate
- Incubate both plates 2 hrs at 37°C, shake at start to mix then stop the shaking
- Continue with the NEFA C procedure

Part 3 - NEFA-C procedure

- Add 10 µl A solution
- Incubate 10 min 37°C, 300 rpm
- Add 20 µl B solution
- Incubate 10 min 37°C, 300 rpm
- Read the plate at 550 nm

Substrate composition - in mM

25 mM PC eller DGDG
10 mM $CaCl_2$
60 mM Triton X 100
15 mM $NaN_3$
20 mM Briton Robinson pH 5.0

EXAMPLE 5 Selected variants

Determination of enzyme activity

To determine the enzymatic activity towards various substrates 4 µl enzyme solution was incubated with 11 µl substrate for 60 minutes at 37°C. Subsequently the amount of free fatty acids was determined using the WACO NEFA-C kit. To the 15 µl enzyme+substrate mix 75 µl NEFA solution A was added and incubated for 15 minutes at 37°C. Subsequently 150 µl NEFA solution B was added and incubated for 15 minutes. Subsequently the optical density (OD) of the sample was measured at 550 nm.

As a control, from each variant 4 µl enzyme solution was incubated with 11 µl HEPES buffer for 60 min at 37°C. Subsequently the amount of free fatty acids was determined as described above. The OD values of this control sample was deducted from the observed OD on each substrate to obtain a corrected activity.

Four different substrates were used, the composition was in general 30 mg lipid, 4.75 ml 50 mM HEPES buffer pH 7, 42.5 µl 0.6 M CaCl2, 200 µl 10% Triton X-100 H2O2-free. The 30 mg lipid was either phosphatidyl choline (PC), PC with cholesterol in a 9 to 1 ratio, digalactosyl diglyceride (DGDG), or DGDG with cholesterol in a 9 to 1 ratio.

Selection of improved variants

Variants with improved activity towards PC

Those variants that showed an increase in the OD relative to the wild type enzyme when incubated on PC were selected as variants with improved phospholipase activity.

Variants with improved activity towards DGDG

Those variants that showed an increase in the OD relative to the wild type enzyme when incubated on DGDG were selected as variants with improved activity towards DGDG.

Variants with improved specificity towards DGDG

The specificity towards DGDG is the ratio between the activity towards DGDG and the activity towards phosphatidylcholine (PC). Those variants that showed a higher ratio between DGDG and PC than the wild type were selected as variants with improved specificity towards DGDG.

Variants with improved transferase activity with PC as the acyl donor

The difference in the amount of free fatty acids formed when one incubates an enzyme on PC and on PC with cholesterol is an indication of the amount of transferase activity relative to the amount of hydrolytic activity. Transferase activity will not cause the formation of free fatty acids. The transferase preference is the ratio between the free fatty acids formed when PC is used as a substrate and the free fatty acids formed when PC with cholesterol is used as a substrate. Those variants that show an increase in the transferase preference and show a higher than wild type activity towards PC were selected as having improved transferase activity.

Variants with improved transferase activity with DGDG as the acyl donor

The difference in the amount of free fatty acids formed when one incubates an enzyme on DGDG and on DGDG with cholesterol is an indication of the amount of transferase activity relative to the amount of hydrolytic activity. Transferase activity will not cause the formation of free fatty acids. The transferase preference is the ratio between the free fatty acids formed when DGDG is used as a substrate and the free fatty acids formed when DGDG with cholesterol is used as a substrate. Those variants that show an increase in the transferase preference and show a higher than wild type activity towards DGDG were selected as having improved transferase activity.

Selected variants

For each of the four selection criteria above a number of variants were selected. The "wild type" enzyme in this example is *A. salmonicida* (SEQ ID No. 28). Variants with improved activity towards PC:

| | PC |
|---|---|
| Thr3Asn | 158,0 |
| Thr3Gln | 151,5 |
| Thr3Lys | 141,5 |
| Thr3Arg | 133,0 |
| Glu309Ala | 106,0 |
| Thr3Pro | 101,5 |
| Thr3Met | 96,0 |
| wild-type | 86,5 |

Variants with improved activity towards DGDG:

| | DGDG |
|---|---|
| Gln182asp | 66,5 |
| Glu309Ala | 60 |
| Tyr230Thr | 59 |
| Tyr230Gly | 57,5 |
| Tyr230Gly | 51 |
| Thr3Gln | 44,5 |
| wild-type | 43,5 |

Variants with improved specificity towards DGDG:

| | $R_{DGDG/PC}$ | PC | DGDG |
|---|---|---|---|
| Gln182Asp | 1,02 | 65,5 | 66,5 |
| Tyr230Gly | 0,79 | 72,5 | 57,5 |
| Tyr230Gly | 0,78 | 65,0 | 51,0 |
| Tyr230Thr | 0,75 | 78,5 | 59,0 |
| Tyr230Val | 0,71 | 58,0 | 41,0 |
| Asp157Cys | 0,69 | 48,0 | 33,0 |
| Glu309Pro | 0,58 | 73,5 | 42,5 |
| Glu309Ala | 0,57 | 106,0 | 60,0 |
| Gly318Ile | 0,53 | 69,5 | 36,5 |
| Tyr230Arg | 0,50 | 63,5 | 32,0 |
| Tyr230Met | 0,50 | 64,5 | 32,5 |
| wild-type | 0,50 | 86,5 | 43,5 |

Variants with improved transferase activity with PC as the acyl donor:

| | $R_{PC+Cho/PC}$ | PC | PC+Cho |
|---|---|---|---|
| Thr3Lys | 0,54 | 142 | 76 |
| Thr3Arg | 0,55 | 133 | 73 |
| Thr3Gln | 0,63 | 152 | 96 |
| Thr3Asn | 0,64 | 158 | 101 |
| Thr3Pro | 0,67 | 102 | 68 |
| Thr3Met | 0,78 | 96 | 75 |
| wild-type | 0,83 | 87 | 72 |

Variants with improved transferase activity with DGDG as the acyl donor:

| | $R_{DGDG+Cho/DG\ DG}$ | DGDG |
|---|---|---|
| Tyr230Thr | 1,10 | 59 |
| Gln182Asp | 1,39 | 67 |

(continued)

| | $R_{DGDG+Cho/DG\ DG}$ | DGDG |
|---|---|---|
| Tyr230Gly | 1,55 | 58 |
| Glu309Ala | 1,78 | 60 |
| wild-type | 1,78 | 44 |

EXAMPLE 6: Transferase assay Phospholipid:cholesterol

Phospholipid can be replaced by DGDG to provide a transferase assay from a galacolipid. Other acceptors for example, glycerol, glucose, hydroxy acids, proteins or maltose can also be used in the same assay.

300 mg Phosphatidylcholine (Avanti #441601):Cholesterol(Sigma C8503) 9:1 is scaled in a Wheaton glass. 10 ml 50 mM HEPES buffer pH 7.0 is added and stirring at 40 °C disperses the substrate
0,5 ml substrate is transferred to a 4 ml vial and placed in a heating block at 40 °C. 0.050 ml transferase solution is added, also a control with 0.050 ml water is analysed in the same way. The reaction mixture is agitated for 4 hours at 40 °C. The sample is then frozen and lyophilised and analysed by GLC.

Calculation:

From the GLC analysis the content of free fatty acids and cholesterol ester is calculated.

The enzymatic activity is calculated as:

$$\%\ \text{Transferase activity} = \frac{\Delta\ \%\ \text{cholesterol ester}/(\text{Mv sterol ester}) \times 100}{\Delta\ \%\ \text{cholesterol ester}/(\text{Mv cholesterol ester}) + \Delta\ \%\ \text{fatty acid}/(\text{Mv fatty acid})}.$$

$$\%\ \text{Hydrolyse activity} = \frac{\Delta\ \%\ \text{fatty acid}/(\text{Mv fatty acid}) \times 100}{\Delta\ \%\ \text{cholesterol ester}/(\text{Mv cholesterol ester}) + \Delta\ \%\ \text{fatty acid}/(\text{Mv fatty acid})}.$$

$$\text{Ratio Transferase/Hydrolyse} = \%\ \text{transferase activity}/\%\ \text{Hydrolyse activity}$$

Where:

$$\Delta\ \%\ \text{cholesterol ester} = \%\ \text{cholesterol ester(sample)} - \%\ \text{cholesterol ester(control)}.$$

$$\Delta\ \%\ \text{fatty acid} = \%\ \text{fatty acid(sample)} - \%\ \text{fatty acid(control)}.$$

Transferase assay Galactolipid:cholesterol.

300 mg Digalactosyldiglyceride (DGDG) (purity >95 galactolipids, the DGDG used is purified from wheat lipid. DGDG from Sigma D4651 is also suitable for use):Cholesterol(Sigma) 9:1 is scaled in a Wheaton glass. 10 ml 50 mM HEPES buffer pH 7.0 is added and stirring at 40 °C disperses the substrate.
0,5 ml substrate is transferred to a 4 ml vial and placed in a heating block at 40 °C. 0.050 ml transferase solution is added, also a control with 0.050 ml water is analysed in the same way. The reaction mixture is agitated for 4 hours at 40 °C. The sample is then frozen and lyophilised and analysed by GLC.

Calculation:

From the GLC analysis the content of free fatty acids and cholesterol ester is calculated.
The enzymatic activity is calculated as:

$$\% \text{ Transferase activity} = \frac{\Delta \% \text{ cholesterol ester/(Mv sterol ester) x 100}}{\Delta \% \text{ cholesterol ester/(Mv cholesterol ester)} + \Delta \% \text{ fatty acid/(Mv fatty acid)}}$$

$$\% \text{ Hydrolyse activity} = \frac{\Delta \% \text{ fatty acid/(Mv fatty acid) x 100}}{\Delta \% \text{ cholesterol ester/(Mv cholesterol ester)} + \Delta \% \text{ fatty acid/(Mv fatty acid)}}$$

$$\text{Ratio Transferase/Hydrolyse} = \% \text{ transferase activity}/\% \text{ Hydrolyse activity}$$

Where:

$$\Delta \% \text{ cholesterol ester} = \% \text{ cholesterol ester(sample)} - \% \text{ cholesterol ester(control)}.$$

$$\Delta \% \text{ fatty acid} = \% \text{ fatty acid(sample)} - \% \text{ fatty acid(control)}$$

EXAMPLE 7: Variants of a lipid acyltransferase for *Aeromonas hydrophila* (SEQ ID No. 26)

Mutations were introduced using the QuikChange™ Multi-Site Directed Mutagenesis kit from Stratagene, La Jolla, CA92037, USA following the instructions provided by Stratagene.
Variants at Tyr256 showed an increased activity towards phospholipids.
Variants at Tyr256 and Tyr260 showed an increased activity towards galactolipids.
Variants at Tyr265 showed an increased transferase activity with galactolipids as the acyl donor.
The numbers indicate positions on the following sequence: An enzyme from *Aenomonas hydrophila* the amino acid sequence of which is shown as SEQ ID No. 26. The nucleotide sequence is as shown as SEQ ID No. 27.

EXAMPLE 8: Screening of mutants of glycerophospholipid:cholesterol acyltransferase GCAT from *Aeromonas salmonicida.*

Mutants from point mutations of glycerophospholipid:cholesterol acyltransferase GCAT from *Aeromonas salmonicida* were screened for transferase activity using phosphatidylcholine or digalactosyldiglyceride as donor and cholesterol as acceptor with the aim to select mutant with better activity towards digalactocyldiglyceride than phosphatidylcholine.
GCAT mutants were screened for transferase activity using digalactosyldiglyceride(DG) and phosphatidylcholine(PC) as donor and cholesterol as acceptor.
DG (purity >95% digalactosyldiglyceride (DGDG used is purified from wheat lipid. DGDG from Sigma D4651 is also suitable for use from Signma D4651),) and cholesterol (Sigma C8503) was scaled in the ratio 9 :1 and dissolved in chloroform and evaporated to dryness.
The substate was prepared by dispersing of 3% DG:Cholesterol 9:1 in 50 mM HEPES buffer pH 7.
0,250 ml substrate was transferred to a 3 ml glass with screw lid. 0,025 ml supernatant from fermentation of mutant GCAT was added an incubated at 40 °C for 2 hours. A reference sample with water instead of enzyme was also prepared. Heating the reaction mixture in a boiling water bath for 10 minutes stopped the enzyme reaction.
2 ml 99% ethanol was added and submitted to cholesterol analysis as well as free fatty acid analysis.

Cholesterol assay.

100 µl substrate containing:1.4 U/ml Cholesterol oxidase(SERVA Electrophoresis GmbH cat. No 17109), 0,4 mg/ml ABTS (Sigma A-1888), 6 U/ml Peroxidase (Sigma 6782) in 0,1 M TRIS,HCl buffer pH 6.6 + 0,5% Triton X 100(Sigma X-100) was incubated at 37°C for 5 minutes. 5µl cholesterol sample was added and mixed. The reaction mixture was incubated for further 5 minutes and OD 405nm measured. The content of cholesterol was calculated from the analyses of standard solutions of cholesterol containing 0,4mg/ml, 0,3mg/ml, 0,2Omg/ml, 0,1mg/ml, 0,05 mg/ml and 0 mg/ml.

Free fatty acid assay.

Free fatty acids in the sample was measured using a NEFA C kit (WAKO Chemicals GmbH)
75 µl NEFA reagent A was incubated for 10 minutes at 37 °C. 15 µl enzyme sample was added and mixed. The reaction mixture was incubated for10 minutes. 150 µl NEFA reagent B was added, mixed and incubated for further 10 minutes and OD 540 nm was measured. Free fatty acid was calculated from standard solutions of 0,4, 0.3, 0.2, 0.1, 0.05 and 0 mM fatty acid.

Transferase assay using phosphatidylcholine as donor was measured in the same way, but using phosphatidylcholine(Avanti #441601) instead of DG (DGDG).

**Transferase activity was expressed as % cholesterol esterified** calculated from the difference in free cholesterol in the reference sample and free cholesterol in the enzyme sample.

**Hydrolytic activity was expressed as % free fatty acid produced** calculated from the difference in free fatty acid in the enzyme sample and free fatty acid in the reference sample.

The relative Transferase activity against DG and PC was calculated as % $T_{DG}/T_{PC}$. The transferase activity $T_{DG}$ relative to the hydrolytic activity $H_{DG}$ on DG for the mutants were calculated:

$$\frac{0.1 \text{ x } \%TDG/386}{\% HDG/280} = \frac{0.1 \text{ x } \%TDG \text{ x } 280}{\% HDG \text{ x } 386}$$

Where 386 = MW for cholesterol and 280 = MW for fatty acid.

$$\text{Mutants with } T_{DG} > 50\% \text{ and } T_{DG}/T_{PC} > 3 \text{ and } \frac{0.1 \text{ x } \%T_{DG}/386}{\% H_{DG}/280} > 2.5$$

were selected as improved mutants.

The data obtained from the above example can be analysed via statistics to identify and prioritise key sites and/or specific amino acid substitutions which provide the desired activity profile, such as increased ration of $T_{DG}$ as compared to $T_{PC}$. For example, the following robust modeling is proposed:

> The information regarding $T_{PC}$ and $T_{DG}$ is carried by the censored responses max(0, $T_{PC}$) and max(0, $T_{DG}$). The objective of the study is to identify settings determining $T_{DG}$ >=Tpc, based on the scores for ln(1+ $T_{DG}$)-ln(1+ $T_{PC}$) with positive values as preference, both in absolute scale and in relative scale compared to a control (native). The preferred settings are identified based on a binary response (Event, Non-Event), where Event is defined as a preferred response in relation to the scores. A binomial GLIM model with complementary log-log link, based on the empirical data structure without prior information included, analyses the binary responses. See the following reference for details of how to perform the statistical analysis: proc LOGISTIC in SAS Institute Inc., SAS/STAT® User's Guide, Version 6, 4.Ed, Vol.2, Cary, NC: SAS Institute Inc., 1989.

| Variants with increased $T_{DG}/T_{PC}$ | Variants with enhanced DGDG transferase activity $T_{DG}$ | Variants with enhanced $T_{DG}$/ $H_{DG}$ activity |
|---|---|---|
| | N80 P, G, or E | Y179 E, R, N, Q |
| K22 E, K | S310 Q, H or S | N215 G |
| G40 L | S3 E, A, G, K, M, Y, R, P, N, T, or Q | L210 D, H, R, E, A, Q, P, N, K, G, R, T, W, I, V or S |
| N87 R, D, E, M | | |
| Y117 A, N, E, H, T | -318 R, S, E, H, Q, N or D | N80G |
| | | Y30L |
| Q182K, T | N215 L, G, V, R or | N87G |
| M209 K, M | Y | H180 I, T |
| L210 N | K82 S | M209 Y |

(continued)

| Variants with increased $T_{DG}/T_{PC}$ | Variants with enhanced DGDG transferase activity $T_{DG}$ | Variants with enhanced $T_{DG}$/ $H_{DG}$ activity |
|---|---|---|
| R211 G<br>N215 H<br>Y230 I<br>-318 Y, H or S<br>N215 H<br>L210 D, Q or T<br>E 3095, Q or R<br>H180 K or Q<br>N 80 N, R or D<br>L210 G, I, H, E, M, S, W, V, A, R, N, S310A, P, T, H, M, K, or G<br>V112 C<br>Y30 G, I, L, S, E, M, A or R<br>V290 R, E, H or A<br>Q 289 R or N<br>K22E<br>G40 L<br>Y179 V<br>M209 L, K, M<br>R211 G, Q, K or D<br>Y230 V<br>S310 P<br>Y179 R<br>H180 T<br>Q289 T or D<br>G40 Q, L or V<br>N88 W<br>N87 R or D | | R211 D, T or G<br>S18 G, M or T<br>G40 R or M<br>N88 W<br>N87 C, D, R, E or G |

EXAMPLE 9: Selection of improved mutants of glycerophospholipid:cholesterol acyltransferase GCAT from *Aeromonas salmonicida.*

The "parent" enzyme in this example is *A. salmonicida* (SEQ ID No. 28).

32 positions of GCAT from *Aeromonas salmonicida* (230 Tyr, 182 Lys, 3 Thr, 157 Asp, 310 Thr, 318 Gly, 309 Glu, 17 Leu, 111 Trp, 117 Tyr, 179 Tyr, 118 Leu, 215 Asn, 22 Lys, 290 Val, 289 Gln, 285 Met, 18 Ser, 23 Met, 180 His, 284 Lys, 181 Asn, 209 Met., 210 Leu, 211 Arg, 40 Gly, 81 Pro,112 Val, 80 Asn, 82 Lys, 88 Asn, 87 Asn were screened according to the experimental outline in Example 8.

Based on the results from the screening and the three selection criteria the following mutants listed in the table 1 were selected.

Table 1

| Position | Amino acid | T,PC | T, DG | T,DG/T,PC | H, PC | H, DG |
|---|---|---|---|---|---|---|
| 210 | GLN | 10,3 | 59,7 | 5,9 | 0,0 | 0,0 |
| 215 | GLY | 15,1 | 55,8 | 4,5 | 3,1 | 1,0 |
| 215 | LEU | 19,4 | 51,9 | 3,3 | 4,3 | 1,1 |
| 215 | TYR | 21,3 | 68,0 | 3,9 | 4,3 | 1,8 |
| 215 | ARG | 16,2 | 62,1 | 4,7 | 5,5 | 2,1 |
| 215 | VAL | 14,7 | 61,6 | 5,2 | 3,5 | 1,7 |
| 215 | HIS | 5,7 | 50,1 | 10,9 | 4,2 | 1,3 |

(continued)

| Position | Amino acid | T,PC | T, DG | T,DG/T,PC | H, PC | H, DG |
|---|---|---|---|---|---|---|
| 215 | ASN | 9,4 | 47,4 | 6,2 | 4,0 | 1,2 |

EXAMPLE 10: Selection of specific amino acid regions of interest for mutation of the glycerophospholipid:cholesterol acyltransferase GCAT from *Aeromonas salmonicida.*

From the pfam alignment (alignment 2; FIGURE 56) and overlay of the P10480 model and 1IVN all amino acids in regions surrounding the glycerol molecule in the active site of 1IVN were selected and used for defining regions of specific interest (loops). (Numbers refer to the amino acids in the P10480 mature sequence (SEQ ID No. 2)):

Thr 20- Arg 41 (Loop 1, L1)
Ile 77- Leu 89 (Loop 2, L2)
Leu 118 - Asp 127 (Loop 3, L3)
Gly 146- Val 176 (Loop 4, L4)
Glu 208 - Trp 287 (Loop 5, L5)

The intervening regions (IVR) were named accordingly:

Ala 1 - Asp 19 (IVR1)

Phe 42 - Lys 76 (IVR2)

Asp 90 - Tyr 117 (IVR3)

Ala 128 - Asn 145 (IVR4)

Ser 177 - Ala 207 (IVR5)

Asp 288 - His 317 (IVR6)

The following table summarizes the allocation of preferred positions for mutation of the glycerophospholipid:cholesterol acyltransferase GCAT from *Aeromonas salmonicida.* The results are based on experimental outlines as set out in Example 8-10.

| | P10480 amino acid positions (SEQ ID No 2) | Preferable sites to produce variants with increased $T_{DG}/T_{PC}$ | 10 Å | Preferred regions for methods of the invention |
|---|---|---|---|---|
| IVR1 | 1-19 | | L17, S18 | |
| Loop1 | 20-41 | K22, G40, Y 30 | K22, M23, G40 | Loop1 |
| IVR2 | 42-76 | | | |
| Loop2 | 77-89 | N80, N87, N88 | N80, P81, K82, N87, N88 | Loop 2 |
| IVR3 | 90-117 | Y117, V112, W111, A114, | W111, V112, A114, Y117 | IVR3<br>IVR3 & 10A from active site. |
| Loop3 | 118-127 | | L118 | |
| IVR4 | 128-145 | | | |
| Loop4 | 146-176 | | P156 | |
| IVR5 | 177-207 | N181, Q182, H180, Y179 | Y179, H180, N181 | IVR5<br>IVR5 & 10A from active site. |

(continued)

| | P10480 amino acid positions (SEQ ID No 2) | Preferable sites to produce variants with increased $T_{DG}/T_{PC}$ | 10 Å | Preferred regions for methods of the invention |
|---|---|---|---|---|
| Loop5 | 208-287 | M209, L210, R211, N215, Y230 | M209, L210, R211, N215, K284, M285, Q289, V290 | Loop 5<br>Loop 5 & 10A from active site. |
| IVR6 | 288-317 | Q 289, V290, E309 S310, -318 | | IVR6 |

**Claims**

1. A method of producing a variant glycolipid acyltransferase enzyme comprising: (a) selecting a parent enzyme which is a lipid acyltransferase enzyme **characterised in that** the enzyme comprises the amino acid sequence motif GDSX, wherein X is one or more of the following amino acid residues L, A, V, I, F, Y, H, Q, T, N, M or S; (b) modifying one or more amino acids to produce a variant lipid acyltransferase; (c) testing the variant lipid acyltransferase for transferase activity, and optionally hydrolytic activity, on a galactolipid substrate, and optionally a phospholipid substrate and/or optionally a triglyceride substrate; (d) selecting a variant enzyme with an enhanced activity towards galactolipids compared with the parent enzyme; and optionally (e) preparing a quantity of the variant enzyme;
wherein the method further comprises one or more of the following steps: structural homology mapping or sequence homology alignment;
wherein the structural homology mapping comprises one or more of the following steps:

    (a) aligning a parent sequence with a structural model of the amino acid sequence SEQ ID No. 2 (P10480) obtained by structural alignment of the crystal structure co-ordinates of SEQ ID No. 2 with 1IVN.PDB shown in figure 52
    (b) selecting one or more amino acids within a 10Å sphere centred on the central carbon atom of the glycerol molecule in the active site;
    (c) determining if one or more amino acid residues selected in accordance with step (b) are highly conserved; and
    (d) modifying one or more amino acids selected in accordance with step (b), excluding conserved regions identified in accordance with step (c) in said parent sequence;

and wherein the sequence homology alignment comprises one or more of the following steps:

    (i) selecting a first parent lipid acyltransferase;
    (ii) identifying a second related lipid acyltransferase having a desirable activity;
    (iii) aligning said first parent lipid acyltransferase and the second related lipid acyltransferase;
    (iv) identifying amino acid residues that differ between the two sequences;
    (v) determining if one or more amino acid residues selected in accordance with step (iv) are highly conserved; and
    (vi) modifying one or more of the amino acid residues identified in accordance with step (iv) excluding conserved regions identified in accordance with step (v) in said parent sequence,

and wherein the following amino acid residues identified by alignment with SEQ ID No. 2 is modified compared with a parent sequence: S18 M or T; Y30 G, I, L, S, E, M, A or R; G40 L, R, M, Q or V; N80 R, D, P G or E; N87 R, D, E, M, C or G; N88W; Y179 V, E, R, N or Q; H180, T, K, Q or I; M209 Y, L, K or M; L210 N, D, H, R, E, A, Q, P, K, G, T, W, I, V, S or M; R211 G, Q, K, D or T; N215 H, L, G, V, R, or Y.

2. A method according to claim 1 wherein the method comprises testing the variant lipid acyltransferase for:

    (i) transferase activity from a galactolipid substrate, and
    (ii) transferase activity from a phospholipids substrate; and

selecting a variant enzyme, which when compared with the parent enzyme, has an enhanced ratio of transferase activity from galactolipids compared with phospholipids.

**3.** A method according to claim 2 wherein the ratio of transferase activity from galactolipids compared with phospholipids is at least 3.

**4.** A method according to any one of the proceedings claims comprising testing the variant lipid acyltransferase for:

(a) transferase activity from a galactolipid substrate, and
(b) hydrolytic activity on a galactolipid substrate; and

selecting a variant enzyme with an enhanced ratio of transferase activity from galactolipids compared with its hydrolytic activity on glycolipids, compared with the parent enzyme.

**5.** A method according to claim 4 wherein the enhanced ratio of transferase activity on galactolipids compared to hydrolytic activity on galactolipids is at least 1.5.

**6.** A method according to any one of the preceding claims wherein the parent enzyme comprises an amino acid sequence as shown as SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45, or an amino acid sequence which has at least 70% identity therewith.

**7.** A method according to any one of the preceding claims wherein the parent enzyme is an enzyme which comprises the amino acid sequence shown as SEQ ID No. 2 and/or SEQ ID No. 28.

**8.** A method according to any one of Claims 1 to 5 wherein the parent lipid acyltransferase enzyme is encoded by any one of the following nucleotide sequences: SEQ ID No. 7, SEQ ID No. 8, SEQ ID No. 9, SEQ ID No. 10, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No. 27, SEQ ID No. 29, SEQ ID No. 31, SEQ ID No. 32, SEQ ID No. 35, SEQ ID No. 38, SEQ ID No. 40, SEQ ID No. 42, SEQ ID No. 44 or SEQ ID No. 46 or a nucleotide sequence which has at least 75% or more identity with any one of the sequences shown as SEQ ID No. 7, SEQ ID No. 8, SEQ ID No. 9, SEQ ID No. 10, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No. 27, SEQ ID No. 29, SEQ ID No. 31, SEQ ID No. 32, SEQ ID No. 35, SEQ ID No. 38, SEQ ID No. 40, SEQ ID No. 42, SEQ ID No. 44 or SEQ ID No. 46.

**9.** A method according to any one of the preceding claims wherein the X of the GDSX motif is L.

**10.** A variant glycolipid acyltransferase enzyme **characterised in that** the enzyme comprises the amino acid sequence motif GDSX, wherein X is one or more of the following amino acid residues L, A, V, I, F, Y, H, Q, T, N, M or S, wherein the variant enzyme comprises an amino acid sequence, which amino acid sequence is shown as SEQ ID No. 2, SEQ ID No. 3, SEQ ID No. 26, SEQ ID No. 28 or SEQ ID No. 30 or is encoded by any one of the following nucleotide sequences: SEQ ID No. 7, SEQ ID No. 8, SEQ ID No. 27, SEQ ID No. 29 or SEQ ID No. 31, except for one or more amino acid modifications at any one or more of the amino acid residues defined S18, M or T; Y30 G, I, L, S, E, M, A or R; G40 L, R, M, Q or V; N80 R, D, P, G or E; N87 R, D, E M, C or G; N88W; Y179 V, E, R, N or Q; H180 T, K, Q or I; M209 Y, L, K or M; L210 N, D, H, R, E, A, Q, P K, G, T, W, I, V, S or M; R211 G, Q, K, D or T; N215 H, L, G, V, R, or Y, wherein the numbering is that obtained from alignment of the variant sequence with the reference sequence shown as SEQ ID No. 2.

**11.** A variant glycolipid acyltransferase enzyme according to claim 10 wherein the variant enzyme has an enhanced ratio of activity on galactolipids to either phospholipids and/or triglycerides when compared with the parent enzyme.

**12.** A variant glycolipid acyltransferase according to any one of claims 10-11 wherein the variant enzyme has a higher galactolipid transferase activity compared with its galactolipid hydrolytic activity compared with the parent enzyme.

**13.** Use of a variant glycolipid acyltransferase enzyme according to any one of claims 10-12 in a substrate for preparing a lyso-glycolipid, for example digalactosyl monoglyceride (DGMG) or monogalactosyl monoglyceride (MGMG) by treatment of a glycolipid (e.g. digalactosyl diglyceride (DGDG) or monogalactosyl diglyceride (MGDG)) with the variant lipolytic enzyme according to claims 10-12 to produce the partial hydrolysis product, i.e. the lyso-glycolipid.

**14.** Use according to claim 13 wherein the substrate is a foodstuff.

**15.** A method of preparing a foodstuff the method comprising adding a variant lipid acyltransferase enzyme according to any one of claims 10-12 to one or more ingredients of the foodstuff.

**16.** A method of preparing a baked product from a dough, the method comprising adding a variant glycolipid acyltransferase enzyme according to any one of claims 10-12 to the dough.

**17.** Use of a variant glycolipid acyltransferase enzyme according to any one of claims 10-12 in a process of treating egg or egg-based products to produce lysophospholipids.

**18.** A process of enzymatic degumming of vegetable or edible oils, comprising treating the edible or vegetable oil with a variant glycolipid acyltransferase enzyme according to any one of claims 10-12 so as to hydrolyse a major part of the polar lipids (e.g. phospholipid and/or glycolipid).

**19.** Use of a variant glycolipid acyltransferase enzyme according to any one of claims 10-12 in a process for reducing the content of a phospholipid in an edible oil, comprising treating the oil with said variant lipolytic enzyme so as to hydrolyse a major part of the phospholipid, and separating an aqueous phase containing the hydrolysed phospholipid from the oil.

**20.** Use of a variant glycolipid acyltransferase enzyme according to any one of claims 10-12 in the bioconversion of polar lipids (preferably glycolipids) to make high value products, such as carbohydrate esters and/or protein esters and/or protein subunit esters and/or a hydroxy acid ester.

**21.** An immobilised variant glycolipid acyltransferase enzyme according to any one of claims 10-12.

## Patentansprüche

**1.** Verfahren zur Herstellung eines varianten Glycolipid-Acyltransferase-Enzyms, umfassend: (a) Auswählen eines parentalen Enzyms, das ein Lipid-Acyltransferase-Enzym ist, **dadurch gekennzeichnet, dass** das Enzym das Aminosäuresequenzmotiv GDSX umfasst, wobei X einer oder mehrere der folgenden Aminosäurereste L, A, V, I, F, Y, H, Q, T, N, M oder S ist; (b) Modifizieren einer oder mehrerer Aminosäuren, um eine variante Lipid-Acyltransferase herzustellen; (c) Testen der varianten Lipid-Acyltransferase auf Transferaseaktivität, und gegebenenfalls hydrolytische Aktivität, auf ein Galaktolipidsubstrat, und gegebenenfalls ein Phospholipidsubstrat und/oder gegebenenfalls ein Triglyceridsubstrat; (d) Auswählen eines varianten Enzyms mit einer gesteigerten Aktivität gegenüber Galaktolipiden im Vergleich zum parentalen Enzym; und gegebenenfalls (e) Zubereiten einer Menge des varianten Enzyms;
wobei das Verfahren weiterhin einen oder mehrere der folgenden Schritte umfasst: Mapping struktureller Homologie oder Sequenzhomologie-Alignierung;
wobei das Mapping struktureller Homologie einen oder mehrere der folgenden Schritte umfasst:

(a) Alignieren einer parentalen Sequenz mit einem strukturellen Modell der Aminosäuresequenz SEQ ID Nr. 2 (P10480), erhalten durch strukturelle Alignierung der Kristallstrukturkoordinaten von SEQ ID Nr. 2 mit 1IVN.PDB, dargestellt in Figur 52;
(b) Auswählen einer oder mehrerer Aminosäuren innerhalb einer 10-Å-Sphäre, zentriert auf das zentrale Kohlenstoffatom des Glycerinmoleküls in der aktiven Stelle;
(c) Bestimmen, ob einer oder mehrere Aminosäurereste, ausgewählt gemäß Schritt (b), stark konserviert sind; und
(d) Modifizieren einer oder mehrerer Aminosäuren, ausgewählt gemäß Schritt (b), unter Ausschluss konservierter Regionen, identifiziert gemäß Schritt (c), in der parentalen Sequenz;

und wobei die Sequenzhomologie-Alignierung einen oder mehrere der folgenden Schritte umfasst:

(i) Auswählen einer ersten parentalen Lipid-Acyltransferase;
(ii) Identifizieren einer zweiten verwandten Lipid-Acyltransferase mit einer erwünschten Aktivität;
(iii) Alignieren der ersten parentalen Lipid-Acyltransferase und der zweiten verwandten Lipid-Acyltransferase;
(iv) Identifizieren von Aminosäureresten, die zwischen den beiden Sequenzen verschieden sind;
(v) Bestimmen, ob einer oder mehrere Aminosäurereste, ausgewählt gemäß Schritt (iv), stark konserviert sind; und

(vi) Modifizieren eines oder mehrerer der Aminosäurereste, identifiziert gemäß Schritt (iv), unter Ausschluss konservierter Regionen, identifiziert gemäß Schritt (v), in der parentalen Sequenz,

und wobei die folgenden Aminosäurereste, identifiziert durch Alignierung mit SEQ ID Nr. 2, gegenüber einer parentalen Sequenz modifiziert werden: S18 M oder T; Y30 G, I, L, S, E, M, A oder R; G40 L, R, M, Q oder V; N80 R, D, P, G oder E; N87 R, D, E, M, C oder G; N88W; Y179 V, E, R, N oder Q; H180 T, K, Q oder I; M209 Y, L, K oder M; L210 N, D, H, R, E, A, Q, P, K, G, T, W, I, V, S oder M; R211 G, Q, K, D oder T; N215 H, L, G, V, R oder Y.

**2.** Verfahren nach Anspruch 1, wobei das Verfahren umfasst, dass die variante Lipid-Acyltransferase getestet wird auf:

(i) Transferaseaktivität aus einem Galaktolipidsubstrat, und
(ii) Transferaseaktivität aus einem Phospholipidsubstrat; und

ein variantes Enzym ausgewählt wird, das, bei Vergleich mit dem parentalen Enzym, ein verbessertes Verhältnis von Transferaseaktivität aus Galaktolipiden gegenüber Phospholipiden aufweist.

**3.** Verfahren nach Anspruch 2, wobei das Verhältnis von Transferaseaktivität aus Galaktolipiden gegenüber Phospholipiden zumindest 3 beträgt.

**4.** Verfahren nach einem der vorstehenden Ansprüche, umfassend Testen der varianten Lipid-Acyltransferase auf:

(a) Transferaseaktivität aus einem Galaktolipidsubstrat; und
(b) hydrolytische Aktivität auf ein Galaktolipidsubstrat; und

Auswählen eines varianten Enzyms mit einem verbesserten Verhältnis von Transferaseaktivität aus Galaktolipiden gegenüber seiner hydrolytischen Aktivität auf Glycolipide, im Vergleich zum parentalen Enzym.

**5.** Verfahren nach Anspruch 4, wobei das verbesserte Verhältnis von Transferaseaktivität auf Galaktolipide gegenüber hydrolytischer Aktivität auf Galaktolipide zumindest 1,5 beträgt.

**6.** Verfahren nach einem der vorstehenden Ansprüche, wobei das parentale Enzym eine Aminosäuresequenz, wie dargestellt als SEQ ID Nr. 2, SEQ ID Nr. 3, SEQ ID Nr. 4, SEQ ID Nr. 5, SEQ ID Nr. 6, SEQ ID Nr. 12, SEQ ID Nr. 14, SEQ ID Nr. 16, SEQ ID Nr. 18, SEQ ID Nr. 20, SEQ ID Nr. 22, SEQ ID Nr. 24, SEQ ID Nr. 26, SEQ ID Nr. 28, SEQ ID Nr. 30, SEQ ID Nr. 33, SEQ ID Nr. 34, SEQ ID Nr. 36, SEQ ID Nr. 37, SEQ ID Nr. 39, SEQ ID Nr. 41, SEQ ID Nr. 43 oder SEQ ID Nr. 45, oder eine Aminosäuresequenz umfasst, die zumindest 70% Identität damit aufweist.

**7.** Verfahren nach einem der vorstehenden Ansprüche, wobei das parentale Enzym ein Enzym ist, das die Aminosäuresequenz, dargestellt als SEQ ID Nr. 2 und/oder SEQ ID Nr. 28, umfasst.

**8.** Verfahren nach einem der Ansprüche 1 bis 5, wobei das parentale Lipid-Acyltransferase-Enzym codiert ist durch eine der folgenden Nukleotidsequenzen: SEQ ID Nr. 7, SEQ ID Nr. 8, SEQ ID Nr. 9, SEQ ID Nr. 10, SEQ ID Nr. 11, SEQ ID Nr. 13, SEQ ID Nr. 15, SEQ ID Nr. 17, SEQ ID Nr. 19, SEQ ID Nr. 21, SEQ ID Nr. 23, SEQ ID Nr. 25, SEQ ID Nr. 27, SEQ ID Nr. 29, SEQ ID Nr. 31, SEQ ID Nr. 32, SEQ ID Nr. 35, SEQ ID Nr. 38, SEQ ID Nr. 40, SEQ ID Nr. 42, SEQ ID Nr. 44 oder SEQ ID Nr. 46, oder eine Nukleotidsequenz, die zumindest 75% oder mehr Identität mit einer der Sequenzen aufweist, dargestellt als SEQ ID Nr. 7, SEQ ID Nr. 8, SEQ ID Nr. 9, SEQ ID Nr. 10, SEQ ID Nr. 11, SEQ ID Nr. 13, SEQ ID Nr. 15, SEQ ID Nr. 17, SEQ ID Nr. 19, SEQ ID Nr. 21, SEQ ID Nr. 23, SEQ ID Nr. 25, SEQ ID Nr. 27, SEQ ID Nr. 29, SEQ ID Nr. 31, SEQ ID Nr. 32, SEQ ID Nr. 35, SEQ ID Nr. 38, SEQ ID Nr. 40, SEQ ID Nr. 42, SEQ ID Nr. 44 oder SEQ ID Nr. 46.

**9.** Verfahren nach einem der vorstehenden Ansprüche, wobei das X des GDSX-Motivs L ist.

**10.** Variantes Glycolipid-Acyltransferase-Enzym, **dadurch gekennzeichnet, dass** das Enzym das Aminosäuresequenzmotiv GDSX umfasst, wobei X einer oder mehrere der folgenden Aminosäurereste L, A, V, I, F, Y, H, Q, T, N, M oder S ist, wobei das variante Enzym eine Aminosäuresequenz umfasst, welche Aminosäuresequenz dargestellt ist als SEQ ID Nr. 2, SEQ ID Nr. 3, SEQ ID Nr. 26, SEQ ID Nr. 28 oder SEQ ID Nr. 30 oder codiert ist durch eine der folgenden Nukleotidsequenzen: SEQ ID Nr. 7, SEQ ID Nr. 8, SEQ ID Nr. 27, SEQ ID Nr. 29 oder SEQ ID Nr. 31, außer einer oder mehrerer Aminosäuremodifikationen an einem oder mehreren der Aminosäurereste, definiert S18, M oder T; Y30 G, I, L, S, E, M, A oder R; G40 L, R, M, Q oder V; N80 R, D, P, G oder E; N87 R, D, E M, C oder G; N88W; Y179 V, E, R, N oder Q; H180 T, K, Q oder I; M209 Y, L, K oder M; L210 N, D, H, R, E, A, Q, P, K, G, T, W, I, V, S oder M; R211 G, Q, K, D oder T; N215 H, L, G, V, R oder Y, wobei die Nummerierung jene ist, die aus Alignierung der varianten Sequenz mit der Referenzsequenz, dargestellt als SEQ ID Nr. 2, erhalten wird.

**11.** Variantes Glycolipid-Acyltransferase-Enzym nach Anspruch 10, wobei das variante Enzym ein verbessertes Verhältnis von Aktivität auf Galaktolipide zu entweder Phospholipiden und/oder Triglyceriden aufweist bei Vergleich mit dem parentalen Enzym.

**12.** Variante Glycolipid-Acyltransferase nach einem der Ansprüche 10-11, wobei das variante Enzym eine höhere Galaktolipid-Transferaseaktivität gegenüber seiner hydrolytischen Galaktolipidaktivität im Vergleich zum parentalen Enzym aufweist.

**13.** Verwendung eines varianten Glycolipid-Acyltransferase-Enzyms nach einem der Ansprüche 10-12 in einem Substrat zur Zubereitung von einem Lysoglycolipid, beispielsweise Digalaktosylmonoglycerid (DGMG) oder Monogalaktosylmonoglycerid (MGMG), durch Behandlung von einem Glycolipid (z. B. Digalaktosyldiglycerid (DGDG) oder Monogalaktosyldiglycerid (MGDG)) mit dem varianten lipolytischen Enzym nach Ansprüchen 10-12, um das Partialhydrolyseprodukt, d. h. das Lysoglycolipid, herzustellen.

**14.** Verwendung nach Anspruch 13, wobei das Substrat ein Lebensmittel ist.

**15.** Verfahren zur Zubereitung eines Lebensmittels, das Verfahren umfassend Zugeben eines varianten Lipid-Acyltransferase-Enzyms nach einem der Ansprüche 10-12 zu einem oder mehreren Inhaltsstoffen des Lebensmittels.

**16.** Verfahren zur Zubereitung einer Backware aus einem Teig, das Verfahren umfassend Zugeben eines varianten Glycolipid-Acyltransferase-Enzyms nach einem der Ansprüche 10-12 zum Teig.

**17.** Verwendung eines varianten Glycolipid-Acyltransferase-Enzyms nach einem der Ansprüche 10-12 in einem Prozess zur Behandlung von Ei oder Produkten auf Eierbasis, um Lysophospholipide herzustellen.

**18.** Prozess zur enzymatischen Degummierung von Pflanzen- oder Speiseölen, umfassend Behandeln des Speise- oder Pflanzenöls mit einem varianten Glycolipid-Acyltransferase-Enzym nach einem der Ansprüche 10-12, um einen Hauptteil von den polaren Lipiden (z. B. Phospholipid und/oder Glycolipid) zu hydrolysieren.

**19.** Verwendung eines varianten Glycolipid-Acyltransferase-Enzyms nach einem der Ansprüche 10-12 in einem Prozess zur Verringerung des Gehalts an einem Phospholipid in einem Speiseöl, umfassend Behandeln des Öls mit dem varianten lipolytischen Enzym, um einen Hauptteil des Phospholipids zu hydrolysieren, und Trennen einer wässrigen Phase, die das hydrolysierte Phospholipid enthält, vom Öl.

**20.** Verwendung eines varianten Glycolipid-Acyltransferase-Enzyms nach einem der Ansprüche 10-12 in der Biokonversion von polaren Lipiden (bevorzugt Glycolipiden), um hochwertige Produkte, wie z. B. Kohlenhydratester und/oder Proteinester und/oder Proteinuntereinheitester und/oder einen Hydroxysäureester, zu machen.

**21.** Immobilisiertes variantes Glycolipid-Acyltransferase-Enzym nach einem der Ansprüche 10-12.

## Revendications

**1.** Procédé de production d'une enzyme glycolipide acyltransférase variante, comprenant: (a) la sélection d'une enzyme parente qui est une enzyme lipide acyltransférase **caractérisée en ce que** l'enzyme comprend le motif de séquence d'acides aminés GDSX, où X est un ou plusieurs des restes d'acides aminés suivants L, A, V, I, F, Y, H, Q, T, N, M ou S; (b) la modification d'un ou plusieurs acides aminés pour produire une lipide acyltransférase variante; (c) le test de la lipide acyltransférase variante quant à l'activité de transférase et facultativement l'activité hydrolytique, sur un substrat de galactolipide, et facultativement un substrat de phospholipide et/ou facultativement un substrat de triglycéride; (d) la sélection d'une enzyme variante ayant une activité accrue vis-à-vis des galactolipides par rapport à l'enzyme parente; et facultativement (e) la préparation d'une quantité de l'enzyme variante;
lequel procédé comprend en outre une ou plusieurs des étapes suivantes: cartographie structurale par homologie ou alignement de séquences par homologie;
dans lequel la cartographie structurale par homologie comprend une ou plusieurs des étapes suivantes:

(a) alignement d'une séquence parente avec un modèle structural de la séquence d'acides aminés SEQ ID No. 2 (P10480) obtenu par alignement structural des coordonnées de structure cristalline de la SEQ ID No. 2 avec 1IVN.PDB présenté à la figure 52;
(b) sélection d'un ou plusieurs acides aminés au sein d'une sphère de 10Å centrée sur l'atome de carbone central de la molécule de glycérol dans le site actif;
(c) détermination du point de savoir si un ou plusieurs restes d'acides aminés sélectionnés d'après l'étape (b) sont hautement conservés; et
(d) modification d'un ou plusieurs acides aminés sélectionnés d'après l'étape (b), en excluant les régions conservées identifiées d'après l'étape (c) dans ladite séquence parente;

et dans lequel l'alignement de séquences par homologie comprend une ou plusieurs des étapes suivantes:

(i) sélection d'une première lipide acyltransférase parente;
(ii) identification d'une seconde lipide acyltransférase apparentée ayant une activité souhaitable;
(iii) alignement de ladite première lipide acyltransférase parente et de la seconde lipide acyltransférase apparentée;
(iv) identification des restes d'acides aminés qui diffèrent entre les deux séquences;
(v) détermination du point de savoir si un ou plusieurs restes d'acides aminés sélectionnés d'après l'étape (iv) sont hautement conservés; et
(vi) modification d'un ou plusieurs des restes d'acides aminés identifiés d'après l'étape (iv) en excluant les régions conservées identifiées d'après l'étape (v) dans ladite séquence parente,

et dans lequel les restes d'acides aminés suivants identifiés par alignement avec la SEQ ID No. 2 sont modifiés par rapport à une séquence parente: S18 M ou T; Y30 G, I, L, S, E, M, A ou R; G40 L, R, M, Q ou V; N80 R, D, P G ou E; N87 R, D, E, M, C ou G; N88W; Y179 V, E, R, N ou Q; H180, T, K, Q ou I; M209 Y, L, K ou M; L210 N, D, H, R, E, A, Q, P, K, G, T, W, I, V, S ou M; R211 G, Q, K, D ou T; N215 H, L, G, V, R ou Y.

**2.** Procédé selon la revendication 1, lequel procédé comprend le test de la lipide acyltransférase variante quant à:

(i) l'activité de transférase à partir d'un substrat de galactolipide, et
(ii) l'activité de transférase à partir d'un substrat de phospholipides; et

la sélection d'une enzyme variante qui, lorsqu'elle est comparée avec l'enzyme parente, a un rapport accru d'activité de transférase à partir des galactolipides par rapport aux phospholipides.

**3.** Procédé selon la revendication 2, dans lequel le rapport d'activité de transférase à partir des galactolipides par rapport aux phospholipides est d'au moins 3.

**4.** Procédé selon l'une quelconque des revendications précédentes, comprenant le test de la lipide acyltransférase variante quant à:

(a) l'activité de transférase à partir d'un substrat de galactolipide, et
(b) l'activité hydrolytique sur un substrat de galactolipide; et

la sélection d'une enzyme variante ayant un rapport accru d'activité de transférase à partir des galactolipides par rapport à son activité hydrolytique sur les glycolipides, par rapport à l'enzyme parente.

**5.** Procédé selon la revendication 4, dans lequel le rapport accru d'activité de transférase sur les galactolipides par rapport à l'activité hydrolytique sur les galactolipides est d'au moins 1,5.

**6.** Procédé selon l'une quelconque des revendications précédentes, dans lequel l'enzyme parente comprend une séquence d'acides aminés telle que présentée dans les SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 ou SEQ ID No. 45, ou une séquence d'acides aminés qui a au moins 70% d'identité avec celle-ci.

**7.** Procédé selon l'une quelconque des revendications précédentes, dans lequel l'enzyme parente est une enzyme qui comprend la séquence d'acides aminés présentée sous la forme de la SEQ ID No. 2 et/ou SEQ ID No. 28.

**8.** Procédé selon l'une quelconque des revendications 1 à 5, dans lequel l'enzyme lipide acyltransférase parente est codée par l'une quelconque des séquences nucléotidiques suivantes: SEQ ID No. 7, SEQ ID No. 8, SEQ ID No. 9, SEQ ID No. 10, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No. 27, SEQ ID No. 29, SEQ ID No. 31, SEQ ID No. 32, SEQ ID No. 35, SEQ ID No. 38, SEQ ID No. 40, SEQ ID No. 42, SEQ ID No. 44 ou SEQ ID No. 46 ou une séquence nucléotidique qui a au moins 75% ou plus d'identité avec l'une quelconque des séquences présentées sous la forme des SEQ ID No. 7, SEQ ID No. 8, SEQ ID No. 9, SEQ ID No. 10, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No. 27, SEQ ID No. 29, SEQ ID No. 31, SEQ ID No. 32, SEQ ID No. 35, SEQ ID No. 38, SEQ ID No. 40, SEQ ID No. 42, SEQ ID No. 44 ou SEQ ID No. 46.

**9.** Procédé selon l'une quelconque des revendications précédentes, dans lequel le X du motif GDSX est L.

**10.** Enzyme glycolipide acyltransférase variante **caractérisée en ce que** l'enzyme comprend le motif de séquence d'acides aminés GDSX, dans lequel X est un ou plusieurs des restes d'acides aminés suivants L, A, V, I, F, Y, H, Q, T, N, M ou S, laquelle enzyme variante comprend une séquence d'acides aminés, laquelle séquence d'acides aminés est présentée sous la forme des SEQ ID No. 2, SEQ ID No. 3, SEQ ID No. 26, SEQ ID No. 28 ou SEQ ID No. 30 ou est codée par l'une quelconque des séquences nucléotidiques suivantes: SEQ ID No. 7, SEQ ID No. 8, SEQ ID No. 27, SEQ ID No. 29 ou SEQ ID No. 31, à l'exception d'une ou plusieurs modifications d'acides aminés à l'un quelconque ou plusieurs des restes d'acides aminés définis S18, M ou T; Y30 G, I, L, S, E, M, A ou R; G40 L, R, M, Q ou V; N80 R, D, P G ou E; N87 R, D, E, M, C ou G; N88W; Y179 V, E, R, N ou Q; H180, T, K, Q ou I; M209 Y, L, K ou M; L210 N, D, H, R, E, A, Q, P, K, G, T, W, I, V, S ou M; R211 G, Q, K, D ou T; N215 H, L, G, V, R ou Y, dans laquelle la numérotation est celle obtenue à partir de l'alignement de la séquence variante avec la séquence de référence présentée sous la forme de la SEQ ID No. 2.

**11.** Enzyme glycolipide acyltransférase variante selon la revendication 10, laquelle enzyme variante a un rapport accru d'activité sur des galactolipides sur soit des phospholipides et/soit des triglycérides par rapport à l'enzyme parente.

**12.** Glycolipide acyltransférase variante selon l'une quelconque des revendications 10-11, dans laquelle l'enzyme variante a une activité de galactolipide transférase plus élevée que son activité hydrolytique de galactolipides par rapport à l'enzyme parente.

**13.** Utilisation d'une enzyme glycolipide acyltransférase variante selon l'une quelconque des revendications 10 à 12, dans un substrat pour préparer un lyso-glycolipide, par exemple le digalactosyl monoglycéride (DGMG) ou le monogalactosyl monoglycéride (MGMG) par traitement d'un glycolipide (par exemple le digalactosyl diglycéride (DGDG) ou le monogalactosyl diglycéride (MGDG)) avec l'enzyme lipolytique variante selon les revendications 10 à 12 pour produire le produit d'hydrolyse partielle, à savoir le lyso-glycolipide.

**14.** Utilisation selon la revendication 13, dans laquelle le substrat est une denrée alimentaire.

**15.** Procédé de préparation d'une denrée alimentaire, le procédé comprenant l'addition d'une enzyme lipide acyltransférase variante selon l'une quelconque des revendications 10 à 12 à un ou plusieurs ingrédients de la denrée alimentaire.

**16.** Procédé de préparation d'un produit de boulangerie à partir d'une pâte, le procédé comprenant l'addition d'une enzyme glycolipide acyltransférase variante selon l'une quelconque des revendications 10 à 12 à la pâte.

**17.** Utilisation d'une enzyme glycolipide acyltransférase variante selon l'une quelconque des revendications 10 à 12 dans un procédé consistant à traiter un oeuf ou des produits à base d'oeuf pour obtenir des lysophospholipides.

**18.** Procédé de démucilagination enzymatique d'huiles végétales ou comestibles, comprenant le traitement de l'huile comestible ou végétale avec une enzyme glycolipide acyltransférase variante selon l'une quelconque des revendications 10 à 12 de façon à hydrolyser une partie majeure des lipides polaires (par exemple phospholipide et/ou glycolipide).

**19.** Utilisation d'une enzyme glycolipide acyltransférase variante selon l'une quelconque des revendications 10 à 12 dans un procédé pour réduire la teneur en un phospholipide dans une huile comestible, comprenant le traitement de l'huile avec ladite enzyme lipolytique variante de façon à hydrolyser une partie majeure du phospholipide, et la séparation d'une phase aqueuse contenant le phospholipide hydrolysé à partir de l'huile.

**20.** Utilisation d'une enzyme glycolipide acyltransférase variante selon l'une quelconque des revendications 10 à 12 dans la bioconversion de lipides polaires (de préférences des glycolipides) pour fabriquer des produits de haute valeur, comme des esters d'hydrates de carbone et/ou des esters de protéines et/ou des esters de sous-unités de protéines et/ou un ester d'hydroxy acide.

**21.** Enzyme glycolipide acyltransférase variante immobilisée selon l'une quelconque des revendications 10 à 12.

Figure 1

SEQ ID No. 1

```
  1 ivafGDSlTd geayygdsdg ggwgagladr Ltallrlrar prgvdvfnrg isGrtsdGrl
 61 ivDalvallF laqslglpnL pPYLsgdflr GANFAsagAt Ilptsgpfli QvqFkdfksq
121 vlelrqalgl lqellrllpv ldakspdlvt imiGtNDlit saffgpkste sdrnvsvpef
181 kdnlrqlikr Lrsnngarii vlitlvilnl gplGClPlkl alalassknv dasgclerln
241 eavadfneal relaiskled qlrkdglpdv kgadvpyvDl ysifqdldgi qnpsayvyGF
301 ettkaCCGyG gryNynrvCG naglcnvtak aCnpssylls flfwDgfHps ekGykavAea
361 l
```

Figure 2

SEQ ID No. 2

ADSRPAFSRIVMFGDSLSDTGKMYSKMRGYLPSSPPYYEGRFSNGPVWLEQLTNEF
PGLTIANEAEGGPTAVAYNKISWNPKYQVINNLDYEVTQFLQKDSFKPDDLVILWVGA
NDYLAYGWNTEQDAKRVRDAISDAANRMVLNGAKEILLFNLPDLGQNPSARSQKVV
EAASHVSAYHNQLLLNLARQLAPTGMVKLFEIDKQFAEMLRDPQNFGLSDQRNACY
GGSYVWKPFASRSASTDSQLSAFNPQERLAIAGNPLLAQAVASPMAARSASTLNCE
GKMFWDQVHPTTVVHAALSEPAATFIESQYEFLAH

Figure 3

SEQ ID No. 3

```
  1 mkkwfvcllg lialtvqaad trpafsrivm fgdslsdtgk myskmrgylp ssppyyegrf
 61 sngpvwleql tkqfpgltia neaeggatav aynkiswnpk yqvynnldye vtqflqkdsf
121 kpddlvilwv gandylaygw nteqdakrvr daisdaanrm vlngakqill fnlpdlgqnp
181 sarsqkvvea vshvsayhnk lllnlarqla ptgmvklfei dkqfaemlrd pqnfglsdve
241 npcydggyvw kpfatrsvst drqlsafspq erlaiagnpl laqavaspma rrsasplnce
301 gkmfwdqvhp ttvvhaalse raatfietqy eflahg
```

Figure 4

SEQ ID No. 4

```
  1 mpkpalrrvm tatvaavgtl algltdatah aapaqatptl dyvalgdsys agsgvlpvdp
 61 anllclrsta nyphviadtt garltdvtcg aaqtadftra qypgvapqld algtgtdlvt
121 ltiggndnst finaitacgt agvlsggkgs pckdrhgtsf ddeieantyp alkeallgvr
181 arapharvaa lgypwitpat adpscflklp laagdvpylr aiqahlndav rraaeetgat
241 yvdfsgvsdg hdaceapgtr wiepllfghs lvpvhpnalg errmaehtmd vlgld
```

Figure 5

SEQ ID No. 5

```
  1 mpkpalrrvm tatvaavgtl algltdatah aapaqatptl dyvalgdsys agsgvlpvdp
 61 anllclrsta nyphviadtt garltdvtcg aaqtadftra qypgvapqld algtgtdlvt
121 ltiggndnst finaitacgt agvlsggkgs pckdrhgtsf ddeieantyp alkeallgvr
181 arapharvaa lgypwitpat adpscflklp laagdvpylr aiqahlndav rraaeetgat
241 yvdfsgvsdg hdaceapgtr wiepllfghs lvpvhpnalg errmaehtmd vlgld
```

Figure 6

SEQ ID No. 6

```
  1 mdyekfllfg dsitefafnt rpiedgkdqy algaalvney trkmdilqrg fkgytsrwal
 61 kilpeilkhe snivmatifl gandacsagp qsvplpefid nirqmvslmk syhirpiiig
121 pglvdrekwe kekseeialg yfrtnenfai ysdalaklan eekvpfvaln kafqqeggda
181 wqqlltdglh fsgkgykifh dellkvietf ypqyhpknmq yklkdwrdvl ddgsnims
```

Figure 7

```
Alignment of pfam00657.6 consensus sequence with P10480
                   *->ivafGDSlTdg................eayygdsdgggwgagladrL
                      iv+fGDSl+d+++  ++ ++  +++++++ +++s+g  w ++l + +
      P10480    28    IVMFGDSLSDTgkmyskmrgylpssppYYEGRFSNGPVWLEQLTNEF 74

                   tall..rlrarprgvdvfnrgisGrtsdGrlivDalvallFlaqslglpn
                   + l    + ++++++++ +n+   +
      P10480    75 PGLTiaNEAEGGPTAVAYNKISWNPK------------------------ 100

                   LpPYLsgdflrGANFAsagAtIlptsgpfliQvqFkdfksqvlelrqalg
                                                               ++  ++
      P10480   101 --------------------------------------------YQVINN 106

                   llqellrllpvldakspdlvtimiGtNDlitsaffgpkstesdrnvsvpe
                   l++e+ ++l +++ k+ dlv++++G+ND+          ++ ++ ++++++
      P10480   107 LDYEVTQFLQKDSFKPDDLVILWVGANDY--------LAYGWNTEQDAKR 148

                   fkdnlrqlikrLrsnngariivlitlvilnlgplGClPlklalalasskn
                   ++d ++++++r+   nga+      ++++nl+ lG+ P+
      P10480   149 VRDAISDAANRMV-LNGAK-----EILLFNLPDLGQNPS----------- 181

                   vdasgclerlneavadfnealrelaiskledqlrkdglpdvkgadvpyvD
                    ++++ +e +  ++a++n++l +la     +ql+++g+++++++d ++++
      P10480   182 ARSQKVVEAASHVSAYHNQLLLNLA-----RQLAPTGMVKLFEIDKQFAE 226

                   lysifqdldgiqnpsayv.y....GFe.ttkaCCGyGgr.yNyn.rv.CG
                   +   +q+++ + + +a+++++    +++ +++a+++++++ +N+++r+ ++
      P10480   227 MLRDPQNFGLSDQRNACYgGsyvwKPFaSRSASTDSQLSaFNPQeRLaIA 276

                   nag.l.c.nvtakaC.npssyll.sflfwDgfHpsekGykavAeal<-*
                   +++ l +  ++++a++ +s+ ++++++fwD++Hp+   ++a+ e
      P10480   277 GNPlLaQaVASPMAArSASTLNCeGKMFWDQVHPTTVVHAALSEPA    322

Alignment of pfam00657.6 consensus sequence with AAG09804
                   *->ivafGDSlTdg................eayygdsdgggwgagladrL
                      iv+fGDSl+d+++  ++ ++  +++++++ +++s+g  w ++l + +
    AAG09804    28    IVMFGDSLSDTgkmyskmrgylpssppYYEGRFSNGPVWLEQLTKQF 74

                   tallrlrarprgvdvfnrgisGrtsdGrlivDalvallFlaqslglpnLp
                             +g+++ n + +G+t
    AAG09804    75 ----------PGLTIANEAEGGAT-------------------------- 88

                   PYLsgdflrGANFAsagAtIlptsgpfliQvqFkdfksqvlelrqa....
                                                        ++++ + ++++ +
    AAG09804    89 ------------------------------------AVAYNKISWNpkyq 102

                   ..lgllqellrllpvldakspdlvtimiGtNDlitsaffgpkstesdrnv
                     ++l++e+ ++l +++ k+ dlv++++G+ND+          ++ ++ ++
    AAG09804   103 vyNNLDYEVTQFLQKDSFKPDDLVILWVGANDY--------LAYGWNTEQ 144

                   svpefkdnlrqlikrLrsnngariivlitlvilnlgplGClPlklalala
                   ++++++d ++++++r+   nga+      +++++nl+ lG+ P+
    AAG09804   145 DAKRVRDAISDAANRMV-LNGAK-----QILLFNLPDLGQNPS------- 181

                   ssknvdasgclerlneavadfnealrelaiskledqlrkdglpdvkgadv
                       ++++ +e +  ++a++n++l +la     +ql+++g+++++++d
    AAG09804   182 ----ARSQKVVEAVSHVSAYHNKLLLNLA-----RQLAPTGMVKLFEIDK 222

                   pyvDlysifqdldgiqnpsayv.y....GFe.ttkaCCGyGgr.yNyn.r
                   +++++   +q+++ +  ++ +++++    +++ t++ +++ +++ + +++r
    AAG09804   223 QFAEMLRDPQNFGLSDVENPCYdGgyvwKPFaTRSVSTDRQLSaFSPQeR 272

                   v.CGnag.l.c.nvtakaC.npssyll.sflfwDgfHpsekGykavAeal
                   + +++++ l +  ++++a++ +s  ++++++fwD++Hp+   ++a+ e+
    AAG09804   273 LaIAGNPlLaQaVASPMARrSASPLNCeGKMFWDQVHPTTVVHAALSERA 322
```

```
                  <-*

AAG09804          -     -

Alignment of pfam00657.6 consensus sequence with NP_631558
                  *->ivafGDSlTdgeayygdsdgggwgagladrLtallrlrarprgvdvf
                     +va+GDS ++g          +g  +  +++L      + + + ++  +
   NP_631558    42   YVALGDSYSAG---------SGVLPVDPANL----LCLRSTANYPHV 75

                  nrgisGrtsdGrlivD.a.l.vallFlaqslglpnLpPYLsgdflrGANF
                   + ++G++        D + + +
   NP_631558    76 IADTTGAR-----LTDvTcGaAQ-------------------------- 93

                  AsagAtIlptsgpfliQvqFkdfksqvlelrqalgllqellrllpvldak
                                                +++    ++ +  ++ +++
   NP_631558    94 -----------------------------TADFTRAQYPGVAPQLDALGT 114

                  spdlvtimiGtNDl...............itsaffgpkstesdrnvsvp
                  + dlvt+ iG+ND ++  +  +  ++ +   ++  + +k   ++ + +++
   NP_631558   115 GTDLVTLTIGGNDNstfinaitacgtagvlSGGKGSPCKDRHGTSFDDEI 164

                  efkdn..lrqlikrLrs.nngariivlitlvilnlg...........plG
                  e  +++ l++++  +r+++ +ar+ +l  ++i+++  +++    + +  G
   NP_631558   165 EANTYpaLKEALLGVRArAPHARVAALGYPWITPATadpscflklplAAG 214

                  ClPlklalalassknvdasgclerlneavadfnealrelaiskledqlrk
                    P+                    l+ ++a  n a+r  a
   NP_631558   215 DVPY-------------------LRAIQAHLNDAVRRAA---------- 234

                  dglpdvkgadvpyvDlysifqdldgiqnpsayvyGFettkaCCGyGgryN
                        ++ + +yvD+ ++
   NP_631558   235 ------EETGATYVDFSGVSDG--------------------------- 250

                  ynrvCGnaglcnvtakaC.npssyll.sflfwDgf...HpsekGykavAe
                                ++aC+ p +++ +   lf + + + Hp++ G +++Ae
   NP_631558   251 --------------HDACeAPGTRWIePLLFGHSLvpvHPNALGERRMAE 286

                  al<-*
                  +
   NP_631558   287 HT    288

Alignment of pfam00657.6 consensus sequence with CAC42140
                  *->ivafGDSlTdgeayygdsdgggwgagladrLtallrlrarprgvdvf
                     +va+GDS ++g          +g  +  +++L      + + + ++  +
    CAC42140    42   YVALGDSYSAG---------SGVLPVDPANL----LCLRSTANYPHV 75

                  nrgisGrtsdGrlivD.a.l.vallFlaqslglpnLpPYLsgdflrGANF
                   + ++G++        D + + +
    CAC42140    76 IADTTGAR-----LTDvTcGaAQ-------------------------- 93

                  AsagAtIlptsgpfliQvqFkdfksqvlelrqalgllqellrllpvldak
                                                +++    ++ +  ++ +++
    CAC42140    94 -----------------------------TADFTRAQYPGVAPQLDALGT 114

                  spdlvtimiGtNDl...............itsaffgpkstesdrnvsvp
                  + dlvt+ iG+ND ++  +  +  ++ +   ++  + +k   ++ + +++
    CAC42140   115 GTDLVTLTIGGNDNstfinaitacgtagvlSGGKGSPCKDRHGTSFDDEI 164

                  efkdn..lrqlikrLrs.nngariivlitlvilnlg...........plG
                  e  +++ l++++  +r+++ +ar+ +l  ++i+++  +++    + +  G
    CAC42140   165 EANTYpaLKEALLGVRArAPHARVAALGYPWITPATadpscflklplAAG 214

                  ClPlklalalassknvdasgclerlneavadfnealrelaiskledqlrk
                    P+                    l+ ++a  n a+r  a
    CAC42140   215 DVPY-------------------LRAIQAHLNDAVRRAA---------- 234

                  dglpdvkgadvpyvDlysifqdldgiqnpsayvyGFettkaCCGyGgryN
                        ++ + +yvD+ ++
    CAC42140   235 ------EETGATYVDFSGVSDG--------------------------- 250
```

```
ynrvCGnaglcnvtakaC.npssyll.sflfwDgf...HpsekGykavAe
                              ++aC+ p +++ +  lf + + + Hp++ G +++Ae
    CAC42140   251 --------------HDACeAPGTRWIePLLFGHSLvpvHPNALGERRMAE 286

                   al<-*
                   +
    CAC42140   287 HT    288

Alignment of pfam00657.6 consensus sequence with P41734
                  *->ivafGDSlTdg....eayygdsdgggwgagladrLtallrlrarprg
                      ++fGDS+T+   +++ + +  d+   ga+l + +         +r+
      P41734      6    FLLFGDSITEFafntRPIEDGKDQYALGAALVNEY---------TRK 43

                   vdvfnrgisGrtsdGrlivDalvallFlaqslglpnLpPYLsgdflrGAN
                   +d+  rg++G+t
      P41734     44 MDILQRGFKGYT-------------------------------------- 55

                   FAsagAtIlptsgpfliQvqFkdfksqvlelrqalgllqellrllpvlda
                                                 +r+al++l+e+l+      +
      P41734     56 ------------------------------SRWALKILPEILKH-----E 70

                   kspdlvtimiGtNDlitsaffgpkstesdrnvsvpefkdnlrqlikrLrs
                    +  + ti++G+ND+         ++ +++ v++pef+dn+rq++++++s
      P41734     71 SNIVMATIFLGANDA---------CSAGPQSVPLPEFIDNIRQMVSLMKS 111

                   nngariivlitlvilnlgplGClPlklalalassknvdasgclerlneav
                    ++++ii++++lv   ++             ++ k ++ +  + r+ne +
      P41734    112 YHIRPIIIGPGLVDREKW-------------EKEKSEEIALGYFRTNENF 148

                   adfnealrelaiskledqlrkdglpdvkgadvpyvDlysifqdldgiqnp
                   a +  al +la                   ++ +vp+v l+++fq+ +g++++
      P41734    149 AIYSDALAKLA----------------NEEKVPFVALNKAFQQEGGDAWQ 182

                   sayvyGFettkaCCGyGgryNynrvCGnaglcnvtakaCnpssyllsflf
                   +                                                l+
      P41734    183 Q------------------------------------------------LL 185

                   wDgfHpsekGykavAeal<-*
                    Dg+H+s kGyk+++++l
      P41734    186 TDGLHFSGKGYKIFHDEL    203
```

Figure 8

```
A.sal  1    MKKWFVCLLGLIALTVQAADTRPAFSRIVMFGDSLSDTGKMYSKMRGYLPSSPPYYEGRF 60
                          +       +
A.hyd  1    MKKWFVCLLGLVALTVQAADSRPAFSRIVMFGDSLSDTGKMYSKMRGYLPSSPPYYEGRF 60

A. sal 61   SNGPVWLEQLTKQFPGLTIANEAEGGATAVAYNKISWNPKYQVINNLDYEVTQFLQKDSF 120
                       ++         +
A. hyd 61   SNGPVWLEQLTNEFPGLTIANEAEGGPTAVAYNKISWNPKYQVINNLDYEVTQFLQKDSF 120

A. sal 121  KPDDLVILWVGANDYLAYGWNTEQDAKRVRDAISDAANRMVLNGAKQILLFNLPDLGQNP 180
                                                                       +
A. hyd 121  KPDDLVILWVGANDYLAYGWNTEQDAKRVRDAISDAANRMVLNGAKEILLFNLPDLGQNP 180

A. sal 181  SARSQKVVEAVSHVSAYHNKLLLNLARQLAPTGMVKLFEIDKQFAEMLRDPQNFGLSDVE 240
                       +       +                                                 ++
A.hyd  181  SARSQKVVEAASHVSAYHNQLLLNLARQLAPTGMVKLFEIDKQFAEMLRDPQNFGLSDQR 240

A. sal 241  NPCYDGGYVWKPFATRSVSTDRQLSAFSPQERLAIAGNPLLAQAVASPMARRSASPLNCE 300
             + ++ +         +  +   +    +                   +   +
A. hyd 241  NACYGGSYVWKPFASRSASTDSQLSAFNPQERLAIAGNPLLAQAVASPMAARSASTLNCE 300

A. sal 301  GKMFWDQVHPTTVVHAALSERAATFIETQYEFLAH 335
                              +     +
A. hyd 301  GKMFWDQVHPTTVVHAALSEPAATFIESQYEFLAH 335
```

Figure 9

(SEQ ID No. 7)

```
  1 ATGAAAAAAT GGTTTGTGTG TTTATTGGGA TTGGTCGCGC TGACAGTTCA GGCAGCCGAC
 61 AGCCGTCCCG CCTTCTCCCG GATCGTGATG TTTGGCGACA GCCTCTCCGA TACCGGCAAG
121 ATGTACAGCA AGATGCGCGG TTACCTCCCC TCCAGCCCCC CCTACTATGA GGGCCGCTTC
181 TCCAACGGGC CCGTCTGGCT GGAGCAGCTG ACCAACGAGT TCCCGGGCCT GACCATAGCC
241 AACGAGGCGG AAGGCGGACC GACCGCCGTG GCTTACAACA AGATCTCCTG GAATCCCAAG
301 TATCAGGTCA TCAACAACCT GGACTACGAG GTCACCCAGT TCCTGCAAAA AGACAGCTTC
361 AAGCCGGACG ATCTGGTGAT CCTCTGGGTC GGCGCCAACG ACTATCTGGC CTATGGCTGG
421 AACACAGAGC AGGATGCCAA GCGGGTGCGC GACGCCATCA GCGATGCGGC CAACCGCATG
481 GTGCTGAACG GCGCCAAGGA GATACTGCTG TTCAACCTGC CGGATCTGGG CCAGAACCCC
541 TCGGCCCGCA GCCAGAAGGT GGTCGAGGCG GCCAGCCATG TCTCCGCCTA CCACAACCAG
601 CTGCTGCTGA ACCTGGCACG CCAGCTGGCT CCCACCGGCA TGGTGAAGCT GTTCGAGATC
661 GACAAGCAGT TTGCCGAGAT GCTGCGTGAT CCGCAGAACT TCGGCCTGAG CGACCAGAGG
721 AACGCCTGCT ACGGTGGCAG CTATGTATGG AAGCCGTTTG CCTCCCGCAG CGCCAGCACC
781 GACAGCCAGC TCTCCGCCTT CAACCCGCAG GAGCGCCTCG CCATCGCCGG CAACCCGCTG
841 CTGGCCCAGG CCGTCGCCAG CCCCATGGCT GCCCGCAGCG CCAGCACCCT CAACTGTGAG
901 GGCAAGATGT TCTGGGATCA GGTCCACCCC ACCACTGTCG TGCACGCCGC CCTGAGCGAG
961 CCCGCCGCCA CCTTCATCGA GAGCCAGTAC GAGTTCCTCG CCCAC
```

Figure 10

(SEQ ID No. 8)

```
  1  ATGAAAAAAT GGTTTGTTTG TTTATTGGGG TTGATCGCGC TGACAGTTCA GGCAGCCGAC
 61  ACTCGCCCCG CCTTCTCCCG GATCGTGATG TTCGGCGACA GCCTCTCCGA TACCGGCAAA
121  ATGTACAGCA AGATGCGCGG TTACCTCCCC TCCAGCCCGC CCTACTATGA GGGCCGTTTC
181  TCCAACGGAC CCGTCTGGCT GGAGCAGCTG ACCAAGCAGT TCCCGGGTCT GACCATCGCC
241  AACGAAGCGG AAGGCGGTGC CACTGCCGTG GCTTACAACA AGATCTCCTG GAATCCCAAG
301  TATCAGGTCT ACAACAACCT GGACTACGAG GTCACCCAGT TCTTGCAGAA AGACAGCTTC
361  AAGCCGGACG ATCTGGTGAT CCTCTGGGTC GGTGCCAATG ACTATCTGGC ATATGGCTGG
421  AATACGGAGC AGGATGCCAA GCGAGTTCGC GATGCCATCA GCGATGCGGC CAACCGCATG
481  GTACTGAACG GTGCCAAGCA GATACTGCTG TTCAACCTGC CGGATCTGGG CCAGAACCCG
541  TCAGCCCGCA GTCAGAAGGT GGTCGAGGCG GTCAGCCATG TCTCCGCCTA TCACAACAAG
601  CTGCTGCTGA ACCTGGCACG CCAGCTGGCC CCCACCGGCA TGGTAAAGCT GTTCGAGATC
661  GACAAGCAAT TTGCCGAGAT GCTGCGTGAT CCGCAGAACT TCGGCCTGAG CGACGTCGAG
721  AACCCCTGCT ACGACGGCGG CTATGTGTGG AAGCCGTTTG CCACCCGCAG CGTCAGCACC
781  GACCGCCAGC TCTCCGCCTT CAGTCCGCAG GAACGCCTCG CCATCGCCGG CAACCCGCTG
841  CTGGCACAGG CCGTTGCCAG TCCTATGGCC CGCCGCAGCG CCAGCCCCCT CAACTGTGAG
901  GGCAAGATGT TCTGGGATCA GGTACACCCG ACCACTGTCG TGCACGCAGC CCTGAGCGAG
961  CGCGCCGCCA CCTTCATCGA GACCCAGTAC GAGTTCCTCG CCCACGGATG A
```

Figure 11

(SEQ ID No. 9)

```
  1  ATGCCGAAGC CTGCCCTTCG CCGTGTCATG ACCGCGACAG TCGCCGCCGT CGGCACGCTC
 61  GCCCTCGGCC TCACCGACGC CACCGCCCAC GCCGCGCCCG CCCAGGCCAC TCCGACCCTG
121  GACTACGTCG CCCTCGGCGA CAGCTACAGC GCCGGCTCCG GCGTCCTGCC CGTCGACCCC
181  GCCAACCTGC TCTGTCTGCG CTCGACGGCC AACTACCCCC ACGTCATCGC GGACACGACG
241  GGCGCCCGCC TCACGGACGT CACCTGCGGC GCCGCGCAGA CCGCCGACTT CACGCGGGCC
301  CAGTACCCGG GCGTCGCACC CCAGTTGGAC GCGCTCGGCA CCGGCACGGA CCTGGTCACG
361  CTCACCATCG GCGGCAACGA CAACAGCACC TTCATCAACG CCATCACGGC CTGCGGCACG
421  GCGGGTGTCC TCAGCGGCGG CAAGGGCAGC CCCTGCAAGG ACAGGCACGG CACCTCCTTC
481  GACGACGAGA TCGAGGCCAA CACGTACCCC GCGCTCAAGG AGGCGCTGCT CGGCGTCCGC
541  GCCAGGGCTC CCCACGCCAG GGTGGCGGCT CTCGGCTACC CGTGGATCAC CCCGGCCACC
601  GCCGACCCGT CCTGCTTCCT GAAGCTCCCC CTCGCCGCCG GTGACGTGCC CTACCTGCGG
661  GCCATCCAGG CACACCTCAA CGACGCGGTC CGGCGGGCCG CCGAGGAGAC CGGAGCCACC
721  TACGTGGACT TCTCCGGGGT GTCCGACGGC CACGACGCCT GCGAGGCCCC CGGCACCCGC
781  TGGATCGAAC CGCTGCTCTT CGGGCACAGC CTCGTTCCCG TCCACCCCAA CGCCCTGGGC
841  GAGCGGCGCA TGGCCGAGCA CACGATGGAC GTCCTCGGCC TGGACTGA
```

Figure 12

(SEQ ID No. 10)

```
  1  TCAGTCCAGG CCGAGGACGT CCATCGTGTG CTCGGCCATG CGCCGCTCGC CCAGGGCGTT
 61  GGGGTGGACG GGAACGAGGC TGTGCCCGAA GAGCAGCGGT TCGATCCAGC GGGTGCCGGG
121  GGCCTCGCAG GCGTCGTGGC CGTCGGACAC CCCGGAGAAG TCCACGTAGG TGGCTCCGGT
181  CTCCTCGGCG GCCCGCCGGA CCGCGTCGTT GAGGTGTGCC TGGATGGCCC GCAGGTAGGG
241  CACGTCACCG GCGGCGAGGG GGAGCTTCAG GAAGCAGGAC GGGTCGGCGG TGGCCGGGGT
301  GATCCACGGG TAGCCGAGAG CCGCCACCCT GGCGTGGGGA GCCCTGGCGC GGACGCCGAG
361  CAGCGCCTCC TTGAGCGCGG GGTACGTGTT GGCCTCGATC TCGTCGTCGA AGGAGGTGCC
421  GTGCCTGTCC TTGCAGGGGC TGCCCTTGCC GCCGCTGAGG ACACCCGCCG TGCCGCAGGC
481  CGTGATGGCG TTGATGAAGG TGCTGTTGTC GTTGCCGCCG ATGGTGAGCG TGACCAGGTC
541  CGTGCCGGTG CCGAGCGCGT CCAACTGGGG TGCGACGCCC GGGTACTGGG CCCGCGTGAA
601  GTCGGCGGTC TGCGCGGCGC CGCAGGTGAC GTCCGTGAGG CGGGCGCCCG TCGTGTCCGC
661  GATGACGTGG GGGTAGTTGG CCGTCGAGCG CAGACAGAGC AGGTTGGCGG GGTCGACGGG
721  CAGGACGCCG GAGCCGGCGC TGTAGCTGTC GCCGAGGGCG ACGTAGTCCA GGGTCGGAGT
781  GGCCTGGGCG GGCGCGGCGT GGGCGGTGGC GTCGGTGAGG CCGAGGGCGA GCGTGCCGAC
841  GGCGGCGACT GTCGCGGTCA TGACACGGCG AAGGGCAGGC TTCGGCAT
```

Figure 13

(SEQ ID No. 11)

```
  1  ATGGATTACG AGAAGTTTCT GTTATTTGGG GATTCCATTA CTGAATTTGC TTTTAATACT
 61  AGGCCCATTG AAGATGGCAA AGATCAGTAT GCTCTTGGAG CCGCATTAGT CAACGAATAT
121  ACGAGAAAAA TGGATATTCT TCAAAGAGGG TTCAAAGGGT ACACTTCTAG ATGGGCGTTG
181  AAAATACTTC CTGAGATTTT AAAGCATGAA TCCAATATTG TCATGGCCAC AATATTTTTG
241  GGTGCCAACG ATGCATGCTC AGCAGGTCCC CAAAGTGTCC CCCTCCCCGA ATTTATCGAT
301  AATATTCGTC AAATGGTATC TTTGATGAAG TCTTACCATA TCCGTCCTAT TATAATAGGA
361  CCGGGGCTAG TAGATAGAGA GAAGTGGGAA AAAGAAAAAT CTGAAGAAAT AGCTCTCGGA
421  TACTTCCGTA CCAACGAGAA CTTTGCCATT TATTCCGATG CCTTAGCAAA ACTAGCCAAT
481  GAGGAAAAAG TTCCCTTCGT GGCTTTGAAT AAGGCGTTTC AACAGGAAGG TGGTGATGCT
541  TGGCAACAAC TGCTAACAGA TGGACTGCAC TTTTCCGGAA AAGGGTACAA AATTTTTCAT
601  GACGAATTAT TGAAGGTCAT TGAGACATTC TACCCCCAAT ATCATCCCAA AAACATGCAG
661  TACAAACTGA AAGATTGGAG AGATGTGCTA GATGATGGAT CTAACATAAT GTCTTGA
```

Figure 14

(SEQ ID No. 12)

```
        10         20         30         40         50         60
         |          |          |          |          |          |
MNLRQWMGAA TAALALGLAA CGGGGTDQSG NPNVAKVQRM VVFGDSLSDI GTYTPVAQAV

        70         80         90        100        110        120
         |          |          |          |          |          |
GGGKFTTNPG PIWAETVAAQ LGVTLTPAVM GYATSVQNCP KAGCFDYAQG GSRVTDPNGI

       130        140        150        160        170        180
         |          |          |          |          |          |
GHNGGAGALT YPVQQQLANF YAASNNTFNG NNDVVFVLAG SNDIFFWTTA AATSGSGVTP

       190        200        210        220        230        240
         |          |          |          |          |          |
AIATAQVQQA ATDLVGYVKD MIAKGATQVY VFNLPDSSLT PDGVASGTTG QALLHALVGT

       250        260        270        280        290        300
         |          |          |          |          |          |
FNTTLQSGLA GTSARIIDFN AQLTAAIQNG ASFGFANTSA RACDATKINA LVPSAGGSSL

       310        320        330        340
         |          |          |          |
FCSANTLVAS GADQSYLFAD GVHPTTAGHR LIASNVLARL LADNVAH
```

Figure 15

(SEQ ID No. 13)

```
atgaacctgc gtcaatggat gggcgccgcc acggctgccc ttgccttggg cttggccgcg        60
tgcgggggcg gtgggaccga ccagagcggc aatcccaatg tcgccaaggt gcagcgcatg       120
gtggtgttcg gcgacagcct gagcgatatc ggcacctaca cccccgtcgc gcaggcggtg       180
ggcggcggca agttcaccac caacccgggc ccgatctggg ccgagaccgt ggccgcgcaa       240
ctgggcgtga cgctcacgcc ggcggtgatg ggctacgcca cctccgtgca gaattgcccc       300
aaggccggct gcttcgacta tgcgcagggc ggctcgcgcg tgaccgatcc gaacggcatc       360
ggccacaacg gcggcgcggg ggcgctgacc tacccggttc agcagcagct cgccaacttc       420
tacgcggcca gcaacaacac attcaacggc aataacgatg tcgtcttcgt gctggccggc       480
agcaacgaca ttttcttctg gaccactgcg gcggccacca gcggctccgg cgtgacgccc       540
gccattgcca cggcccaggt gcagcaggcc gcgacggacc tggtcggcta tgtcaaggac       600
atgatcgcca agggtgcgac gcaggtctac gtgttcaacc tgcccgacag cagcctgacg       660
ccggacggcg tggcaagcgg cacgaccggc caggcgctgc tgcacgcgct ggtgggcacg       720
ttcaacacga cgctgcaaag cgggctggcc ggcacctcgg cgcgcatcat cgacttcaac       780
gcacaactga ccgcggcgat ccagaatggc gcctcgttcg gcttcgccaa caccagcgcc       840
cgggcctgcg acgccaccaa gatcaatgcc ctggtgccga gcgccggcgg cagctcgctg       900
ttctgctcgg ccaacacgct ggtggcttcc ggtgcggacc agagctacct gttcgccgac       960
ggcgtgcacc cgaccacggc cggccatcgc ctgatcgcca gcaacgtgct ggcgcgcctg      1020
ctggcggata acgtcgcgca ctga                                            1044
```

Figure 16 (SEQ ID No. 14)

```
  1 migsyvavgd sftegvgdpg pdgafvgwad rlavlladrr pegdftytnl avrgrlldqi
 61 vaeqvprvvg lapdlvsfaa ggndiirpgt dpdevaerfe lavaaltaaa gtvlvttgfd
121 trgvpvlkhl rgkiatyngh vraiadrygc pvldlwslrs vqdrrawdad rlhlspeght
181 rvalragqal glrvpadpdq pwpplpprgt ldvrrddvhw areylvpwig rrlrgessgd
241 hvtakgtlsp daiktriaav a
```

Figure 17 (SEQ ID No. 15)

```
  1 gtgatcgggt cgtacgtggc ggtgggggac agcttcaccg agggcgtcgg cgaccccggc
 61 cccgacgggg cgttcgtcgg ctgggccgac cggctcgccg tactgctcgc ggaccggcgc
121 cccgagggcg acttcacgta cacgaacctc gccgtgcgcg gcaggctcct cgaccagatc
181 gtggcggaac aggtcccgcg ggtcgtcgga ctcgcgcccg acctcgtctc gttcgcggcg
241 ggcggcaacg acatcatccg gcccggcacc gatcccgacg aggtcgccga gcggttcgag
301 ctggcggtgg ccgcgctgac cgccgcggcc ggaaccgtcc tggtgaccac cgggttcgac
361 acccgggggg tgcccgtcct caagcacctg cgcggcaaga tcgccacgta caacgggcac
421 gtccgcgcca tcgccgaccg ctacggctgc ccggtgctcg acctgtggtc gctgcggagc
481 gtccaggacc gcagggcgtg ggacgccgac cggctgcacc tgtcgccgga ggggcacacc
541 cgggtggcgc tgcgcgcggg gcaggccctg ggcctgcgcg tcccggccga ccctgaccag
601 ccctggccgc ccctgccgcc gcgcggcacg ctcgacgtcc ggcgcgacga cgtgcactgg
661 gcgcgcgagt acctggtgcc gtggatcggg cgccggctgc ggggcgagtc gtcgggcgac
721 cacgtgacgg ccaaggggac gctgtcgccg gacgccatca agacgcggat cgccgcggtg
781 gcctga
```

Figure 18
(SEQ ID No. 16)

```
  1 mqtnpaytsl vavgdsfteg msdllpdgsy rgwadllatr maarspgfry anlavrgkli
 61 gqivdeqvdv aaamgadvit lvgglndtlr pkcdmarvrd lltqaverla phceqlvlmr
121 spgrqgpvle rfrprmealf aviddlagrh gavvvdlyga qsladprmwd vdrlhltaeg
181 hrrvaeavwq slghepedpe whapipatpp pgwvtrrtad vrfarqhllp wigrrltgrs
241 sgdglpakrp dllpyedpar
```

Figure 19 (SEQ ID No. 17)

```
  1 atgcagacga accccgcgta caccagtctc gtcgccgtcg gcgactcctt caccgagggc
 61 atgtcggacc tgctgcccga cggctcctac cgtggctggg ccgacctcct cgccacccgg
121 atggcggccc gctcccccgg cttccggtac gccaacctgg cggtgcgcgg gaagctgatc
181 ggacagatcg tcgacgagca ggtggacgtg gccgccgcca tgggagccga cgtgatcacg
241 ctggtcggcg ggctcaacga cacgctgcgg cccaagtgcg acatggcccg ggtgcgggac
301 ctgctgaccc aggccgtgga acggctcgcc ccgcactgcg agcagctggt gctgatgcgc
361 agtcccggtc gccagggtcc ggtgctggag cgcttccggc cccgcatgga ggccctgttc
421 gccgtgatcg acgacctggc cgggcggcac ggcgccgtgg tcgtcgacct gtacggggcc
481 cagtcgctgg ccgaccctcg gatgtgggac gtggaccggc tgcacctgac cgccgagggc
541 caccgccggg tcgcggaggc ggtgtggcag tcgctcggcc acgagcccga ggaccccgag
601 tggcacgcgc cgatcccggc gacgccgccg ccggggtggg tgacgcgcag gaccgcggac
661 gtccggttcg cccggcagca cctgctgccc tggataggcc gcaggctgac cgggcgctcg
721 tccggggacg gcctgccggc caagcgcccg gacctgctgc cctacgagga ccccgcacgg
781 tga
```

Figure 20 (SEQ ID No. 18)

```
  1 mtrgrdggag apptkhrall aaivtlivai saaiyagasa ddgsrdhalq aggrlprgda
 61 apastgawvg awatapaaae pgtettglag rsvrnvvhts vggtgaritl snlygqsplt
121 vthasialaa gpdtaaaiad tmrrltfggs arviipaggq vmsdtarlai pyganvlvtt
181 yspipsgpvt yhpqarqtsy ladgdrtadv tavayttptp ywryltaldv lsheadgtvv
241 afgdsitdga rsqsdanhrw tdvlaarlhe aagdgrdtpr ysvvnegisg nrlltsrpgr
301 padnpsglsr fqrdvlertn vkavvvvlgv ndvlnspela drdailtglr tlvdraharg
361 lrvvgatitp fggyggytea retmrqevne eirsgrvfdt vvdfdkalrd pydprrmrsd
421 ydsgdhlhpg dkgyarmgav idlaalkgaa pvka
```

Figure 21 (SEQ ID No. 19)

```
   1 atgacccggg gtcgtgacgg gggtgcgggg gcgcccccca ccaagcaccg tgccctgctc
  61 gcggcgatcg tcaccctgat agtggcgatc tccgcggcca tatacgccgg agcgtccgcg
 121 gacgacggca gcagggacca cgcgctgcag gccggaggcc gtctcccacg aggagacgcc
 181 gcccccgcgt ccaccggtgc ctgggtgggc gcctgggcca ccgcaccggc cgcggccgag
 241 ccgggcaccg agacgaccgg cctggcgggc cgctccgtgc gcaacgtcgt gcacacctcg
 301 gtcggcggca ccggcgcgcg gatcaccctc tcgaacctgt acgggcagtc gccgctgacc
 361 gtcacacacg cctcgatcgc cctggccgcc gggcccgaca ccgccgccgc gatcgccgac
 421 accatgcgcc ggctcacctt cggcggcagc gcccgggtga tcatcccggc gggcggccag
 481 gtgatgagcg acaccgcccg cctcgccatc ccctacgggg cgaacgtcct ggtcaccacg
 541 tactccccca tcccgtccgg gccggtgacc taccatccgc aggcccggca gaccagctac
 601 ctggccgacg gcgaccgcac ggcggacgtc accgccgtcg cgtacaccac ccccacgccc
 661 tactggcgct acctgaccgc cctcgacgtg ctgagccacg aggccgacgg cacggtcgtg
 721 gcgttcggcg actccatcac cgacggcgcc cgctcgcaga gcgacgccaa ccaccgctgg
 781 accgacgtcc tcgccgcacg cctgcacgag gcggcgggcg acggccggga cacgccccgc
 841 tacagcgtcg tcaacgaggg catcagcggc aaccggctcc tgaccagcag gccggggcgg
 901 ccggccgaca acccgagcgg actgagccgg ttccagcggg acgtgctgga acgcaccaac
 961 gtcaaggccg tcgtcgtcgt cctcggcgtc aacgacgtcc tgaacagccc ggaactcgcc
1021 gaccgcgacg ccatcctgac cggcctgcgc accctcgtcg accgggcgca cgcccgggga
1081 ctgcgggtcg tcggcgccac gatcacgccg ttcggcggct acggcggcta caccgaggcc
1141 cgcgagacga tgcggcagga ggtcaacgag gagatccgct ccggccgggt cttcgacacg
1201 gtcgtcgact tcgacaaggc cctgcgcgac ccgtacgacc cgcgccggat gcgctccgac
1261 tacgacagcg gcgaccacct gcaccccggc gacaaggggt acgcgcgcat gggcgcggtc
1321 atcgacctgg ccgcgctgaa gggcgcggcg ccggtcaagg cgtag
```

# Figure 22 (SEQ ID No. 20)

```
  1 mtsmsrarva rriaagaayg gggiglagaa avglvvaevq larrrvgvgt ptrvpnaqgl
 61 yggtlptagd pplrlmmlgd staagqgvhr agqtpgalla sglaavaerp vrlgsvaqpg
121 acsddldrqv alvlaepdrv pdicvimvga ndvthrmpat rsvrhlssav rrlrtagaev
181 vvgtcpdlgt iervrqplrw larrasrqla aaqtigaveq ggrtvslgdl lgpefaqnpr
241 elfgpdnyhp saegyataam avlpsvcaal glwpadeehp dalrregflp varaaaeaas
301 eagtevaaam ptgprgpwal lkrrrrrrvs eaepsspsgv
```

# Figure 23 (SEQ ID No. 21)

```
   1 atgacgagca tgtcgagggc gagggtggcg cggcggatcg cggccggcgc ggcgtacggc
  61 ggcggcggca tcggcctggc gggagcggcg gcggtcggtc tggtggtggc cgaggtgcag
 121 ctggccagac gcagggtggg ggtgggcacg ccgacccggg tgccgaacgc gcagggactg
 181 tacggcggca ccctgcccac ggccggcgac ccgccgctgc ggctgatgat gctgggcgac
 241 tccacggccg ccgggcaggg cgtgcaccgg gccgggcaga cgccgggcgc gctgctggcg
 301 tccgggctcg cggcggtggc ggagcggccg gtgcggctgg ggtcggtcgc ccagccgggg
 361 gcgtgctcgg acgacctgga ccggcaggtg gcgctggtgc tcgccgagcc ggaccgggtg
 421 cccgacatct gcgtgatcat ggtcggcgcc aacgacgtca cccaccggat gccggcgacc
 481 cgctcggtgc ggcacctgtc ctcggcggta cggcggctgc gcacggccgg tgcggaggtg
 541 gtggtcggca cctgtccgga cctgggcacg atcgagcggg tgcggcagcc gctgcgctgg
 601 ctggcccggc gggcctcacg gcagctcgcg gcggcacaga ccatcggcgc cgtcgagcag
 661 ggcgggcgca cggtgtcgct gggcgacctg ctgggtccgg agttcgcgca gaacccgcgg
 721 gagctcttcg gccccgacaa ctaccacccc tccgccgagg ggtacgccac ggccgcgatg
 781 gcggtactgc cctcggtgtg cgccgcgctc ggcctgtggc cggccgacga ggagcacccg
 841 gacgcgctgc gccgcgaggg cttcctgccg gtggcgcgcg cggcggcgga ggcggcgtcc
 901 gaggcgggta cggaggtcgc cgccgccatg cctacggggc ctcgggggcc ctgggcgctg
 961 ctgaagcgcc ggagacggcg tcgggtgtcg gaggcggaac cgtccagccc gtccggcgtt
1021 tga
```

## Figure 24 (SEQ ID No. 22)

```
  1 mgrgtdqrtr ygrrrarval aaltaavlgv gvagcdsvgg dspapsgsps krtrtapawd
 61 tspasvaavg dsitrgfdac avlsdcpevs watgssakvd slavrllgka daaehswnya
121 vtgarmadlt aqvtraaqre pelvavmaga ndacrsttsa mtpvadfraq feeamatlrk
181 klpkaqvyvs sipdlkrlws qgrtnplgkq vwklglcpsm lgdadsldsa atlrrntvrd
241 rvadynevlr evcakdrrcr sddgavhefr fgtdqlshwd wfhpsvdgqa rlaeiayrav
301 taknp
```

## Figure 25 (SEQ ID No. 23)

```
  1 atgggtcgag ggacggacca gcggacgcgg tacggccgtc gccgggcgcg tgtcgcgctc
 61 gccgccctga ccgccgccgt cctgggcgtg ggcgtggcgg gctgcgactc cgtgggcggc
121 gactcacccg ctccttccgg cagcccgtcg aagcggacga ggacggcgcc cgcctgggac
181 accagcccgg cgtccgtcgc cgccgtgggc gactccatca cgcgcggctt cgacgcctgt
241 gcggtgctgt cggactgccc ggaggtgtcg tgggcgaccg gcagcagcgc gaaggtcgac
301 tcgctggccg tacggctgct ggggaaggcg gacgcggccg agcacagctg gaactacgcg
361 gtcaccgggg cccggatggc ggacctgacc gctcaggtga cgcgggcggc gcagcgcgag
421 ccggagctgg tggcggtgat ggccggggcg aacgacgcgt gccggtccac gacctcggcg
481 atgacgccgg tggcggactt ccgggcgcag ttcgaggagg cgatggccac cctgcgcaag
541 aagctcccca aggcgcaggt gtacgtgtcg agcatcccgg acctcaagcg gctctggtcc
601 cagggccgca ccaacccgct gggcaagcag gtgtggaagc tcggcctgtg cccgtcgatg
661 ctgggcgacg cggactccct ggactcggcg gcgaccctgc ggcgcaacac ggtgcgcgac
721 cgggtggcgg actacaacga ggtgctgcgg gaggtctgcg cgaaggaccg gcggtgccgc
781 agcgacgacg gcgcggtgca cgagttccgg ttcggcacgg accagttgag ccactgggac
841 tggttccacc cgagtgtgga cggccaggcc cggctggcgg agatcgccta ccgcgcggtc
901 accgcgaaga atccctga
```

## Figure 26 (SEQ ID No. 24)

```
  1 mrlsrraata sallltpala lfgasaavsa priqatdyva lgdsyssgvg agsydsssgs
 61 ckrstksypa lwaashtgtr fnftacsgar tgdvlakqlt pvnsgtdlvs itiggndagf
121 adtmttcnlq gesaclaria karayiqqtl paqldqvyda idsrapaaqv vvlgyprfyk
181 lggscavgls eksraainaa addinavtak raadhgfafg dvnttfaghe lcsgapwlhs
241 vtlpvensyh ptangqskgy lpvlnsat
```

## Figure 27 (SEQ ID No. 25)

```
   1 ttcatcacaa cgatgtcaca acaccggcca tccgggtcat ccctgatcgt gggaatgggt
  61 gacaagcctt cccgtgacga aagggtcctg ctacatcaga aatgacagaa atcctgctca
 121 gggaggttcc atgagactgt cccgacgcgc ggccacggcg tccgcgctcc tcctcacccc
 181 ggcgctcgcg ctcttcggcg cgagcgccgc cgtgtccgcg ccgcgaatcc aggccaccga
 241 ctacgtggcc ctcggcgact cctactcctc ggggtcggc gcgggcagct acgacagcag
 301 cagtggctcc tgtaagcgca gcaccaagtc ctacccggcc ctgtgggccg cctcgcacac
 361 cggtacgcgg ttcaacttca ccgcctgttc gggcgcccgc acaggagacg tgctggccaa
 421 gcagctgacc ccggtcaact ccggcaccga cctggtcagc attaccatcg gcggcaacga
 481 cgcgggcttc gccgacacca tgaccacctg caacctccag ggcgagagcg cgtgcctggc
 541 gcggatcgcc aaggcgcgcg cctacatcca gcagacgctg cccgcccagc tggaccaggt
 601 ctacgacgcc atcgacagcc gggcccccgc agcccaggtc gtcgtcctgg gctacccgcg
 661 cttctacaag ctgggcggca gctgcgccgt cggtctctcg gagaagtccc gcgcggccat
 721 caacgccgcc gccgacgaca tcaacgccgt caccgccaag cgcgccgccg accacggctt
 781 cgccttcggg gacgtcaaca cgaccttcgc cgggcacgag ctgtgctccg gcgccccctg
 841 gctgcacagc gtcacccttc ccgtggagaa ctcctaccac cccacggcca acggacagtc
 901 caagggctac ctgcccgtcc tgaactccgc cacctgatct cgcggctact ccgcccctga
 961 cgaagtcccg cccccgggcg gggcttcgcc gtaggtgcgc gtaccgccgt cgcccgtcgc
1021 gccggtggcc ccgccgtacg tgccgccgcc cccggacgcg gtcggttc
```

Figure 28 (SEQ ID No. 26)

```
  1  MKKWFVCLLG LVALTVQAAD SRPAFSRIVM FGDSLSDTGK MYSKMRGYLP
 51  SSPPYYEGRF SNGPVWLEQL TKQFPGLTIA NEAEGGATAV AYNKISWNPK
101  YQVINNLDYE VTQFLQKDSF KPDDLVILWV GANDYLAYGW NTEQDAKRVR
151  DAISDAANRM VLNGAKQILL FNLPDLGQNP SARSQKVVEA VSHVSAYHNQ
201  LLLNLARQLA PTGMVKLFEI DKQFAEMLRD PQNFGLSDVE NPCYDGGYVW
251  KPFATRSVST DRQLSAFSPQ ERLAIAGNPL LAQAVASPMA RRSASPLNCE
301  GKMFWDQVHP TTVVHAALSE RAATFIANQY EFLAH*
```

# Figure 29 (SEQ ID No. 27)

```
   1  ATGAAAAAAT GGTTTGTGTG TTTATTGGGA TTGGTCGCGC TGACAGTTCA
      TACTTTTTTA CCAAACACAC AAATAACCCT AACCAGCGCG ACTGTCAAGT

  51  GGCAGCCGAC AGTCGCCCCG CCTTTTCCCG GATCGTGATG TTCGGCGACA
      CCGTCGGCTG TCAGCGGGGC GGAAAAGGGC CTAGCACTAC AAGCCGCTGT

 101  GCCTCTCCGA TACCGGCAAA ATGTACAGCA AGATGCGCGG TTACCTCCCC
      CGGAGAGGCT ATGGCCGTTT TACATGTCGT TCTACGCGCC AATGGAGGGG

 151  TCCAGCCCGC CCTACTATGA GGGCCGTTTC TCCAACGGAC CCGTCTGGCT
      AGGTCGGGCG GGATGATACT CCCGGCAAAG AGGTTGCCTG GGCAGACCGA

 201  GGAGCAGCTG ACCAAACAGT TCCCGGGTCT GACCATCGCC AACGAAGCGG
      CCTCGTCGAC TGGTTTGTCA AGGGCCCAGA CTGGTAGCGG TTGCTTCGCC

 251  AAGGCGGTGC CACTGCCGTG GCTTACAACA AGATCTCCTG GAATCCCAAG
      TTCCGCCACG GTGACGGCAC CGAATGTTGT TCTAGAGGAC CTTAGGGTTC

 301  TATCAGGTCA TCAACAACCT GGACTACGAG GTCACCCAGT TCTTGCAGAA
      ATAGTCCAGT AGTTGTTGGA CCTGATGCTC CAGTGGGTCA AGAACGTCTT

 351  AGACAGCTTC AAGCCGGACG ATCTGGTGAT CCTCTGGGTC GGTGCCAATG
      TCTGTCGAAG TTCGGCCTGC TAGACCACTA GGAGACCCAG CCACGGTTAC

 401  ACTATCTGGC CTATGGCTGG AACACGGAGC AGGATGCCAA GCGGGTTCGC
      TGATAGACCG GATACCGACC TTGTGCCTCG TCCTACGGTT CGCCCAAGCG

 451  GATGCCATCA GCGATGCGGC CAACCGCATG GTACTGAACG GTGCCAAGCA
      CTACGGTAGT CGCTACGCCG GTTGGCGTAC CATGACTTGC CACGGTTCGT

 501  GATACTGCTG TTCAACCTGC CGGATCTGGG CCAGAACCCG TCAGCTCGCA
      CTATGACGAC AAGTTGGACG GCCTAGACCC GGTCTTGGGC AGTCGAGCGT

 551  GTCAGAAGGT GGTCGAGGCG GTCAGCCATG TCTCCGCCTA TCACAACCAG
      CAGTCTTCCA CCAGCTCCGC CAGTCGGTAC AGAGGCGGAT AGTGTTGGTC

 601  CTGCTGCTGA ACCTGGCACG CCAGCTGGCC CCCACCGGCA TGGTAAAGCT
      GACGACGACT TGGACCGTGC GGTCGACCGG GGGTGGCCGT ACCATTTCGA

 651  GTTCGAGATC GACAAGCAAT TTGCCGAGAT GCTGCGTGAT CCGCAGAACT
      CAAGCTCTAG CTGTTCGTTA AACGGCTCTA CGACGCACTA GGCGTCTTGA

 701  TCGGCCTGAG CGACGTCGAG AACCCCTGCT ACGACGGCGG CTATGTGTGG
      AGCCGGACTC GCTGCAGCTC TTGGGGACGA TGCTGCCGCC GATACACACC

 751  AAGCCGTTTG CCACCCGCAG CGTCAGCACC GACCGCCAGC TCTCCGCCTT
      TTCGGCAAAC GGTGGGCGTC GCAGTCGTGG CTGGCGGTCG AGAGGCGGAA

 801  CAGTCCGCAG GAACGCCTCG CCATCGCCGG CAACCCGCTG CTGGCACAGG
      GTCAGGCGTC CTTGCGGAGC GGTAGCGGCC GTTGGGCGAC GACCGTGTCC

 851  CCGTTGCCAG TCCTATGGCC CGCCGCAGCG CCAGCCCCCT CAACTGTGAG
      GGCAACGGTC AGGATACCGG GCGGCGTCGC GGTCGGGGGA GTTGACACTC

 901  GGCAAGATGT TCTGGGATCA GGTACACCCG ACCACTGTCG TGCACGCAGC
      CCGTTCTACA AGACCCTAGT CCATGTGGGC TGGTGACAGC ACGTGCGTCG

 951  CCTGAGCGAG CGCGCCGCCA CCTTCATCGC GAACCAGTAC GAGTTCCTCG
      GGACTCGCTC GCGCGGCGGT GGAAGTAGCG CTTGGTCATG CTCAAGGAGC

1001  CCCAC TGA
      GGGTG ACT
```

Figure 30 (SEQ ID No. 28)

```
  1  MKKWFVCLLG LIALTVQAAD TRPAFSRIVM FGDSLSDTGK MYSKMRGYLP
 51  SSPPYYEGRF SNGPVWLEQL TKQFPGLTIA NEAEGGATAV AYNKISWNPK
101  YQVINNLDYE VTQFLQKDSF KPDDLVILWV GANDYLAYGW NTEQDAKRVR
151  DAISDAANRM VLNGAKQILL FNLPDLGQNP SARSQKVVEA VSHVSAYHNK
201  LLLNLARQLA PTGMVKLFEI DKQFAEMLRD PQNFGLSDVE NPCYDGGYVW
251  KPFATRSVST DRQLSAFSPQ ERLAIAGNPL LAQAVASPMA RRSASPLNCE
301  GKMFWDQVHP TTVVHAALSE RAATFIETQY EFLAHG*
```

Figure 31 (SEQ ID No. 29)

```
   1  ATGAAAAAAT GGTTTGTTTG TTTATTGGGG TTGATCGCGC TGACAGTTCA
      TACTTTTTTA CCAAACAAAC AAATAACCCC AACTAGCGCG ACTGTCAAGT

  51  GGCAGCCGAC ACTCGCCCCG CCTTCTCCCG GATCGTGATG TTCGGCGACA
      CCGTCGGCTG TGAGCGGGGC GGAAGAGGGC CTAGCACTAC AAGCCGCTGT

 101  GCCTCTCCGA TACCGGCAAA ATGTACAGCA AGATGCGCGG TTACCTCCCC
      CGGAGAGGCT ATGGCCGTTT TACATGTCGT TCTACGCGCC AATGGAGGGG

 151  TCCAGCCCGC CCTACTATGA GGGCCGTTTC TCCAACGGAC CCGTCTGGCT
      AGGTCGGGCG GGATGATACT CCCGGCAAAG AGGTTGCCTG GGCAGACCGA

 201  GGAGCAGCTG ACCAAGCAGT TCCCGGGTCT GACCATCGCC AACGAAGCGG
      CCTCGTCGAC TGGTTCGTCA AGGGCCCAGA CTGGTAGCGG TTGCTTCGCC

 251  AAGGCGGTGC CACTGCCGTG GCTTACAACA AGATCTCCTG GAATCCCAAG
      TTCCGCCACG GTGACGGCAC CGAATGTTGT TCTAGAGGAC CTTAGGGTTC

 301  TATCAGGTCA TCAACAACCT GGACTACGAG GTCACCCAGT TCTTGCAGAA
      ATAGTCCAGT AGTTGTTGGA CCTGATGCTC CAGTGGGTCA AGAACGTCTT

 351  AGACAGCTTC AAGCCGGACG ATCTGGTGAT CCTCTGGGTC GGTGCCAATG
      TCTGTCGAAG TTCGGCCTGC TAGACCACTA GGAGACCCAG CCACGGTTAC

 401  ACTATCTGGC ATATGGCTGG AATACGGAGC AGGATGCCAA GCGAGTTCGC
      TGATAGACCG TATACCGACC TTATGCCTCG TCCTACGGTT CGCTCAAGCG

 451  GATGCCATCA GCGATGCGGC CAACCGCATG GTACTGAACG GTGCCAAGCA
      CTACGGTAGT CGCTACGCCG GTTGGCGTAC CATGACTTGC CACGGTTCGT

 501  GATACTGCTG TTCAACCTGC CGGATCTGGG CCAGAACCCG TCAGCCCGCA
      CTATGACGAC AAGTTGGACG GCCTAGACCC GGTCTTGGGC AGTCGGGCGT

 551  GTCAGAAGGT GGTCGAGGCG GTCAGCCATG TCTCCGCCTA TCACAACAAG
      CAGTCTTCCA CCAGCTCCGC CAGTCGGTAC AGAGGCGGAT AGTGTTGTTC

 601  CTGCTGCTGA ACCTGGCACG CCAGCTGGCC CCCACCGGCA TGGTAAAGCT
      GACGACGACT TGGACCGTGC GGTCGACCGG GGGTGGCCGT ACCATTTCGA

 651  GTTCGAGATC GACAAGCAAT TTGCCGAGAT GCTGCGTGAT CCGCAGAACT
      CAAGCTCTAG CTGTTCGTTA AACGGCTCTA CGACGCACTA GGCGTCTTGA

 701  TCGGCCTGAG CGACGTCGAG AACCCCTGCT ACGACGGCGG CTATGTGTGG
      AGCCGGACTC GCTGCAGCTC TTGGGGACGA TGCTGCCGCC GATACACACC

 751  AAGCCGTTTG CCACCCGCAG CGTCAGCACC GACCGCCAGC TCTCCGCCTT
      TTCGGCAAAC GGTGGGCGTC GCAGTCGTGG CTGGCGGTCG AGAGGCGGAA

 801  CAGTCCGCAG GAACGCCTCG CCATCGCCGG CAACCCGCTG CTGGCACAGG
      GTCAGGCGTC CTTGCGGAGC GGTAGCGGCC GTTGGGCGAC GACCGTGTCC

 851  CCGTTGCCAG TCCTATGGCC CGCCGCAGCG CCAGCCCCCT CAACTGTGAG
      GGCAACGGTC AGGATACCGG GCGGCGTCGC GGTCGGGGGA GTTGACACTC

 901  GGCAAGATGT TCTGGGATCA GGTACACCCG ACCACTGTCG TGCACGCAGC
      CCGTTCTACA AGACCCTAGT CCATGTGGGC TGGTGACAGC ACGTGCGTCG

 951  CCTGAGCGAG CGCGCCGCCA CCTTCATCGA GACCCAGTAC GAGTTCCTCG
      GGACTCGCTC GCGCGGCGGT GGAAGTAGCT CTGGGTCATG CTCAAGGAGC

1001  CCCACGGATG A
      GGGTGCCTAC T
```

Figure 32

```
            1       10        20        30        40        50
            |--------+---------+---------+---------+---------|
      satA     ADTRPAFSRIVMFGDSLSDTGKMYSKMRGYLPSSPPYYEGRFSN--G
     R.sol  QSGNPNVAKVQRMVVFGDSLSDIGT-------YTPVAQAVGGGKFTTNPG
 Consensus  ...adnraafqRiVmFGDSLSDiGk.......YlPsaqayggeGrFsn..G

            51      60        70        80        90       100
            |--------+---------+---------+---------+---------|
      satA  PVWLEQLTKQFPGLTIANEAEGGATAVAYNKISWNPKYQVINNLDYEVTQ
     R.sol  PIWAETVAAQL-GVTLTPAVHGYATSVQNCPKAGCFDYAQGGSRVTDPNG
 Consensus  P!WaEqlaaQl.GlTianaaeGgATaVannkiagnfdYaqgnnrdt#pnq

            101     110       120       130       140      150
            |--------+---------+---------+---------+---------|
      satA  FLQKDSFKPDDLVILWVGANDYLAYG--WNTEQDAKRVRDAISDAANRMV
     R.sol  IGHNGGAGALTYPVQQQLANFYAASNNTFNGNNDVVFVLAGSNDIFFWTT
 Consensus  igqndgagaddlp!qqqgANdYaAsn..fNg##DakrVraainDaanrmt

            151     160       170       180       190      200
            |--------+---------+---------+---------+---------|
      satA  LNGAKQILLFNLPDLGQNPSARSQKVVEAVSHVSAYHNKL-LLNLARQLA
     R.sol  AAATSGSGVTPAIATAQVQQAATDLVGYVKDMIAKGATQVVVFNLPDSSL
 Consensus  aaaakqiglfnaialaQnqqAas#lVgeakdh!aaganql.llNLarqla

            201     210       220       230       240      250
            |--------+---------+---------+---------+---------|
      satA  PTGMVKLFEIDKQFAEMLRDPQNFGLSDVENPCYDGGYVWKPFATRSVST
     R.sol  TPDGVASGTTGQALLHALVGTFNTTLQSGLAGTSARIIDFNAQLTAAIQN
 Consensus  ppdgValgeidqalaeaLrdpqNfgLqdgeagcsargidfnaqaTaa!qn

            251     260       270       280       290      300
            |--------+---------+---------+---------+---------|
      satA  DRQLSAFSPQERLAIAG--NPLLAQAVASPM---ARRSASPLNCEGKMFW
     R.sol  GASFGFANTSARACDATKINALVPSAGGSSLFCSANTLVASGADQSYLFA
 Consensus  daqlgaanpqaRaadAg..NaLlaqAgaSp$...Arrlaapgad#gk$Fa

            301     310       320       330
            |--------+---------+---------|
      satA  DQVHPTTVVHAALSERAATFIETQYEFLAH
     R.sol  DGVHPTTAGHRLIASNVLARLLA--DNVAH
 Consensus  DqVHPTTagHaaiaeraaariea..#nlAH
```

Figure 33

```
                            ▼
Pfam         *->ivafGDSltdggg..............ayygdsdgggwgagladrltsla..rlrargrgvdv
Srim1   38      YVALGDSYSSGVG............agSYDSSSGSCKRSTKSYPALWAAS..-----HTGTRF 81
Scoe1    5      YVAVGDSFTEG--..............--VGDPGPDGAFVGWADRLAVLL..ADRRPEGDFTY 47
Scoe2   10      LVAVGDSFTEG--..............--MSDLLPDGSYRGWADLLATRM..--AARSPGFRY 50
Scoe3  239      VVAFGDSITDG--..............ARSQSDANHRWTDVLAARLHEAA..GDGRDTPRYSV 283
Scoe4   75      LMMLGDSTAAG--..............------QGVHRAGQTPGALLASG..LAAVAERPVRL 113
Scoe5   66      VAAVGDSITRGFD............acAVLSDCPEVSWATGSSAKVDSLAvrLLGKADAAEHS 116
Ahyd1   28      IVMFGDSLSDTGKmyskmrgylpssppyYEGRFSNGPVWLEQLTNEFPGLTiaNEAEGGPTAVA 91
Asal1   28      IVMFGDSLSDTGKmyskmrgylpssppyYEGRFSNGPVWLEQLTKQF----..------PGLTI 79
Ahyd2   40      IVMFGDSLSDTGKmyskmrgylpssppyYEGRFSNGPVWLEQLTKQFPGLTiaNEAEGGATAVA 103

Pfam         fnrgisGrtsdGrlvvDarlvatllFlaqflGlnlpPYLsgdflrGANFAsagAtIlgtslipflni
Srim1   82 NFTACSGAR---------------------------------------------------------- 90
Scoe1   48 TNLAVRGRL---------------------------------------------------------- 56
Scoe2   51 ANLAVRGKL---------------------------------------------------------- 59
Scoe3  284 VNEGISGNR---------------------------------------------------------- 292
Scoe4  114 GSVAQPGAC---------------------------------------------------------- 122
Scoe5  117 WNYAVTGAR---------------------------------------------------------- 125
Ahyd1     92 YNKISWNPK---------------------------------------------------------- 100
Asal1     80 ANEAEGGAT---------------------------------------------------------- 88
Ahyd2  104 YNKISWNPK---------------------------------------------------------- 112

                                                          ▼
Pfam         QvqFkdfkskvlelrqa......lgllqellrlvpvldakspdlvtimiGtNDl...itvakfgpks
Srim1   91 -----------------......---TGDVLAKQLTPVNSGTDLVSITIGGNDAgfaDTMTTCNLQG 131
Scoe1   57 -----------------......--LDQIVAEQVPRVVGLAPDLVSFAAGGNDI...-----I---- 86
Scoe2   60 -----------------......--IGQIVDEQVDVAAAMGADVITLVGGLNDT...---------- 88
Scoe3  293 -------LLTSRPGRPA......DNPSGLSRFQRDVLERTNVKAVVVVLGVNDV...---------- 333
Scoe4  123 -----------------......SDDLDRQVALVLAEPDRVPDICVIMVGANDV...---------- 153
Scoe5  126 -----------------......---MADLTAQVTRAAQREPELVAVMAGANDA...--------CR 155
Ahyd1  101 -------------YQVI......NNLDYEVTQFLQKDSFKPDDLVILWVGANDY...--------LA 137
Asal1     89 -------AVAYNKISWNpkyqvyNNLDYEVTQFLQKDSFKPDDLVILWVGANDY...--------LA 137
Ahyd2  113 -------------YQVI......NNLDYEVTQFLQKDSFKPDDLVILWVGANDY...--------LA 149

Pfam         ......tksdrnvsvpefrdnlrklikrLrsangariiilitlvllnlpl..........plGCl
Srim1  132 esaclarIAKARAYIQQTLPAQLDQVYDAIDSRAPAA-----QVVVLGYP-..........----- 176
Scoe1   87 ......---RPGTDPDEVAERFELAVAALT-AAAGTVLVTTGFDTRGVP-..........----- 125
Scoe2   89 ......---------LRPKCDMARVRDLLTQAVERLAPHCEQLVLMRSP-..........----- 122
Scoe3  334 ......LNSPELADRDAILTGLRTLVDRAHARGLRVVGATITPFGGYGG-..........----- 376
Scoe4  154 ......---THRMPATRSVRHLSSAVRRLR-TAGAEVVVGTCPDLGTIE-..........----- 192
Scoe5  156 ......STTSAMTPVADFRAQFEEAMATLR-KKLPKAQVYVSSIPDLKRLwsqgrtnplgkQVWKL 214
Ahyd1  138 ......YGWNTEQDAKRVRDAISDAANRMV-LNGAK-----EILLFNLP-..........----- 174
Asal1  138 ......YGWNTEQDAKRVRDAISDAANRMV-LNGAK-----QILLFNLP-..........----- 174
Ahyd2  150 ......YGWNTEQDAKRVRDAISDAANRMV-LNGAK-----QILLFNLP-..........----- 186

Pfam         pq.klalalassknvdatgclerlneavadynealrelaei.ek.l.q.aqlrkdglpdlkeanvpy
Srim1  177 --.RFYKLGGSCAVGLSEKSRAAINAAADDINAVTAKRA--.--.-.-.-----------ADHGFAF 219
Scoe1  126 --.----------------VLKHLRGKIATYNGHVRAIA--.--.-.-.-----------DRYGCPV 152
Scoe2  123 --.------------GRQGPVLERFRPRMEALFAVIDDLA--.--.-.-.-----------GRHGAVV 154
Scoe3  377 --.YTEARETMRQEVNEEIRSGRVFDTVVDFDKALRDPY--.--.-.-.------------------ 412
Scoe4  193 --.----------------------------RVRQPLRWLaRRaSrQlAAAQTIGAVEQGGRTVSL 227
Scoe5  215 GLcPSMLGDADSLDSAATLRRNTVRDRVADYNEVLREVC--.--.-.AkDRRCRSDDGAVHEFRFGT 273
Ahyd1  175 --.----DLGQNPSARSQKVVEAASHVSAYHNQLLLNLA--.--.-.-.RQLAPTGMVKLFEIDKQF 224
Asal1  175 --.----DLGQNPSARSQKVVEAVSHVSAYHNKLLLNLA--.--.-.-.RQLAPTGMVKLFEIDKQF 224
Ahyd2  187 --.----DLGQNPSARSQKVVEAVSHVSAYHNQLLLNLA--.--.-.-.RQLAPTGMVKLFEIDKQF 236

Pfam         VDlysifqdldgiqnpsayv.y....GFeet.kaCCGyGgr.yNyn.rv.CGnag.l.ck.vtakaC
Srim1  220 GDVNT---------------.-....-----.---------.-TFAgHElCSGAPwL.HS.VT---- 242
Scoe1  153 LDLWSLRSVQDRRA------.-....-----.---------.----.--.-----.-.--.------ 166
Scoe2  155 VDLYGAQSLADPRM------.-....-----.---------.----.--.-----.-.--.------ 168
```

```
Scoe3  413 -------------------.-...-----   .---------.----.--.-----.-.--.------ 413
Scoe4 228 GDLLGPEFAQNPREL-----.-...-----.---------.----.--.-----.-.--.------ 242
Scoe5   274 DQL-----------------.-...-----.---------.----.--.-----.-.--.------ 276
Ahyd1 225 AEMLRDPQNFGLSDQRNACYgGsyvwKPFASrSASTDSQLSaFNPQeRLaIAGNPlLaQAvASPMAA 291
Asal1 225 AEMLRDPQNFGLSDVENPCYdGgyvwKPFATrSVSTDRQLSaFSPQeRLaIAGNPlLaQAvASPMAR 291
Ahyd2 237 AEMLRDPQNFGLSDVENPCYdGgyvwKPFATrSVSTDRQLSaFSPQeRLaIAGNPlLaQAvASPMAR 303

                            ▼
Pfam      .dassyll.atlfwDgf.HpsekGykavAeal<-*
Srim1 243 .-------.--LPVENSyHPTANGQSKGYLPV    263
Scoe1 167 .-------.--WDADRL.HLSPEGHTRVALRA    186
Scoe2 169 .-------.--WDVDRL.HLTAEGHRRVAEAV    188
Scoe3 413 .-DPRRMRsDYDSGDHL.HPGDKGYARMGAVI    441
Scoe4 243 .-------.--FGPDNY.HPSAEGYATAAMAV    262
Scoe5 277 .-------.--SHWDWF.HPSVDGQARLAEIA    296
Ahyd1 292 rSASTLNCeGKMFWDQV.HPTTVVHAALSEPA    322
Asal1 292 rSASPLNCeGKMFWDQV.HPTTVVHAALSERA    322
Ahyd2 304 rSASPLNCeGKMFWDQV.HPTTVVHAALSERA    334
```

# Figure 34

```
                                         ▼
Pfam      *->ivafGDSltdggg..............ayygdsdgggwgagladrltsla..rlrargrgvdv
Sriml  38   YVALGDSYSSGVG............agSYDSSSGSCKRSTKSYPALWAAS..-----HTGTRF 81
Scoel   5   YVAVGDSFTEG--...............--VGDPGPDGAFVGWADRLAVLL..ADRRPEGDFTY 47
Scoe2  10   LVAVGDSFTEG--...............--MSDLLPDGSYRGWADLLATRM..--AARSPGFRY 50
Ahyd1  28   IVMFGDSLSDTGKmyskmrgylpssppyYEGRFSNGPVWLEQLTNEFPGLTiaNEAEGGPTAVA 91
Asal1  28   IVMFGDSLSDTGKmyskmrgylpssppyYEGRFSNGPVWLEQLTKQF----.------PGLTI 79
Ahyd2  40   IVMFGDSLSDTGKmyskmrgylpssppyYEGRFSNGPVWLEQLTKQFPGLTiaNEAEGGATAVA 103

Pfam      fnrgisGrtsdGrlvvDarlvatllFlaqflGlnlpPYLsgdflrGANFAsagAtIlgtslipflni
Sriml  82 NFTACSGAR---------------------------------------------------------- 90
Scoel  48 TNLAVRGRL---------------------------------------------------------- 56
Scoe2  51 ANLAVRGKL---------------------------------------------------------- 59
Ahyd1  92 YNKISWNPK---------------------------------------------------------- 100
Asal1  80 ANEAEGGAT---------------------------------------------------------- 88
Ahyd2 104 YNKISWNPK---------------------------------------------------------- 112

                                                                ▼
Pfam      QvqFkdfkskvlelrqa......lgllqellrlvpvldakspdlvtimiGtNDl...itvakfgpks
Sriml  91 ----------------......---TGDVLAKQLTPVNSGTDLVSITIGGNDAgfaDTMTTCNLQG 131
Scoel  57 ----------------......--LDQIVAEQVPRVVGLAPDLVSFAAGGNDI...-----I---- 86
Scoe2  60 ----------------......--IGQIVDEQVDVAAAMGADVITLVGGLNDT...---------- 88
Ahyd1 101 ------------YQVI......NNLDYEVTQFLQKDSFKPDDLVILWVGANDY...---------LA 137
Asal1  89 -------AVAYNKISWNpkyqvyNNLDYEVTQFLQKDSFKPDDLVILWVGANDY...---------LA 137
Ahyd2 113 ------------YQVI......NNLDYEVTQFLQKDSFKPDDLVILWVGANDY...---------LA 149

Pfam      .......tksdrnvsvpefrdnlrklikrLrsangariiilitlvllnlplplGCl
Sriml 132 esaclarIAKARAYIQQTLPAQLDQVYDAIDSRAPAA-----QVVVLGYP------ 176
Scoel  87 .......---RPGTDPDEVAERFELAVAALT-AAAGTVLVTTGFDTRGVP------ 125
Scoe2  89 .......---------LRPKCDMARVRDLLTQAVERLAPHCEQLVLMRSP------ 122
Ahyd1 138 .......YGWNTEQDAKRVRDAISDAANRMV-LNGAK-----EILLFNLP------ 174
Asal1 138 .......YGWNTEQDAKRVRDAISDAANRMV-LNGAK-----QILLFNLP------ 174
Ahyd2 150 .......YGWNTEQDAKRVRDAISDAANRMV-LNGAK-----QILLFNLP------ 186

Pfam      pqklalalassknvdatgclerlneavadynealrelaeieklqaqlrkdglpdlkeanvpy
Sriml 177 --RFYKLGGSCAVGLSEKSRAAINAAADDINAVTAKRA-----------------ADHGFAF 219
Scoel 126 ------------------VLKHLRGKIATYNGHVRAIA-----------------DRYGCPV 152
Scoe2 123 --------------GRQGPVLERFRPRMEALFAVIDDLA------------------GRHGAVV 154
Ahyd1 175 ------DLGQNPSARSQKVVEAASHVSAYHNQLLLNLA------RQLAPTGMVKLFEIDKQF 224
Asal1 175 ------DLGQNPSARSQKVVEAVSHVSAYHNKLLLNLA------RQLAPTGMVKLFEIDKQF 224
Ahyd2 187 ------DLGQNPSARSQKVVEAVSHVSAYHNQLLLNLA------RQLAPTGMVKLFEIDKQF 236

Pfam      VDlysifqdldgiqnpsayv.y....GFeet.kaCCGyGgr.yNyn.rv.CGnag.l.ck.vtakaC
Sriml 220 GDVNT---------------.-...-----.---------.-TFAgHElCSGAPwL.HS.VT---- 242
Scoel 153 LDLWSLRSVQDRRA------.-...-----.---------.----.--.-----.-.--.------ 166
Scoe2 155 VDLYGAQSLADPRM------.-...-----.---------.----.--.-----.-.--.------ 168
Ahyd1 225 AEMLRDPQNFGLSDQRNACYgGsyvwKPFASrSASTDSQLSaFNPQeRLaIAGNPlLaQAvASPMAA 291
Asal1 225 AEMLRDPQNFGLSDVENPCYdGgyvwKPFATrSVSTDRQLSaFSPQeRLaIAGNPlLaQAvASPMAR 291
Ahyd2 237 AEMLRDPQNFGLSDVENPCYdGgyvwKPFATrSVSTDRQLSaFSPQeRLaIAGNPlLaQAvASPMAR 303

                         ▼
Pfam      .dassyll.atlfwDgf.HpsekGykavAeal<-*
Sriml 243 .-------.--LPVENSyHPTANGQSKGYLPV    263
Scoel 167 .-------.--WDADRL.HLSPEGHTRVALRA    186
Scoe2 169 .-------.--WDVDRL.HLTAEGHRRVAEAV    188
Ahyd1 292 rSASTLNCeGKMFWDQV.HPTTVVHAALSEPA    322
Asal1 292 rSASPLNCeGKMFWDQV.HPTTVVHAALSERA    322
Ahyd2 304 rSASPLNCeGKMFWDQV.HPTTVVHAALSERA    334
```

Figure 35

(SEQ ID No. 30)

```
  1 MFKFKKNFLV GLSAALMSIS LFSATASAAS ADSRPAFSRI VMFGDSLSDT
 51 GKMYSKMRGY LPSSPPYYEG RFSNGPVWLE QLTKQFPGLT IANEAEGGAT
101 AVAYNKISWN PKYQVINNLD YEVTQFLQKD SFKPDDLVIL WVGANDYLAY
151 GWNTEQDAKR VRDAISDAAN RMVLNGAKQI LLFNLPDLGQ NPSARSQKVV
201 EAVSHVSAYH NQLLLNLARQ LAPTGMVKLF EIDKQFAEML RDPQNFGLSD
251 VENPCYDGGY VWKPFATRSV STDRQLSAFS PQERLAIAGN PLLAQAVASP
301 MARRSASPLN CEGKMFWDQV HPTTVVHAAL SERAATFIAN QYEFLAH**
```

Figure 36 (SEQ ID No. 31)

```
   1  ATGTTTAAGT TTAAAAAGAA TTTCTTAGTT GGATTATCGG CAGCTTTAAT
      TACAAATTCA AATTTTTCTT AAAGAATCAA CCTAATAGCC GTCGAAATTA

  51  GAGTATTAGC TTGTTTTCGG CAACCGCCTC TGCAGCTAGC GCCGACAGCC
      CTCATAATCG AACAAAAGCC GTTGGCGGAG ACGTCGATCG CGGCTGTCGG

 101  GTCCCGCCTT TTCCCGGATC GTGATGTTCG GCGACAGCCT CTCCGATACC
      CAGGGCGGAA AAGGGCCTAG CACTACAAGC CGCTGTCGGA GAGGCTATGG

 151  GGCAAAATGT ACAGCAAGAT GCGCGGTTAC CTCCCCTCCA GCCCGCCCTA
      CCGTTTTACA TGTCGTTCTA CGCGCCAATG GAGGGGAGGT CGGGCGGGAT

 201  CTATGAGGGC CGTTTCTCCA ACGGACCCGT CTGGCTGGAG CAGCTGACCA
      GATACTCCCG GCAAAGAGGT TGCCTGGGCA GACCGACCTC GTCGACTGGT

 251  AACAGTTCCC GGGTCTGACC ATCGCCAACG AAGCGGAAGG CGGTGCCACT
      TTGTCAAGGG CCCAGACTGG TAGCGGTTGC TTCGCCTTCC GCCACGGTGA

 301  GCCGTGGCTT ACAACAAGAT CTCCTGGAAT CCCAAGTATC AGGTCATCAA
      CGGCACCGAA TGTTGTTCTA GAGGACCTTA GGGTTCATAG TCCAGTAGTT

 351  CAACCTGGAC TACGAGGTCA CCCAGTTCTT GCAGAAAGAC AGCTTCAAGC
      GTTGGACCTG ATGCTCCAGT GGGTCAAGAA CGTCTTTCTG TCGAAGTTCG

 401  CGGACGATCT GGTGATCCTC TGGGTCGGTG CCAATGACTA TCTGGCCTAT
      GCCTGCTAGA CCACTAGGAG ACCCAGCCAC GGTTACTGAT AGACCGGATA

 451  GGCTGGAACA CGGAGCAGGA TGCCAAGCGG GTTCGCGATG CCATCAGCGA
      CCGACCTTGT GCCTCGTCCT ACGGTTCGCC CAAGCGCTAC GGTAGTCGCT

 501  TGCGGCCAAC CGCATGGTAC TGAACGGTGC CAAGCAGATA CTGCTGTTCA
      ACGCCGGTTG GCGTACCATG ACTTGCCACG GTTCGTCTAT GACGACAAGT

 551  ACCTGCCGGA TCTGGGCCAG AACCCGTCAG CTCGCAGTCA GAAGGTGGTC
      TGGACGGCCT AGACCCGGTC TTGGGCAGTC GAGCGTCAGT CTTCCACCAG

 601  GAGGCGGTCA GCCATGTCTC CGCCTATCAC AACCAGCTGC TGCTGAACCT
      CTCCGCCAGT CGGTACAGAG GCGGATAGTG TTGGTCGACG ACGACTTGGA

 651  GGCACGCCAG CTGGCCCCCA CCGGCATGGT AAAGCTGTTC GAGATCGACA
      CCGTGCGGTC GACCGGGGGT GGCCGTACCA TTTCGACAAG CTCTAGCTGT

 701  AGCAATTTGC CGAGATGCTG CGTGATCCGC AGAACTTCGG CCTGAGCGAC
      TCGTTAAACG GCTCTACGAC GCACTAGGCG TCTTGAAGCC GGACTCGCTG

 751  GTCGAGAACC CCTGCTACGA CGGCGGCTAT GTGTGGAAGC CGTTTGCCAC
      CAGCTCTTGG GGACGATGCT GCCGCCGATA CACACCTTCG GCAAACGGTG

 801  CCGCAGCGTC AGCACCGACC GCCAGCTCTC CGCCTTCAGT CCGCAGGAAC
      GGCGTCGCAG TCGTGGCTGG CGGTCGAGAG GCGGAAGTCA GGCGTCCTTG

 851  GCCTCGCCAT CGCCGGCAAC CCGCTGCTGG CACAGGCCGT TGCCAGTCCT
      CGGAGCGGTA GCGGCCGTTG GGCGACGACC GTGTCCGGCA ACGGTCAGGA

 901  ATGGCCCGCC GCAGCGCCAG CCCCCTCAAC TGTGAGGGCA AGATGTTCTG
      TACCGGGCGG CGTCGCGGTC GGGGGAGTTG ACACTCCCGT TCTACAAGAC

 951  GGATCAGGTA CACCCGACCA CTGTCGTGCA CGCAGCCCTG AGCGAGCGCG
      CCTAGTCCAT GTGGGCTGGT GACAGCACGT GCGTCGGGAC TCGCTCGCGC

1001  CCGCCACCTT CATCGCGAAC CAGTACGAGT TCCTCGCCCA CTGATGA
      GGCGGTGGAA GTAGCGCTTG GTCATGCTCA AGGAGCGGGT GACTACT
```

Figure 37

SEQ ID NO. 32:

ACAGGCCGATGCACGGAACCGTACCTTTCCGCAGTGAAGCGCTCTCCCCCCATCGTTCGC
CGGGACTTCATCCGCGATTTTGGCATGAACACTTCCTTCAACGCGCGTAGCTTGCTACAA
GTGCGGCAGCAGACCCGCTCGTTGGAGGCTCAGTGAGATTGACCCGATCCCTGTCGGCCG
CATCCGTCATCGTCTTCGCCCTGCTGCTCGCGCTGCTGGGCATCAGCCCGGCCCAGGCAG
CCGGCCCGGCCTATGTGGCCCTGGGGGATTCCTATTCCTCGGGCAACGGCGCCGGAAGTT
ACATCGATTCGAGCGGTGACTGTCACCGCAGCAACAACGCGTACCCCGCCCGCTGGGCGG
CGGCCAACGCACCGTCCTCCTTCACCTTCGCGGCCTGCTCGGGAGCGGTGACCACGGATG
TGATCAACAATCAGCTGGGCGCCCTCAACGCGTCCACCGGCCTGGTGAGCATCACCATCG
GCGGCAATGACGCGGGCTTCGCGGACGCGATGACCACCTGCGTCACCAGCTCGGACAGCA
CCTGCCTCAACCGGCTGGCCACCGCCACCAACTACATCAACACCACCCTGCTCGCCCGGC
TCGACGCGGTCTACAGCCAGATCAAGGCCCGTGCCCCCAACGCCCGCGTGGTCGTCCTCG
GCTACCCGCGCATGTACCTGGCCTCGAACCCCTGGTACTGCCTGGGCCTGAGCAACACCA
AGCGCGCGGCCATCAACACCACCGCCGACACCCTCAACTCGGTGATCTCCTCCCGGGCCA
CCGCCCACGGATTCCGATTCGGCGATGTCCGCCCGACCTTCAACAACCACGAACTGTTCT
TCGGCAACGACTGGCTGCACTCACTCACCCTGCCGGTGTGGGAGTCGTACCACCCCACCA
GCACGGGCCATCAGAGCGGCTATCTGCCGGTCCTCAACGCCAACAGCTCGACCTGATCAA
CGCACGGCCGTGCCCGCCCCGCGCGTCACGCTCGGCGCGGGCGCCGCAGCGCGTTGATCA
GCCCACAGTGCCGGTGACGGTCCCACCGTCACGGTCGAGGGTGTACGTCACGGTGGCGCC
GCTCCAGAAGTGGAACGTCAGCAGGACCGTGGAGCCGTCCCTGACCTCGTCGAAGAACTC
CGGGGTCAGCGTGATCACCCCTCCCCCGTAGCCGGGGGCGAAGGCGGCGCCGAACTCCTT
GTAGGACGTCCAGTCGTGCGGCCCGGCGTTGCCACCGTCCGCGTAGACCGCTTCCATGGT
CGCCAGCCGGTCCCCGCGGAACTCGGTGGGGATGTCCGTGCCCAAGGTGGTCCCGGTGGT
GTCCGAGAGCACCGGGGGCTCGTACCGGATGATGTGCAGATCCAAAGAATT

FIGURE 38

SEQ ID NO. 33:

MRLTRSLSAASVIVFALLLALLGISPAQAAGPAYVALGDSYSSGNGAGSYIDSSGDCHRSN
NAYPARWAAANAPSSFTFAACSGAVTTDVINNQLGALNASTGLVSITIGGNDAGFADAMTT
CVTSSDSTCLNRLATATNYINTTLLARLDAVYSQIKARAPNARVVVLGYPRMYLASNPWYC
LGLSNTKRAAINTTADTLNSVISSRATAHGFRFGDVRPTFNNHELFFGNDWLHSLTLPVWE
SYHPTSTGHQSGYLPVLNANSST

Figure 39

SEQ ID No. 34

ZP_00058717
1 mlphpagerg evgaffallv gtpqdrrlrl echetrplrg rcgcgerrvp pltlpgdgvl
61 cttsstrdae tvwrkhlqpr pdggfrphlg vgcllagqgs pgvlwcgreg crfevcrrdt
121 pglsrtrngd ssppfragws lppkcgeisq sarktpavpr ysllrtdrpd gprgrfvgsg
181 praatrrrlf lgipalvlvt altlvlavpt gretlwrmwc eatqdwclgv pvdsrgqpae
241 dgeflllspv qaatwgnyya lgdsyssgdg ardyypgtav kggcwrsana ypelvaeayd
301 faghlsflac sgqrgyamld aidevgsqld wnsphtslvt igiggndlgf stvlktcmvr
361 vplldskact dqedairkrm akfettfeel isevrtrapd arilvvgypr ifpeeptgay
421 ytltasnqrw lnetiqefnq qlaeavavhd eeiaasggvg svefvdvyha ldgheigsde
481 pwvngvqlrd latgvtvdrs tfhpnaaghr avgervieqi etgpgrplya tfavvagatv
541 dtlagevg

FIGURE 40

SEQ ID No. 35

1 ggtggtgaac cagaacaccc ggtcgtcggc gtgggcgtcc aggtgcaggt gcaggttctt
61 caactgctcc agcaggatgc cgccgtggcc gtgcacgatg gccttgggca ggcctgtggt
121 ccccgacgag tacagcaccc atagcggatg gtcgaacggc agcggggtga actccagttc
181 cgcgccttcg cccgcggctt cgaactccgc ccaggacagg gtgtcggcga cagggccgca
241 gcccaggtac ggcaggacga cggtgtgctg caggctgggc atgccgtcgc gcagggcttt
301 gagcacgtca cggcggtcga agtccttacc gccgtagcgg tagccgtcca cggccagcag
361 cactttcggt tcgatctgcg cgaaccggtc gaggacgctg cgcaccccga agtcggggga
421 acaggacgac caggtcgcac cgatcgcggc gcaggcgagg aatgcggccg tcgcctcggc
481 gatgttcggc aggtaggcca cgacccggtc gccggggccc accccgaggc tgcggagggc
541 cgcagcgatc gcggcggtgc gggtccgcag ttctccccag gtccactcgg tcaacggccg
601 gagttcggac gcgtgccgga tcgccacggc tgatgggtca cggtcgcgga agatgtgctc
661 ggcgtagttg agggtggcgc cggggaacca gacggcgccg ggcatggcgt cggaggcgag
721 cactgtggtg tacggggtgg cggcgcgcac ccggtagtac tcccagatcg cggaccagaa
781 tccttcgagg tcggttaccg accagcgcca cagtgcctcg tagtccggtg cgtccacacc
841 gcggtgctcc cgcacccagc gggtgaacgc ggtgaggttg gcgcgttctt tgcgctcctc
901 gtcgggactc cacaggatcg gcggctgcgg cttgagtgtc atgaaacgcg accccttcgt
961 ggacggtgcg gatgcggtga gcgtcgggtg cctcccctaa cgctccccgg tgacggagtg
1021 ttgtgcacca catctagcac gcgggacgcg gaaaccgtat ggagaaaaca cctacaaccc
1081 cggccggacg gtgggtttcg gccacactta ggggtcgggt gcctgcttgc cgggcagggc
1141 agtcccgggg tgctgtggtg cgggcgggag ggctgtcgct tcgaggtgtg ccggcgggac
1201 actccgggcc tcagccgtac ccgcaacggg gacagttctc ctcccttccg ggctggatgg
1261 tcccttcccc cgaaatgcgg cgagatctcc cagtcagccc ggaaaacacc cgctgtgccc
1321 aggtactctt tgcttcgaac agacaggccg gacggtccac gggggaggtt tgtgggcagc
1381 ggaccacgtg cggcgaccag acgacggttg ttcctcggta tccccgctct tgtacttgtg
1441 acagcgctca cgctggtctt ggctgtcccg acggggcgcg agacgctgtg gcgcatgtgg
1501 tgtgaggcca cccaggactg gtgcctgggg gtgccggtcg actcccgcgg acagcctgcg
1561 gaggacggcg agtttctgct gctttctccg gtccaggcag cgacctgggg gaactattac
1621 gcgctcgggg attcgtactc ttcgggggac ggggcccgcg actactatcc cggcaccgcg
1681 gtgaagggcg gttgctggcg gtccgctaac gcctatccgg agctggtcgc cgaagcctac
1741 gacttcgccg gacacttgtc gttcctggcc tgcagcggcc agcgcggcta cgccatgctt
1801 gacgctatcg acgaggtcgg ctcgcagctg gactggaact cccctcacac gtcgctggtg
1861 acgatcggga tcggcggcaa cgatctgggg ttctccacgg ttttgaagac ctgcatggtg
1921 cgggtgccgc tgctggacag caaggcgtgc acggaccagg aggacgctat ccgcaagcgg
1981 atggcgaaat tcgagacgac gtttgaagag ctcatcagcg aagtgcgcac ccgcgcgccg
2041 gacgcccgga tccttgtcgt gggctacccc cggatttttc cggaggaacc gaccggcgcc
2101 tactacacgc tgaccgcgag caaccagcgg tggctcaacg aaaccattca ggagttcaac
2161 cagcagctcg ccgaggctgt cgcggtccac gacgaggaga ttgccgcgtc gggcggggtg
2221 ggcagcgtgg agttcgtgga cgtctaccac gcgttggacg gccacgagat cggctcggac
2281 gagccgtggg tgaacggggt gcagttgcgg gacctcgcca ccggggtgac tgtggaccgc
2341 agtaccttcc accccaacgc cgctgggcac cgggcggtcg gtgagcgggt catcgagcag
2401 atcgaaaccg gcccgggccg tccgctctat gccactttcg cggtggtggc gggggcgacc
2461 gtggacactc tcgcgggcga ggtggggtga cccggcttac cgtccggccc gcaggtctgc
2521 gagcactgcg gcgatctggt ccactgccca gtgcagttcg tcttcggtga tgaccagcgg
2581 cggggagagc cggatcgttg agccgtgcgt gtctttgacg agcacacccc gctgcaggag
2641 ccgttcgcac agttctcttc cggtggccag agtcgggtcg acgtcgatcc cagcccacag
2701 gccgatgctg cgggccgcga ccacgccgtt gccgaccagt tggtcgaggc gggcgcgcag
2761 cacgggggcg agggcgcgga catggtccag gtaagggccg tcgcggacga ggctcaccac
2821 ggcagtgccg accgcgcagg cgagggcgtt gccgccgaag gtgctgccgt gctggccggg
2881 gcggatcacg tcgaagactt ccgcgtcgcc taccgccgcc gccacgggca ggatgccgcc
2941 gcccagcgct ttgccgaaca ggtagatatc ggcgtcgact ccgctgtggt cgcaggcccg
//

FIGURE 41

SEQ ID No. 36

1 vgsgpraatr rrlflgipal vlvtaltlvl avptgretlw rmwceatqdw clgvpvdsrg
61 qpaedgefll lspvqaatwg nyyalgdsys sgdgardyyp gtavkggcwr sanaypelva
121 eaydfaghls flacsgqrgy amldaidevg sqldwnspht slvtigiggn dlgfstvlkt
181 cmvrvpllds kactdqedai rkrmakfett feelisevrt rapdarilvv gyprifpeep
241 tgayytltas nqrwlnetiq efnqqlaeav avhdeeiaas ggvgsvefvd vyhaldghei
301 gsdepwvngv qlrdlatgvt vdrstfhpna aghravgerv ieqietgpgr plyatfavva
361 gatvdtlage vg

FIGURE 42

SEQ ID No. 37

1 mrttviaasa llllagcadg areetagapp gessggiree gaeastsitd vyialgdsya
61 amggrdqplr gepfclrssg nypellhaev tdltcqgavt gdlleprtlg ertlpaqvda
121 ltedttlvtl siggndlgfg evagcireri agenaddcvd llgetigeql dqlppqldrv
181 heairdragd aqvvvtgylp lvsagdcpel gdvseadrrw aveltgqine tvreaaerhd
241 alfvlpddad ehtscappqq rwadiqgqqt dayplhptsa gheamaaavr dalglepvqp

//

FIGURE 43

SEQ ID No. 38

1 ttctggggtg ttatggggtt gttatcggct cgtcctgggt ggatcccgcc aggtggggta
61 ttcacggggg actttgtgt ccaacagccg agaatgagtg ccctgagcgg tgggaatgag
121 gtgggcgggg ctgtgtcgcc atgagggggc ggcgggctct gtggtgcccc gcgacccccg
181 gccccggtga gcggtgaatg aaatccggct gtaatcagca tcccgtgccc accccgtcgg
241 ggaggtcagc gcccggagtg tctacgcagt cggatcctct cggactcggc catgctgtcg
301 gcagcatcgc gctcccgggt cttggcgtcc ctcggctgtt ctgcctgctg tccctggaag
361 gcgaaatgat caccggggag tgatacaccg gtggtctcat cccggatgcc cacttcggcg
421 ccatccggca attcgggcag ctccgggtgg aagtaggtgg catccgatgc gtcggtgacg
481 ccatagtggg cgaagatctc atcctgctcg agggtgctca ggccactctc cggatcgata
541 tcgggggcgt ccttgatggc gtccttgctg aaaccgaggt gcagcttgtg ggcttccaat
601 ttcgcaccac ggagcgggac gaggctggaa tgacggccga agagcccgtg gtggacctca
661 acgaaggtgg gtagtcccgt gtcatcattg aggaacacgc cctccaccgc acccagcttg
721 tggccggagt tgtcgtaggc gctggcatcc agaagggaaa cgatctcata tttgtcggtg
781 tgctcagaca tgatcttcct ttgctgtcgg tgtctggtac taccacggta gggctgaatg
841 caactgttat ttttctgtta ttttaggaat tggtccatat cccacaggct ggctgtggtc
901 aaatcgtcat caagtaatcc ctgtcacaca aaatgggtgg tgggagccct ggtcgcggtt
961 ccgtgggagg cgccgtgccc cgcaggatcg tcggcatcgg cggatctggc cggtaccccg
1021 cggtgaataa aatcattctg taaccttcat cacggttggt tttaggtatc cgcccctttc
1081 gtcctgaccc cgtccccggc gcgcgggagc ccgcgggttg cggtagacag gggagacgtg
1141 gacaccatga ggacaacggt catcgcagca agcgcattac tccttctcgc cggatgcgcg
1201 gatggggccc gggaggagac cgccggtgca ccgccgggtg agtcctccgg gggcatccgg
1261 gaggaggggg cggaggcgtc gacaagcatc accgacgtct acatcgccct cggggattcc
1321 tatgcggcga tgggcgggcg ggatcagccg ttacggggtg agccgttctg cctgcgctcg
1381 tccggtaatt acccggaact cctccacgca gaggtcaccg atctcacctg ccaggggcg
1441 gtgaccgggg atctgctcga acccaggacg ctgggggagc gcacgctgcc ggcgcaggtg
1501 gatgcgctga cggaggacac caccctggtc accctctcca tcgggggcaa tgacctcgga
1561 ttcggggagg tggcgggatg catccgggaa cggatcgccg ggagagaacgc tgatgattgc
1621 gtggacctgc tgggggaaac catcggggag cagctcgatc agcttccccc gcagctggac
1681 cgcgtgcacg aggctatccg ggaccgcgcc ggggacgcgc aggttgtggt caccggttac
1741 ctgccgctcg tgtctgccgg ggactgcccc gaactggggg atgtctccga ggcggatcgt
1801 cgttgggcgg ttgagctgac cggcagatc aacgagaccg tgcgcgaggc ggccgaacga
1861 cacgatgccc tctttgtcct gcccgacgat gccgatgagc acaccagttg tgcaccccca
1921 cagcagcgct gggcggatat ccagggccaa cagaccgatg cctatccgct gcacccgacc
1981 tccgccggcc atgaggcgat ggccgccgcc gtccgggacg cgctgggcct ggaaccggtc
2041 cagccgtagc gccgggcgcg cgcttgtcga cgaccaaccc atgccaggct gcagtcacat
2101 ccgcacatag cgcgcgcggg cgatggagta cgcaccatag aggatgagcc cgatgccgac
2161 gatgatgagc agcacactgc cgaagggttg ttccccgagg gtgcgcagag ccgagtccag
2221 acctgcggcc tgctccggat catgggccca accggcgatg acgatcaaca cccccaggat
2281 cccgaaggcg ataccacggg cgacataacc ggctgttccg gtgatgatga tcgcggtccc
2341 gacctgccct gaccccgcac ccgcctccag atcctcccgg aaatcccggg tggccccctt
2401 ccagaggttg tagacacccg cccccagtac caccagcccg gcgaccacaa ccagcaccac
2461 accccagggt tgggatagga cggtggcggt gacatcggtg gcggtctccc catcggaggt
2521 gctgccgccc cgggcgaagg tggaggtggt caccgccagg gagaagtaga ccatggccat
2581 gaccgccccc ttggcccttt ccttgaggtc ctcgcccgcc agcagctggc tcaattgcca
2641 gagtcccagg gccgccaggg cgatgacggc aacccacagg aggaactgcc cacccggagc
2701 ctccgcgatg gtggccaggg cacctgaatt cgaggcctca tcacccgaac cgccggatcc
2761 agtggcgatg cgcaccgcga tccacccgat gaggatgtgc agtatgccca ggacaatgaa
2821 accacctctg gccagggtgg tcagcgcggg gtggtcctcg gcctggtcgg cagcccgttc
2881 gatcgtccgt ttcgcggatc tggtgtcgcc cttatccata gctcccattg aaccgccttg
2941 aggggtgggc ggccactgtc agggcggatt gtgatctgaa ctgtgatgtt ccatcaaccc
//

FIGURE 44

SEQ ID No. 39

ZP_00094165

1 mgqvklfarr capvllalag lapaatvare aplaegaryv algssfaagp gvgpnapgsp
61 ercgrgtlny phllaealkl dlvdatcsga tthhvlgpwn evppqidsvn gdtrlvtlti
121 ggndvsfvgn ifaaacekma spdprcgkwr eiteeewqad eermrsivrq iharaplarv
181 vvvdyitvlp psgtcaamai spdrlaqsrs aakrlarita rvareegasl lkfshisrrh
241 hpcsakpwsn glsapaddgi pvhpnrlgha eaaaalvklv klmk
//

FIGURE 45

SEQ ID No. 40

1 tgccggaact caagcggcgt ctagccgaac tcatgcccga aagcgcgtgg cactatcccg
61 aagaccaggt ctcggacgcc agcgagcgcc tgatggccgc cgaaatcacg cgcgaacagc
121 tctaccgcca gctccacgac gagctgccct atgacagtac cgtacgtccc gagaagtacc
181 tccatcgcaa ggacggttcg atcgagatcc accagcagat cgtgattgcc cgcgagacac
241 agcgtccgat cgtgctgggc aagggtggcg cgaagatcaa ggcgatcgga gaggccgcac
301 gcaaggaact ttcgcaattg ctcgacacca aggtgcacct gttcctgcat gtgaaggtcg
361 acgagcgctg ggccgacgcc aaggaaatct acgaggaaat cggcctcgaa tgggtcaagt
421 gaagctcttc gcgcgccgct gcgccccagt acttctcgcc cttgccgggc tggctccggc
481 ggctacggtc gcgcgggaag caccgctggc cgaaggcgcg cgttacgttg cgctgggaag
541 ctccttcgcc gcaggtccgg gcgtggggcc caacgcgccc ggatcgcccg aacgctgcgg
601 ccggggcacg ctcaactacc cgcacctgct cgccgaggcg ctcaagctcg atctcgtcga
661 tgcgacctgc agcggcgcga cgacccacca cgtgctgggc ccctggaacg aggttccccc
721 tcagatcgac agcgtgaatg gcgacacccg cctcgtcacc ctgaccatcg gcggaaacga
781 tgtgtcgttc gtcggcaaca tcttcgccgc cgcttgcgag aagatggcgt cgcccgatcc
841 gcgctgcggc aagtggcggg agatcaccga ggaagagtgg caggccgacg aggagcggat
901 gcgctccatc gtacgccaga tccacgcccg cgcgcctctc gcccgggtgg tggtggtcga
961 ttacatcacg gtcctgccgc catcaggcac ttgcgctgcc atggcgattt cgccggaccg
1021 gctggcccag agccgcagcg ccgcgaaacg gcttgcccgg attaccgcac gggtcgcgcg
1081 agaagagggt gcatcgctgc tcaagttctc gcatatctcg cgccggcacc atccatgctc
1141 tgccaagccc tggagcaacg gcctttccgc cccggccgac gacggcatcc cggtccatcc
1201 gaaccggctc ggacatgctg aagcggcagc ggcgctggtc aagcttgtga aattgatgaa
1261 gtagctactg cactgatttc aaatagtatt gcctgtcagc tttccagccc ggattgttgc
1321 agcgcaacag aaacttgtcc gtaatggatt gatggtttat gtcgctcgca aattgccgtc
1381 gaagggaacg ggcgcgtcgc tcgttaacgt cctgggtgca gcagtgacgg agcgcgtgga
1441 tgagtgatac tggcggtgtc atcggtgtac gcgccgccat tcccatgcct gtacgcgccg
//

FIGURE 46

SEQ ID No. 41

NP_625998.

1 mrrfrlvgfl sslvlaagaa ltgaataqaa qpaaadgyva lgdsyssgvg agsyisssgd
61 ckrstkahpy lwaaahspst fdftacsgar tgdvlsgqlg plssgtglvs isiggndagf
121 adtmttcvlq sessclsria taeayvdstl pgkldgvysa isdkapnahv vvigyprfyk
181 lgttciglse tkrtainkas dhlntvlaqr aaahgftfgd vrttftghel csgspwlhsv
241 nwlnigesyh ptaagqsggy lpvlngaa
//

FIGURE 47

SEQ ID No. 42

1 cccggcggcc cgtgcaggag cagcagccgg cccgcgatgt cctcgggcgt cgtcttcatc
61 aggccgtcca tcgcgtcggc gaccggcgcc gtgtagttgg cccggacctc gtcccaggtg
121 cccgcggcga tctggcgggt ggtgcggtgc gggccgcgcc gaggggagac gtaccagaag
181 cccatcgtca cgttctccgg ctgcggttcg ggctcgtccg ccgctccgtc cgtcgcctcg
241 ccgagcacct tctcggcgag gtcggcgctg gtcgccgtca ccgtgacgtc ggcgccccgg
301 ctccagcgcg agatcagcag cgtccagccg tcgccctccg ccagcgtcgc gctgcggtcg
361 tcgtcgcggg cgatccgcag cacgcgcgcg ccgggcggca gcagcgtggc gccggaccgt
421 acgcggtcga tgttcgccgc gtgcgagtac ggctgctcac ccgtggcgaa acggccgagg
481 aacagcgcgt cgacgacgtc ggacggggag tcgctgtcgt ccacgttgag ccggatcggc
541 agggcttcgt gcgggttcac ggacatgtcg ccatgatcgg gcacccggcc gccgcgtgca
601 cccgctttcc cggcacgca cgacaggggc tttctcgccg tcttccgtcc gaacttgaac
661 gagtgtcagc catttcttgg catggacact tccagtcaac gcgcgtagct gctaccacgg
721 ttgtggcagc aatcctgcta agggaggttc catgagacgt ttccgacttg tcggcttcct
781 gagttcgctc gtcctcgccg ccggcgccgc cctcaccggg gcagcgaccg cccaggcggc
841 ccaacccgcc gccgccgacg gctatgtggc cctcggcgac tcctactcct ccggggtcgg
901 agcgggcagc tacatcagct cgagcggcga ctgcaagcgc agcacgaagg cccatcccta
961 cctgtgggcg gccgcccact cgccctccac gttcgacttc accgcctgtt ccggcgcccg
1021 tacgggtgat gttctctccg gacagctcgg cccgctcagc tccggcaccg gcctcgtctc
1081 gatcagcatc ggcggcaacg acgccggttt cgccgacacc atgacgacct gtgtgctcca
1141 gtccgagagc tcctgcctgt cgcggatcgc caccgccgag gcgtacgtcg actcgacgct
1201 gcccggcaag ctcgacggcg tctactcggc aatcagcgac aaggcgccga acgcccacgt
1261 cgtcgtcatc ggctacccgc gcttctacaa gctcggcacc acctgcatcg gcctgtccga
1321 gaccaagcgg acggcgatca acaaggcctc cgaccacctc aacaccgtcc tcgcccagcg
1381 cgccgccgcc cacggcttca ccttcggcga cgtacgcacc accttcaccg gccacgagct
1441 gtgctccggc agcccctggc tgcacagcgt caactggctg aacatcggcg agtcgtacca
1501 ccccaccgcg gccggccagt ccggtggcta cctgccggtc ctcaacggcg ccgcctgacc
1561 tcaggcggaa ggagaagaag aaggagcgga gggagacgag gagtgggagg ccccgcccga
1621 cggggtcccc gtccccgtct ccgtctccgt cccggtcccg caagtcaccg agaacgccac
1681 cgcgtcggac gtggcccgca ccggactccg cacctccacg cgcacggcac tctcgaacgc
1741 gccggtgtcg tcgtgcgtcg tcaccaccac gccgtcctgg cgcgagcgct cgccgcccga
1801 cgggaaggac agcgtccgcc accccggatc ggagaccgac ccgtccgcgg tcacccaccg
1861 gtagccgacc tccgcgggca gccgcccgac cgtgaacgtc gccgtgaacg cgggtgcccg
1921 gtcgtgcggc ggcggacagg cccccgagta gtgggtgcgc gagcccacca cggtcacctc
1981 caccgactgc gctgcggggc
//

FIGURE 48

SEQ ID No. 43

NP_827753.

1 mrrsritayv tslllavgca ltgaataqas paaaatgyva lgdsyssgvg agsylsssgd
61 ckrsskaypy lwqaahspss fsfmacsgar tgdvlanqlg tlnsstglvs ltiggndagf
121 sdvmttcvlq sdsaclsrin takayvdstl pgqldsvyta istkapsahv avlgyprfyk
181 lggsclagls etkrsainda adylnsaiak raadhgftfg dvkstftghe icssstwlhs
241 ldllnigqsy hptaagqsgg ylpvmnsva

//

FIGURE 49

SEQ ID No. 44

1 ccaccgccgg gtcggcggcg agtctcctgg cctcggtcgc ggagaggttg gccgtgtagc
61 cgttcagcgc ggcgccgaac gtcttcttca ccgtgccgcc gtactcgttg atcaggccct
121 tgcccttgct cgacgcggcc ttgaagccgg tgcccttctt gagcgtgacg atgtagctgc
181 ccttgatcgc ggtgggggag ccggcggcga gcaccgtgcc ctcggccggg gtggcctggg
241 cgggcagtgc ggtgaatccg cccacgaggg cgccggtcgc cacggcggtt atcgcggcga
301 tccggatctt cttgctacgc agctgtgcca tacgagggag tcctcctctg ggcagcggcg
361 cgcctgggtg gggcgcacgg ctgtgggggg tgcgcgcgtc atcacgcaca cggccctgga
421 gcgtcgtgtt ccgccctggg ttgagtaaag cctcggccat ctacgggggt ggctcaaggg
481 agttgagacc ctgtcatgag tctgacatga gcacgcaatc aacggggccg tgagcacccc
541 ggggcgaccc cggaaagtgc cgagaagtct tggcatggac acttcctgtc aacacgcgta
601 gctggtacga cggttacggc agagatcctg ctaaagggag gttccatgag acgttcccga
661 attacggcat acgtgacctc actcctcctc gccgtcggct gcgccctcac cggggcagcg
721 acggcgcagg cgtccccagc cgccgcggcc acgggctatg tggccctcgg cgactcgtac
781 tcgtccggtg tcggcgccgg cagctacctc agctccagcg gcgactgcaa gcgcagttcg
841 aaggcctatc cgtacctctg gcaggccgcg cattcaccct cgtcgttcag tttcatggct
901 tgctcgggcg ctcgtacggg tgatgtcctg gccaatcagc tcggcaccct gaactcgtcc
961 accggcctgg tctccctcac catcggaggc aacgacgcgg gcttctccga cgtcatgacg
1021 acctgtgtgc tccagtccga cagcgcctgc ctctcccgca tcaacacggc gaaggcgtac
1081 gtcgactcca ccctgcccgg ccaactcgac agcgtgtaca cggcgatcag cacgaaggcc
1141 ccgtcggccc atgtggccgt gctgggctac ccccgcttct acaaactggg cggctcctgc
1201 ctcgcgggcc tctcggagac caagcggtcc gccatcaacg acgcggccga ctatctgaac
1261 agcgccatcg ccaagcgcgc cgccgaccac ggcttcacct tcggcgacgt caagagcacc
1321 ttcaccggcc atgagatctg ctccagcagc acctggctgc acagtctcga cctgctgaac
1381 atcggccagt cctaccaccc gaccgcggcc ggccagtccg gcggctatct gccggtcatg
1441 aacagcgtgg cctgagctcc cacggcctga atttttaagg cctgaatttt taaggcgaag
1501 gtgaaccgga agcggaggcc ccgtccgtcg ggtctccgt cgcacaggtc accgagaacg
1561 gcacggagtt ggacgtcgtg cgcaccgggt cgcgcacctc gacggcgatc tcgttcgaga
1621 tcgttccgct cgtgtcgtac gtggtgacga acacctgctt ctgctgggtc tttccgccgc
1681 tcgccgggaa ggacagcgtc ttccagcccg gatccgggac ctcgcccttc ttggtcaccc
1741 agcggtactc cacctcgacc ggcacccggc ccaccgtgaa ggtcgccgtg aacgtgggcg
1801 cctgggcggt gggcggcggg caggcaccgg agtagtcggt gtgcacgccg gtgaccgtca
1861 ccttcacgga ctgggccggc ggggtcgtcg taccgccgcc gccaccgccg cctcccggag
1921 tggagcccga gctgtggtcg cccccgccgt cggcgttgtc gtcctcgggg gttttcgaac

//

FIGURE 50

SEQ ID No. 45

MRLTRSLSAASVIVFALLLALLGISPAQAAGPAYVALGDSYSSGNGAGSYIDSSGDCHRSN
NAYPARWAAANAPSSFTFAACSGAVTTDVINNQLGALNASTGLVSITIGGNDAGFADAMTT
CVTSSDSTCLNRLATATNYINTTLLARLDAVYSQIKARAPNARVVVLGYPRMYLASNPWYC
LGLSNTKRAAINTTADTLNSVISSRATAHGFRFGDVRPTFNNHELFFGNDWLHSLTLPVWE
SYHPTSTGHQSGYLPVLNANSST

FIGURE 51

SEQ ID No. 46

ACAGGCCGATGCACGGAACCGTACCTTTCCGCAGTGAAGCGCTCTCCCCCCATCGTTCGC
CGGGACTTCATCCGCGATTTTGGCATGAACACTTCCTTCAACGCGCGTAGCTTGCTACAA
GTGCGGCAGCAGACCCGCTCGTTGGAGGCTCAGTGAGATTGACCCGATCCCTGTCGGCCG
CATCCGTCATCGTCTTCGCCCTGCTGCTCGCGCTGCTGGGCATCAGCCCGGCCCAGGCAG
CCGGCCCGGCCTATGTGGCCCTGGGGGATTCCTATTCCTCGGGCAACGGCGCCGGAAGTT
ACATCGATTCGAGCGGTGACTGTCACCGCAGCAACAACGCGTACCCCGCCCGCTGGGCGG
CGGCCAACGCACCGTCCTCCTTCACCTTCGCGGCCTGCTCGGGAGCGGTGACCACGGATG
TGATCAACAATCAGCTGGGCGCCCTCAACGCGTCCACCGGCCTGGTGAGCATCACCATCG
GCGGCAATGACGCGGGCTTCGCGGACGCGATGACCACCTGCGTCACCAGCTCGGACAGCA
CCTGCCTCAACCGGCTGGCCACCGCCACCAACTACATCAACACCACCCTGCTCGCCCGGC
TCGACGCGGTCTACAGCCAGATCAAGGCCCGTGCCCCCAACGCCCGCGTGGTCGTCCTCG
GCTACCCGCGCATGTACCTGGCCTCGAACCCCTGGTACTGCCTGGGCCTGAGCAACACCA
AGCGCGCGGCCATCAACACCACCGCCGACACCCTCAACTCGGTGATCTCCTCCCGGGCCA
CCGCCCACGGATTCCGATTCGGCGATGTCCGCCCGACCTTCAACAACCACGAACTGTTCT
TCGGCAACGACTGGCTGCACTCACTCACCCTGCCGGTGTGGGAGTCGTACCACCCCACCA
GCACGGGCCATCAGAGCGGCTATCTGCCGGTCCTCAACGCCAACAGCTCGACCTGATCAA
CGCACGGCCGTGCCCGCCCCGCGCGTCACGCTCGGCGCGGGCGCCGCAGCGCGTTGATCA
GCCCACAGTGCCGGTGACGGTCCCACCGTCACGGTCGAGGGTGTACGTCACGGTGGCGCC
GCTCCAGAAGTGGAACGTCAGCAGGACCGTGGAGCCGTCCCTGACCTCGTCGAAGAACTC
CGGGGTCAGCGTGATCACCCCTCCCCCGTAGCCGGGGGCGAAGGCGGCGCCGAACTCCTT
GTAGGACGTCCAGTCGTGCGGCCCGGCGTTGCCACCGTCCGCGTAGACCGCTTCCATGGT
CGCCAGCCGGTCCCCGCGGAACTCGGTGGGGATGTCCGTGCCCAAGGTGGTCCCGGTGGT
GTCCGAGAGCACCGGGGGCTCGTACCGGATGATGTGCAGATCCAAAGAATT

FIGURE 52

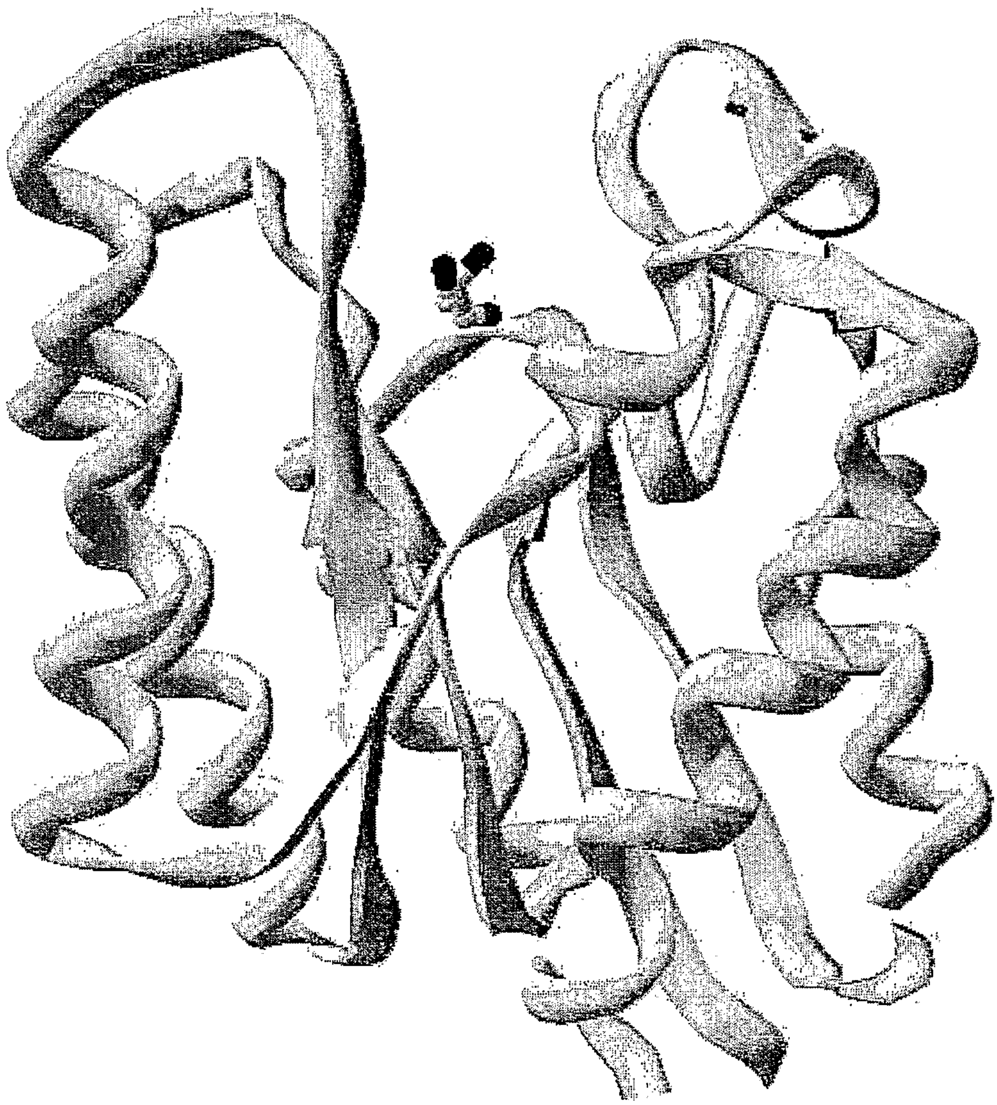

FIGURE 53

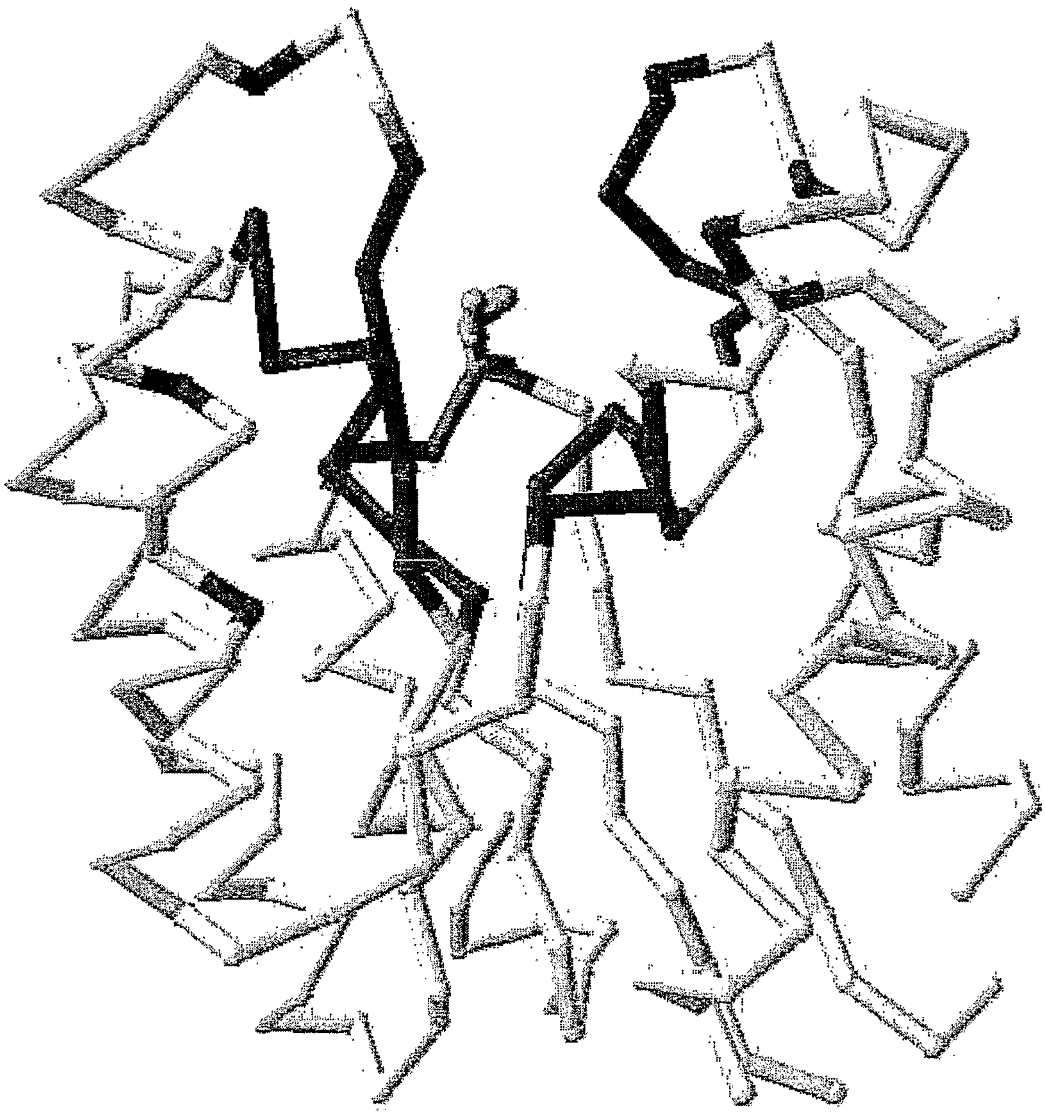

FIGURE 54

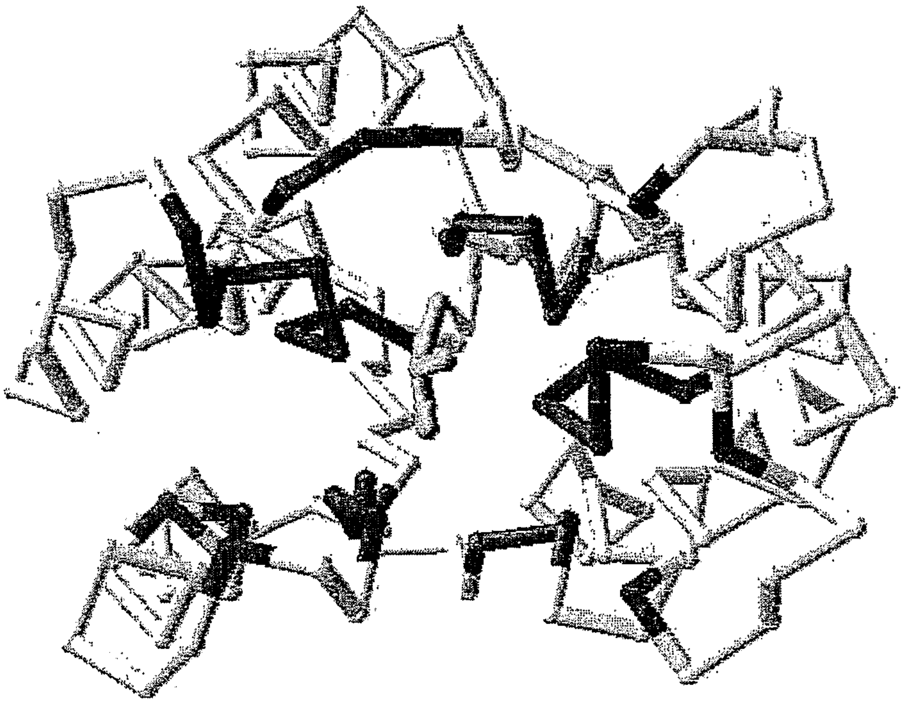

FIGRURE 55

```
1DEO      T T V Y  L  A G D S T M A K n - - - - - - - - - - - - - - - - - - - - - G G G S G T N G W G E Y L
          s1s1s1s1 s1 s1s1h?h?h?h?                                                 h1h1h1h1h1
1IVN    A D T L L  I  L G D S L S A G - - - - - - - - - - - - - - - - - - - - - Y R M S A S A A W P A L L
          s1s1s1s1 s1 s1s1h h h h                                                    h1h1h1h1h1
P10480      I V  M  F G D S L S D T g k m y s k m r g y l p s s p p y y e G R F S N G P V W L E Q L

1DEO    A S Y L S  A  T V - - - - - - - - - - - - - - - V N D A V A G R S - - - A R S Y T R E G R F E N I A D VV
        h1h1h1  s2 s2 s2                          s2s2 s2 s2 s2   h3   h3h3 h3h3h3h3h3h3h3h3h3h3h3h3h3
1IVN    N D K W q  s  k - - - - - - - - - - - - t s v V N A S I S G D T - - - - - - S Q Q G L A R L P A L L KQ
        h1h1h1  s2?s2?                       s2?s2s2s2s2 s2 s2 s2                 h3h3h3h3h3h3h3h3h3h3h3h3h3
P10480  T N E F P  G  L T i a n e a e g g p t a y a Y N K I S W N P K y q v I N N L D Y E V T Q F L Q K D SF

1DEO    T A G D Y  V  I V E F G H N D G g s l s t d n g r t d c s g t g a E v C Y S V Y D G V N E T I L T FP
           s4s4   s4  s4s4s4          ? ? ? ? ? ? ? ? ? ? ? ? ? ? ? ? ? ? ? ? s?s?s?s?s?s?s?s?s?s?s?h4h4h4
1IVN    H Q P R W  V  L V E L G G N D G - - - - - - - - - - - - - - - - - - - - - - - - - - L R G F Q P Q Q TE
        h3  s4s4s4 s4 s4s4                                                                     h4h4h4h4h4
P10480  K P D D L  V  I L W V G A N D Y - - - - - - - - - - - - - - - - - - - - - - - L A Y G W N T E Q D A K R VR

1DEO    A Y L E N  A  A K L F T - A K G A K - - - - - V I L S S Q T P - - - - - - N N P W E T G T F V N S P TR
        h4h4h4h4h4 h4 h4h4h4h4h4  h4   s5        s5 s5 s5 s5 s5
1IVN    Q T L R Q  I  L Q D V K a A N A E P l l m q i R L P A N Y G R - - - - - - - - - - - - - - - - - - - RY
        h4h4h4h4h4 h4 h4h4h4h4h4h4    s5s5s5 s5s5s5s5?s5?s5?s5?                                        h5
P10480  D A I S D  A  A N R M V - L N G A K - - - - - E I L L F N L P d l g q n P S A R S Q K V V E A A S HV

1DEO    F V E Y A  E  L A A E V A - - - - - - - - - - - - - - G V E Y V D H W S Y V D S I Y E T L G N A t vn
        h5h5h5h5h5 h5 h5h5h5h5h5h5                          s6s6s6s6?h6h6h6h6h6h6h6h6h6h6h6   h h h h
1IVN    N E A F S  A  I Y P K L A k e - - - - - - - - - - - - f D V P L L P F F M E E V Y L K P Q W - - - - - --
        h5h5h5h5h5 h5 h5h5h5h5h5h5h5h5                    h5   s6s6s6s6?  h6h6h6h6h6     s
P10480  S A Y H N  Q  L L L N L A r q l a p t g m v k l f e i D K Q F A E M L R D P Q N F G L S D Q R N a cy

1DEO    - - - - -  -  - - - - - - - - - - - - - - - - - - - - - - - - - - - - - - - - - - - - - - - - - --

1IVN    - - - - -  -  - - - - - - - - - - - - - - - - - - - - - - - - - - - - - - - - - - - - - - - - - --

P10480  g g s y v  w  k p f a s r s a s t d s q l s a f n p q e r l a i a g n p l l a q a v a s p m a a r sa

1DEO    - - - - -  -  s y F P I D H T H T S P A G A E V V A E A F L K A V V C T G T S L K S V L T T T S F EG
                      h             s?s?s? h7h7h7h7 h7 h7 h7 h7h7h7h7h7h7h7 h7h7h7h7   h?h?h?
1IVN    - - - - -  -  - - M Q D D G I H P N R D A Q P F I A D W M A K Q L Q P L V N H D S L E
                          s s         s s s  h7h7h7 h7 h7 h7 h7h7h7h7h7h7h7 h7h7h7h7
P10480  s t l n c  e  g k M F W D Q V H P T T V V H A A L S E P A A T F I E S Q Y E F L A H -
```

FIGURE 56

```
1DEOm     T T V Y  L  A G D S T M A K n - - - - - - - - - - - - - - - - - - - - - G G G S G T N G W G E Y L
          s1s1s1s1 s1 s1s1h?h?h?h?                                                    h1h1h1h1h1
1IVNm   A D T L L  I  L G D S L S A G - - - - - - - - - - - - - - - - - - - - - - Y R M S A S A A W P A L L
          s1s1s1s1 s1 s1s1h h h h                                                      h1h1h1h1h1
P10480m      I V   M  F G D S L S D T g k m y s k m r g y l p s s p p y y e G R F S N G P V W L E Q L

1DEOm   A S Y L S  A  T V - - - - - - - - - - - - - - - V N D A  V  A  G  R  S - - -     A R S Y T R E G R F E N IA
        h1h1h1  s2 s2 s2                        s2s2s2s2s2      h3             h3h3h3h3h3h3h3h3h3h3h3h3
1IVNm   N D K W q  s  k - - - - - - - - - - - - t s v V N A S  I  S  G  D  T - - - - - - S Q Q G L A   R L P A LL
        h1h1h1  s2?s2?                  s2?s2s2s2s2s2s2s2                   h3h3h3h3h3  h3h3h3h3h3h3
P10480m T N E F P  G  L T i a n e a e g g p t a v a Y N K I  S  W  N  P  K      y q v I N N L D Y E V T Q F LQ

1DEOm   D   V V T  A  G D Y V I V E F G H N D G g s l s t d  n  g  r  t  d c s g t g a E v C Y S V Y D G V N E TI
        h3h3       s4 s4s4s4s4s4         ? ? ? ? ? ? ? ? ? ? ? ? ? ? ? ? ? ? ? ? ? s?s?s?s?s?s?s?s?s?s?s?s?
1IVNm   K Q H Q P     R W V L V E L G G N D G - - - - - - -  -  -  -  -  - - - - - - - - - - - - - - - - L R G F QP
        h3h3h3        s4s4s4s4s4s4                                                                      h4
P10480m K D S F K  P  D D L V I L W V G A N D Y - - - - - -  -  -  -  -  - - - - - - - - - - L A Y G W N T E Q DA

1DEOm   L T F P A  Y  L E N A A K L F T A K G A K V I L S S  Q  T  P  N  N P W E T G T F V N S P T R
          h4h4h4h4 h4 h4h4h4h4h4h4h4h4h4h4   s5s5s5s5s5s5s5
1IVNm   Q Q T E Q  T  L R Q I L Q D V K a A N A E P l l m q  i  R  L  P  A N Y G R - - - - - - - - - R Y N E A
        h4h4h4h4h4 h4 h4h4h4h4h4h4h4h4h4h4   s5s5s5s5s5s5s5?s5?s5?s5?                            h5h5h5h5h5h5
P10480m K R V R D  A  I S D A A N R M V L N G A K E I L L F  N  L  P  d  l g q n P S A R S Q K V V E A A S H V SA

1DEOm     F V E Y  A  E L A A E V A - - - - - - - - - - - G  V  E  Y  V  D H W S Y V D S I Y E T L G N A t v n --
          h5h5h5h5 h5 h5h5h5h5h5h5h5                        s6 s6 s6 s6?h6h6h6h6h6h6h6h6h6h6h6   h h h h
1IVNm     F S A I  Y  P K L A k e - - - - - - - - - - - f D  V  P  L  L  P F F M E E V Y        L K P Q W - - --
        h5h5h5h5h5 h5 h5h5h5                   h5  s6s6s6s6?  h6 h6 h6 h6h6      s
P10480m Y H N Q L  L  L N L A r q l a p t g m v k l f e i D  K  Q  F  A  E M L R D P Q N F G L S D Q R N a c y gg

1DEOm   - - - - -  -  - - - - - - - - - - - - - - - - - - -  -  -  -  -  - - - - - - - - - - - - - - - - - - --

1IVNm   - - - - -  -  - - - - - - - - - - - - - - - - - - -  -  -  -  -  - - - - - - - - - - - - - - - - - - --

P10480m s y v w k  p  f a s r s a s t d s q l s a f n p q e  r  l  a  i  a g n p l l a q a v a s p m a a r s a st

1DEOm   - - - - s  y  F P I D H T H T S P A G A E V V A E A  F  L  K  A  V V C T G T S L K S V L T T T S F E G TC
                h               s?s?s?h7h7 h7h7h7h7h7h7h7h7 h7 h7 h7 h7 h7h7h7h7    h?h?h?
1IVNm   - - - - -  -  M Q D D G I H P N R D A Q P F I A D W  M  A  K  Q  L Q P L V N H D S L E
                s  s s  h7h7h7h7h7h7h7h7 h7h7h7h7h7h7h7h7 h7 h7 h7 h7 h7
P10480m l n c e g  k  M F W D Q V H P T T V V H A A L S E P  A  A  T  F  I E S Q Y E F L A H -
```

FIGURE 57

```
1DEO     T T V Y  L  A G D S T M A K n - - - - - - - - - - - - - - - - - - - - - - - - G G G S G T N G W G E Y L
           s1s1s1s1  s1  s1s1h?h?h?h?                                                          h1h1h1h1h1
1IVN     A D T L L  I  L G D S L S A G - - - - - - - - - - - - - - - - - - - - - - - - Y R M S A S A A W P A L L
           s1s1s1s1  s1  s1s1h h h h                                                             h1h1h1h1h1
P10480       I V  M  F G D S L S D T g k m  y s k m  r  g  y  l  p  s  s  p  p y  y e G R F S N G P V W L E Q L

1DEOm    T T V Y  L  A G D S T M A K n - - - - - - - - - - - - - - - - - - - - - - - - G G G S G T N G W G E Y L
           s1s1s1s1  s1  s1s1h?h?h?h?                                                          h1h1h1h1h1
1IVNm    A D T L L  I  L G D S L S A G - - - - - - - - - - - - - - - - - - - - - - - - Y R M S A S A A W P A L L
           s1s1s1s1  s1  s1s1h h h h                                                             h1h1h1h1h1
P10480m      I V  M  F G D S L S D T g k m  y s k m  r  g  y  l  p  s  s  p  p y  y e G R F S N G P V W L E Q L


1DEO     A S Y L S  A  T V - - - - - - - - - - - - - - -  V  N  D  A  V  A  G  R  S -  - - A R S Y T R E G R F E N I A D VV
         h1h1h1  s2 s2 s2                          s2s2 s2 s2 s2     h3       h3h3 h3h3h3h3h3h3h3h3h3h3h3h3h3
1IVN     N D K W q  s  k - - - - - - - - - - - - t s v  V  N  A  S  I  S  G  D  T -  - - - - - S Q Q G L A R L P A L L KQ
         h1h1h1  s2?s2?                  s2?s2s2s2s2 s2 s2 s2                            h3h3h3h3h3h3h3h3h3h3h3h3
P10480   T N E F P  G  L T i a n e a e g g p  t a v a  Y  N  K  I  S  W  N  P  K y  q v I N N L D Y E V T Q F L Q K D SF

1DEOm    A S Y L S  A  T V - - - - - - - - - - - - - - -  V  N  D  A  V  A  G  R  S -  - -       A R S Y T R E G R F E N IA
         h1h1h1  s2 s2 s2                          s2s2 s2 s2 s2     h3                h3h3h3h3h3h3h3h3h3h3h3h3h3
1IVNm    N D K W q  s  k - - - - - - - - - - - - t s v  V  N  A  S  I  S  G  D  T -  - - - - - S Q Q G L A    R L P A LL
         h1h1h1  s2?s2?                  s2?s2s2s2s2 s2 s2 s2                            h3h3h3h3h3  h3h3h3h3h3h3
P10480m  T N E F P  G  L T i a n e a e g g p  t a v a  Y  N  K  I  S  W  N  P  K          y q v I N N L D Y E V T Q F LQ


1DEO     T A G D Y  V  I V E F G H N D G g s  l s t d  n  g  r  t  d  c  s  g  t g  a E v C Y S V Y D G V N E T I L T FP
              s4s4 s4  s4s4s4           ? ? ? ?  ? ? ? ?  ?  ?  ?  ?  ?  ?  ?  ?  ? ?  ? ? s?s?s?s?s?s?s?s?s?s?s?s?h4h4h4
1IVN     H Q P R W  V  L V E L G G N D G - - - - - - - - - - - - - - - - - - - - - - - - - - - - - - - L R G F Q P Q Q TE
         h3  s4s4s4 s4  s4s4                                                                      h4h4h4h4h4
P10480   K P D D L  V  I L W V G A N D Y - - - - - - - - - - - - - - - - - - - - - - - - - - - L A Y G W N T E Q D A K R VR

1DEOm    D  V V T  A  G D Y V I V E F G H N  D G g s  l  s  t  d  n  g  r  t  d c  s g t g a E v C Y S V Y D G V N E TI
         h3h3         s4  s4s4s4s4s4           ? ?  ? ? ? ?  ?  ?  ?  ?  ?  ?  ?  ? ?  ? ? ? ? s?s?s?s?s?s?s?s?s?s?s?s?
1IVNm    K Q H Q P       R W V L V E L G G N  D G - - - - - - - - - - - - - - - - - - - - - - - - - - - - - - L R G F QP
         h3h3h3          s4s4s4s4s4s4                                                                            h4
P10480m  K D S F K  P  D D L V I L W V G A N  D Y - - - - - - - - - - - - - - - - - - - - - - - - - L A Y G W N T E Q DA


1DEO     A Y L E N  A  A K L F T - A K G A K  - - - - -  V  I  L  S  S  Q  T  P -  - - - - N N P W E T G T F V N S P TR
         h4h4h4h4h4 h4 h4h4h4h4h4  h4     s5          s5 s5 s5 s5 s5
1IVN     Q T L R Q  I  L Q D V K a A N A E P  l l m q  i  R  L  P  A  N  Y  G  R -  - - - - - - - - - - - - - - - - - - RY
         h4h4h4h4h4 h4 h4h4h4h4h4h4     s5s5s5  s5s5s5s5?s5?s5?s5?                                                 h5
P10480   D A I S D  A  A N R M V - L N G A K  - - - - -  E  I  L  L  F  N  L  P d  l g q n P S A R S Q K V V E A A S HV

1DEOm    L T F P A  Y  L E N A A K L F T A K  G A K V  I  L  S  S  Q  T  P  N  N P  W E T G T F V N S P T R
           h4h4h4h4 h4 h4h4h4h4h4h4h4h4h4h4       s5s5s5 s5 s5 s5
```

```
1IVNm    Q Q T E Q  T  L R Q I L Q D V K a A  N A E P  l  l  m  q  i  R  L  P  A N  Y G R - - - - - - - - - - R Y N E A
         h4h4h4h4h4 h4 h4h4h4h4h4h4h4h4h4h4    s5s5s5 s5 s5 s5 s5?s5?s5?s5?                              h5h5h5h5h5h5
P10480m K R V R D  A  I S D A A N R M V L N  G A K E  I  L  L  F  N  L  P  d  l  g  q n P S A R S Q K V V E A A S H V SA

1DEO     F V E Y A  E  L A A E V A - - - - -  - - - -  -  -  -  -  -  G  V  E  Y V  D H W S Y V D S I Y E T L G N A t vn
         h5h5h5h5h5 h5 h5h5h5h5h5h5                                   s6 s6 s6s6?h6h6h6h6h6h6h6h6h6h6h6   h h h h
1IVN     N E A F S  A  I Y P K L A k e - - - -  - - - -  -  -  -  -  f  D  V  P  L L  P F F M E E V Y L K P Q W - - - - - --
         h5h5h5h5h5 h5 h5h5h5h5h5h5h5h5                         h5    s6 s6 s6s6?  h6h6h6h6h6       s
P10480   S A Y H N  Q  L L L N L A r q l a p  t g m v  k  l  f  e  i  D  K  Q  F A  E M L R D P Q N F G L S D Q R N a cy

1DEOm      F V E Y  A  E L A A E V A - - - -  - - - -  -  -  -  G  V  E  Y  V  D H  W S Y V D S I Y E T L G N A t v n --
         h5h5h5h5   h5 h5h5h5h5h5h5h5                              s6 s6 s6 s6?h6h6 h6h6h6h6h6h6h6h6h6   h h h h
1IVNm      F S A I  Y  P K L A k e - - - - -  - - - -  -  -  f  D  V  P  L  L  P F  F M E E V Y       L K P Q W - - --
         h5h5h5h5h5 h5 h5h5h5                    h5    s6 s6 s6 s6?   h6 h6 h6 h6h6       s
P10480m Y H N Q L  L  L N L A r q l a p t g  m v k l  f  e  i  D  K  Q  F  A  E M  L R D P Q N F G L S D Q R N a c y gg

1DEO     - - - - -  -  - - - - - - - - - - -  - - - -  -  -  -  -  -  -  -  -  - -  - - - - - - - - - - - - - - - - - --
1IVN     - - - - -  -  - - - - - - - - - - -  - - - -  -  -  -  -  -  -  -  -  - -  - - - - - - - - - - - - - - - - - --
P10480   g g s y v  w  k p f a s r s a s t d  s q l s  a  f  n  p  q  e  r  l  a i  a g n p l l a q a v a s p m a a r sa
1DEOm    - - - - -  -  - - - - - - - - - - -  - - - -  -  -  -  -  -  -  -  -  - -  - - - - - - - - - - - - - - - - - --
1IVNm    - - - - -  -  - - - - - - - - - - -  - - - -  -  -  -  -  -  -  -  -  - -  - - - - - - - - - - - - - - - - - --
P10480m s y v w k  p  f a s r s a s t d s q  l s a f  n  p  q  e  r  l  a  i  a g  n p l l a q a v a s p m a a r s a st

1DEO     - - - - -  -  s y F P I D H T H T S  P A G A  E  V  V  A  E  A  F  L  K A  V V C T G T S L K S V L T T T S F EG
                       h             s?s?s? h7h7h7h7 h7 h7 h7 h7 h7 h7 h7 h7 h7h7 h7h7h7h7   h?h?h?
1IVN     - - - - -  -  - - M Q D D G I H P N  R D A Q  P  F  I  A  D  W  M  A  K Q  L Q P L V N H D S L E
                           s s         s s s   h7h7h7h7 h7 h7 h7 h7 h7 h7 h7 h7 h7h7 h7h7h7h7
P10480   s t l n c  e  g k M F W D Q V H P T  T V V H  A  A  L  S  E  P  A  A  T F  I E S Q Y E F L A H -

1DEOm    - - - - s  y  F P I D H T H T S P A  G A E V  V  A  E  A  F  L  K  A  V V  C T G T S L K S V L T T T S F E G TC
                 h               s?s?s?h7h7 h7h7h7h7 h7 h7 h7 h7 h7 h7 h7 h7 h7h7 h7h7   h?h?h?
1IVNm    - - - - -  -  M Q D D G I H P N R D  A Q P F  I  A  D  W  M  A  K  Q  L Q  P L V N H D S L E
                    s  s s   h7h7h7h7h7h7h7h7 h7h7h7h7 h7 h7 h7 h7 h7 h7 h7 h7 h7
P10480m l n c e g  k  M F W D Q V H P T T V  V H A A  L  S  E  P  A  A  T  F  I E  S Q Y E F L A H -
```

FIGURE 58

```
                              10        20        30        40        50        60
                     ....*....|....*....|....*....|....*....|....*....|....*....|
1IVN_A            4  LLILGDSLSAG-------------------YRMSASAAWPALLNDKWqsk---------- 34
P10480           28  IVMFGDSLSDTgkmyskmrgylpssppyyeGRFSNGPVWLEQLTNEFPGLTianeaeggp 87

                              70        80        90       100       110       120
                     ....*....|....*....|....*....|....*....|....*....|....*....|
1IVN_A           35  -tsvVNASISGDT----------------------------SQQGLARLPALLKQHQPRW 65
P10480           88  tavaYNKISWNPKyq----------------------vINNLDYEVTQFLQKDSFKPDDL 125

                             130       140       150       160       170       180
                     ....*....|....*....|....*....|....*....|....*....|....*....|
1IVN_A           66  VLVELGGNDG--------------------------------------LRGFQPQQTEQT 87
P10480          126  VILWVGANDY-------------------------------LA--YGWNTEQDAKRVRDA 152

                             190       200       210       220       230       240
                     ....*....|....*....|....*....|....*....|....*....|....*....|
1IVN_A           88  LRQILQDVKaANAEPllmqiRLPANYGR-------------------------------- 115
P10480          153  ISDAANRMV-LNGAK-----EILLFNLPdlg--------------------------qnP 180

                             250       260       270       280       290       300
                     ....*....|....*....|....*....|....*....|....*....|....*....|
1IVN_A          116  ------------RYNEAFSAIYPKLAke---------------------fDVPLLPFFME 142
P10480          181  SARSQKVVEAASHVSAYHNQLLLNLArqlaptg----------mvklfeiDKQFAEMLRD 230

                             310       320       330       340       350       360
                     ....*....|....*....|....*....|....*....|....*....|....*....|
1IVN_A          143  EVYLKPQW---------------------------------------------------- 150
P10480          231  PQNFGLSDQRNacyggsyvwkpfasrsastdsqlsafnpqerlaiagnpllaqavaspma 290

                             370       380       390       400
                     ....*....|....*....|....*....|....*....|
1IVN_A          151  ------------MQDDGI--------HPNRDAQPFIADWM 170
P10480          291  arsastlncegkMFWDQV--------HPTTVVHAALSEPA 322
```

FIGURE 59

```
                       1                                                50
   P10480      (1)  MKKWFVCLLGLVALTVQAADSRPAFSRIVMFGDSLSDTGKMYSKMRGYLP
   A. sal      (1)  ------------------ADTRPAFSRIVMFGDSLSDTGKMYSKMRGYLP
   A. hyd      (1)  ------------------ADSRPAFSRIVMFGDSLSDTGKMYSKMRGYLP
Consensus      (1)                    AD*RPAFSRIVMFGDSLSDTGKMYSKMRGYLP
                    51                                               100
   P10480     (51)  SSPPYYEGRFSNGPVWLEQLTNEFPGLTIANEAEGGPTAVAYNKISWNPK
   A. sal     (33)  SSPPYYEGRFSNGPVWLEQLTKQFPGLTIANEAEGGATAVAYNKISWNPK
   A. hyd     (33)  SSPPYYEGRFSNGPVWLEQLTKQFPGLTIANEAEGGATAVAYNKISWNPK
Consensus     (51)  SSPPYYEGRFSNGPVWLEQLT**FPGLTIANEAEGG*TAVAYNKISWNPK
                    101                                              150
   P10480    (101)  YQVINNLDYEVTQFLQKDSFKPDDLVILWVGANDYLAYGWNTEQDAKRVR
   A. sal     (83)  YQVINNLDYEVTQFLQKDSFKPDDLVILWVGANDYLAYGWNTEQDAKRVR
   A. hyd     (83)  YQVINNLDYEVTQFLQKDSFKPDDLVILWVGANDYLAYGWNTEQDAKRVR
Consensus    (101)  YQVINNLDYEVTQFLQKDSFKPDDLVILWVGANDYLAYGWNTEQDAKRVR
                    151                                              200
   P10480    (151)  DAISDAANRMVLNGAKEILLFNLPDLGQNPSARSQKVVEAASHVSAYHNQ
   A. sal    (133)  DAISDAANRMVLNGAKQILLFNLPDLGQNPSARSQKVVEAVSHVSAYHNK
   A. hyd    (133)  DAISDAANRMVLNGAKQILLFNLPDLGQNPSARSQKVVEAVSHVSAYHNQ
Consensus    (151)  DAISDAANRMVLNGAK*ILLFNLPDLGQNPSARSQKVVEA*SHVSAYHN*
                    201                                              250
   P10480    (201)  LLLNLARQLAPTGMVKLFEIDKQFAEMLRDPQNFGLSDQRNACYGGSYVW
   A. sal    (183)  LLLNLARQLAPTGMVKLFEIDKQFAEMLRDPQNFGLSDVENPCYDGGYVW
   A. hyd    (183)  LLLNLARQLAPTGMVKLFEIDKQFAEMLRDPQNFGLSDVENPCYDGGYVW
Consensus    (201)  LLLNLARQLAPTGMVKLFEIDKQFAEMLRDPQNFGLSD**N*CY*G*YVW
                    251                                              300
```

```
 P10480   (251) KPFASRSASTDSQLSAFNPQERLAIAGNPLLAQAVASPMAARSASTLNCE
 A. sal   (233) KPFATRSVSTDRQLSAFSPQERLAIAGNPLLAQAVASPMARRSASPLNCE
 A. hyd   (233) KPFATRSVSTDRQLSAFSPQERLAIAGNPLLAQAVASPMARRSASPLNCE
Consensus (251) KPFA*RS*STD*QLSAF*PQERLAIAGNPLLAQAVASPMA*RSAS*LNCE
                301                                 336
 P10480   (301) GKMFWDQVHPTTVVHAALSEPAATFIESQYEFLAH-
 A. sal   (283) GKMFWDQVHPTTVVHAALSERAATFIETQYEFLAHG
 A. hyd   (283) GKMFWDQVHPTTVVHAALSERAATFIANQYEFLAH-
Consensus (301) GKMFWDQVHPTTVVHAALSE*AATFI**QYEFLAH*
```

FIGURE 60

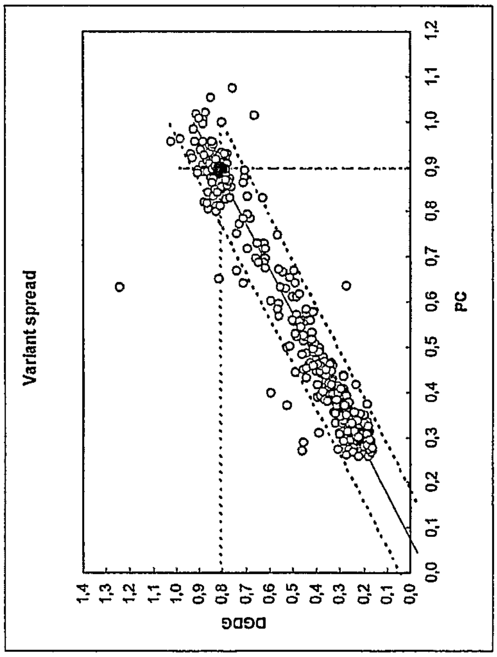
Variant spread
DGDG
1,4
1,3
1,2
1,1
1,0
0,9
0,8
0,7
0,6
0,5
0,4
0,3
0,2
0,1
0,0
0,0
0,1
0,2
0,3
0,4
0,5
0,6
0,7
0,8
0,9
1,0
1,1
1,2
PC

FIGURE 61

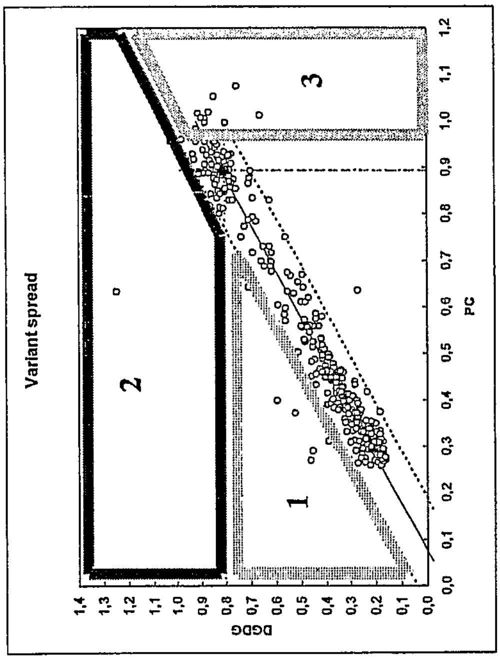

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 09750990 B
- US 10409391 B
- US 60489441 B
- GB 0330016 A
- WO IB2004000655 A
- WO 02065854 A
- EP 0583265 A
- EP 0866796 A
- WO 0206457 A
- EP 0752008 A
- EP 1138763 A
- EP 1103606 A
- US 6180406 B
- WO 0134835 A
- EP 1131416 A
- WO 0214490 A
- WO 00061771 A
- WO 9299640 A
- US 4683202 A
- WO 0058517 A
- US 6344328 B
- US 6361974 B
- WO 9117243 A
- EP 0238023 A
- EP 0449375 A
- WO 04064537 A
- WO 02214490 A
- US 5741665 A
- US 5674707 A
- WO 0139544 A
- WO 0116308 A
- US 3817837 A
- US 3850752 A
- US 3939350 A
- US 3996345 A
- US 4277437 A
- US 4275149 A
- US 4366241 A
- US 4816567 A


### Non-patent literature cited in the description


- **BUCKLEY.** *Biochemistry,* 1983, vol. 22, 5490-5493
- **UPTON ; BUCKLEY.** *TIBS,* 20 May 1995, 178-179
- **BRUMLIK ; BUCKLEY.** *J. of Bacteriology,* April 1996, 2060-2064
- **THORNTON et al.** *Biochem. et Biophys. Acta.,* 1988, vol. 959, 153-159
- **HILTON ; BUCKLEY.** *J. Biol. Chem.,* 1991, vol. 266, 997-1000
- **BUCKLEY et al.** *J. Bacteriol,* 1996, vol. 178 (7), 2060-4
- **ROBERTSON et al.** *J. Biol. Chem.,* 1994, vol. 269, 2146-50
- **BRUMLIK ; BUCKLEY.** *Journal of Bacteriology,* April 1996, vol. 178 (7), 2060-2064
- **BATEMAN A et al.** *Nucleic Acids Res.,* 2002, vol. 30, 276-280
- **BATEMAN A ; HAFT DH.** *Brief Bioinform,* 2002, vol. 3, 236-245
- probabilistic models of proteins and nucleic acids. **DURBIN R ; EDDY S ; KROGH A.** Biological sequence analysis. Cambridge University Press, 1998
- **BEUCAGE S.L. et al.** *Tetrahedron Letters,* 1981, vol. 22, 1859-1869
- **MATTHES et al.** *EMBO J.,* 1984, vol. 3, 801-805
- **SAIKI R K et al.** *Science,* 1988, vol. 239, 487-491
- **CARUTHERS MH et al.** *Nuc Acids Res Symp Ser,* 1980, 215-23
- **HORN T et al.** *Nuc Acids Res Symp Ser,* 1980, 225-232
- **MORINAGA et al.** *Biotechnology,* 1984, vol. 2, 646-649
- **NELSON ; LONG.** *Analytical Biochemistry,* 1989, vol. 180, 147-151
- **HILTON ; BUCKLEY.** *J Biol. Chem.,* 15 January 1991, vol. 266 (2), 997-1000
- **ROBERTSON et al.** *J. Biol. Chem.,* 21 January 1994, vol. 269 (3), 2146-50
- **BRUMLIK et al.** *J. Bacteriol,* April 1996, vol. 178 (7), 2060-4
- **PEELMAN et al.** *Protein Sci.,* March 1998, vol. 7 (3), 587-99
- **DEVEREUX et al.** *Nuc. Acids Research,* 1984, vol. 12, 387

- **AUSUBEL et al.** Short Protocols in Molecular Biology. 1999
- **ALTSCHUL et al.** *J. Mol. Biol.,* 1990, 403-410
- *FEMS Microbiol Lett,* 1999, vol. 174 (2), 247-50
- *FEMS Microbiol Lett,* 1999, vol. 177 (1), 187-8
- **HIGGINS DG ; SHARP PM.** *Gene,* 1988, vol. 73 (1), 237-244
- **SIMON RJ et al.** *PNAS,* 1992, vol. 89 (20), 9367-9371
- **HORWELL DC.** *Trends Biotechnol.,* 1995, vol. 13 (4), 132-134
- Guide to Molecular Cloning Techniques. **BERGER ; KIMMEL.** Methods in Enzymology. Academic Press, 1987, vol. 152
- **SAMBROOK et al.** Molecular Cloning: A Laboratory Manual. Cold Spring Harbor Laboratory Press, 1989
- **POTRYKUS.** *Annu Rev Plant Physiol Plant Mol Biol,* 1991, vol. 42, 205-225
- **CHRISTOU.** *Agro-Food-Industry Hi-Tech,* March 1994, 17-27
- **DAVIS ; DE SERRES.** *Methods Enzymol,* 1971, vol. 17A, 79-143
- Vectors for genetic manipulation. **TURNER G.** Aspergillus: 50 years on. Progress in industrial microbiology. Elsevier, 1994, vol. 29, 641-666
- **PUNT et al.** *Trends Biotechnol,* May 2002, vol. 20 (5), 200-6
- **ARCHER ; PEBERDY.** *Crit Rev Biotechnol,* 1997, vol. 17 (4), 273-306
- *Methods Mol Biol,* 1995, vol. 49, 341-54
- *Curr Opin Biotechnol,* October 1997, vol. 8 (5), 554-60
- *FEMS Microbiol Rev,* 2000, vol. 24 (1), 45-66
- Yeast as a vehicle for the expression of heterologous genes. **E HINCHELIFFE ; E KENNY.** Yeasts. Academic Press Ltd, 1993, vol. 5
- **HINNEN et al.** *Proceedings of the National Academy of Sciences of the USA,* 1978, vol. 75, 1929
- **BEGGS, J D.** *Nature,* 1978, vol. 275, 104
- **ITO, H et al.** *J Bacteriology,* 1983, vol. 153, 163-168
- *Curr. Opin. Biotechnol.,* 1995, vol. 6 (5), 501-6
- **UPTON C ; BUCKLEY JT.** *Trends Biochem Sci,* 1995, vol. 20, 179-179
- **PENNINAGA et al.** *Biochemistry,* 1995, 3368-3376
- **KAWAMURA ; DOI.** *J. of Bacteriology,* October 1984, 442-444
- Molecular Biological Methods for Bacillus. John Wiley & Sons Ltd, 1990